# EUROPEAN PATENT APPLICATION

(11) **EP 2 363 480 A2**
(43) Date of publication of application: **07.09.2011**
(21) Application number: 11158293.8
(22) Date of filing: 20.01.2005
(51) Int. Cl.: C12N 15/113, A61K 31/7088, C07H 21/00, A61P 3/00

(54) **Modulation of glucocorticoid receptor expression**

(30) Priority: 03.03.2004 US 550191 P; 20.01.2004 US 538173 P
(62) Divisional of application: 05722546.8
(71) Applicant: ISIS PHARMACEUTICALS, INC., Carlsbad, CA 92008 (US)
(72) Inventor: Bhanot, Sanjay, Carlsbad, CA 92009 (US); Dobie, Kenneth W., Delmar, CA 92014 (US); Freier, Susan M., San Diego, CA 92122 (US); Dean, Nicholas, M., Olivenhain, CA 92024 (US); Bennett, C. Frank, Carlsbad, CA 92009 (US)
(74) Representative: Hallybone, Huw George

(57) **Abstract**

Compounds, compositions and methods are provided for modulating the expression of glucocorticoid receptor. The compositions comprise oligonucleotides, targeted to nucleic acid encoding glucocorticoid receptor. Methods of using these compounds for modulation of glucocorticoid receptor expression and for diagnosis and treatment of diseases and conditions associated with expression of glucocorticoid receptor are provided.

## Description

### FIELD OF THE INVENTION

The present invention provides compositions and methods for modulating the expression of glucocorticoid receptor. In particular, this invention relates to antisense compounds, particularly oligonucleotide compounds, which, in preferred embodiments, hybridize with nucleic acid molecules encoding glucocorticoid receptor. Such compounds are shown herein to modulate the expression of glucocorticoid receptor.

### BACKGROUND OF THE INVENTION

Glucocorticoids were among the first steroid hormones to be identified and are responsible for a multitude of physiological functions, including the stimulation of gluconeogenesis, decreased glucose uptake and utilization in peripheral tissues, increased glycogen deposition, suppression of immune and inflammatory responses, inhibition of cytokine synthesis and acceleration of various developmental events. Glucocorticoids are also especially important for combating stress. Stress-induced elevation of glucocorticoid synthesis and release leads to, among other responses, increased ventricular workload, inhibition of inflammatory mediators, inhibition of cytokine synthesis and increased glucose production (Karin, Cell, 1998, 93, 487-490).

Both natural glucocorticoids and their synthetic derivatives exert their action through the glucocorticoid receptor, a ubiquitously expressed cytoplasmic member of the nuclear hormone superfamily of receptors. Complementary DNA clones encoding the human glucocorticoid receptor (also known as nuclear receptor subfamily 3, group C, member 1; NR3C1; GCCR; GCR; GRL; Glucocorticoid receptor, lymphocyte) were first isolated in 1985 (Hollenberg et al., Nature, 1985, 318, 635-641; Weinberger et al., Science, 1985, 228, 740-742). The gene is located on human chromosome Sq11-q13 and consists of 9 exons (Encio and Detera-Wadleigh, J Biol Chem, 1991, 266, 7182-7188; Gehring et al., Proc Natl Acad Sci USA, 1985, 82, 3751-3755). Multiple forms of human glucocorticoid receptor mRNA exist: a 5.5 kb human glucocorticoid receptor α cDNA containing exons 1-8 and exon 9α; a 4.3 kb human glucocorticoid receptor β cDNA containing exons 1-8 and exon 9β; and a 7.0 kb human glucocorticoid receptor α cDNA containing exons 1-8 and the entire exon 9, which includes exon 9α, exon 9β and the 'J region', which is flanked by exons 9α and 9β (Hollenberg et al., Nature, 1985, 318, 635-641; Oakley et al., J Biol Chem, 1996, 271, 9550-9559). Human glucocorticoid receptor α is the predominant isoform of the receptor and the one that exhibits steroid binding activity (Hollenberg et al., Nature, 1985, 318, 635-641). Additionally, through usage of three different promoters three different exons 1 can be transcribed, and alternative splicing of one exon 1 variant can result in three different versions of this exon. Thus, human glucocorticoid receptor mRNA may contain 5 different versions of exon 1 (Breslin et al., Mol Endocrinol, 2001, 15, 1381-1395).

Examination of the expression patterns of the α and β isoforms of human glucocorticoid receptor mRNA reveals that the α isoform is more abundantly expressed. Both isoforms are expressed in similar tissues and cell types, including lung, kidney, heart, liver, skeletal muscle, macrophages, neutrophils and peripheral blood mononuclear cells. Only human glucocorticoid receptor α is expressed in colon. At the level of protein, while the α isoform is detected in all tissues examined, the β isoform is undetectable, suggesting that under physiological conditions, the default splicing pathway is the one that produces the α isoform (Pujols et al., Am J Physiol Cell Physiol, 2002, 283, C1324-1331). The β isoform of glucocorticoid receptor binds neither a glucocorticoid agonist nor an antagonist. Furthermore, the β isoform is localized primarily in the nucleus in transfected cells, independent of hormone stimulation. When both isoforms are expressed in the same cell, the glucocorticoid receptor β inhibits the hormone-induced, glucocorticoid receptor α-mediated stimulation of gene expression, suggesting that the β isoform functions as an inhibitor of glucocorticoid receptor α activity (Oakley et al., J Biol Chem, 1996, 271, 9550-9559). Unless otherwise noted, the human glucocorticoid receptor described herein is defined as the ubiquitous product(s) of the gene located on chromosome 5q11-q13.

The human glucocorticoid receptor is comprised of three major domains, the N-terminal activation domain, the central DNA-binding domain and the C-terminal ligand-binding domain (Giguere et al., Cell, 1986, 46, 645-652). In the absence of ligand, the glucocorticoid receptor forms a large heteromeric complex with several other proteins, from which it dissociates upon ligand binding. The heat shock protein 90 (hsp90) performs a key role in this complex, keeping the receptor in a conformation capable of binding to steroid by incapable of activating transcription (Cadepond et al., J Biol Chem, 1991, 266, 5834-5841). The glucocorticoid receptor is phosphorylated in the absence of ligand, and becomes hyperphosphorylated after the binding of an agonist, such as a steroid, but not an antagonist, such as the antiglucocorticoid compound RU-486 (Orti et al., J Biol Chem, 1989, 264, 9728-9731).

The phosphorylated glucocorticoid receptor subsequently translocates to the nucleus through the action of two domains which participate in nuclear localization, NL 1, localized in the region bridging the DNA-binding and ligand-binding domains and NL2, localized completely within the ligand-binding domain. The function of NL1 is inhibited by the ligand-binding domain, and this inhibition can be abrogated by ligand binding (Picard and Yamamoto, Embo J, 1987, 6, 3333-3340). Nuclear translocation occurs in a hormone-dependent manner.

Once activated, the glucocorticoid receptor forms a homodimer. Studies of the purified activated glucocorticoid receptor demonstrate that it exists as a homodimer in the presence and absence of DNA, suggesting that dimerization occurs before DNA binding (Wrange et al., J Biol Chem, 1989, 264, 5253-5259). The dimerized glucocorticoid receptor binds to specific palindromic DNA sequences named glucocorticoid-responsive elements (GREs) in its target genes, and consequently affects transcription (Schaaf and Cidlowski, J Steroid Biochem Mol Biol, 2002, 83, 37-48). The regulatory regions of the tyrosine aminotransferase, alanine aminotransferase and phosphoenolpyruvate carboxykinase (PEPCK) genes, among others, contain positive GREs, which serve to enhance transcription. In addition to activating transcription following binding to positive GREs, the glucocorticoid receptor can also repress transcription through binding to negative GREs, which represses transcription, or through transcription interference via interactions of the glucocorticoid receptor with other transcription factors (Karin, Cell, 1998, 93, 487-490). The latter is a DNA-binding independent activity. Thus, the glucocorticoid receptor can influence transcription through both DNA-independent and DNA-dependent mechanisms. While the glucocorticoid receptor gene is itself essential for survival, as demonstrated by the lack of viability in glucocorticoid receptor-deficient mice, the DNA binding activity of the glucocorticoid receptor is not essential for survival. Mice bearing a point mutation in the glucocorticoid receptor that impairs dimerization and consequently GRE-dependent transactivation are viable, revealing the in vivo relevance of the DNA-binding-independent activities of the glucocorticoid receptor (Reichardt et al., Cell, 1998, 93, 531-541).

Owing to the ubiquitous expression of the glucocorticoid receptor, and to its ability to both activate and repress transcription, the glucocorticoid receptor often requires cofactors to confer transcriptional specificity. Certain cofactors facilitate transcription through the recruitment of the basal transcription machinery or the remodeling of chromatin. The CREB-binding protein (CBP) and its homolog p300 function as coactivators for the glucocorticoid receptor, enhancing transcription of glucocorticoid receptor responsive genes (Chakravarti et al., Nature, 1996, 383, 99-103). Another class of coactivators include the vitamin D receptor-interacting proteins (DRIP) DRIP150 and DRIP205, both of which facilitate glucocorticoid receptor transcriptional activation (Hittelman et al., Embo J, 1999, 18, 5380-5388). Human glucocorticoid receptor also associates with the chromatin remodeling complex BRG, which removes histone H1 from chromatin and allows general transcription factors to access their binding sites (Fryer and Archer, Nature, 1998, 393, 88-91). In this case, the glucocorticoid receptor appears to recruit the BRG complex to promoters via interactions with the BRG-associated factor BAF250, a subunit of the BRG complex. Once escorted to the promoter, BRG induces chromatin remodeling and transcription proceeds (Deroo and Archer, Oncogene, 2001, 20, 3039-3046; Nie et al., Mol Cell Biol, 2000, 20, 8879-8888). A transcription factor whose activity is negatively regulated by the glucocorticoid receptor is NF-kB. Dexamethasone, a ligand of the glucocorticoid receptor, promotes the binding of the glucocorticoid receptor to the p65 subunit of NFkB, which inhibits the activation of the interleukin-6 promoter (Ray and Prefontaine, Proc Natl Acad Sci USA, 1994, 91, 752-756).

Cell lines transfected with a complementary glucocorticoid receptor antisense RNA strand exhibited a reduction in glucocorticoid receptor mRNA levels and a decreased response to the glucocorticoid receptor agonist dexamethasone (Pepin and Barden, Mol Cell Biol, 1991, 11, 1647-1653). Transgenic mice bearing an antisense glucocorticoid receptor gene construct were used to study the glucocorticoid feedback effect on the hypothalamus-pituitary-adrenal axis (Pepin et al., Nature, 1992, 355, 725-728). In another study of similarly genetically engineered mice, energy intake and expenditure, heart and vastus lateralis muscle lipoprotein lipase activty, and heart and brown adipose tissue norepinephrine were lower than in control animals. Conversely, fat content and total body energy were significantly higher than in control animals. These results suggest that a defective glucocorticoid receptor system may affect energy balance through increasing energetic efficiency, and they emphasize the modulatory effects of hypothalamic-pituitary-adrenal axis changes on muscle lipoprotein lipase activity (Richard et al., Am JPhysiol, 1993, 265, R146-150).

Behavorial effects of glucocorticoid receptor antagonists have been measured in animal models designed to assess anxiety, learning and memory. Reduced expression of glucocorticoid receptor in rats long-term intracerebroventricularly infused with antisense oligodeoxynucleotides targeting glucocorticoid receptor mRNA did not interfere with spatial navigation in the Morris water maze test (Engelmann et al., Eur JPharmacol, 1998, 361, 17-26). Bilateral infusion of an antisense oligodeoxynucleotide targeting the glucocorticoid receptor mRNA into the dentate gyrus of the rat hippocampus reduced the immobility of rats in the Porsolt forced swim test (Korte et al., Eur J Pharmacol, 1996, 301, 19-25).

Glucocorticoids are frequently used for their immunosuppressive, anti-inflammatory effects in the treatment of diseases such as allergies, athsma, rheumatoid arthritis, AIDS, systemic lupus erythematosus and degenerative osteoarthritis. Negative regulation of gene expression, such as that caused by the interaction of glucocorticoid receptor with NF-κB, is proposed to be at least partly responsible for the anti-inflammatory action of glucocorticoids in vivo. Interleukin-6, tumor necrosis factor α and interleukin-1 are the three cytokines that account for most of the hypothalamic-pituitary-adrenal (HPA) axis stimulation during the stress of inflammation. The HPA axis and the systemic sympathetic and adrenomedullary system are the peripheral components of the stress system, responsible for maintaining basal and stress-related homeostasis. Glucocorticoids, the end products of the HPA axis, inhibit the production of all three inflammatory cytokines and also inhibit their effects on target tissues, with the exception of interleukin-6, which acts synergistically with glucocorticoids to stimulate the production of acute-phase reactants. Glucocorticoid treatment decreases the activity of the HPA axis (Chrousos, N Engl J Med, 1995, 332, 1351-1362).

In some cases, patients are refractory to glucocorticoid treatment. One reason for this resistance to steroids lies with mutations or polymorphisms present in the glucocorticoid receptor gene. A total of 15 missense, three nonsense, three frameshift, one splice site, and two alternative spliced mutations, as well as 16 polymorphisms, have been reported in the NR3C1 gene in association with glucocorticoid resistance (Bray and Cotton, Hum Mutat, 2003, 21, 557-568). Additional studies in humans have suggested a positive association between metabolic syndrome incidence and progression, with alleles at the glucocorticoid receptor (GR) gene (Rosmond, Obes Res, 2002, 10, 1078-1086).

Other cases of glucocorticoid insensitivity are associated with altered expression of glucocorticoid receptor isoforms. A study of human glucocorticoid receptor β isoform mRNA expression in glucocorticoid-resistant ulcerative colitis patients revealed the presence of this mRNA was significantly higher than in the glucocorticoid-sensitive patients, suggesting that the expression of human glucocorticoid receptor β mRNA in the peripheral blood mononuclear cells may serve as a predictor of glucocorticoid response in ulcerative colitis (Honda et al., Gastroenterology, 2000, 118, 859-866). Increased expression of glucocorticoid receptor β is also observed in a significantly high number of glucocorticoid-insensitive asthmatics. Additionally, cytokine-induced abnormalities in the DNA binding capacity of the glucocorticoid receptor were found in peripheral blood mononuclear cells from glucocorticoid-insensitive patients transfection, and HepG2 cells with the glucocorticoid receptor β gene resulted in a significant reduction of glucocorticoid receptor α DNA-binding capacity (Leung et al., J Exp Med, 1997, 186, 1567-1574). Dexamethasone binding studies demonstrate that human glucocorticoid receptor β does not alter the affinity of glucocorticoid receptor α for hormonal ligands, but rather its ability to bind to the GRE (Bamberger et al., J Clin Invest, 1995, 95, 2435-2441). Taken together, these results illustrate that glucocorticoid receptor β, through competition with glucocorticoid receptor α for GRE target sites, may function as a physiologically and pathophysiologically relevant endogenous inhibitor of glucocorticoid action.

In the liver, glucocorticoid agonists increase hepatic glucose production by activating the glucocorticoid receptor, which subsequently leads to increased expression of the gluconeogenic enzymes phosphoenolpyruvate carboxykinase (PEPCK) and glucose-6-phosphatase. Through gluconeogenesis, glucose is formed through non-hexose precursors, such as lactate, pyruvate and alanine (Link, Curr Opin Investig Drugs, 2003,4,421-429). Steroidal glucocorticoid receptor antagonists such as RU 486 have been tested in rodent models of diabetes. Mice deficient in the leptin receptor gene, termed db/db mice, are genetically obese, diabetic and hyperinsulinemic. Treatment of hyperglycemic db/db mice with RU 486 decreased blood glucose levels by approximately 49%, without affecting plasma insulin levels. Additionally, RU 486 treatment reduced the expression of glucocorticoid receptor responsive genes PEPCK, glucose-6-phosphatase, glucose transporter type 2 and tyrosine aminotransferase in db/db mice as compared to untreated animals (Friedman et al., J Biol Chem, 1997, 272, 31475-31481). RU 486 also ameliorates diabetes in the ob/ob mouse model of diabetes, obesity and hyperinsulinemia, through a reduction in serum insulin and blood glucose levels (Gettys et al., Int J Obes Relat Metab Disord, 1997, 21, 865-873).

As increased gluconeogenesis is considered to be the major source of increased glucose production in diabetes, a number of therapeutic targets for the inhibition of hepatic glucose production have been investigated. Due to the ability of antagonists of the glucocorticoid receptor to ameliorate diabetes in animal models, such compounds are among the potential therapies being explored. However, there are detrimental systemic effects of glucocorticoid receptor antagonists, including activation of the HPA axis (Link, Curr Opin Investig Drugs, 2003, 4, 421-429). Increased HPA axis activity is associated with suppression of immune-related inflammatory action, which can increase susceptibility to infectious agents and neoplasms. Conditions associated with suppression of immune-mediated inflammation through defects in the HPA axis, or its target tissues, include Cushing's syndrome, chronic stress, chronic alcoholism and melancholic depression (Chrousos, N Engl J Med, 1995, 332, 1351-1362). Thus, it is of great value to develop liver-specific glucocorticoid receptor antagonists. Steroidal glucocorticoid receptor antagonists have been conjugated to bile acids for the purpose of targeting them to the liver (Apelqvist et al., 2000). Currently, there are no known therapeutic agents that target the glucocorticoid receptor without undesired peripheral effects (Link, Curr Opin Investig Drugs, 2003, 4, 421-429). Consequently, there remains a long felt need for agents capable of effectively inhibiting hepatic glucocorticoid receptor.

The US patent 6,649,341 discloses antisense primers targeted to a nucleotide sequence comprising human glucocorticoid receptor 1Ap/e transcript, as well as a method of preventing apoptosis in neurons by expressing an antisense transgene to the human glucocorticoid receptor exon 1A transcripts (Vedeckis and Breslin, 2003).

The US Pre-grant publication 20030092616 and the PCT publication WO 02/096943 disclose a nucleotide sequence encoding human glucocorticoid receptor, as well as an antisense oligonucleotide complementary to said polynucleotide; a ribozyme which inhibits STAT6 activation by cleavage of an RNA comprising said polynucleotide; and a method for treating a disease, which comprises administering to a subject an amount of an antisense oligonucleotide or a ribozyme effective to treat a disease selected from the group consisting of allergic disease, inflammation, autoimmune diseases, diabetes, hyperlipidemia, infectious disease and cancers (Honda et al., 2002).

The PCT publication WO 88/00975 discloses an antisense oligonucleotide targeted to a nucleic acid sequence encoding human glucocorticoid receptor.

The PCT publication WO 01/42307 discloses antisense oligonucleotides targeted to a nucleic acid sequence encoding human glucocorticoid receptor.

The PCT publication WO 01/77344 discloses an antisense oligonucleotide targeted to a nucleic acid sequence encoding human glucocorticoid receptor.

The PCT publication WO 03/008583 discloses a carcinoma cancer inhibitor which is an antisense molecule including antisense or sense oligonucleotides comprising a single-stranded nucleic acid sequence (either RNA or DNA) capable of binding to target mRNA (sense) or DNA (antisense) sequences for carcinoma cancer molecules, including a nucleic acid sequence encoding human glucocorticoid receptor (Morris and Engelhard, 2003).

Antisense technology is an effective means of reducing the expression of specific gene products and therefore is uniquely useful in a number of therapeutic, diagnostic and research applications for the modulation of glucocorticoid receptor expression. Furthermore, liver is one of the tissues in which the highest concentrations of antisense oligonucleotides are found following administration (Geary et al., Curr. Opin. Investig. Drugs, 2001, 2, 562-573). Therefore, antisense technology represents an attractive method for the liver-specific inhibition of glucocorticoid receptor. In addition to diabetes, particularly type 2 diabetes, glucocorticoid receptor modulators are useful to treat diseases such as obesity, Metabolic syndrome X, Cushing's Syndrome, Addison's disease, inflammatory diseases such as asthma, rhinitis and arthritis, allergy, autoimmune disease, immunodeficiency, anorexia, cachexia, bone loss or bone frailty, and wound healing. Metabolic syndrome, metabolic syndrome X or simply Syndrome X refers to a cluster of risk factors that include obesity, dyslipidemia, particularly high blood triglycerides, glucose intolerance, high blood sugar and high blood pressure. Scott, C.L., Am J Cardiol. 2003 Jul 3;92(1A):35i-42i. Glucocorticoid receptor inhibitors such as the compounds described herein are also believed to be useful for amelioration of hyperglycemia induced by systemic steroid therapy.

Moreover, antisense technology provides a means of inhibiting the expression of the glucocorticoid receptor β isoform, demonstrated to be overexpressed in patients refractory to glucocorticoid treatment.

The present invention provides compositions and methods for inhibiting glucocorticoid receptor expression.

### SUMMARY OF THE INVENTION

The present invention is directed to antisense compounds, especially nucleic acid and nucleic acid-like oligomers, which are targeted to a nucleic acid encoding glucocorticoid receptor, and which modulate the expression of glucocorticoid receptor. Pharmaceutical and other compositions comprising the compounds of the invention are also provided. Further provided are methods of screening for modulators of glucocorticoid receptor and methods of modulating the expression of glucocorticoid receptor in cells, tissues or animals comprising contacting said cells, tissues or animals with one or more of the compounds or compositions of the invention. Methods of treating an animal, particularly a human, suspected of having or being prone to a disease or condition associated with expression of glucocorticoid receptor are also set forth herein. Such methods comprise administering a therapeutically or prophylactically effective amount of one or more of the compounds or compositions of the invention to the person in need of treatment.

### DETAILED DESCRIPTION OF THE INVENTION

### A. Overview of the Invention

The present invention employs antisense compounds, preferably oligonucleotides and similar species for use in modulating the function or effect of nucleic acid molecules encoding glucocorticoid receptor. This is accomplished by providing oligonucleotides which specifically hybridize with one or more nucleic acid molecules encoding glucocorticoid receptor. As used herein, the terms "target nucleic acid" and "nucleic acid molecule encoding glucocorticoid receptor" have been used for convenience to encompass DNA encoding glucocorticoid receptor, RNA (including pre-mRNA and mRNA or portions thereof) transcribed from such DNA, and also cDNA derived from such RNA. The hybridization of a compound of this invention with its target nucleic acid is generally referred to as "antisense". Consequently, the preferred mechanism believed to be included in the practice of some preferred embodiments of the invention is referred to herein as "antisense inhibition." Such antisense inhibition is typically based upon hydrogen bonding-based hybridization of oligonucleotide strands or segments such that at least one strand or segment is cleaved, degraded, or otherwise rendered inoperable. In this regard, it is presently preferred to target specific nucleic acid molecules and their functions for such antisense inhibition.

The functions of DNA to be interfered with can include replication and transcription. Replication and transcription, for example, can be from an endogenous cellular template, a vector, a plasmid construct or otherwise. The functions of RNA to be interfered with can include functions such as translocation of the RNA to a site of protein translation, translocation of the RNA to sites within the cell which are distant from the site of RNA synthesis, translation of protein from the RNA, splicing of the RNA to yield one or more RNA species, and catalytic activity or complex formation involving the RNA which may be engaged in or facilitated by the RNA. One preferred result of such interference with target nucleic acid function is modulation of the expression of glucocorticoid receptor. In the context of the present invention, "modulation" and "modulation of expression" mean either an increase (stimulation) or a decrease (inhibition) in the amount or levels of a nucleic acid molecule encoding the gene, e.g., DNA or RNA. mRNA is often a preferred target nucleic acid. Inhibition is often the preferred form of modulation of expression; it is understood that "inhibition" does not have to be absolute inhibition, but is intended to mean a decrease or reduction in target expression.

In the context of this invention, "hybridization" means the pairing of complementary strands of oligomeric compounds. In the present invention, the preferred mechanism of pairing involves hydrogen bonding, which may be Watson-Crick, Hoogsteen or reversed Hoogsteen hydrogen bonding, between complementary nucleoside or nucleotide bases (nucleobases) of the strands of oligomeric compounds. For example, adenine and thymine are complementary nucleobases which pair through the formation of hydrogen bonds. Hybridization can occur under varying circumstances.

An antisense compound is specifically hybridizable when binding of the compound to the target nucleic acid interferes with the normal function of the target nucleic acid to cause a loss of activity, and there is a sufficient degree of complementarity to avoid non-specific binding of the antisense compound to non-target nucleic acid sequences under conditions in which specific binding is desired, i.e., under physiological conditions in the case of *in vivo* assays or therapeutic treatment, and under conditions in which assays are performed in the case of *in vitro* assays.

In the present invention the phrase "stringent hybridization conditions" or "stringent conditions" refers to conditions under which a compound of the invention will hybridize to its target sequence, but to a minimal number of other sequences. Stringent conditions are sequence-dependent and will be different in different circumstances and in the context of this invention, "stringent conditions" under which oligomeric compounds hybridize to a target sequence are determined by the nature and composition of the oligomeric compounds and the assays in which they are being investigated.

"Complementary," as used herein, refers to the capacity for precise pairing between two nucleobases of an oligomeric compound. For example, if a nucleobase at a certain position of an oligonucleotide (an oligomeric compound), is capable of hydrogen bonding with a nucleobase at a certain position of a target nucleic acid, said target nucleic acid being a DNA, RNA, or oligonucleotide molecule, then the position of hydrogen bonding between the oligonucleotide and the target nucleic acid is considered to be a complementary position. The oligonucleotide and the further DNA, RNA, or oligonucleotide molecule are complementary to each other when a sufficient number of complementary positions in each molecule are occupied by nucleobases which can hydrogen bond with each other. Thus, "specifically hybridizable" and "complementary" are terms which are used to indicate a sufficient degree of precise pairing or complementarity over a sufficient number of nucleobases such that stable and specific binding occurs between the oligonucleotide and a target nucleic acid.

It is understood in the art that the sequence of an antisense compound need not be 100% complementary to that of its target nucleic acid to be specifically hybridizable. Moreover, an oligonucleotide may hybridize over one or more segments such that intervening or adjacent segments are not involved in the hybridization event (e.g., a loop structure or hairpin structure). It is preferred that the antisense compounds of the present invention comprise at least 70%, or at least 75%, or at least 80%, or at least 85% sequence complementarity to a target region within the target nucleic acid, more preferably that they comprise at least 90% sequence complementarity and even more preferably comprise at least 95% or at least 99% sequence complementarity to the target region within the target nucleic acid sequence to which they are targeted. For example, an antisense compound in which 18 of 20 nucleobases of the antisense compound are complementary to a target region, and would therefore specifically hybridize, would represent 90 percent complementarity. In this example, the remaining noncomplementary nucleobases may be clustered or interspersed with complementary nucleobases and need not be contiguous to each other or to complementary nucleobases. As such, an antisense compound which is 18 nucleobases in length having 4 (four) noncomplementary nucleobases which are flanked by two regions of complete complementarity with the target nucleic acid would have 77.8% overall complementarity with the target nucleic acid and would thus fall within the scope of the present invention. Percent complementarity of an antisense compound with a region of a target nucleic acid can be determined routinely using BLAST programs (basic local alignment search tools) and PowerBLAST programs known in the art (Altschul et al., J. Mol. Biol., 1990, 215, 403-410; Zhang and Madden, Genome Res., 1997, 7, 649-656).

Percent homology, sequence identity or complementarity, can be determined by, for example, the Gap program (Wisconsin Sequence Analysis Package, Version 8 for Unix, Genetics Computer Group, University Research Park, Madison WI), using default settings, which uses the algorithm of Smith and Waterman (Adv. Appl. Math., 1981, 2, 482-489). In some preferred embodiments, homology, sequence identity or complementarity, between the oligomeric and target is between about 50% to about 60%. In some embodiments, homology, sequence identity or complementarity, is between about 60% to about 70%. In preferred embodiments, homology, sequence identity or complementarity, is between about 70% and about 80%. In more preferred embodiments, homology, sequence identity or complementarity, is between about 80% and about 90%. In some preferred embodiments, homology, sequence identity or complementarity, is about 90%, about 92%, about 94%, about 95%, about 96%, about 97%, about 98%, about 99% or about 100%.

### B. Compounds of the Invention

According to the present invention, antisense compounds include antisense oligomeric compounds, antisense oligonucleotides, ribozymes, external guide sequence (EGS) oligonucleotides, alternate splicers and other oligomeric compounds which hybridize to at least a portion of the target nucleic acid and modulate its expression. As such, these compounds may be introduced in the form of single-stranded, double-stranded, circular or hairpin oligomeric compounds and may contain structural elements such as internal or terminal bulges or loops. Once introduced to a system, the compounds of the invention may elicit the action of one or more enzymes or structural proteins to effect modification of the target nucleic acid.

One non-limiting example of such an enzyme is RNAse H, a cellular endonuclease which cleaves the RNA strand of an RNA:DNA duplex. It is known in the art that single-stranded antisense compounds which are "DNA-like" elicit RNAse H. Activation of RNase H, therefore, results in cleavage of the RNA target, thereby greatly enhancing the efficiency of oligonucleotide-mediated inhibition of gene expression. Similar roles have been postulated for other ribonucleases such as those in the RNase III and ribonuclease L family of enzymes.

While the preferred form of antisense compound is a single-stranded antisense oligonucleotide, in many species the introduction of double-stranded structures, such as double-stranded RNA (dsRNA) molecules, has been shown to induce potent and specific antisense-mediated reduction of the function of a gene or its associated gene products. This phenomenon occurs in both plants and animals and is believed to have an evolutionary connection to viral defense and transposon silencing.

The first evidence that dsRNA could lead to gene silencing in animals came in 1995 from work in the nematode, *Caenorhabditis elegans* (Guo and Kempheus, Cell, 1995, 81, 611-620). Montgomery et al. have shown that the primary interference effects of dsRNA are posttranscriptional (Montgomery et al., Proc. Natl. Acad. Sci. USA, 1998, 95, 15502-15507). The posttranscriptional antisense mechanism defined in *Caenorhabditis elegans* resulting from exposure to double-stranded RNA (dsRNA) has since been designated RNA interference (RNAi). This term has been generalized to mean antisense-mediated gene silencing involving the introduction of dsRNA leading to the sequence-specific reduction of endogenous targeted mRNA levels (Fire et al., Nature, 1998, 391, 806-811). Recently, it has been shown that it is, in fact, the single-stranded RNA oligomers of antisense polarity of the dsRNAs which are the potent inducers of RNAi (Tijsterman et al., Science, 2002, 295, 694-697).

The antisense compounds of the present invention also include modified compounds in which a different base is present at one or more of the nucleotide positions in the compound. For example, if the first nucleotide is an adenosine, modified compounds may be produced which contain thymidine, guanosine or cytidine at this position. This may be done at any of the positions of the antisense compound. These compounds are then tested using the methods described herein to determine their ability to inhibit expression of glucocorticoid receptor mRNA.

In the context of this invention, the term "oligomeric compound" refers to a polymer or oligomer comprising a plurality of monomeric units. In the context of this invention, the term "oligonucleotide" refers to an oligomer or polymer of ribonucleic acid (RNA) or deoxyribonucleic acid (DNA) or mimetics, chimeras, analogs and homologs thereof. This term includes oligonucleotides composed of naturally occurring nucleobases, sugars and covalent internucleoside (backbone) linkages as well as oligonucleotides having non-naturally occurring portions which function similarly. Such modified or substituted oligonucleotides are often preferred over native forms because of desirable properties such as, for example, enhanced cellular uptake, enhanced affinity for a target nucleic acid and increased stability in the presence of nucleases.

While oligonucleotides are a preferred form of the antisense compounds of this invention, the present invention comprehends other families of antisense compounds as well, including but not limited to oligonucleotide analogs and mimetics such as those described herein.

The antisense compounds in accordance with this invention preferably comprise from about 8 to about 80 nucleobases (i.e. from about 8 to about 80 linked nucleosides). One of ordinary skill in the art will appreciate that the invention embodies compounds of 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 nucleobases in length.

In one preferred embodiment, the antisense compounds of the invention are 12 to 50 nucleobases in length. One having ordinary skill in the art will appreciate that this embodies compounds of 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleobases in length.

In another preferred embodiment, the antisense compounds of the invention are 15 to 30 nucleobases in length. One having ordinary skill in the art will appreciate that this embodies compounds of 15, 16, 17, 18, 19, 20, 21, 22, 23, 24, 25, 26, 27, 28, 29, or 30 nucleobases in length.

Particularly preferred compounds are oligonucleotides from about 12 to about 50 nucleobases, even more preferably those comprising from about 15 to about 30 nucleobases.

Antisense compounds 8-80 nucleobases in length comprising a stretch of at least eight (8) consecutive nucleobases selected from within the illustrative antisense compounds are considered to be suitable antisense compounds as well. Exemplary preferred antisense compounds include oligonucleotide sequences that comprise at least the 8 consecutive nucleobases from the 5'-terminus of one of the illustrative preferred antisense compounds (the remaining nucleobases being a consecutive stretch of the same oligonucleotide beginning immediately upstream of the 5'-terminus of the antisense compound which is specifically hybridizable to the target nucleic acid and continuing until the oligonucleotide contains about 8 to about 80 nucleobases). Similarly preferred antisense compounds are represented by oligonucleotide sequences that comprise at least the 8 consecutive nucleobases from the 3'-terminus of one of the illustrative preferred antisense compounds (the remaining nucleobases being a consecutive stretch of the same oligonucleotide beginning immediately downstream of the 3'-terminus of the antisense compound which is specifically hybridizable to the target nucleic acid and continuing until the oligonucleotide contains about 8 to about 80 nucleobases). It is also understood that preferred antisense compounds may be represented by oligonucleotide sequences that comprise at least 8 consecutive nucleobases from an internal portion of the sequence of an illustrative preferred antisense compound, and may extend in either or both directions until the oligonucleotide contains about 8 to about 80 nucleobases. One having skill in the art armed with the preferred antisense compounds illustrated herein will be able, without undue experimentation, to identify further preferred antisense compounds.

### C. Targets of the Invention

"Targeting" an antisense compound to a particular nucleic acid molecule, in the context of this invention, can be a multistep process. The process usually begins with the identification of a target nucleic acid whose function is to be modulated. This target nucleic acid may be, for example, a cellular gene (or mRNA transcribed from the gene) whose expression is associated with a particular disorder or disease state, or a nucleic acid molecule from an infectious agent. In the present invention, the target nucleic acid encodes glucocorticoid receptor.

The targeting process usually also includes determination of at least one target region, segment, or site within the target nucleic acid for the antisense interaction to occur such that the desired effect, e.g., modulation of expression, will result. Within the context of the present invention, the term "region" is defined as a portion of the target nucleic acid having at least one identifiable structure, function, or characteristic. Within regions of target nucleic acids are segments. "Segments" are defined as smaller or sub-portions of regions within a target nucleic acid. "Sites," as used in the present invention, are defined as positions within a target nucleic acid.

Since, as is known in the art, the translation initiation codon is typically 5'-AUG (in transcribed mRNA molecules; 5'-ATG in the corresponding DNA molecule), the translation initiation codon is also referred to as the "AUG codon," the "start codon" or the "AUG start codon". A minority of genes have a translation initiation codon having the RNA sequence 5'-GUG, 5'-UUG or 5'-CUG, and 5'-AUA, 5'-ACG and 5'-CUG have been shown to function *in vivo.* Thus, the terms "translation initiation codon" and "start codon" can encompass many codon sequences, even though the initiator amino acid in each instance is typically methionine (in eukaryotes) or formylmethionine (in prokaryotes). It is also known in the art that eukaryotic and prokaryotic genes may have two or more alternative start codons, any one of which may be preferentially utilized for translation initiation in a particular cell type or tissue, or under a particular set of conditions. In the context of the invention, "start codon" and "translation initiation codon" refer to the codon or codons that are used *in vivo* to initiate translation of an mRNA transcribed from a gene encoding glucocorticoid receptor, regardless of the sequence(s) of such codons. It is also known in the art that a translation termination codon (or "stop codon") of a gene may have one of three sequences, i.e., 5'-UAA, 5'-UAG and 5'-UGA (the corresponding DNA sequences are 5'-TAA, 5'-TAG and 5'-TGA, respectively).

The terms "start codon region" and "translation initiation codon region" refer to a portion of such an mRNA or gene that encompasses from about 25 to about 50 contiguous nucleotides in either direction (i.e., 5' or 3') from a translation initiation codon. Similarly, the terms "stop codon region" and "translation termination codon region" refer to a portion of such an mRNA or gene that encompasses from about 25 to about 50 contiguous nucleotides in either direction (i.e., 5' or 3') from a translation termination codon. Consequently, the "start codon region" (or "translation initiation codon region") and the "stop codon region" (or "translation termination codon region") are all regions which may be targeted effectively with the antisense compounds of the present invention.

The open reading frame (ORF) or "coding region," which is known in the art to refer to the region between the translation initiation codon and the translation termination codon, is also a region which may be targeted effectively. Within the context of the present invention, a preferred region is the intragenic region encompassing the translation initiation or termination codon of the open reading frame (ORF) of a gene.

Other target regions include the 5' untranslated region (5'UTR), known in the art to refer to the portion of an mRNA in the 5' direction from the translation initiation codon, and thus including nucleotides between the 5' cap site and the translation initiation codon of an mRNA (or corresponding nucleotides on the gene), and the 3' untranslated region (3'UTR), known in the art to refer to the portion of an mRNA in the 3' direction from the translation termination codon, and thus including nucleotides between the translation termination codon and 3' end of an mRNA (or corresponding nucleotides on the gene). The 5' cap site of an mRNA comprises an N7-methylated guanosine residue joined to the 5'-most residue of the mRNA via a 5'-5' triphosphate linkage. The 5' cap region of an mRNA is considered to include the 5' cap structure itself as well as the first 50 nucleotides adjacent to the cap site. It is also preferred to target the 5' cap region.

Although some eukaryotic mRNA transcripts are directly translated, many contain one or more regions, known as "introns," which are excised from a transcript before it is translated. The remaining (and therefore translated) regions are known as "exons" and are spliced together to form a continuous mRNA sequence. Targeting splice sites, i.e., intron-exon junctions or exon-intron junctions, may also be particularly useful in situations where aberrant splicing is implicated in disease, or where an overproduction of a particular splice product is implicated in disease. Aberrant fusion junctions due to rearrangements or deletions are also preferred target sites. mRNA transcripts produced via the process of splicing of two (or more) mRNAs from different gene sources are known as "fusion transcripts". It is also known that introns can be effectively targeted using antisense compounds targeted to, for example, DNA or pre-mRNA.

It is also known in the art that alternative RNA transcripts can be produced from the same genomic region of DNA. These alternative transcripts are generally known as "variants". More specifically, "pre-mRNA variants" are transcripts produced from the same genomic DNA that differ from other transcripts produced from the same genomic DNA in either their start or stop position and contain both intronic and exonic sequence.

Upon excision of one or more exon or intron regions, or portions thereof during splicing, pre-mRNA variants produce smaller "mRNA variants". Consequently, mRNA variants are processed pre-mRNA variants and each unique pre-mRNA variant must always produce a unique mRNA variant as a result of splicing. These mRNA variants are also known as "alternative splice variants". If no splicing of the pre-mRNA variant occurs then the pre-mRNA variant is identical to the mRNA variant.

It is also known in the art that variants can be produced through the use of alternative signals to start or stop transcription and that pre-mRNAs and mRNAs can possess more that one start codon or stop codon. Variants that originate from a pre-mRNA or mRNA that use alternative start codons are known as "alternative start variants" of that pre-mRNA or mRNA. Those transcripts that use an alternative stop codon are known as "alternative stop variants" of that pre-mRNA or mRNA. One specific type of alternative stop variant is the "polyA variant" in which the multiple transcripts produced result from the alternative selection of one of the "polyA stop signals" by the transcription machinery, thereby producing transcripts that terminate at unique polyA sites. Within the context of the invention, the types of variants described herein are also preferred target nucleic acids.

The locations on the target nucleic acid to which the preferred antisense compounds hybridize are hereinbelow referred to as "preferred target segments." As used herein the term "preferred target segment" is defined as at least an 8-nucleobase portion of a target region to which an active antisense compound is targeted. While not wishing to be bound by theory, it is presently believed that these target segments represent portions of the target nucleic acid which are accessible for hybridization.

One of skill in the art will recognize that the active antisense compound sequences and their target segments ("preferred target segments") serve to illustrate and describe particular embodiments within the scope of the present invention. Additional active antisense compounds and preferred target segments may be identified by one having ordinary skill.

Once one or more target regions, segments or sites have been identified, antisense compounds are chosen which are sufficiently complementary to the target, i.e., hybridize sufficiently well and with sufficient specificity, to give the desired effect.

The oligomeric antisense compounds may also be targeted to regions of the target nucleobase sequence (e.g., such as those disclosed in Example 13 and other examples herein) comprising nucleobases 1-80, 81-160, 161-240, 241-320, 321-400, 401-480, 481-560, 561-640, 641-720, 721-800, 801-880, 881-960, 961-1040, 1041-1120, 1121-1200, 1201-1280, 1281-1360, 1361-1440, 1441-1520, 1521-1600, 1601-1680, 1681-1760, 1761-1840, 1841-1920, 1921-2000, 2001-2080, 2081-2160, 2161-2240, 2241-2320, 2321-2400, 2401-2480, 2481-2560, 2561-2640, 2641-2720, 2721-2800, 2801-2880, 2881-2960, 2961-3040, 3041-3120, 3121-3200, 3201-3280, 3281-3360, 3361-3440, 3441-3520, 3521-3600, 3601-3680, 3681-3760, 3761-3840, 3841-3920, 3921-4000, 4001-4080, 4081-4160, 4161-4240, 4241-4320, 4321-4400, 4401-4480, 4481-4560, 4561-4640, 4641-4720 or 4721-4788 of SEQ ID NO: 4, or any combination thereof.

In one embodiment of the present invention, antisense compounds are targeted to nucleotides 13-119 in the 5' UTR, nucleotides 114-151 in the start codon region, nucleotides 351-533, 667-845, 877-1243, 1356-1488, 1552-1756, 1819-1999, 2008-2139, 2146-2194, 2201-2301, or 2386-2416 in the coding region or nucleotides 2488-2685, 2723-3435, 3499-3789, 3826-3860, 3886-3905, 3918-3937, 4031-4072, 4082-4193 or 4244-4758 in the 3' UTR, all of SEQ ID NO: 4; or nucleotides 104562-104648 in the 3' UTR of SEQ ID NO: 25.

In another embodiment of the present invention, antisense compounds are targeted to nucleotides 2-20 in the start codon region, 301-1405, 1459-2043 or 2050-2309 in the coding region, nucleotides 2376-2433 or 2521-2546 in the 3' UTR, all of SEQ ID NO: 11; nucleotides 227-297 in the 5' UTR of SEQ ID NO: 219; or nucleotides 14909-18389 in the 3' UTR of SEQ ID NO: 220.

In a further embodiment of the present invention, antisense compounds are targeted to nucleotides 150-2129 or 2136-2395 in the coding region, or nucleotides 2472-3705, 4576-4867, 5039-5293, 5680-5877 or 6214-6263 in the 3' UTR, all of SEQ ID NO: 18; or nucleotides 278-304 in the coding region of SEQ ID NO: 256.

### D. Screening and Target Validation

In a further embodiment, the "preferred target segments" identified herein may be employed in a screen for additional compounds that modulate the expression of glucocorticoid receptor. "Modulators" are those compounds that decrease or increase the expression of a nucleic acid molecule encoding glucocorticoid receptor and which comprise at least an 8-nucleobase portion which is complementary to a preferred target segment. The screening method comprises the steps of contacting a preferred target segment of a nucleic acid molecule encoding glucocorticoid receptor with one or more candidate modulators, and selecting for one or more candidate modulators which decrease or increase the expression of a nucleic acid molecule encoding glucocorticoid receptor. Once it is shown that the candidate modulator or modulators are capable of modulating (e.g. either decreasing or increasing) the expression of a nucleic acid molecule encoding glucocorticoid receptor, the modulator may then be employed in further investigative studies of the function of glucocorticoid receptor, or for use as a research, diagnostic, or therapeutic agent in accordance with the present invention.

The preferred target segments of the present invention may be also be combined with their respective complementary antisense compounds of the present invention to form stabilized double-stranded (duplexed) oligonucleotides. Such double stranded oligonucleotide moieties have been shown in the art to modulate target expression and regulate translation as well as RNA processsing via an antisense mechanism. Moreover, the double-stranded moieties may be subject to chemical modifications (Fire et al., Nature, 1998, 391, 806-811; Timmons and Fire, Nature 1998, 395, 854; Timmons et al., Gene, 2001, 263, 103-112; Tabara et al., Science, 1998, 282, 430-431; Montgomery et al., Proc. Natl. Acad. Sci. USA, 1998, 95, 15502-15507; Tuschl et al., Genes Dev., 1999,13, 3191-3197; Elbashir et al., Nature, 2001, 411, 494-498; Elbashir et al., Genes Dev. 2001, 15, 188-200). For example, such double-stranded moieties have been shown to inhibit the target by the classical hybridization of antisense strand of the duplex to the target, thereby triggering enzymatic degradation of the target (Tijsterman et al., Science, 2002, 295, 694-697).

The antisense compounds of the present invention can also be applied in the areas of drug discovery and target validation. The present invention comprehends the use of the compounds and preferred target segments identified herein in drug discovery efforts to elucidate relationships that exist between glucocorticoid receptor and a disease state, phenotype, or condition. These methods include detecting or modulating glucocorticoid receptor comprising contacting a sample, tissue, cell, or organism with the compounds of the present invention, measuring the nucleic acid or protein level of glucocorticoid receptor and/or a related phenotypic or chemical endpoint at some time after treatment, and optionally comparing the measured value to a non-treated sample or sample treated with a further compound of the invention. These methods can also be performed in parallel or in combination with other experiments to determine the function of unknown genes for the process of target validation or to determine the validity of a particular gene product as a target for treatment or prevention of a particular disease, condition, or phenotype.

### E. Kits, Research Reagents, Diagnostics, and Therapeutics

The antisense compounds of the present invention can be utilized for diagnostics, therapeutics, prophylaxis and as research reagents and kits. Furthermore, antisense oligonucleotides, which are able to inhibit gene expression with exquisite specificity, are often used by those of ordinary skill to elucidate the function of particular genes or to distinguish between functions of various members of a biological pathway.

For use in kits and diagnostics, the compounds of the present invention, either alone or in combination with other compounds or therapeutics, can be used as tools in differential and/or combinatorial analyses to elucidate expression patterns of a portion or the entire complement of genes expressed within cells and tissues.

As one nonlimiting example, expression patterns within cells or tissues treated with one or more antisense compounds are compared to control cells or tissues not treated with antisense compounds and the patterns produced are analyzed for differential levels of gene expression as they pertain, for example, to disease association, signaling pathway, cellular localization, expression level, size, structure or function of the genes examined. These analyses can be performed on stimulated or unstimulated cells and in the presence or absence of other compounds which affect expression patterns.

Examples of methods of gene expression analysis known in the art include DNA arrays or microarrays (Brazma and Vilo, FEBSLett., 2000, 480, 17-24; Celis, et al., FEBSLett., 2000, 480, 2-16), SAGE (serial analysis of gene expression)(Madden, et al., Drug Discov. Today, 2000, 5, 415-425), READS (restriction enzyme amplification of digested cDNAs) (Prashar and Weissman, Methods Enzymol., 1999, 303, 258-72), TOGA (total gene expression analysis) (Sutcliffe, et al., Proc. Natl. Acad. Sci. U.S.A., 2000, 97, 1976-81), protein arrays and proteomics (Celis, et al., FEBSLett., 2000, 480, 2-16; Jungblut, et al., Electrophoresis, 1999, 20, 2100-10), expressed sequence tag (**EST**) sequencing (Celis, et al., FEBS Lett., 2000, 480, 2-16; Larsson, et al., J. Biotechnol., 2000, 80, 143-57), subtractive RNA fingerprinting (SuRF) (Fuchs, et al., Areal. Biochem., 2000, 286, 91-98; Larson, et al., Cytometry, 2000, 41, 203-208), subtractive cloning, differential display (DD) (Jurecic and Belmont, Curr. Opin. Microbiol., 2000, 3, 316-21), comparative genomic hybridization (Carulli, et al., J. Cell Biochem. Suppl., 1998, 31, 286-96), **FISH** (fluorescent *in situ* hybridization) techniques (Going and Gusterson, Eur. J. Cancer, 1999, 35, 1895-904) and mass spectrometry methods (To, Comb. Chem. High Throughput Screen, 2000, 3, 235-41).

The antisense compounds of the invention are useful for research and diagnostics, because these compounds hybridize to nucleic acids encoding glucocorticoid receptor and modulate the expression of glucocorticoid receptor. The specificity and sensitivity of antisense is also harnessed by those of skill in the art for therapeutic uses. Antisense compounds have been employed as therapeutic moieties in the treatment of disease states in animals, including humans. Antisense oligonucleotide drugs, including ribozymes, have been safely and effectively administered to humans and numerous clinical trials are presently underway. It is thus established that antisense compounds can be useful therapeutic modalities that can be configured to be useful in treatment regimes for the treatment of cells, tissues and animals, especially humans.

For therapeutics, an animal, preferably a human, suspected of having a disease or disorder which can be treated by modulating the expression of glucocorticoid receptor is treated by administering antisense compounds in accordance with this invention. For example, in one non-limiting embodiment, the methods comprise the step of administering to the animal in need of treatment, a therapeutically effective amount of a glucocorticoid receptor inhibitor. The glucocorticoid receptor inhibitors of the present invention effectively inhibit the activity of the glucocorticoid receptor protein or inhibit the expression of the glucocorticoid receptor protein. In one embodiment, the activity or expression of glucocorticoid receptor in an animal is inhibited by about 10%. Preferably, the activity or expression of glucocorticoid receptor in an animal is inhibited by about 30%. More preferably, the activity or expression of glucocorticoid receptor in an animal is inhibited by 50% or more. Thus, the oligomeric antisense compounds modulate expression of glucocorticoid receptor mRNA by at least 10%, by at least 20%, by at least 25%, by at least 30%, by at least 40%, by at least 50%, by at least 60%, by at least 70%, by at least 75%, by at least 80%, by at least 85%, by at least 90%, by at least 95%, by at least 98%, by at least 99%, or by 100%.

For example, the reduction of the expression of glucocorticoid receptor may be measured in serum, adipose tissue, liver or any other body fluid, tissue or organ of the animal. Preferably, the cells contained within said fluids, tissues or organs being analyzed contain a nucleic acid molecule encoding glucocorticoid receptor protein and/or the glucocorticoid receptor protein itself.

The antisense compounds of the invention can be utilized in pharmaceutical compositions by adding an effective amount of a compound to a suitable pharmaceutically acceptable diluent or carrier. Use of the compounds and methods of the invention may also be useful prophylactically.

The compounds of the present inventions are inhibitors of glucocorticoid receptor expression. Thus, the compounds of the present invention are believed to be useful for treating metabolic diseases and conditions, particularly diabetes, hyperglycemia induced by systemic steroid therapy, obesity, hyperlipidemia or metabolic syndrome X. The compounds of the present invention may also be useful for treating Cushing's Syndrome, Addison's disease, allergy, autoimmune disease, immunodeficiency, anorexia, cachexia, bone loss or bone frailty, for treating inflammatory diseases such as asthma, rhinitis and arthritis, and for promoting wound healing. The compounds of the invention are also believed to be useful for preventing or delaying the onset of metabolic diseases and conditions, particularly diabetes, obesity, hyperlipidemia or metabolic syndrome X, and for preventing or delaying the onset of Cushing's Syndrome, Addison's disease, allergy, autoimmune disease, immunodeficiency, anorexia, cachexia, bone loss, or inflammatory diseases such as asthma, rhinitis and arthritis.

The compounds of the invention have been found to be effective for lowering blood glucose, including plasma glucose, and for lowering blood lipids, including serum lipids, particularly serum cholesterol and serum triglycerides. The compounds of the invention are therefore particularly useful for the treatment, prevention and delay of onset of type 2 diabetes, high blood glucose and hyperlipidemia. Surprisingly, the compounds of the invention have been found to have these therapeutic effects in the absence of certain side effects such as, for example, elevated corticosterone levels or lymphopenia which are associated with systemic inhibition of glucocorticoid receptor signaling.

### F. Modifications

As is known in the art, a nucleoside is a base-sugar combination. The base portion of the nucleoside is normally a heterocyclic base sometimes referred to as a "nucleobase" or simply a "base". The two most common classes of such heterocyclic bases are the purines and the pyrimidines. Nucleotides are nucleosides that further include a phosphate group covalently linked to the sugar portion of the nucleoside. For those nucleosides that include a pentofuranosyl sugar, the phosphate group can be linked to either the 2', 3' or 5' hydroxyl moiety of the sugar. In forming oligonucleotides, the phosphate groups covalently link adjacent nucleosides to one another to form a linear polymeric compound. In turn, the respective ends of this linear polymeric compound can be further joined to form a circular compound, however, linear compounds are generally preferred. In addition, linear compounds may have internal nucleobase complementarity and may therefore fold in a manner as to produce a fully or partially double-stranded compound. Within oligonucleotides, the phosphate groups are commonly referred to as forming the internucleoside backbone of the oligonucleotide. The normal linkage or backbone of RNA and DNA is a 3' to 5' phosphodiester linkage.

### Modified Internucleoside Linkages (Backbones)

Specific examples of preferred antisense compounds useful in this invention include oligonucleotides containing modified backbones or non-natural internucleoside linkages. As defined in this specification, oligonucleotides having modified backbones include those that retain a phosphorus atom in the backbone and those that do not have a phosphorus atom in the backbone. For the purposes of this specification, and as sometimes referenced in the art, modified oligonucleotides that do not have a phosphorus atom in their internucleoside backbone can also be considered to be oligonucleosides.

Preferred modified oligonucleotide backbones containing a phosphorus atom therein include, for example, phosphorothioates, chiral phosphorothioates, phosphorodithioates, phosphotriesters, aminoalkylphosphotriaminoalkylphosphotriesters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, selenophosphates and boranophosphates having normal 3'-5' linkages, 2'-5' linked analogs of these, and those having inverted polarity wherein one or more internucleotide linkages is a 3' to 3', 5' to 5' or 2' to 2' linkage. Preferred oligonucleotides having inverted polarity comprise a single 3' to 3' linkage at the 3'-most internucleotide linkage i.e. a single inverted nucleoside residue which may be abasic (the nucleobase is missing or has a hydroxyl group in place thereof). Various salts, mixed salts and free acid forms are also included.

Representative United States patents that teach the preparation of the above phosphorus-containing linkages include, but are not limited to, U.S.: 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; 5,194,599; 5,565,555; 5,527,899; 5,721,218; 5,672,697 and 5,625,050, certain of which are commonly owned with this application, and each of which is herein incorporated by reference.

Preferred modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH₂ component parts.

Representative United States patents that teach the preparation of the above oligonucleosides include, but are not limited to, U.S.: 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; 5,792,608; 5,646,269 and 5,677,439, certain of which are commonly owned with this application, and each of which is herein incorporated by reference.

### Modified sugar and internucleoside linkages-Mimetics

In other preferred antisense compounds, e.g., oligonucleotide mimetics, both the sugar and the internucleoside linkage (i.e. the backbone), of the nucleotide units are replaced with novel groups. The nucleobase units are maintained for hybridization with an appropriate target nucleic acid. One such compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA compounds include, but are not limited to, U.S.: 5,539,082; 5,714,331; and 5,719,262, each of which is herein incorporated by reference. Further teaching of PNA compounds can be found in Nielsen et al., Science, 1991, 254, 1497-1500.

Preferred embodiments of the invention are oligonucleotides with phosphorothioate backbones and oligonucleosides with heteroatom backbones, and in particular -CH₂-NH-O-CH₂-, -CH₂-N(CH₃)-O-CH₂- [known as a methylene (methylimino) or MMI backbone], -CH₂-O-N(CH₃)-CH₂-, -CH₂-N(CH₃)-N(CH₃)-CH₂- and -O-N(CH₃)-CH₂-CH₂- [wherein the native phosphodiester backbone is represented as - O-P-O-CH₂-] of the above referenced U.S. patent 5,489,677, and the amide backbones of the above referenced U.S. patent 5,602,240. Also preferred are oligonucleotides having morpholino backbone structures of the above-referenced U.S. patent 5,034,506.

### Modified sugars

Modified antisense compounds may also contain one or more substituted sugar moieties. Preferred are antisense compounds, preferably antisense oligonucleotides, comprising one of the following at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C₁ to C₁₀ alkyl or C₂ to C₁₀ alkenyl and alkynyl. Particularly preferred are O[(CH₂)ₙO]ₘCH₃, O(CH₂)ₙOCH₃, O(CH₂)ₙNH₂, O(CH₂)ₙCH₃, O(CH₂)ₙONH₂, and O(CH₂)ₙON[(CH₂)ₙCH₃]₂, where n and m are from 1 to about 10. Other preferred oligonucleotides comprise one of the following at the 2' position: C₁ to C₁₀ lower alkyl, substituted lower alkyl, alkenyl, alkynyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂ N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred modification includes 2'-methoxyethoxy (2'-O-CH₂CH₂OCH₃, also known as 2'-O-(2-methoxyethyl) or 2'-MOE) (Martin et al., Helv. Chim. Acta, 1995, 78, 486-504) i.e., an alkoxyalkoxy group. A further preferred modification includes 2'-dimethylaminooxyethoxy, i.e., a O(CH₂)₂ON(CH₃)₂ group, also known as 2'-DMAOE, as described in examples hereinbelow, and 2'-dimethylaminoethoxyethoxy (also known in the art as 2'-O-dimethyl-amino-ethoxy-ethyl or 2'-DMAEOE), i.e., 2'-O-CH₂-O-CH₂-N(CH₃)₂, also described in examples hereinbelow.

Other preferred modifications include 2'-methoxy (2'-O-CH₃), 2'-aminopropoxy (2'-OCH₂CH₂CH₂NH₂), 2'-allyl (2'-CH₂-CH=CH₂), 2'-O-allyl (2'-O-CH₂-CH=CH₂) and 2'-fluoro (2'-F). The 2'-modification may be in the arabino (up) position or ribo (down) position. A preferred 2'-arabino modification is 2'-F. Similar modifications may also be made at other positions on the oligonucleotide, particularly the 3' position of the sugar on the 3' terminal nucleotide or in 2'-5' linked oligonucleotides and the 5' position of 5' terminal nucleotide. Antisense compounds may also have sugar mimetics such as cyclobutyl moieties in place of the pentofuranosyl sugar. Representative United States patents that teach the preparation of such modified sugar structures include, but are not limited to, U.S.: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; 5,792,747; and 5,700,920, certain of which are commonly owned with the instant application, and each of which is herein incorporated by reference in its entirety.

A further preferred modification of the sugar includes Locked Nucleic Acids (LNAs) in which the 2'-hydroxyl group is linked to the 3' or 4' carbon atom of the sugar ring, thereby forming a bicyclic sugar moiety. The linkage can be a methylene (-CH₂-) group bridging the 2' oxygen atom and the 4' carbon atom, for which the term LNA is used for the bicyclic moiety.. LNAs and preparation thereof are described in WO 98/39352 and WO 99/14226. In the case of an ethylene group in this position, the term ENA is used (Singh et al., Chem. Commun., 1998, 4, 455-456; ENA™: Morita et al., Bioorganic Medicinal Chemistry, 2003,11, 2211-2226).

### Natural and Modified Nucleobases

Antisense compounds may also include nucleobase (often referred to in the art as heterocyclic base or simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine and 3-deazaguanine and 3-deazaadenine. Further modified nucleobases include tricyclic pyrimidines such as phenoxazine cytidine(1H-pyrimido[5,4-b][1,4]benzoxazin-2(3H)-one), phenothiazine cytidine (1H-pyrimido[5,4-b][1,4]benzothiazin-2(3H)-one), G-clamps such as a substituted phenoxazine cytidine (e.g. 9-(2-aminoethoxy)-H-pyrimido[5,4-b][1,4]benzoxazin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in United States Patent No. 3,687,808, those disclosed in The Concise Encyclopedia Of Polymer Science And Engineering, pages 858-859, Kroschwitz, J.I., ed. John Wiley & Sons, 1990, those disclosed by Englisch et al., Angewandte Chemie, International Edition, 1991, 30, 613, and those disclosed by Sanghvi, Y.S., Chapter 15, Antisense Research and Applications, pages 289-302, Crooke, S.T. and Lebleu, B. , ed., CRC Press, 1993. Certain of these nucleobases are particularly useful for increasing the binding affinity of the compounds of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2 °C and are presently preferred base substitutions, even more particularly when combined with 2'-O-methoxyethyl sugar modifications.

Representative United States patents that teach the preparation of certain of the above noted modified nucleobases as well as other modified nucleobases include, but are not limited to, the above noted U.S. 3,687,808, as well as U.S.: 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; 5,645,985; 5,830,653; 5,763,588; 6,005,096; and 5,681,941, certain of which are commonly owned with the instant application, and each of which is herein incorporated by reference, and United States patent 5,750,692, which is commonly owned with the instant application and also herein incorporated by reference.

### Conjugates

Another modification of the antisense compounds of the invention involves chemically linking to the antisense compound one or more moieties or conjugates which enhance the activity, cellular distribution or cellular uptake of the oligonucleotide. These moieties or conjugates can include conjugate groups covalently bound to functional groups such as primary or secondary hydroxyl groups. Conjugate groups of the invention include intercalators, reporter molecules, polyamines, polyamides, polyethylene glycols, polyethers, groups that enhance the pharmacodynamic properties of oligomers, and groups that enhance the pharmacokinetic properties of oligomers. Typical conjugate groups include cholesterols, lipids, phospholipids, biotin, phenazine, folate, phenanthridine, anthraquinone, acridine, fluoresceins, rhodamines, coumarins, and dyes. Groups that enhance the pharmacodynamic properties, in the context of this invention, include groups that improve uptake, enhance resistance to degradation, and/or strengthen sequence-specific hybridization with the target nucleic acid. Groups that enhance the pharmacokinetic properties, in the context of this invention, include groups that improve uptake, distribution, metabolism or excretion of the compounds of the present invention. Representative conjugate groups are disclosed in International Patent Application PCT/US92/09196, filed October 23, 1992, and U.S. Patent 6,287,860, the entire disclosure of which are incorporated herein by reference. Conjugate moieties include but are not limited to lipid moieties such as a cholesterol moiety, cholic acid, a thioether, e.g., hexyl-S-tritylthiol, a thiocholesterol, an aliphatic chain, e.g., dodecandiol or undecyl residues, a phospholipid, e.g., di-hexadecyl-rac-glycerol or triethylammonium 1,2-di-O-hexadecyl-rac-glycero-3-H-phosphonate, a polyamine or a polyethylene glycol chain, or adamantane acetic acid, a palmityl moiety, or an octadecylamine or hexylamino-carbonyl-oxycholesterol moiety. Antisense compounds of the invention may also be conjugated to active drug substances, for example, aspirin, warfarin, phenylbutazone, ibuprofen, suprofen, fenbufen, ketoprofen, (*S*)-(+)-pranoprofen, carprofen, dansylsarcosine, 2,3,5-triiodobenzoic acid, flufenamic acid, folinic acid, a benzothiadiazide, chlorothiazide, a diazepine, indomethicin, a barbiturate, a cephalosporin, a sulfa drug, an antidiabetic, an antibacterial or an antibiotic. Oligonucleotide-drug conjugates and their preparation are described in United States Patent Application 09/334,130 (filed June 15, 1999) which is incorporated herein by reference in its entirety.

Representative United States patents that teach the preparation of such oligonucleotide conjugates include, but are not limited to, U.S.: 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941, certain of which are commonly owned with the instant application, and each of which is herein incorporated by reference.

### Chimeric compounds

It is not necessary for all positions in a given compound to be uniformly modified, and in fact more than one of the aforementioned modifications may be incorporated in a single compound or even at a single nucleoside within an oligonucleotide.

The present invention also includes antisense compounds which are chimeric compounds. "Chimeric" antisense compounds or "chimeras," in the context of this invention, are antisense compounds, particularly oligonucleotides, which contain two or more chemically distinct regions, each made up of at least one monomer unit, i.e., a nucleotide in the case of an oligonucleotide compound. Chimeric antisense oligonucleotides are thus a form of antisense compound. These oligonucleotides typically contain at least one region wherein the oligonucleotide is modified so as to confer upon the oligonucleotide increased resistance to nuclease degradation, increased cellular uptake, increased stability and/or increased binding affinity for the target nucleic acid. An additional region of the oligonucleotide may serve as a substrate for enzymes capable of cleaving RNA:DNA or RNA:RNA hybrids. By way of example, RNAse H is a cellular endonuclease which cleaves the RNA strand of an RNA:DNA duplex. Activation of RNase H, therefore, results in cleavage of the RNA target, thereby greatly enhancing the efficiency of oligonucleotide-mediated inhibition of gene expression. The cleavage of RNA:RNA hybrids can, in like fashion, be accomplished through the actions of endoribonucleases, such as RNAseL which cleaves both cellular and viral RNA. Cleavage of the RNA target can be routinely detected by gel electrophoresis and, if necessary, associated nucleic acid hybridization techniques known in the art.

Chimeric antisense compounds of the invention may be formed as composite structures of two or more oligonucleotides, modified oligonucleotides, oligonucleosides and/or oligonucleotide mimetics as described above. Such compounds have also been referred to in the art as hybrids or gapmers. Representative United States patents that teach the preparation of such hybrid structures include, but are not limited to, U.S.: 5,013,830; 5,149,797; 5,220,007; 5,256,775; 5,366,878; 5,403,711; 5,491,133; 5,565,350; 5,623,065; 5,652,355; 5,652,356; and 5,700,922, certain of which are commonly owned with the instant application, and each of which is herein incorporated by reference in its entirety.

### G. Formulations

The compounds of the invention may also be admixed, encapsulated, conjugated or otherwise associated with other molecules, molecule structures or mixtures of compounds, as for example, liposomes, receptor-targeted molecules, oral, rectal, topical or other formulations, for assisting in uptake, distribution and/or absorption. Representative United States patents that teach the preparation of such uptake, distribution and/or absorption-assisting formulations include, but are not limited to, U.S.: 5,108,921; 5,354,844; 5,416,016; 5,459,127; 5,521,291; 5,543,158; 5,547,932; 5,583,020; 5,591,721; 4,426,330; 4,534,899; 5,013,556; 5,108,921; 5,213,804; 5,227,170; 5,264,221; 5,356,633; 5,395,619; 5,416,016; 5,417,978; 5,462,854; 5,469,854; 5,512,295; 5,527,528; 5,534,259; 5,543,152; 5,556,948; 5,580,575; and 5,595,756, each of which is herein incorporated by reference.

The antisense compounds of the invention encompass any pharmaceutically acceptable salts, esters, or salts of such esters, or any other compound which, upon administration to an animal, including a human, is capable of providing (directly or indirectly) the biologically active metabolite or residue thereof.

The term "pharmaceutically acceptable salts" refers to physiologically and pharmaceutically acceptable salts of the compounds of the invention: i.e., salts that retain the desired biological activity of the parent compound and do not impart undesired toxicological effects thereto. For oligonucleotides, preferred examples of pharmaceutically acceptable salts and their uses are further described in U.S. Patent 6,287,860, which is incorporated herein in its entirety. For oligonucleotides, presently preferred examples of pharmaceutically acceptable salts include but are not limited to (a) salts formed with cations such as sodium, potassium, ammonium, magnesium, calcium, polyamines such as spermine and spermidine, *etc*.; (b) acid addition salts formed with inorganic acids, for example hydrochloric acid, hydrobromic acid, sulfuric acid, phosphoric acid, nitric acid and the like; (c) salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, naphthalenesulfonic acid, methanesulfonic acid, p-toluenesulfonic acid, naphthalenedisulfonic acid, polygalacturonic acid, and the like; and (d) salts formed from elemental anions such as chlorine, bromine, and iodine. Sodium salts are presently believed to be more preferred.

The present invention also includes pharmaceutical compositions and formulations which include the antisense compounds of the invention. The pharmaceutical compositions of the present invention may be administered in a number of ways depending upon whether local or systemic treatment is desired and upon the area to be treated. Administration may be topical (including ophthalmic and to mucous membranes including vaginal and rectal delivery), pulmonary, e.g., by inhalation or insufflation of powders or aerosols, including by nebulizer; intratracheal, intranasal, epidermal and transdermal), oral or parenteral. Parenteral administration includes intravenous, intraarterial, subcutaneous, intraperitoneal or intramuscular injection or infusion; or intracranial, e.g., intrathecal or intraventricular, administration. Oligonucleotides with at least one 2'-O-methoxyethyl modification are believed to be particularly useful for oral administration. Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful.

The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers or both, and then, if necessary, shaping the product.

The compositions of the present invention may be formulated into any of many possible dosage forms such as, but not limited to, tablets, capsules, gel capsules, liquid syrups, soft gels, suppositories, and enemas. The compositions of the present invention may also be formulated as suspensions in aqueous, non-aqueous or mixed media. Aqueous suspensions may further contain substances which increase the viscosity of the suspension including, for example, sodium carboxymethylcellulose, sorbitol and/or dextran. The suspension may also contain stabilizers.

Pharmaceutical compositions of the present invention include, but are not limited to, solutions, emulsions, foams and liposome-containing formulations. The pharmaceutical compositions and formulations of the present invention may comprise one or more penetration enhancers, carriers, excipients or other active or inactive ingredients.

Emulsions are typically heterogenous systems of one liquid dispersed in another in the form of droplets usually exceeding 0.1 µm in diameter. Emulsions may contain additional components in addition to the dispersed phases, and the active drug which may be present as a solution in either the aqueous phase, oily phase or itself as a separate phase. Microemulsions are included as an embodiment of the present invention. Emulsions and their uses are well known in the art and are further described in U.S. Patent 6,287,860, which is incorporated herein in its entirety.

Formulations of the present invention include liposomal formulations. As used in the present invention, the term "liposome" means a vesicle composed of amphiphilic lipids arranged in a spherical bilayer or bilayers. Liposomes are unilamellar or multilamellar vesicles which have a membrane formed from a lipophilic material and an aqueous interior that contains the composition to be delivered. Cationic liposomes are positively charged liposomes which are believed to interact with negatively charged DNA molecules to form a stable complex. Liposomes that are pH-sensitive or negatively-charged are believed to entrap DNA rather than complex with it. Both cationic and noncationic liposomes have been used to deliver DNA to cells.

Liposomes also include "sterically stabilized" liposomes, a term which, as used herein, refers to liposomes comprising one or more specialized lipids that, when incorporated into liposomes, result in enhanced circulation lifetimes relative to liposomes lacking such specialized lipids. Examples of sterically stabilized liposomes are those in which part of the vesicle-forming lipid portion of the liposome comprises one or more glycolipids or is derivatized with one or more hydrophilic polymers, such as a polyethylene glycol (PEG) moiety. Liposomes and their uses are further described in U.S. Patent 6,287,860, which is incorporated herein in its entirety.

The pharmaceutical formulations and compositions of the present invention may also include surfactants. The use of surfactants in drug products, formulations and in emulsions is well known in the art. Surfactants and their uses are further described in U.S. Patent 6,287,860, which is incorporated herein in its entirety.

In one embodiment, the present invention employs various penetration enhancers to effect the efficient delivery of nucleic acids, particularly oligonucleotides. In addition to aiding the diffusion of non-lipophilic drugs across cell membranes, penetration enhancers also enhance the permeability of lipophilic drugs. Penetration enhancers may be classified as belonging to one of five broad categories, *i.e.,* surfactants, fatty acids, bile salts, chelating agents, and non-chelating non-surfactants. Penetration enhancers and their uses are further described in U.S. Patent 6,287,860, which is incorporated herein in its entirety.

One of skill in the art will recognize that formulations are routinely designed according to their intended use, i.e. route of administration.

Preferred formulations for topical administration include those in which the oligonucleotides of the invention are in admixture with a topical delivery agent such as lipids, liposomes, fatty acids, fatty acid esters, steroids, chelating agents and surfactants. Preferred lipids and liposomes include neutral (e.g. dioleoylphosphatidyl DOPE ethanolamine, dimyristoylphosphatidyl choline DMPC, distearolyphosphatidyl choline) negative (e.g. dimyristoylphosphatidyl glycerol DMPG) and cationic (e.g. dioleoyltetramethylaminopropyl DOTAP and dioleoylphosphatidyl ethanolamine DOTMA).

For topical or other administration, oligonucleotides of the invention may be encapsulated within liposomes or may form complexes thereto, in particular to cationic liposomes. Alternatively, oligonucleotides may be complexed to lipids, in particular to cationic lipids. Preferred fatty acids and esters, pharmaceutically acceptable salts thereof, and their uses are further described in U.S. Patent 6,287,860, which is incorporated herein in its entirety. Topical formulations are described in detail in United States patent application 09/315,298 filed on May 20, 1999, which is incorporated herein by reference in its entirety.

Compositions and formulations for oral administration include powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders may be desirable. Preferred oral formulations are those in which oligonucleotides of the invention are administered in conjunction with one or more penetration enhancers surfactants and chelators. Preferred surfactants include fatty acids and/or esters or salts thereof, bile acids and/or salts thereof. Preferred bile acids/salts and fatty acids and their uses are further described in U.S. Patent 6,287,860, which is incorporated herein in its entirety. Also preferred are combinations of penetration enhancers, for example, fatty acids/salts in combination with bile acids/salts. A particularly preferred combination is the sodium salt of lauric acid, capric acid and UDCA. Further penetration enhancers include polyoxyethylene-9-lauryl ether, polyoxyethylene-20-cetyl ether. Oligonucleotides of the invention may be delivered orally, in granular form including sprayed dried particles, or complexed to form micro or nanoparticles. Oligonucleotide complexing agents and their uses are further described in U.S. Patent 6,287,860, which is incorporated herein in its entirety. Oral formulations for oligonucleotides and their preparation are described in detail in United States applications 09/108,673 (filed July 1, 1998), 09/315,298 (filed May 20, 1999) and 10/071,822, filed February 8, 2002, each of which is incorporated herein by reference in their entirety.

Compositions and formulations for parenteral, intrathecal or intraventricular administration may include sterile aqueous solutions which may also contain buffers, diluents and other suitable additives such as, but not limited to, penetration enhancers, carrier compounds and other pharmaceutically acceptable carriers or excipients.

Certain embodiments of the invention provide pharmaceutical compositions containing one or more oligomeric compounds and one or more other chemotherapeutic agents which function by a non-antisense mechanism. Examples of such chemotherapeutic agents include but are not limited to cancer chemotherapeutic drugs such as daunorubicin, daunomycin, dactinomycin, doxorubicin, epirubicin, idarubicin, esorubicin, bleomycin, mafosfamide, ifosfamide, cytosine arabinoside, bis-chloroethylnitrosurea, busulfan, mitomycin C, actinomycin D, mithramycin, prednisone, hydroxyprogesterone, testosterone, tamoxifen, dacarbazine, procarbazine, hexamethylmelamine, pentamethylmelamine, mitoxantrone, amsacrine, chlorambucil, methylcyclohexylnitrosurea, nitrogen mustards, melphalan, cyclophosphamide, 6-mercaptopurine, 6-thioguanine, cytarabine, 5-azacytidine, hydroxyurea, deoxycoformycin, 4-hydroxyperoxycyclophosphoramide, 5-fluorouracil (5-FU), 5-fluorodeoxyuridine (5-FUdR), methotrexate (MTX), colchicine, taxol, vincristine, vinblastine, etoposide (VP-16), trimetrexate, irinotecan, topotecan, gemcitabine, teniposide, cisplatin and diethylstilbestrol (DES). When used with the compounds of the invention, such chemotherapeutic agents may be used individually (e.g., 5-FU and oligonucleotide), sequentially (*e.g.*, 5-FU and oligonucleotide for a period of time followed by MTX and oligonucleotide), or in combination with one or more other such chemotherapeutic agents (*e.g.*, 5-FU, MTX and oligonucleotide, or 5-FU, radiotherapy and oligonucleotide). Anti-inflammatory drugs, including but not limited to nonsteroidal anti-inflammatory drugs and corticosteroids, and antiviral drugs, including but not limited to ribivirin, vidarabine, acyclovir and ganciclovir, may also be combined in compositions of the invention. Combinations of antisense compounds and other non-antisense drugs are also within the scope of this invention. Two or more combined compounds may be used together or sequentially.

In another related embodiment, compositions of the invention may contain one or more antisense compounds, particularly oligonucleotides, targeted to a first nucleic acid and one or more additional antisense compounds targeted to a second nucleic acid target. Alternatively, compositions of the invention may contain two or more antisense compounds targeted to different regions of the same nucleic acid target. Numerous examples of antisense compounds are known in the art. Two or more combined compounds may be used together or sequentially.

### H. Dosing

The formulation of therapeutic compositions and their subsequent administration (dosing) is believed to be within the skill of those in the art. Dosing is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. Persons of ordinary skill can easily determine optimum dosages, dosing methodologies and repetition rates. Optimum dosages may vary depending on the relative potency of individual oligonucleotides, and can generally be estimated based on EC₅₀s found to be effective in *in vitro* and *in vivo* animal models. In general, dosage is from 0.0001 ug to 100 g per kg of body weight, and may be given once or more daily, weekly, monthly or yearly, or even once every 2 to 20 years. Persons of ordinary skill in the art can easily estimate repetition rates for dosing based on measured residence times and concentrations of the drug in bodily fluids or tissues. Following successful treatment, it may be desirable to have the patient undergo maintenance therapy to prevent the recurrence of the disease state, wherein the oligonucleotide is administered in maintenance doses, ranging from 0.0001 ug to 100 g per kg of body weight, once or more daily, to once every 20 years.

While the present invention has been described with specificity in accordance with certain of its preferred embodiments, the following examples serve only to illustrate the invention and are not intended to limit the same. Each of the references, GenBank accession numbers, and the like recited in the present application is incorporated herein by reference in its entirety.

### EXAMPLES

### Example 1

### Synthesis of Nucleoside Phosphoramidites

The following compounds, including amidites and their intermediates were prepared as described in US Patent 6,426,220 and published PCT WO 02/36743; 5'-O-Dimethoxytrityl-thymidine intermediate for 5-methyl dC amidite, 5'-O-Dimethoxytrityl-2'-deoxy-5-methylcytidine intermediate for 5-methyl-dC amidite, 5'-O-Dimethoxytrityl-2'-deoxy-N4-benzoyl-5-methylcytidine penultimate intermediate for 5-methyl dC amidite, [5'-*O*-(4,4'-Dimethoxytriphenylmethyl)-2'-deoxy-N⁴-benzoyl-5-methylcytidin-3'-*O-*yl]-2-cyanoethyl-*N,N*-diisopropylphosphoramidite (5-methyl dC amidite), 2'-Fluorodeoxyadenosine, 2'-Fluorodeoxyguanosine, 2'-Fluorouridine, 2'-Fluorodeoxycytidine, 2'-O-(2-Methoxyethyl) modified amidites, 2'-O-(2-methoxyethyl)-5-methyluridine intermediate, 5'-O-DMT-2'-O-(2-methoxyethyl)-5-methyluridine penultimate intermediate, [5'-*O*-(4,4'-Dimethoxytriphenylmethyl)-2'-*O*-(2-methoxyethyl)-5-methyluridin-3'-*O*-yl]-2-cyanoethyl-*N,N*-diisopropylphosphoramidite (MOE T amidite), 5'-O-Dimethoxytrityl-2'-O-(2-methoxyethyl)-5-methylcytidine intermediate, 5'-O-dimethoxytrityl-2'-*O*-(2-methoxyethyl)-N⁴-benzoyl-5-methyl-cytidine penultimate intermediate, [5'-*O*-(4,4'-Dimethoxytriphenylmethyl)-2'-*O*-(2-methoxyethyl)-N⁴-benzoyl-5-methylcytidin-3'-*O*-yl]-2-cyanoethyl-*N,N-*diisopropylphosphoramidite (MOE 5-Me-C amidite), [5'-*O*-(4,4'-Dimethoxytriphenylmethyl)-2'-*O-*(2-methoxyethyl)-N⁶-benzoyladenosin-3'-*O*-yl]-2-cyanoethyl-*N,N*-diisopropylphosphoramidite (MOE A amidite), [5'-*O*-(4,4'-Dimethoxytriphenylmethyl)-2'-*O*-(2-methoxyethyl)-N⁴-isobutyrylguanosin-3'-*O*-yl]-2-cyanoethyl-*N,N*-diisopropylphosphoramidite (MOE G amidite), 2'-O-(Aminooxyethyl) nucleoside amidites and 2'-O-(dimethylaminooxyethyl) nucleoside amidites, 2'-(Dimethylaminooxyethoxy) nucleoside amidites, 5'-O-tert-Butyldiphenylsilyl-O²-2'-anhydro-5-methyluridine , 5'-O-tert-Butyldiphenylsilyl-2'-O-(2-hydroxyethyl)-5-methyluridine, 2'-O-([2-phthalimidoxy)ethyl]-5'-*t-*butyldiphenylsilyl-5-methyluridine , 5'-O-*tert*-butyldiphenylsilyl-2'-O-[(2-formadoximinooxy)ethyl]-5-methyluridine, 5'-O-*tert*-Butyldiphenylsilyl-2'-O-[N,N-dimethylaminooxyethyl]-5-methyluridine, 2'-O-(dimethylaminooxyethyl)-5-methyluridine, 5'-O-DMT-2'-O-(dimethylaminooxyethyl)-5-methyluridine, 5'-O-DMT-2'-O-(2-N,N-dimethylaminooxyethyl)-5-methyluridine-3'-[(2-cyanoethyl)-N,N-diisopropylphosphoramidite], 2'-(Aminooxyethoxy) nucleoside amidites, N2-isobutyryl-6-O-diphenylcarbamoyl-2'-O-(2-ethylacetyl)-5'-O-(4,4'-dimethoxytrityl)guanosine-3'-[(2-cyanoethyl)-N,N-diisopropylphosphoramidite], 2'-dimethylaminoethoxyethoxy (2'-DMAEOE) nucleoside amidites, 2'-O-[2(2-N,N-dimethylaminoethoxy)ethyl]-5-methyl uridine, 5'-O-dimethoxytrityl-2'-O-[2(2-N,N-dimethyl-aminoethoxy)-ethyl)]-5-methyl uridine and 5'-O-Dimethoxytrityl-2'-O-[2(2-N,N-dimethylaminoethoxy)-ethyl)]-5-methyl uridine-3'-O-(cyanoethyl-N,N-diisopropyl)phosphoramidite.

### Example 2

### Oligonucleotide and oligonucleoside synthesis

The antisense compounds used in accordance with this invention may be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives.

Oligonucleotides: Unsubstituted and substituted phosphodiester (P=O) oligonucleotides are synthesized on an automated DNA synthesizer (Applied Biosystems model 394) using standard phosphoramidite chemistry with oxidation by iodine.

Phosphorothioates (P=S) are synthesized similar to phosphodiester oligonucleotides with the following exceptions: thiation was effected by utilizing a 10% w/v solution of 3,H-1,2-benzodithiole-3-one 1,1-dioxide in acetonitrile for the oxidation of the phosphite linkages. The thiation reaction step time was increased to 180 sec and preceded by the normal capping step. After cleavage from the CPG column and deblocking in concentrated ammonium hydroxide at 55°C (12-16 hr), the oligonucleotides were recovered by precipitating with >3 volumes of ethanol from a 1 M NH₄OAc solution. Phosphinate oligonucleotides are prepared as described in U.S. Patent 5,508,270, herein incorporated by reference. Alkyl phosphonate oligonucleotides are prepared as described in U.S. Patent 4,469,863, herein incorporated by reference.

3'-Deoxy-3'-methylene phosphonate oligonucleotides are prepared as described in U.S. Patents 5,610,289 or 5,625,050, herein incorporated by reference.

Phosphoramidite oligonucleotides are prepared as described in U.S. Patent, 5,256,775 or U.S.

Patent 5,366,878, herein incorporated by reference.

Alkylphosphonothioate oligonucleotides are prepared as described in published PCT applications PCT/US94/00902 and PCT/US93/06976 (published as WO 94/17093 and WO 94/02499, respectively), herein incorporated by reference.

3'-Deoxy-3'-amino phosphoramidate oligonucleotides are prepared as described in U.S. Patent 5,476,925, herein incorporated by reference.

Phosphotriester oligonucleotides are prepared as described in U.S. Patent 5,023,243, herein incorporated by reference.

Borano phosphate oligonucleotides are prepared as described in U.S. Patents 5,130,302 and 5,177,198, both herein incorporated by reference.

Oligonucleosides: Methylenemethylimino linked oligonucleosides, also identified as MMI linked oligonucleosides, methylenedimethylhydrazo linked oligonucleosides, also identified as MDH linked oligonucleosides, and methylenecarbonylamino linked oligonucleosides, also identified as amide-3 linked oligonucleosides, and methyleneaminocarbonyl linked oligonucleosides, also identified as amide-4 linked oligonucleosides, as well as mixed backbone compounds having, for instance, alternating MMI and P=O or P=S linkages are prepared as described in U.S. Patents 5,378,825, 5,386,023, 5,489,677, 5,602,240 and 5,610,289, all of which are herein incorporated by reference.

Formacetal and thioformacetal linked oligonucleosides are prepared as described in U.S. Patents 5,264,562 and 5,264,564, herein incorporated by reference.

Ethylene oxide linked oligonucleosides are prepared as described in U.S. Patent 5,223,618, herein incorporated by reference.

### Example 3

### RNA Synthesis

In general, RNA synthesis chemistry is based on the selective incorporation of various protecting groups at strategic intermediary reactions. Although one of ordinary skill in the art will understand the use of protecting groups in organic synthesis, a useful class of protecting groups includes silyl ethers. In particular bulky silyl ethers are used to protect the 5'-hydroxyl in combination with an acid-labile orthoester protecting group on the 2'-hydroxyl. This set of protecting groups is then used with standard solid-phase synthesis technology. It is important to lastly remove the acid labile orthoester protecting group after all other synthetic steps. Moreover, the early use of the silyl protecting groups during synthesis ensures facile removal when desired, without undesired deprotection of 2' hydroxyl.

Following this procedure for the sequential protection of the 5'-hydroxyl in combination with protection of the 2'-hydroxyl by protecting groups that are differentially removed and are differentially chemically labile, RNA oligonucleotides were synthesized.

RNA oligonucleotides are synthesized in a stepwise fashion. Each nucleotide is added sequentially (3'- to 5'-direction) to a solid support-bound oligonucleotide. The first nucleoside at the 3'-end of the chain is covalently attached to a solid support. The nucleotide precursor, a ribonucleoside phosphoramidite, and activator are added, coupling the second base onto the 5'-end of the first nucleoside. The support is washed and any unreacted 5'-hydroxyl groups are capped with acetic anhydride to yield 5'-acetyl moieties. The linkage is then oxidized to the more stable and ultimately desired P(V) linkage. At the end of the nucleotide addition cycle, the 5'-silyl group is cleaved with fluoride. The cycle is repeated for each subsequent nucleotide.

Following synthesis, the methyl protecting groups on the phosphates are cleaved in 30 minutes utilizing 1 M disodium-2-carbamoyl-2-cyanoethylene-1,1-dithiolate trihydrate (S₂Na₂) in DMF. The deprotection solution is washed from the solid support-bound oligonucleotide using water. The support is then treated with 40% methylamine in water for 10 minutes at 55 °C. This releases the RNA oligonucleotides into solution, deprotects the exocyclic amines, and modifies the 2'- groups. The oligonucleotides can be analyzed by anion exchange HPLC at this stage.

The 2'-orthoester groups are the last protecting groups to be removed. The ethylene glycol monoacetate orthoester protecting group developed by Dharmacon Research, Inc. (Lafayette, CO), is one example of a useful orthoester protecting group which, has the following important properties. It is stable to the conditions of nucleoside phosphoramidite synthesis and oligonucleotide synthesis. However, after oligonucleotide synthesis the oligonucleotide is treated with methylamine which not only cleaves the oligonucleotide from the solid support but also removes the acetyl groups from the orthoesters. The resulting 2-ethyl-hydroxyl substituents on the orthoester are less electron withdrawing than the acetylated precursor. As a result, the modified orthoester becomes more labile to acid-catalyzed hydrolysis. Specifically, the rate of cleavage is approximately 10 times faster after the acetyl groups are removed. Therefore, this orthoester possesses sufficient stability in order to be compatible with oligonucleotide synthesis and yet, when subsequently modified, permits deprotection to be carried out under relatively mild aqueous conditions compatible with the final RNA oligonucleotide product.

Additionally, methods of RNA synthesis are well known in the art (Scaringe, S. A. Ph.D. Thesis, University of Colorado, 1996; Scaringe, S. A., et al., J. Am. Chem. Soc., 1998, 120, 11820-11821; Matteucci, M. D. and Caruthers, M. H. J. Am. Chem. Soc., 1981,103, 3185-3191; Beaucage, S. L. and Caruthers, M. H. Tetrahedron Lett., 1981, 22, 1859-1862; Dahl, B. J., et al., Acta Chem. Scand,. 1990, 44, 639-641; Reddy, M. P., et al., Tetrahedron Lett., 1994, 25, 4311-4314; Wincott, F. et al., Nucleic Acids Res., 1995, 23, 2677-2684; Griffin, B. E., et al., Tetrahedron, 1967, 23, 2301-2313; Griffin, B. E., et al., Tetrahedron, 1967, 23, 2315-2331).

RNA antisense compounds (RNA oligonucleotides) of the present invention can be synthesized by the methods herein or purchased from Dharmacon Research, Inc (Lafayette, CO). Once synthesized, complementary RNA antisense compounds can then be annealed by methods known in the art to form double stranded (duplexed) antisense compounds. For example, duplexes can be formed by combining 30 µl of each of the complementary strands of RNA oligonucleotides (50 uM RNA oligonucleotide solution) and 15 µl of 5X annealing buffer (100 mM potassium acetate, 30 mM HEPES-KOH pH 7.4, 2 mM magnesium acetate) followed by heating for 1 minute at 90°C, then 1 hour at 37°C. The resulting duplexed antisense compounds can be used in kits, assays, screens, or other methods to investigate the role of a target nucleic acid, or for diagnostic or therapeutic purposes.

### Example 4

### Synthesis of Chimeric Compounds

Chimeric oligonucleotides, oligonucleosides or mixed oligonucleotides/oligonucleosides of the invention can be of several different types. These include a first type wherein the "gap" segment of linked nucleosides is positioned between 5' and 3' "wing" segments of linked nucleosides and a second "open end" type wherein the "gap" segment is located at either the 3' or the 5' terminus of the oligomeric compound. Oligonucleotides of the first type are also known in the art as "gapmers" or gapped oligonucleotides. Oligonucleotides of the second type are also known in the art as "hemimers" or "wingmers".

### [2'-O-Me]--[2'-deoxy]--[2'-O-Me] Chimeric Phosphorothioate Oligonucleotides

Chimeric oligonucleotides having 2'-O-alkyl phosphorothioate and 2'-deoxy phosphorothioate oligonucleotide segments are synthesized using an Applied Biosystems automated DNA synthesizer Model 394, as above. Oligonucleotides are synthesized using the automated synthesizer and 2'-deoxy-5'-dimethoxytrityl-3'-O-phosphoramidite for the DNA portion and 5'-dimethoxytrityl-2'-O-methyl-3'-O-phosphoramidite for 5' and 3' wings. The standard synthesis cycle is modified by incorporating coupling steps with increased reaction times for the 5'-dimethoxytrityl-2'-O-methyl-3'-O-phosphoramidite. The fully protected oligonucleotide is cleaved from the support and deprotected in concentrated ammonia (NH₄OH) for 12-16 hr at 55°C. The deprotected oligo is then recovered by an appropriate method (precipitation, column chromatography, volume reduced *in vacuo* and analyzed spetrophotometrically for yield and for purity by capillary electrophoresis and by mass spectrometry.

**[2'-O-(2-Methoxyethyl)]--[2'-deoxy]--[2'-O-(Methoxyethyl)] Chimeric Phosphorothioate Oligonucleotides**

[2'-O-(2-methoxyethyl)]--[2'-deoxy]--[-2'-O-(methoxyethyl)] chimeric phosphorothioate oligonucleotides were prepared as per the procedure above for the 2'-O-methyl chimeric oligonucleotide, with the substitution of 2'-O-(methoxyethyl) amidites for the 2'-O-methyl amidites.

### [2'-O-(2-Methoxyethyl)Phosphodiester]--[2'-deoxy Phosphorothioate]--[2'-O-(2-Methoxyethyl) Phosphodiester] Chimeric Oligonucleotides

[2'-O-(2-methoxyethyl phosphodiester]--[2'-deoxy phosphorothioate]--[2'-O-(methoxyethyl) phosphodiester] chimeric oligonucleotides are prepared as per the above procedure for the 2'-O-methyl chimeric oligonucleotide with the substitution of 2'-O-(methoxyethyl) amidites for the 2'-O-methyl amidites, oxidation with iodine to generate the phosphodiester internucleotide linkages within the wing portions of the chimeric structures and sulfurization utilizing 3,H-1,2 benzodithiole-3-one 1,1 dioxide (Beaucage Reagent) to generate the phosphorothioate internucleotide linkages for the center gap.

Other chimeric oligonucleotides, chimeric oligonucleosides and mixed chimeric oligonucleotides/oligonucleosides are synthesized according to United States patent 5,623,065, herein incorporated by reference.

### Example 5

### Design and screening of duplexed antisense compounds targeting glucocorticoid receptor

In accordance with the present invention, a series of nucleic acid duplexes comprising the antisense compounds of the present invention and their complements can be designed to target glucocorticoid receptor. The nucleobase sequence of the antisense strand of the duplex comprises at least an 8-nucleobase portion of an oligonucleotide in Table 1. The ends of the strands may be modified by the addition of one or more natural or modified nucleobases to form an overhang. The sense strand of the dsRNA is then designed and synthesized as the complement of the antisense strand and may also contain modifications or additions to either terminus. For example, in one embodiment, both strands of the dsRNA duplex would be complementary over the central nucleobases, each having overhangs at one or both termini.

For example, a duplex comprising an antisense strand having the sequence CGAGAGGCGGACGGGACCG and having a two-nucleobase overhang of deoxythymidine(dT) would have the following structure:

cgagaggcggacgggaccgTT Antisense Strand
|||||||||||||||||||
TTgctctccgcctgccctggc Complement

In another embodiment, a duplex comprising an antisense strand having the same sequence CGAGAGGCGGACGGGACCG may be prepared with blunt ends (no single stranded overhang) as shown:

cgagaggcggacgggaccg          Antisense Strand
|||||||||||||||||||
gctctccgcctgccctggc          Complement

RNA strands of the duplex can be synthesized by methods disclosed herein or purchased from Dharmacon Research Inc., (Lafayette, CO). Once synthesized, the complementary strands are annealed. The single strands are aliquoted and diluted to a concentration of 50 uM. Once diluted, 30 uL of each strand is combined with 15uL of a 5X solution of annealing buffer. The final concentration of said buffer is 100 mM potassium acetate, 30 mM HEPES-KOH pH 7.4, and 2mM magnesium acetate. The final volume is 75 uL. This solution is incubated for 1 minute at 90°C and then centrifuged for 15 seconds. The tube is allowed to sit for 1 hour at 37°C at which time the dsRNA duplexes are used in experimentation. The final concentration of the dsRNA duplex is 20 uM. This solution can be stored frozen (-20°C) and freeze-thawed up to 5 times.

Once prepared, the duplexed antisense compounds are evaluated for their ability to modulate glucocorticoid receptor expression.When cells reached 80% confluency, they are treated with duplexed antisense compounds of the invention. For cells grown in 96-well plates, wells are washed once with 200 µL OPTI-MEM-1 reduced-serum medium (Gibco BRL) and then treated with 130 µL of OPTI-MEM-1 containing 12 µg/mL LIPOFECTIN (Gibco BRL) and the desired duplex antisense compound at a final concentration of 200 nM. After 5 hours of treatment, the medium is replaced with fresh medium. Cells are harvested 16 hours after treatment, at which time RNA is isolated and target reduction measured by RT-PCR.

### Example 6

### Oligonucleotide Isolation

After cleavage from the controlled pore glass solid support and deblocking in concentrated ammonium hydroxide at 55°C for 12-16 hours, the oligonucleotides or oligonucleosides are recovered by precipitation out of 1 M NH₄OAc with >3 volumes of ethanol. Synthesized oligonucleotides were analyzed by electrospray mass spectroscopy (molecular weight determination) and by capillary gel electrophoresis and judged to be at least 70% full length material. The relative amounts of phosphorothioate and phosphodiester linkages obtained in the synthesis was determined by the ratio of correct molecular weight relative to the -16 amu product (+/-32 +/-48). For some studies oligonucleotides were purified by HPLC, as described by Chiang et al., J. Biol. Chem. 1991, 266, 18162-18171. Results obtained with HPLC-purified material were similar to those obtained with non-HPLC purified material.

### Example 7

### Oligonucleotide Synthesis - 96 Well Plate Format

Oligonucleotides were synthesized via solid phase P(III) phosphoramidite chemistry on an automated synthesizer capable of assembling 96 sequences simultaneously in a 96-well format. Phosphodiester internucleotide linkages were afforded by oxidation with aqueous iodine. Phosphorothioate internucleotide linkages were generated by sulfurization utilizing 3,H-1,2 benzodithiole-3-one 1,1 dioxide (Beaucage Reagent) in anhydrous acetonitrile. Standard base-protected beta-cyanoethyl-diiso-propyl phosphoramidites were purchased from commercial vendors (e.g. PE-Applied Biosystems, Foster City, CA, or Pharmacia, Piscataway, NJ). Non-standard nucleosides are synthesized as per standard or patented methods. They are utilized as base protected beta-cyanoethyldiisopropyl phosphoramidites.

Oligonucleotides were cleaved from support and deprotected with concentrated NH₄OH at elevated temperature (55-60°C) for 12-16 hours and the released product then dried *in vacuo.* The dried product was then re-suspended in sterile water to afford a master plate from which all analytical and test plate samples are then diluted utilizing robotic pipettors.

### Example 8

### Oligonucleotide Analysis - 96-Well Plate Format

The concentration of oligonucleotide in each well was assessed by dilution of samples and UV absorption spectroscopy. The full-length integrity of the individual products was evaluated by capillary electrophoresis (CE) in either the 96-well format (Beckman P/ACE™ MDQ) or, for individually prepared samples, on a commercial CE apparatus (e.g., Beckman P/ACE™ 5000, ABI 270). Base and backbone composition was confirmed by mass analysis of the compounds utilizing electrospray-mass spectroscopy. All assay test plates were diluted from the master plate using single and multi-channel robotic pipettors. Plates were judged to be acceptable if at least 85% of the compounds on the plate were at least 85% full length.

### Example 9

### Cell culture and oligonucleotide treatment

The effect of antisense compounds on target nucleic acid expression can be tested in any of a variety of cell types provided that the target nucleic acid is present at measurable levels. This can be routinely determined using, for example, PCR or Northern blot analysis. The following cell types are provided for illustrative purposes, but other cell types can be routinely used, provided that the target is expressed in the cell type chosen. This can be readily determined by methods routine in the art, for example Northern blot analysis, ribonuclease protection assays, or RT-PCR.

T-24 cells:
The human transitional cell bladder carcinoma cell line T-24 was obtained from the American Type Culture Collection (ATCC) (Manassas, VA). T-24 cells were routinely cultured in complete McCoy's 5A basal media (Invitrogen Corporation, Carlsbad, CA) supplemented with 10% fetal calf serum (Invitrogen Corporation, Carlsbad, CA), penicillin 100 units per mL, and streptomycin 100 micrograms per mL (Invitrogen Corporation, Carlsbad, CA). Cells were routinely passaged by trypsinization and dilution when they reached 90% confluence. Cells were seeded into 96-well plates (Falcon-Primaria #353872) at a density of 7000 cells/well for use in RT-PCR analysis.

For Northern blotting or other analysis, cells may be seeded onto 100 mm or other standard tissue culture plates and treated similarly, using appropriate volumes of medium and oligonucleotide.

A549 cells:
The human lung carcinoma cell line A549 was obtained from the American Type Culture Collection (ATCC) (Manassas, VA). A549 cells were routinely cultured in DMEM basal media (Invitrogen Corporation, Carlsbad, CA) supplemented with 10% fetal calf serum (Invitrogen Corporation, Carlsbad, CA), penicillin 100 units per mL, and streptomycin 100 micrograms per mL (Invitrogen Corporation, Carlsbad, CA). Cells were routinely passaged by trypsinization and dilution when they reached 90% confluence.

NHDF cells:
Human neonatal dermal fibroblast (NHDF) were obtained from the Clonetics Corporation (Walkersville, MD). NHDFs were routinely maintained in Fibroblast Growth Medium (Clonetics Corporation, Walkersville, MD) supplemented as recommended by the supplier. Cells were maintained for up to 10 passages as recommended by the supplier.

HEK cells:
Human embryonic keratinocytes (HEK) were obtained from the Clonetics Corporation (Walkersville, MD). HEKs were routinely maintained in Keratinocyte Growth Medium (Clonetics Corporation, Walkersville, MD) formulated as recommended by the supplier. Cells were routinely maintained for up to 10 passages as recommended by the supplier.

HepG2 cells:
The human hepatoblastoma cell line HepG2 was obtained from the American Type Culture Collection (Manassas, VA). HepG2 cells were routinely cultured in Eagle's MEM supplemented with 10% fetal calf serum, non-essential amino acids, and 1 mM sodium pyruvate (Gibco/Life Technologies, Gaithersburg, MD). Cells were routinely passaged by trypsinization and dilution when they reached 90% confluence. Cells were seeded into 96-well plates (Falcon-Primaria #3872) at a density of 7000 cells/well for use in RT-PCR analysis.

For Northern blotting or other analyses, cells may be seeded onto 100 mm or other standard tissue culture plates and treated similarly, using appropriate volumes of medium and oligonucleotide. b.END cells:
The mouse brain endothelial cell line b.END was obtained from Dr. Werner Risau at the Max Plank Instititute (Bad Nauheim, Germany). b.END cells were routinely cultured in DMEM, high glucose (Gibco/Life Technologies, Gaithersburg, MD) supplemented with 10% fetal calf serum (Gibco/Life Technologies, Gaithersburg, MD). Cells were routinely passaged by trypsinization and dilution when they reached 90% confluence. Cells were seeded into 96-well plates (Falcon-Primaria #3872) at a density of 3000 cells/well for use in RT-PCR analysis.

For Northern blotting or other analyses, cells may be seeded onto 100 mm or other standard tissue culture plates and treated similarly, using appropriate volumes of medium and oligonucleotide. NRK cells:
Normal rat kidney (NRK) cells were obtained from American Type Culture Collection (Manassus, VA). They were grown in serial monolayer culture in Minimum Essential Media (Invitrogen Life Technologies, Carlsbad, CA) supplemented with 10% fetal bovine serum, (Invitrogen Life Technologies, Carlsbad, CA), 100 ug/ml penicillin and 100 ug/ml streptomycin and 0.1 mM non-essential amino acids (all supplements from Invitrogen Life Technologies, Carlsbad, CA) in a humidified atmosphere of 90% air-10% CO² at 37°C. Cells were routinely passaged by trypsinization and dilution when they reached 85-90% confluencey. Cells were seeded into 96-well plates (Falcon-Primaria #353872, BD Biosciences, Bedford, MA) at a density of 6000 cells/well for use in antisense oligonucleotide transfection.

Primary mouse hepatocytes:
Primary mouse hepatocytes are prepared from CD-1 mice purchased from Charles River Labs. Primary mouse hepatocytes are routinely cultured in Hepatocyte Attachment Media (Invitrogen Life Technologies, Carlsbad, CA) supplemented with 10% Fetal Bovine Serum (Invitrogen Life Technologies, Carlsbad, CA), 250 nM dexamethasone (Sigma-Aldrich Corporation, St. Louis, MO), 10 nM bovine insulin (Sigma-Aldrich Corporation, St. Louis, MO). Cells are seeded into 96-well plates (Falcon-Primaria #353872, BD Biosciences, Bedford, MA) at a density of 4000-6000 cells/well for treatment with the oligomeric compounds of the invention.

Treatment with antisense compounds:
When cells reached 65-75% confluency, they were treated with oligonucleotide. For cells grown in 96-well plates, wells were washed once with 100 µL OPTI-MEM™-1 reduced-serum medium (Invitrogen Corporation, Carlsbad, CA) and then treated with 130 µL of OPTI-MEM™-1 containing 3.75 µg/mL LIPOFECTIN™ (Invitrogen Corporation, Carlsbad, CA) and the desired concentration of oligonucleotide. Cells are treated and data are obtained in triplicate. After 4-7 hours of treatment at 37°C, the medium was replaced with fresh medium. Cells were harvested 16-24 hours after oligonucleotide treatment.

The concentration of oligonucleotide used varies from cell line to cell line. To determine the optimal oligonucleotide concentration for a particular cell line, the cells are treated with a positive control oligonucleotide at a range of concentrations. For human cells the positive control oligonucleotide is selected from either ISIS 13920 (**TCC**GTCATCGCT**CCTCAGGG**, SEQ ID NO: 1) which is targeted to human H-ras, or ISIS 18078, (**GTG**CGCGCGAGCCCG**AAATC**, SEQ ID NO: 2) which is targeted to human Jun-N-terminal kinase-2 (JNK2). Both controls are 2'-O-methoxyethyl gapmers (2'-O-methoxyethyls shown in bold) with a phosphorothioate backbone. For mouse or rat cells the positive control oligonucleotide is ISIS 15770, **ATGCA**TTCTGCCCCC**AAGGA**, SEQ ID NO: 3, a 2'-O-methoxyethyl gapmer (2'-O-methoxyethyls shown in bold) with a phosphorothioate backbone which is targeted to both mouse and rat c-raf. The concentration of positive control oligonucleotide that results in 80% inhibition of c-H-ras (for ISIS 13920), JNK2 (for ISIS 18078) or c-raf (for ISIS 15770) mRNA is then utilized as the screening concentration for new oligonucleotides in subsequent experiments for that cell line. If 80% inhibition is not achieved, the lowest concentration of positive control oligonucleotide that results in 60% inhibition of c-H-ras, JNK2 or c-rafmRNA is then utilized as the oligonucleotide screening concentration in subsequent experiments for that cell line. If 60% inhibition is not achieved, that particular cell line is deemed as unsuitable for oligonucleotide transfection experiments. The concentrations of antisense oligonucleotides used herein are from 50 nM to 300 nM.

### Example 10

### Analysis of oligonucleotide inhibition of glucocorticoid receptor expression

Antisense modulation of glucocorticoid receptor expression can be assayed in a variety of ways known in the art. For example, glucocorticoid receptor mRNA levels can be quantitated by, e.g., Northern blot analysis, competitive polymerase chain reaction (PCR), or real-time PCR (RT-PCR). Real-time quantitative PCR is presently preferred. RNA analysis can be performed on total cellular RNA or poly(A)+ mRNA. The preferred method of RNA analysis of the present invention is the use of total cellular RNA as described in other examples herein. Methods of RNA isolation are well known in the art. Northern blot analysis is also routine in the art. Real-time quantitative (PCR) can be conveniently accomplished using the commercially available ABI PRISM™ 7600, 7700, or 7900 Sequence Detection System, available from PE-Applied Biosystems, Foster City, CA and used according to manufacturer's instructions.

Protein levels of glucocorticoid receptor can be quantitated in a variety of ways well known in the art, such as immunoprecipitation, Western blot analysis (immunoblotting), enzyme-linked immunosorbent assay (ELISA) or fluorescence-activated cell sorting (FACS). Antibodies directed to glucocorticoid receptor can be identified and obtained from a variety of sources, such as the MSRS catalog of antibodies (Aerie Corporation, Birmingham, MI), or can be prepared via conventional monoclonal or polyclonal antibody generation methods well known in the art.

### Example 11

### Design of phenotypic assays for the use of glucocorticoid receptor inhibitors

### Phenotypic assays

Once glucocorticoid receptor inhibitors have been identified by the methods disclosed herein, the compounds are further investigated in one or more phenotypic assays, each having measurable endpoints predictive of efficacy in the treatment of a particular disease state or condition.

Phenotypic assays, kits and reagents for their use are well known to those skilled in the art and are herein used to investigate the role and/or association of glucocorticoid receptor in health and disease. Representative phenotypic assays, which can be purchased from any one of several commercial vendors, include those for determining cell viability, cytotoxicity, proliferation or cell survival (Molecular Probes, Eugene, OR; PerkinElmer, Boston, MA), protein-based assays including enzymatic assays (Panvera, LLC, Madison, WI; BD Biosciences, Franklin Lakes, NJ; Oncogene Research Products, San Diego, CA), cell regulation, signal transduction, inflammation, oxidative processes and apoptosis (Assay Designs Inc., Ann Arbor, MI), triglyceride accumulation (Sigma-Aldrich, St. Louis, MO), angiogenesis assays, tube formation assays, cytokine and hormone assays and metabolic assays (Chemicon International Inc., Temecula, CA; Amersham Biosciences, Piscataway, NJ).

In one non-limiting example, cells determined to be appropriate for a particular phenotypic assay (i.e., MCF-7 cells selected for breast cancer studies; adipocytes for obesity studies) are treated with glucocorticoid receptor inhibitors identified from the *in vitro* studies as well as control compounds at optimal concentrations which are determined by the methods described above. At the end of the treatment period, treated and untreated cells are analyzed by one or more methods specific for the assay to determine phenotypic outcomes and endpoints.

Phenotypic endpoints include changes in cell morphology over time or treatment dose as well as changes in levels of cellular components such as proteins, lipids, nucleic acids, hormones, saccharides or metals. Measurements of cellular status which include pH, stage of the cell cycle, intake or excretion of biological indicators by the cell, are also endpoints of interest.

Analysis of the genotype of the cell (measurement of the expression of one or more of the genes of the cell) after treatment is also used as an indicator of the efficacy or potency of the glucocorticoid receptor inhibitors. Hallmark genes, or those genes suspected to be associated with a specific disease state, condition, or phenotype, are measured in both treated and untreated cells.

### Example 12

### RNA Isolation

### Poly(A)+ mRNA isolation

Poly(A)+ mRNA was isolated according to Miura et al., (Clin. Chem., 1996, 42, 1758-1764). Other methods for poly(A)+ mRNA isolation are routine in the art. Briefly, for cells grown on 96-well plates, growth medium was removed from the cells and each well was washed with 200 µL cold PBS. 60 µL lysis buffer (10 mM Tris-HCl, pH 7.6, 1 mM EDTA, 0.5 M NaCl, 0.5% NP-40, 20 mM vanadyl-ribonucleoside complex) was added to each well, the plate was gently agitated and then incubated at room temperature for five minutes. 55 µL of lysate was transferred to Oligo d(T) coated 96-well plates (AGCT Inc., Irvine CA). Plates were incubated for 60 minutes at room temperature, washed 3 times with 200 µL of wash buffer (10 mM Tris-HCl pH 7.6, 1 mM EDTA, 0.3 M NaCl). After the final wash, the plate was blotted on paper towels to remove excess wash buffer and then air-dried for 5 minutes. 60 µL of elution buffer (5 mM Tris-HCl pH 7.6), preheated to 70°C, was added to each well, the plate was incubated on a 90°C hot plate for 5 minutes, and the eluate was then transferred to a fresh 96-well plate.

Cells grown on 100 mm or other standard plates may be treated similarly, using appropriate volumes of all solutions.

### Total RNA Isolation

Total RNA was isolated using an RNEASY 96™ kit and buffers purchased from Qiagen Inc. (Valencia, CA) following the manufacturer's recommended procedures. Briefly, for cells grown on 96-well plates, growth medium was removed from the cells and each well was washed with 200 µL cold PBS. 150 µL Buffer RLT was added to each well and the plate vigorously agitated for 20 seconds. 150 µL of 70% ethanol was then added to each well and the contents mixed by pipetting three times up and down. The samples were then transferred to the RNEASY 96™ well plate attached to a QIAVAC™ manifold fitted with a waste collection tray and attached to a vacuum source. Vacuum was applied for 1 minute. 500 µL of Buffer RW1 was added to each well of the RNEASY 96™ plate and incubated for 15 minutes and the vacuum was again applied for 1 minute. An additional 500 µL of Buffer RW1 was added to each well of the RNEASY 96™ plate and the vacuum was applied for 2 minutes. 1 mL of Buffer RPE was then added to each well of the RNEASY 96™ plate and the vacuum applied for a period of 90 seconds. The Buffer RPE wash was then repeated and the vacuum was applied for an additional 3 minutes. The plate was then removed from the QIAVAC™ manifold and blotted dry on paper towels. The plate was then re-attached to the QIAVAC™ manifold fitted with a collection tube rack containing 1.2 mL collection tubes. RNA was,then eluted by pipetting 140 µL of RNAse free water into each well, incubating 1 minute, and then applying the vacuum for 3 minutes.

The repetitive pipetting and elution steps may be automated using a QIAGEN Bio-Robot 9604 (Qiagen, Inc., Valencia CA). Essentially, after lysing of the cells on the culture plate, the plate is transferred to the robot deck where the pipetting, DNase treatment and elution steps are carried out.

### Example 13

### Real-time Quantitative PCR Analysis of glucocorticoid receptor mRNA Levels

Quantitation of glucocorticoid receptor mRNA levels was accomplished by real-time quantitative PCR using the ABI PRISM™ 7600, 7700, or 7900 Sequence Detection System (PE-Applied Biosystems, Foster City, CA) according to manufacturer's instructions. This is a closed-tube, non-gel-based, fluorescence detection system which allows high-throughput quantitation of polymerase chain reaction (PCR) products in real-time. As opposed to standard PCR in which amplification products are quantitated after the PCR is completed, products in real-time quantitative PCR are quantitated as they accumulate. This is accomplished by including in the PCR reaction an oligonucleotide probe that anneals specifically between the forward and reverse PCR primers, and contains two fluorescent dyes. A reporter dye (e.g., FAM or JOE, obtained from either PE-Applied Biosystems, Foster City, CA, Operon Technologies Inc., Alameda, CA or Integrated DNA Technologies Inc., Coralville, IA) is attached to the 5' end of the probe and a quencher dye (e.g., TAMRA, obtained from either PE-Applied Biosystems, Foster City, CA, Operon Technologies Inc., Alameda, CA or Integrated DNA Technologies Inc., Coralville, IA) is attached to the 3' end of the probe. When the probe and dyes are intact, reporter dye emission is quenched by the proximity of the 3' quencher dye. During amplification, annealing of the probe to the target sequence creates a substrate that can be cleaved by the 5'-exonuclease activity of Taq polymerase. During the extension phase of the PCR amplification cycle, cleavage of the probe by Taq polymerase releases the reporter dye from the remainder of the probe (and hence from the quencher moiety) and a sequence-specific fluorescent signal is generated. With each cycle, additional reporter dye molecules are cleaved from their respective probes, and the fluorescence intensity is monitored at regular intervals by laser optics built into the ABI PRISM™ Sequence Detection System. In each assay, a series of parallel reactions containing serial dilutions of mRNA from untreated control samples generates a standard curve that is used to quantitate the percent inhibition after antisense oligonucleotide treatment of test samples.

Prior to quantitative PCR analysis, primer-probe sets specific to the target gene being measured are evaluated for their ability to be "multiplexed" with a GAPDH amplification reaction. In multiplexing, both the target gene and the internal standard gene GAPDH are amplified concurrently in a single sample. In this analysis, mRNA isolated from untreated cells is serially diluted. Each dilution is amplified in the presence of primer-probe sets specific for GAPDH only, target gene only ("single-plexing"), or both (multiplexing). Following PCR amplification, standard curves of GAPDH and target mRNA signal as a function of dilution are generated from both the single-plexed and multiplexed samples. If both the slope and correlation coefficient of the GAPDH and target signals generated from the multiplexed samples fall within 10% of their corresponding values generated from the single-plexed samples, the primer-probe set specific for that target is deemed multiplexable. Other methods of PCR are also known in the art.

PCR reagents were obtained from Invitrogen Corporation, (Carlsbad, CA). RT-PCR reactions were carried out by adding 20 µL PCR cocktail (2.5x PCR buffer minus MgCl₂, 6.6 mM MgCl₂, 375 µM each of dATP, dCTP, dCTP and dGTP, 375 nM each of forward primer and reverse primer, 125 nM of probe, 4 Units RNAse inhibitor, 1.25 Units PLATINUM® Taq, 5 Units MuLV reverse transcriptase, and 2.5x ROX dye) to 96-well plates containing 30 µL total RNA solution (20-200 ng). The RT reaction was carried out by incubation for 30 minutes at 48°C. Following a 10 minute incubation at 95°C to activate the PLATINUM® Taq, 40 cycles of a two-step PCR protocol were carried out: 95°C for 15 seconds (denaturation) followed by 60°C for 1.5 minutes (annealing/extension).

Gene target quantities obtained by real time RT-PCR are normalized using either the expression level of GAPDH, a gene whose expression is constant, or by quantifying total RNA using RiboGreen^{™} (Molecular Probes, Inc. Eugene, OR). GAPDH expression is quantified by real time RT-PCR, by being run simultaneously with the target, multiplexing, or separately. Total RNA is quantified using RiboGreen^{™} RNA quantification reagent (Molecular Probes, Inc. Eugene, OR). Methods of RNA quantification by RiboGreen^{™} are taught in Jones, L.J., et al, (Analytical Biochemistry, 1998, 265, 368-374).

In this assay, 170 µL of RiboGreen™ working reagent (RiboGreen™ reagent diluted 1:350 in 10mM Tris-HCl, 1 mM EDTA, pH 7.5) is pipetted into a 96-well plate containing 30 µL purified, cellular RNA. The plate is read in a CytoFluor 4000 (PE Applied Biosystems) with excitation at 485nm and emission at 530nm.

Probes and primers to human glucocorticoid receptor were designed to hybridize to a human glucocorticoid receptor sequence, using published sequence information (GenBank accession number NM_000176.1, incorporated herein as SEQ ID NO: 4). For human glucocorticoid receptor the PCR primers were:
forward primer: AGGTTGTGCAAATTAACAGTCCTAACT (SEQ ID NO: 5)
reverse primer: TAGTCTTTTGCAACCATCATCCA (SEQ ID NO: 6) and the PCR probe was: FAM-AGCACCTAGTCCAGTGACCTGCTGGGTAAA-TAMRA (SEQ ID NO: 7) where FAM is the fluorescent dye and TAMRA is the quencher dye. For human GAPDH the PCR primers were:
forward primer: GAAGGTGAAGGTCGGAGTC(SEQ ID NO: 8)
reverse primer: GAAGATGGTGATGGGATTTC (SEQ ID NO: 9) and the PCR probe was: 5' JOE-CAAGCTTCCCGTTCTCAGCC- TAMRA 3' (SEQ ID NO: 10) where JOE is the fluorescent reporter dye and TAMRA is the quencher dye.

Probes and primers to mouse glucocorticoid receptor were designed to hybridize to a mouse glucocorticoid receptor sequence, using published sequence information (GenBank accession number NM_008173.1, incorporated herein as SEQ ID NO: 11). For mouse glucocorticoid receptor the PCR primers were:
forward primer: GACATCTTGCAGGATTTGGAGTT (SEQ ID NO: 12)
reverse primer: AACAGGTCTGACCTCCAAGGACT (SEQ ID NO: 13) and the PCR probe was: FAM-CGGGTCCCCAGGTAAAGAGACAAACGA-TAMRA
(SEQ ID NO: 14) where FAM is the fluorescent reporter dye and TAMRA is the quencher dye. For mouse GAPDH the PCR primers were:
   forward primer: GGCAAATTCAACGGCACAGT(SEQ ID NO: 15)
reverse primer: GGGTCTCGCTCCTGGAAGAT(SEQ ID NO: 16) and the PCR probe was: 5' JOE-AAGGCCGAGAATGGGAAGCTTGTCATC- TAMRA 3' (SEQ ID NO: 17) where JOE is the fluorescent reporter dye and TAMRA is the quencher dye.

Probes and primers to rat glucocorticoid receptor were designed to hybridize to a rat glucocorticoid receptor sequence, using published sequence information (GenBank accession number NM_012576.1, incorporated herein as SEQ ID NO: 18). For rat glucocorticoid receptor the PCR primers were:
forward primer: AAACAATAGTTCCTGCAGCATTACC (SEQ ID NO: 19) reverse primer: CATACAACACCTCGGGTTCAATC (SEQ ID NO: 20) and the PCR probe was: FAM-ACCCCTACCTTGGTGTCACTGCT-TAMRA

(SEQ ID NO: 21) where FAM is the fluorescent reporter dye and TAMRA is the quencher dye. For rat GAPDH the PCR primers were:
forward primer: TGTTCTAGAGACAGCCGCATCTT(SEQ ID NO: 22) reverse primer: CACCGACCTTCACCATCTTGT(SEQ ID NO: 23) and the PCR probe was: 5' JOE-TTGTGCAGTGCCAGCCTCGTCTCA- TAMRA 3' (SEQ ID NO: 24) where JOE is the fluorescent reporter dye and TAMRA is the quencher dye.

### Example 14

### Northern blot analysis of glucocorticoid receptor mRNA levels

Eighteen hours after antisense treatment, cell monolayers were washed twice with cold PBS and lysed in 1 mL RNAZOL™ (TEL-TEST "B" Inc., Friendswood, TX). Total RNA was prepared following manufacturer's recommended protocols. Twenty micrograms of total RNA was fractionated by electrophoresis through 1.2% agarose gels containing 1.1% formaldehyde using a MOPS buffer system (AMRESCO, Inc. Solon, OH). RNA was transferred from the gel to HYBOND™-N+ nylon membranes (Amersham Pharmacia Biotech, Piscataway, NJ) by overnight capillary transfer using a Northern/Southern Transfer buffer system (TEL-TEST "B" Inc., Friendswood, TX). RNA transfer was confirmed by UV visualization. Membranes were fixed by UV cross-linking using a STRATALINKER™ UV Crosslinker 2400 (Stratagene, Inc, La Jolla, CA) and then probed using QUICKHYB™ hybridization solution (Stratagene, La Jolla, CA) using manufacturer's recommendations for stringent conditions.

To detect human glucocorticoid receptor, a human glucocorticoid receptor specific probe was prepared by PCR using the forward primer AGGTTGTGCAAATTAACAGTCCTAACT (SEQ ID NO: 5) and the reverse primer TAGTCTTTTGCAACCATCATCCA (SEQ ID NO: 6). To normalize for variations in loading and transfer efficiency membranes were stripped and probed for human glyceraldehyde-3-phosphate dehydrogenase (GAPDH) RNA (Clontech, Palo Alto, CA).

To detect mouse glucocorticoid receptor, a mouse glucocorticoid receptor specific probe was prepared by PCR using the forward primer GACATCTTGCAGGATTTGGAGTT (SEQ ID NO: 12) and the reverse primer AACAGGTCTGACCTCCAAGGACT (SEQ ID NO: 13). To normalize for variations in loading and transfer efficiency membranes were stripped and probed for mouse glyceraldehyde-3-phosphate dehydrogenase (GAPDH) RNA (Clontech, Palo Alto, CA).

To detect rat glucocorticoid receptor, a rat glucocorticoid receptor specific probe was prepared by PCR using the forward primer AAACAATAGTTCCTGCAGCATTACC (SEQ ID NO: 19) and the reverse primer CATACAACACCTCGGGTTCAATC (SEQ ID NO: 20). To normalize for variations in loading and transfer efficiency membranes were stripped and probed for rat glyceraldehyde-3-phosphate dehydrogenase (GAPDH) RNA (Clontech, Palo Alto, CA).

Hybridized membranes were visualized and quantitated using a PHOSPHORIMAGER™ and IMAGEQUANT™ Software V3.3 (Molecular Dynamics, Sunnyvale, CA). Data was normalized to GAPDH levels in untreated controls.

### Example 15

### Antisense inhibition of human glucocorticoid receptor expression by chimeric phosphorothioate oligonucleotides having 2'-MOE wings and a deoxy gap

In accordance with the present invention, a series of antisense compounds was designed to target different regions of the human glucocorticoid receptor RNA, using published sequences (GenBank accession number NM_000176.1, incorporated herein as SEQ ID NO: 4, nucleotides 1 to 106000 of the sequence with GenBank accession number AC012634, incorporated herein as SEQ ID NO: 25, GenBank accession number X03348.1, incorporated herein as SEQ ID NO: 26 and GenBank accession number U01351.1, incorporated herein as SEQ ID NO: 27). The compounds are shown in Tables 1 and 2. "Target site" indicates the first (5'-most) nucleotide number on the particular target sequence to which the compound binds. All compounds in Tables 1 and 2 are chimeric oligonucleotides ("gapmers") 20 nucleotides in length, composed of a central "gap" region consisting often 2'-deoxynucleotides, which is flanked on both sides (5' and 3' directions) by five-nucleotide "wings". The wings are composed of 2'-methoxyethyl (2'-MOE)nucleotides. The internucleoside (backbone) linkages are phosphorothioate (P=S) throughout the oligonucleotide. All cytosine residues are 5-methylcytosines.

The compounds in Table 1 were analyzed for their effect on human glucocorticoid receptor mRNA levels in T-24 cells by quantitative real-time PCR as described in other examples herein. Data, shown in Table 1, are averages from two experiments in which T-24 cells were treated with 100 nM of the antisense oligonucleotides of the present invention. The positive control for each datapoint is identified in the table by sequence ID number. If present, "N.D." indicates "no data".

**Table 1**

| **Inhibition of human glucocorticoid receptor mRNA levels by chimeric phosphorothioate oligonucleotides having 2'-MOE wings and a deoxy gap** | | | | | | | |
|---|---|---|---|---|---|---|---|
| **ISIS #** | **REGION** | **TARGET SEQ ID NO** | **TARGET SITE** | **SEQUENCE** | **% INHIB** | **SEQ ID NO** | **CONTROL SEQ ID NO** |
| 153080 | Coding | 4 | 2197 | ttgatgtaggtcattctaat | 28 | 30 | 2 |
| 153081 | 3'UTR | 4 | 3875 | tggcttagtaaatatgttaa | 21 | 31 | 2 |
| 153082 | 3'UTR | 4 | 4031 | cttcccttcccagattagtg | 39 | 32 | 2 |
| 153083 | 3'UTR | 4 | 3336 | aaccatcatccacagtttac | 57 | 33 | 2 |
| 153084 | 3'UTR | 4 | 3838 | agttggtaaggtgcacacag | 54 | 34 | 2 |
| 153085 | Coding | 4 | 1865 | gaaacctggtattgcctttg | 24 | 35 | 2 |
| 153086 | 3'UTR | 4 | 2965 | accagacagtaatagctata | 78 | 36 | 2 |
| 153087 | 5'UTR | 4 | 35 | tagcttgtgaacgcagaagg | 61 | 37 | 2 |
| 153088 | Coding | 4 | 851 | cttgcagtcctcattcgagt | 31 | 38 | 2 |
| 153089 | 3'UTR | 4 | 4289 | ttcactgcacacaggaccag | 58 | 39 | 2 |
| 153090 | 5'UTR | 4 | 38 | acttagcttgtgaacgcaga | 68 | 40 | 2 |
| 153091 | Coding | 4 | 2286 | tagaatccaagagttttgtc | 19 | 41 | 2 |
| 153092 | 3'UTR | 4 | 4020 | agattagtgaataccaatat | 32 | 42 | 2 |
| 153093 | Coding | 4 | 1822 | tgccgccctcctaacatgtt | 66 | 43 | 2 |
| 153094 | Coding | 4 | 275 | tgattgagaagcgacagcca | 65 | 44 | 2 |
| 153095 | 3'UTR | 4 | 2828 | gaaaatttcatccagccaac | 19 | 45 | 2 |
| 153096 | 3'UTR | 4 | 3549 | gtgagaggaattactttgtc | 67 | 46 | 2 |
| 153097 | 3'UTR | 4 | 2635 | cgactcaactgcttctgttg | 7 | 47 | 2 |
| 153098 | 3'UTR | 4 | 3291 | ctataccagttaggactgtt | 90 | 48 | 2 |
| 153099 | 3'UTR | 4 | 3787 | aataattttcaacagtgaag | 19 | 49 | 2 |
| 153100 | Coding | 4 | 1662 | taccaggattttcagaggtt | 76 | 50 | 2 |
| 153101 | 3'UTR | 4 | 3826 | gcacacagaaagggctacta | 66 | 51 | 2 |
| 153102 | Coding | 4 | 1946 | tctccaccccagagcaaatg | 44 | 52 | 2 |
| 153103 | Coding | 4 | 1675 | actattgttttgttaccagg | 66 | 53 | 2 |
| 153104 | Coding | 4 | 2018 | agtcattctctgctcattaa | 51 | 54 | 2 |
| 153105 | Coding | 4 | 2338 | tccaaaaatgtttggaagca | 48 | 55 | 2 |
| 153106 | Coding | 4 | 631 | tggcccttcaaatgttgctg | 1 | 56 | 2 |
| 153107 | 3'UTR | 4 | 2829 | agaaaatttcatccagccaa | 70 | 57 | 2 |
| 153108 | 3'UTR | 4 | 3515 | tcagctgtgttacagctggt | 46 | 58 | 2 |
| 153109 | Coding | 4 | 351 | tggacagatctggctgctgc | 73 | 59 | 2 |
| 153110 | 3'UTR | 4 | 3252 | attctccactgaagcagata | 81 | 60 | 2 |
| 153111 | 3'UTR | 4 | 4253 | cccctagagcaaactgtttg | 70 | 61 | 2 |
| 153112 | 3'UTR | 4 | 4581 | attgctggtacctctatgca | 72 | 62 | 2 |
| 153113 | Start Codon | 4 | 114 | tcagtgaatatcaactctgg | 54 | 63 | 2 |
| 153114 | 3'UTR | 4 | 2716 | cacatattaaggtttctaat | 34 | 64 | 2 |
| 153115 | 3'UTR | 4 | 4142 | atatataacatgtcatgata | 38 | 65 | 2 |
| 153116 | Coding | 4 | 1744 | aacacttcaggttcaataac | 3 | 66 | 2 |

As shown in Table 1, SEQ ID NOs 32, 33, 34, 36, 37, 39, 40, 43, 44, 46, 48, 50, 51, 52, 53, 54, 55, 57, 58, 59, 60, 61, 62, 63 and 65 demonstrated at least 38% inhibition of human glucocorticoid receptor expression in this assay and are therefore preferred. The target regions to which these preferred sequences are complementary are herein referred to as "preferred target segments" and are therefore preferred for targeting by compounds of the present invention. SEQ ID NO: 55 is a cross species oligonucleotide which is also complementary to the mouse glucocorticoid nucleic acid target.

The compounds in Table 2 were analyzed for their effect on human glucocorticoid receptor mRNA levels by quantitative real-time PCR as described in other examples herein. Data, shown in Table 2, are averages from two experiments in which HepG2 cells were treated with 150 nM the antisense oligonucleotides of the present invention. The positive control for each datapoint is identified in the table by sequence ID number. If present, "N.D." indicates "no data".

**Table 2**

| **Inhibition of human glucocorticoid receptor mRNA levels by chimeric phosphorothioate oligonucleotides having 2'-MOE wings and a deoxy gap** | | | | | | | |
|---|---|---|---|---|---|---|---|
| **ISIS #** | **REGION** | **TARGET SEQ ID NO** | **TARGET SITE** | **SEQUENCE** | **% INHIB** | **SEQ ID NO** | **CONTROL SEQ ID NO** |
| 180270 | Coding | 4 | 251 | gggtgaagacgcagaaacct | 47 | 67 | 2 |
| 180271 | Coding | 4 | 388 | tctcccatatacagtcccat | 70 | 68 | 2 |
| 180272 | Coding | 4 | 497 | gtttgcaatgctttcttcca | 45 | 69 | 2 |
| 180273 | Coding | 4 | 507 | acctattgaggtttgcaatg | 69 | 70 | 2 |
| 180274 | Coding | 4 | 514 | ctggtcgacctattgaggtt | 67 | 71 | 2 |
| 180275 | Coding | 4 | 672 | ctgtggtatacaatttcaca | 94 | 72 | 2 |
| 180276 | Coding | 4 | 679 | ctttggtctgtggtatacaa | 90 | 73 | 2 |
| 180277 | Coding | 4 | 687 | caaaggtgctttggtctgtg | 89 | 74 | 2 |
| 180278 | Coding | 4 | 712 | gaaaactccaaatcctgcaa | 64 | 75 | 2 |
| 180279 | Coding | 4 | 877 | ggtttagtgtccggtaaaat | 45 | 76 | 2 |
| 180280 | Coding | 4 | 1000 | ttctcttgcttaattacccc | 69 | 77 | 2 |
| 180281 | Coding | 4 | 1007 | gcccagtttctcttgcttaa | 76 | 78 | 2 |
| 180282 | Coding | 4 | 1072 | gaaatggcagacattttatt | 67 | 79 | 2 |
| 180283 | Coding | 4 | 1081 | ccatgaacagaaatggcaga | 92 | 80 | 2 |
| 180284 | Coding | 4 | 1102 | tgtcctccagaggtactcac | 87 | 81 | 2 |
| 180285 | Coding | 4 | 1112 | gtggtacatctgtcctccag | 56 | 82 | 2 |
| 180286 | Coding | 4 | 1122 | tcatgtcatagtggtacatc | 82 | 83 | 2 |
| 180287 | Coding | 4 | 1132 | gatgctgtattcatgtcata | 21 | 84 | 2 |
| 180288 | Coding | 4 | 1141 | tgagaaagggatgctgtatt | 78 | 85 | 2 |
| 180289 | Coding | 4 | 1181 | tggtggaatgacattaaaaa | 81 | 86 | 2 |
| 180290 | Coding | 4 | 1186 | ggaattggtggaatgacatt | 50 | 87 | 2 |
| 180291 | Coding | 4 | 1387 | gagcacaccaggcagagttt | 47 | 88 | 2 |
| 180292 | Coding | 4 | 1469 | ctgtccttccactgctcttt | 61 | 89 | 2 |
| 180293 | Coding | 4 | 1479 | ggtaattgtgctgtccttcc | 21 | 90 | 2 |
| 180294 | Coding | 4 | 1552 | tttcgatagcggcatgctgg | 78 | 91 | 2 |
| 180295 | Coding | 4 | 1561 | tgaagacattttcgatagcg | 63 | 92 | 2 |
| 180296 | Coding | 4 | 1591 | gtttttcgagcttccaggtt | 55 | 93 | 2 |
| 180297 | Coding | 4 | 1680 | caggaactattgttttgtta | 73 | 94 | 2 |
| 180298 | Coding | 4 | 1852 | gcctttgcccatttcactgc | 53 | 95 | 2 |
| 180300 | Coding | 4 | 2001 | taataatcagatcaggagca | 34 | 96 | 2 |
| 180301 | Coding | 4 | 2008 | tgctcattaataatcagatc | 73 | 97 | 2 |
| 180302 | Coding | 4 | 2015 | cattctctgctcattaataa | 42 | 98 | 2 |
| 180303 | Coding | 4 | 2026 | cagggtagagtcattctctg | 82 | 99 | 2 |
| 180304 | Coding | 4 | 2053 | agcatgtgtttacattggtc | 63 | 100 | 2 |
| 180305 | Coding | 4 | 2110 | atacagagatactcttcata | 59 | 101 | 2 |
| 180306 | Coding | 4 | 2120 | taaggttttcatacagagat | 68 | 102 | 2 |
| 180307 | Coding | 4 | 2131 | gagagaagcagtaaggtttt | 22 | 103 | 2 |
| 180309 | Coding | 4 | 2213 | ggcttttcctagctctttga | 41 | 104 | 2 |
| 180310 | Coding | 4 | 2221 | ttgacaatggcttttcctag | 76 | 105 | 2 |
| 180311 | Coding | 4 | 2386 | gtgatgatttcagctaacat | 57 | 106 | 2 |
| 180315 | 3'UTR | 4 | 2617 | tgccaagtcttggccctcta | 80 | 107 | 2 |
| 180316 | 3'UTR | 4 | 2627 | ctgcttctgttgccaagtct | 75 | 108 | 2 |
| 305186 | 5'UTR | 4 | 13 | caggagggaaatatattttt | 48 | 109 | 2 |
| 305187 | 5'UTR | 4 | 41 | acaacttagcttgtgaacgc | 54 | 110 | 2 |
| 305188 | 5'UTR | 4 | 100 | ctctggcagaggagccgctc | 77 | 111 | 2 |
| 305189 | Start Codon | 4 | 118 | tccatcagtgaatatcaact | 58 | 112 | 2 |
| 305190 | Start Codon | 4 | 125 | tttggagtccatcagtgaat | 72 | 113 | 2 |
| 305191 | Start Codon | 4 | 132 | atgattctttggagtccatc | 76 | 114 | 2 |
| 305192 | Coding | 4 | 205 | ttatagaagtccatcacatc | 55 | 115 | 2 |
| 305193 | Coding | 4 | 243 | acgcagaaaccttcacagta | 67 | 116 | 2 |
| 305194 | Coding | 4 | 358 | actgctttggacagatctgg | 60 | 117 | 2 |
| 305195 | Coding | 4 | 667 | gtatacaatttcacattgcc | 79 | 118 | 2 |
| 305196 | Coding | 4 | 695 | caaaatgtcaaaggtgcttt | 81 | 119 | 2 |
| 305197 | Coding | 4 | 763 | aggtctgatctccaaggact | 77 | 120 | 2 |
| 305198 | Coding | 4 | 826 | tccaaaaggaatgaatcgtc | 85 | 121 | 2 |
| 305199 | Coding | 4 | 1067 | ggcagacattttattaccaa | 68 | 122 | 2 |
| 305200 | Coding | 4 | 1150 | tcctgctgttgagaaaggga | 85 | 123 | 2 |
| 305201 | Coding | 4 | 1224 | cagatccttggcacctattc | 89 | 124 | 2 |
| 305202 | Coding | 4 | 1250 | ccccagagaagtcaagttgt | 0 | 125 | 2 |
| 305203 | Coding | 4 | 1356 | ctgttgttgctgttgaggag | 72 | 126 | 2 |
| 305204 | Coding | 4 | 1737 | caggttcaataacctccaac | 71 | 127 | 2 |
| 305205 | Coding | 4 | 1819 | cgccctcctaacatgttgag | 60 | 128 | 2 |
| 305206 | Coding | 4 | 1870 | ttcctgaaacctggtattgc | 45 | 129 | 2 |
| 305207 | Coding | 4 | 1980 | aacacagcaggtttgcactt | 69 | 130 | 2 |
| 305208 | Coding | 4 | 2146 | tccttaggaactgaagagag | 67 | 131 | 2 |
| 305209 | Coding | 4 | 2175 | catcaaatagctcttggctc | 56 | 132 | 2 |
| 305210 | Coding | 4 | 2201 | ctctttgatgtaggtcattc | 62 | 133 | 2 |
| 305211 | Coding | 4 | 2282 | atccaagagttttgtcagtt | 39 | 134 | 2 |
| 305212 | Coding | 4 | 2304 | tttcaaccacttcatgcata | 71 | 135 | 2 |
| 305213 | Coding | 4 | 2397 | gtatctgattggtgatgatt | 75 | 136 | 2 |
| 305214 | Stop Codon | 4 | 2455 | taaggcagtcacttttgatg | 74 | 137 | 2 |
| 305215 | 3'UTR | 4 | 2488 | taattcgactttctttaagg | 64 | 138 | 2 |
| 305216 | 3'UTR | 4 | 2519 | acaaactgatagtttataca | 41 | 139 | 2 |
| 305217 | 3'UTR | 4 | 2584 | gtgcgtatttaaaacaaaac | 56 | 140 | 2 |
| 305218 | 3'UTR | 4 | 4739 | taatttctccaaaatactga | 53 | 141 | 2 |
| 305219 | 3'UTR | 4 | 2646 | aaaagtgatgacgactcaac | 55 | 142 | 2 |
| 305220 | 3'UTR | 4 | 2723 | ttacgtccacatattaaggt | 67 | 143 | 2 |
| 305221 | 3'UTR | 4 | 2753 | ttaggtgccatccttctttg | 87 | 144 | 2 |
| 305222 | 3'UTR | 4 | 2764 | gcactggtggtttaggtgcc | 77 | 145 | 2 |
| 305223 | 3'UTR | 4 | 2769 | tttgggcactggtggtttag | 82 | 146 | 2 |
| 305224 | 3'UTR | 4 | 2824 | atttcatccagccaactgtg | 82 | 147 | 2 |
| 305225 | 3'UTR | 4 | 2850 | ggatacaccaacagaaagtc | 62 | 148 | 2 |
| 305226 | 3'UTR | 4 | 2939 | acaacttcccttttctgata | 62 | 149 | 2 |
| 305227 | 3'UTR | 4 | 2959 | cagtaatagctataaaaggc | 77 | 150 | 2 |
| 305228 | 3'UTR | 4 | 3004 | agcaagcgtagttcactaaa | 88 | 151 | 2 |
| 305229 | 3'UTR | 4 | 3063 | gctgcccatcttaaacagct | 61 | 152 | 2 |
| 305230 | 3'UTR | 4 | 3132 | aagcaccaacccattttcac | 63 | 153 | 2 |
| 305231 | 3'UTR | 4 | 3144 | ccatcaggttagaagcacca | 72 | 154 | 2 |
| 305232 | 3'UTR | 4 | 3160 | ttctgatagctaagtgccat | 80 | 155 | 2 |
| 305233 | 3'UTR | 4 | 3195 | aagaatactggagatttgag | 75 | 156 | 2 |
| 305234 | 3'UTR | 4 | 3294 | gctctataccagttaggact | 94 | 157 | 2 |
| 305235 | 3'UTR | 4 | 3320 | ttacccagcaggtcactgga | 92 | 158 | 2 |
| 305236 | 3'UTR | 4 | 3330 | catccacagtttacccagca | 86 | 159 | 2 |
| 305237 | 3'UTR | 4 | 3347 | tagtcttttgcaaccatcat | 89 | 160 | 2 |
| 305238 | 3'UTR | 4 | 3375 | agggcctcttggtagttatt | 83 | 161 | 2 |
| 305239 | 3'UTR | 4 | 3409 | tagccattgcaaaaataggg | 71 | 162 | 2 |
| 305240 | 3'UTR | 4 | 3416 | tgccatatagccattgcaaa | 89 | 163 | 2 |
| 305241 | 3'UTR | 4 | 3445 | ctgaaagacaaatagtttac | 29 | 164 | 2 |
| 305242 | 3'UTR | 4 | 3484 | acaacttttaagaagttata | 32 | 165 | 2 |
| 305243 | 3'UTR | 4 | 3499 | tggttatctggaatcacaac | 82 | 166 | 2 |
| 305244 | 3'UTR | 4 | 3504 | acagctggttatctggaatc | 81 | 167 | 2 |
| 305245 | 3'UTR | 4 | 3521 | agtctctcagctgtgttaca | 81 | 168 | 2 |
| 305246 | 3'UTR | 4 | 3610 | gtgaaaatgggtgtctagcc | 51 | 169 | 2 |
| 305247 | 3'UTR | 4 | 3624 | tgacagatgggaatgtgaaa | 67 | 170 | 2 |
| 305248 | 3'UTR | 4 | 3641 | aaagattaaccaattggtga | 80 | 171 | 2 |
| 305249 | 3'UTR | 4 | 3658 | tttcctgtaccatcaggaaa | 83 | 172 | 2 |
| 305250 | 3'UTR | 4 | 3743 | tctatggcacacattaggga | 67 | 173 | 2 |
| 305251 | 3'UTR | 4 | 3754 | ttgtgttaaactctatggca | 74 | 174 | 2 |
| 305252 | 3'UTR | 4 | 3770 | aagaaattcacaggacttgt | 76 | 175 | 2 |
| 305253 | 3'UTR | 4 | 3841 | gaaagttggtaaggtgcaca | 84 | 176 | 2 |
| 305254 | 3'UTR | 4 | 3886 | caaatttcttgtggcttagt | 69 | 177 | 2 |
| 305255 | 3'UTR | 4 | 3898 | ttgaatagaaatcaaatttc | 0 | 178 | 2 |
| 305256 | 3'UTR | 4 | 3918 | acacaaataatttggccacc | 58 | 179 | 2 |
| 305257 | 3'UTR | 4 | 3923 | ctattacacaaataatttgg | 22 | 180 | 2 |
| 305258 | 3'UTR | 4 | 4038 | agtagcccttcccttcccag | 33 | 181 | 2 |
| 305259 | 3'UTR | 4 | 4046 | aaagctgcagtagcccttcc | 56 | 182 | 2 |
| 305260 | 3'UTR | 4 | 4053 | tgcatgtaaagctgcagtag | 77 | 183 | 2 |
| 305261 | 3'UTR | 4 | 4065 | attttaataaattgcatgta | 24 | 184 | 2 |
| 305262 | 3'UTR | 4 | 4082 | caagctattttacaatcatt | 57 | 185 | 2 |
| 305263 | 3'UTR | 4 | 4174 | atccatcagcatttctttga | 74 | 186 | 2 |
| 305264 | 3'UTR | 4 | 4191 | tataaatcatataggttatc | 26 | 187 | 2 |
| 305265 | 3'UTR | 4 | 4244 | caaactgtttggtttctgag | 77 | 188 | 2 |
| 305266 | 3'UTR | 4 | 4311 | ctgggtcagagcctcagcaa | 84 | 189 | 2 |
| 305267 | 3'UTR | 4 | 4319 | taatctcactgggtcagagc | 79 | 190 | 2 |
| 305268 | 3'UTR | 4 | 4365 | aatgagaagggtggtcagaa | 77 | 191 | 2 |
| 305269 | 3'UTR | 4 | 4376 | ctcactgttggaatgagaag | 82 | 192 | 2 |
| 305270 | 3'UTR | 4 | 4401 | agtaaacwaacctgcgctg | 74 | 193 | 2 |
| 305271 | 3'UTR | 4 | 4442 | ctgtttacatactttacata | 68 | 194 | 2 |
| 305272 | 3'UTR | 4 | 4483 | agatggtgcctttaaggatg | 70 | 195 | 2 |
| 305273 | 3'UTR | 4 | 4504 | atgtgaaagtaacccgctat | 74 | 196 | 2 |
| 305274 | 3'UTR | 4 | 4547 | ttctgaagcttctgttgtca | 93 | 197 | 2 |
| 305275 | 3'UTR | 4 | 4577 | ctggtacctctatgcaaact | 90 | 198 | 2 |
| 305276 | 3'UTR | 4 | 4602 | gagattctgcactatttaca | 81 | 199 | 2 |
| 305277 | 3'UTR | 4 | 4624 | tagtgtattattggcaacct | 79 | 200 | 2 |
| 305278 | 3'UTR | 4 | 4664 | ttatttggaaataaactctt | 27 | 201 | 2 |
| 305279 | 3'UTR | 4 | 4680 | aaaacatgtcctcattttat | 62 | 202 | 2 |
| 305280 | Intron | 25 | 103636 | gaagctctttttgaaactta | 83 | 203 | 2 |
| 305281 | Coding | 26 | 2315 | tggttttaaccacataacat | 79 | 204 | 2 |
| 305282 | Coding | 26 | 2304 | acataacattttcatgcata | 33 | 205 | 2 |
| 305283 | 3'UTR | 25 | 104039 | tttgttgtgagtaaccaact | 78 | 206 | 2 |
| 305284 | 3'UTR | 25 | 104061 | acactaaaaatacttttcag | 24 | 207 | 2 |
| 305285 | 3'UTR | 25 | 104562 | aactccacccaaagggttta | 71 | 208 | 2 |
| 305286 | 3'UTR | 25 | 104629 | ttcctgaaaacctggtcact | 54 | 209 | 2 |
| 305287 | Intron | 25 | 24125 | attaatctgcataggaagca | 73 | 210 | 2 |
| 305288 | Exon 7: | 25 | | ttctaccaacctgaagagag | 54 | 211 | |
| | intron 8 junction | | 87671 | | | | 2 |
| 305289 | Intron | 25 | 89336 | agaagaactcgtgatattat | 77 | 212 | 2 |
| 305290 | Intron 7: | 25 | | tccttaggaactaaaaggtt | 43 | 213 | |
| | exon 8 junction | | 100360 | | | | 2 |
| 305291 | Intron 8: | 25 | | tttcaaccacctgcaagaga | 68 | 214 | |
| | exon 9 junction | | 101044 | | | | 2 |
| 305292 | Intron | 27 | 196 | ggtcccagctgcttcggccg | 36 | 215 | 2 |
| 305293 | Intron | 27 | 304 | ggagagcccctatttaagaa | 47 | 216 | 2 |

As shown in Table 2, SEQ ID NOs 67, 68, 69, 70, 71, 72, 73, 74, 75, 76, 77, 78, 79, 80, 81, 82, 83, 85, 86, 87, 88, 89, 91, 92, 93, 94, 95, 97, 98, 99, 100, 101, 102, 104, 105, 106, 107, 108, 109, 110, 111,112,113,114,115,116,117,118,119,120,121,122,123,124,126,127,128,129,130,131,132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 166, 167, 168, 169, 170, 171, 172, 173, 174, 175, 176, 177, 179, 182, 183, 185, 186, 188, 189, 190, 191, 192, 193, 194, 195, 196, 197, 198, 199, 200, 202, 203, 204, 206, 208, 209, 210, 211, 212, 213, 214 and 216 demonstrated at least 39% inhibition of human glucocorticoid receptor expression in this assay and are therefore preferred. The target regions to which these preferred sequences are complementary are herein referred to as "preferred target segments" and are therefore preferred for targeting by compounds of the present invention.

SEQ ID NOs 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107 and 108 are cross species oligonucleotides which are also complementary to the mouse glucocorticoid receptor nucleic acid target.

### Example 16

Antisense inhibition of mouse glucocorticoid receptor expression by chimeric phosphorothioate oligonucleotides having 2'-MOE wings and a deoxy gap.

In accordance with the present invention, a second series of antisense compounds was designed to target different regions of the mouse glucocorticoid receptor RNA, using published sequences (GenBank accession number NM_008173.1, incorporated herein as SEQ ID NO: 11, GenBank accession number X66367.1, incorporated herein as SEQ ID NO: 217, GenBank accession number BF181849.1, incorporated herein as SEQ ID NO: 218, GenBank accession number BE373661.1, incorporated herein as SEQ ID NO: 219, and the complement ofnucleotides 145001 to 164000 of the sequence with GenBank accession number AC007995.19, incorporated herein as SEQ ID NO: 220). The compounds

are shown in Table 3. "Target site" indicates the first (5'-most) nucleotide number on the particular target nucleic acid to which the compound binds. All compounds in Table 3 are chimeric oligonucleotides ("gapmers") 20 nucleotides in length, composed of a central "gap" region consisting often 2'-deoxynucleotides, which is flanked on both sides (5' and 3' directions) by five-nucleotide "wings". The wings are composed of 2'-methoxyethyl (2'-MOE)nucleotides. The internucleoside (backbone) linkages are phosphorothioate (P=S) throughout the oligonucleotide. All cytosine residues are 5-methylcytosines. The compounds were analyzed for their effect on mouse glucocorticoid receptor mRNA levels by quantitative real-time PCR as described in other examples herein. Data are averages from two experiments in which b.END cells were treated with 150 nM of the antisense oligonucleotides of the present invention. The positive control for each datapoint is identified in the table by sequence ID number. If present, "N.D." indicates "no data".

**Table 3**

| **Inhibition of mouse glucocorticoid receptor mRNA levels by chimeric phosphorothioate oligonucleotides having 2'-MOE wings and a deoxy gap** | | | | | | | |
|---|---|---|---|---|---|---|---|
| **ISIS #** | **REGION** | **TARGET SEQ ID NO** | **TARGET SITE** | **SEQUENCE** | **% INHIB** | **SEQ ID NO** | **CONTROL SEQ ID NO** |
| 153105 | Coding | 11 | 2242 | tccaaaaatgtttggaagca | 64 | 55 | 2 |
| 180268 | Start Codon | 11 | 2 | cattggcaaatattaacttc | 53 | 221 | 2 |
| 180269 | Start Codon | 11 | 11 | tttggagtccattggcaaat | 70 | 222 | 2 |
| 180270 | Coding | 11 | 140 | gggtgaagacgcagaaacct | 58 | 67 | 2 |
| 180271 | Coding | 11 | 301 | tctcccatatacagtcccat | 86 | 68 | 2 |
| 180272 | Coding | 11 | 410 | gtttgcaatgctttcttcca | 86 | 69 | 2 |
| 180273 | Coding | 11 | 420 | acctattgaggtttgcaatg | 65 | 70 | 2 |
| 180274 | Coding | 11 | 427 | ctggtcgacctattgaggtt | 69 | 71 | 2 |
| 180275 | Coding | 11 | 585 | ctgtggtatacaatttcaca | 84 | 72 | 2 |
| 180276 | Coding | 11 | 592 | ctttggtctgtggtatacaa | 84 | 73 | 2 |
| 180277 | Coding | 11 | 600 | caaaggtgctttggtctgtg | 82 | 74 | 2 |
| 180278 | Coding | 11 | 625 | gaaaactccaaatcctgcaa | 93 | 75 | 2 |
| 180279 | Coding | 11 | 787 | ggtttagtgtccggtaaaat | 75 | 76 | 2 |
| 180280 | Coding | 11 | 910 | ttctcttgcttaattacccc | 87 | 77 | 2 |
| 180281 | Coding | 11 | 917 | gcccagtttctcttgcttaa | 84 | 78 | 2 |
| 180282 | Coding | 11 | 982 | gaaatggcagacattttatt | 72 | 79 | 2 |
| 180283 | Coding | 11 | 991 | ccatgaacagaaatggcaga | 74 | 80 | 2 |
| 180284 | Coding | 11 | 1012 | tgtcctccagaggtactcac | 82 | 81 | 2 |
| 180285 | Coding | 11 | 1022 | gtggtacatctgtcctccag | 66 | 82 | 2 |
| 180286 | Coding | 11 | 1032 | tcatgtcatagtggtacatc | 69 | 83 | 2 |
| 180287 | Coding | 11 | 1042 | gatgctgtattcatgtcata | 70 | 84 | 2 |
| 180288 | Coding | 11 | 1051 | tgagaaagggatgctgtatt | 62 | 85 | 2 |
| 180289 | Coding | 11 | 1091 | tggtggaatgacattaaaaa | 71 | 86 | 2 |
| 180290 | Coding | 11 | 1096 | ggaattggtggaatgacatt | 63 | 87 | 2 |
| 180291 | Coding | 11 | 1294 | gagcacaccaggcagagttt | 54 | 88 | 2 |
| 180292 | Coding | 11 | 1376 | ctgtccttccactgctcttt | 63 | 89 | 2 |
| 180293 | Coding | 11 | 1386 | ggtaattgtgctgtccttcc | 57 | 90 | 2 |
| 180294 | Coding | 11 | 1459 | tttcgatagcggcatgctgg | 55 | 91 | 2 |
| 180295 | Coding | 11 | 1468 | tgaagacattttcgatagcg | 57 | 92 | 2 |
| 180296 | Coding | 11 | 1498 | gtttttcgagcttccaggtt | 59 | 93 | 2 |
| 180297 | Coding | 11 | 1584 | caggaactattgttttgtta | 41 | 94 | 2 |
| 180298 | Coding | 11 | 1756 | gcctttgcccatttcactgc | 41 | 95 | 2 |
| 180299 | Coding | 11 | 1774 | tttctgaatcctggtatcgc | 48 | 223 | 2 |
| 180300 | Coding | 11 | 1905 | taataatcagatcaggagca | 43 | 96 | 2 |
| 180301 | Coding | 11 | 1912 | tgctcattaataatcagatc | 62 | 97 | 2 |
| 180302 | Coding | 11 | 1919 | cattctctgctcattaataa | 50 | 98 | 2 |
| 180303 | Coding | 11 | 1930 | cagggtagagtcattctctg | 63 | 99 | 2 |
| 180304 | Coding | 11 | 1957 | agcatgtgtttacattggtc | 71 | 100 | 2 |
| 180305 | Coding | 11 | 2014 | atacagagatactcttcata | 49 | 101 | 2 |
| 180306 | Coding | 11 | 2024 | taaggttttcatacagagat | 47 | 102 | 2 |
| 180307 | Coding | 11 | 2035 | gagagaagcagtaaggtttt | 26 | 103 | 2 |
| 180308 | Coding | 11 | 2050 | tccttaggaactgaggagag | 46 | 224 | 2 |
| 180309 | Coding | 11 | 2117 | ggcttttcctagctctttga | 69 | 104 | 2 |
| 180310 | Coding | 11 | 2125 | ttgacaatggcttttcctag | 65 | 105 | 2 |
| 180311 | Coding | 11 | 2290 | gtgatgatttcagctaacat | 59 | 106 | 2 |
| 180312 | Stop Codon | 11 | 2359 | taaggcagtcatttctgatg | 58 | 225 | 2 |
| 180313 | 3'UTR | 11 | 2376 | aaggcagcctttcttagtaa | 51 | 226 | 2 |
| 180314 | 3'UTR | 11 | 2414 | aagtttgtacagtaaaagct | 85 | 227 | 2 |
| 180315 | 3'UTR | 11 | 2511 | tgccaagtcttggccctcta | 13 | 107 | 2 |
| 180316 | 3'UTR | 11 | 2521 | ctgcttctgttgccaagtct | 52 | 108 | 2 |
| 180317 | 3'UTR | 11 | 2527 | gctcatctgcttctgttgcc | 68 | 228 | 2 |
| 180318 | 5'UTR | 217 | 1386 | gcatacatactgtgagcccg | 0 | 229 | 2 |
| 180319 | 5'UTR | 218 | 37 | ctgggcggccccgtctgcag | 14 | 230 | 2 |
| 180320 | 5'UTR | 218 | 104 | ttggcaaatattaatgtgag | 20 | 231 | 2 |
| 180321 | 5'UTR | 219 | 227 | agccagataaacaagtcggc | 64 | 232 | 2 |
| 180322 | 5'UTR | 219 | 278 | atattaactcagcaccggcg | 37 | 233 | 2 |
| 180323 | Intron | 220 | 4092 | agaatcttagctatagggct | 35 | 234 | 2 |
| 180324 | Exon 5: Intron 5 junction | 220 | 7968 | catgccttacctggtatcgc | 8 | 235 | 2 |
| 180325 | Exon 6: Intron 6 junction | 220 | 9049 | tgtaacttactcattaataa | 32 | 236 | 2 |
| 180326 | intron | 220 | 13238 | tcacatagtctgcgattgtt | 63 | 237 | 2 |
| 180327 | Intron 7: Exon 8 junction | 220 | 14602 | tccttaggaactaaaaggta | 13 | 238 | 2 |
| 180328 | 3'UTR | 220 | 14909 | tatctctgactgtcctggca | 59 | 239 | 2 |
| 180329 | 3'UTR | 220 | 15984 | agcctttcttagtaaggcag | 36 | 240 | 2 |
| 180330 | 3'UTR | 220 | 16177 | acatcactgtctgctttcct | 59 | 241 | 2 |
| 180331 | 3'UTR | 220 | 16227 | ggacatgtctccactaactg | 65 | 242 | 2 |
| 180332 | 3'UTR | 220 | 16268 | tttgggcactggtggttcag | 63 | 243 | 2 |
| 180333 | 3'UTR | 220 | 16548 | catcttaaacagctatacaa | 64 | 244 | 2 |
| 180334 | 3'UTR | 220 | 16639 | ctgatagctgagtgccatca | 55 | 245 | 2 |
| 180335 | 3'UTR | 220 | 16868 | agagatggtgcattgggtgc | 48 | 246 | 2 |
| 180336 | 3'UTR | 220 | 17166 | cctgacattcagttctaaat | 70 | 247 | 2 |
| 180337 | 3'UTR | 220 | 17178 | caaacatggatgcctgacat | 70 | 248 | 2 |
| 180338 | 3'UTR | 220 | 17215 | ttagattctatggcacatgt | 52 | 249 | 2 |
| 180339 | 3'UTR | 220 | 17327 | gttaagctttgagtcacaga | 64 | 250 | 2 |
| 180340 | 3'UTR | 220 | 17729 | ctctccctagcttagagcaa | 71 | 251 | 2 |
| 180341 | 3'UTR | 220 | 17909 | tggacggtgcctctaagtac | 65 | 252 | 2 |
| 180342 | 3'UTR | 220 | 18076 | gaaatggactcttgtaggat | 65 | 253 | 2 |
| 180343 | 3'UTR | 220 | 18283 | ataaatttcacatccagctg | 62 | 254 | 2 |
| 180344 | 3'UTR | 220 | 18370 | taaatgtacaataatctatt | 14 | 255 | 2 |

As shown in Table 3, SEQ ID NOs 55, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 104, 105, 106, 108, 221, 222, 223, 224, 225, 226, 227, 228, 232, 233, 234, 236, 237, 239, 240, 241, 242, 243, 244, 245, 246, 247, 248, 249, 250, 251, 252, 253 and 254 demonstrated at least 32% inhibition of mouse glucocorticoid receptor expression in this experiment and are therefore preferred. The target regions to which these preferred sequences are complementary are herein referred to as "preferred target segments" and are therefore preferred for targeting by compounds of the present invention.

SEQ ID NOs, 69, 70, 71, 74, 76, 77, 78, 79, 80, 81, 82, 83, 84, 85, 86, 87, 89, 90, 94, 95, 96, 97, 98, 100, 101, 102, 103, 104, 106, 222, 224, 227, 231, 254 and 255 are cross species oligonucleotides which are also complementary to the rat glucocorticoid receptor nucleic acid target.

### Example 17

### Antisense inhibition of rat glucocorticoid receptor expression by chimeric phosphorothioate oligonucleotides having 2'-MOE wings and a deoxy gap

In accordance with the present invention, a third series of antisense compounds was designed to target different regions of the rat glucocorticoid receptor RNA, using published sequences (GenBank accession number NM_0125 76.1, incorporated herein as SEQ ID NO: 18, and GenBank accession number Y00489.1, incorporated herein as SEQ ID NO: 256). The compounds are shown in Table 4. "Target site" indicates the first (5'-most) nucleotide number on the particular target nucleic acid to which the compound binds. All compounds in Table 4 are chimeric oligonucleotides ("gapmers") 20 nucleotides in length, composed of a central "gap" region consisting of ten 2'-deoxynucleotides, which is flanked on both sides (5' and 3' directions) by five-nucleotide "wings". The wings are composed of 2'-methoxyethyl (2'-MOE)nucleotides. The internucleoside (backbone) linkages are phosphorothioate (P=S) throughout the oligonucleotide. All cytosine residues are 5-methylcytosines. The compounds were analyzed for their effect on rat glucocorticoid receptor mRNA levels by quantitative real-time PCR as described in other examples herein. Data are averages from two experiments in which NRK cells were treated with 150 nM of the antisense oligonucleotides of the present invention. The positive control for each datapoint is identified in the table by sequence ID number. If present, "N.D." indicates "no data".

**Table 4**

| **Inhibition of rat glucocorticoid receptor mRNA levels by chimeric phosphorothioate oligonucleotides having 2'-MOE wings and a deoxy gap** | | | | | | | |
|---|---|---|---|---|---|---|---|
| **ISIS #** | **REGION** | **TARGET SEQ ID NO** | **TARGET SITE** | **SEQUENCE** | **% INHIB** | **SEQ ID NO** | **CONTROL SEQ ID NO** |
| 180269 | Start Codon | 18 | 61 | tttggagtccattggcaaat | 42 | 222 | 2 |
| 180272 | Coding | 18 | 496 | gtttgcaatgctttcttcca | 60 | 69 | 2 |
| 180273 | Coding | 18 | 506 | acctattgaggtttgcaatg | 59 | 70 | 2 |
| 180274 | Coding | 18 | 513 | ctggtcgacctattgaggtt | 49 | 71 | 2 |
| 180277 | Coding | 18 | 686 | caaaggtgctttggtctgtg | 68 | 74 | 2 |
| 180279 | Coding | 18 | 873 | ggtttagtgtccggtaaaat | 58 | 76 | 2 |
| 180280 | Coding | 18 | 996 | ttctcttgcttaattacccc | 56 | 77 | 2 |
| 180281 | Coding | 18 | 1003 | gcccagtttctcttgcttaa | 74 | 78 | 2 |
| 180282 | Coding | 18 | 1068 | gaaatggcagacattttatt | 28 | 79 | 2 |
| 180283 | Coding | 18 | 1077 | ccatgaacagaaatggcaga | 52 | 80 | 2 |
| 180284 | Coding | 18 | 1098 | tgtcctccagaggtactcac | 54 | 81 | 2 |
| 180285 | Coding | 18 | 1108 | gtggtacatctgtcctccag | 58 | 82 | 2 |
| 180286 | Coding | 18 | 1118 | tcatgtcatagtggtacatc | 46 | 83 | 2 |
| 180287 | Coding | 18 | 1128 | gatgctgtattcatgtcata | 27 | 84 | 2 |
| 180288 | Coding | 18 | 1137 | tgagaaagggatgctgtatt | 64 | 85 | 2 |
| 180289 | Coding | 18 | 1177 | tggtggaatgacattaaaaa | 47 | 86 | 2 |
| 180290 | Coding | 18 | 1182 | ggaattggtggaatgacatt | 53 | 87 | 2 |
| 180292 | Coding | 18 | 1462 | ctgtccttccactgctcttt | 37 | 89 | 2 |
| 180293 | Coding | 18 | 1472 | ggtaattgtgctgtccttcc | 30 | 90 | 2 |
| 180297 | Coding | 18 | 1670 | caggaactattgttttgtta | 56 | 94 | 2 |
| 180298 | Coding | 18 | 1842 | gcctttgcccatttcactgc | 44 | 95 | 2 |
| 180300 | Coding | 18 | 1991 | taataatcagatcaggagca | 26 | 96 | 2 |
| 180301 | Coding | 18 | 1998 | tgctcattaataatcagatc | 38 | 97 | 2 |
| 180302 | Coding | 18 | 2005 | cattctctgctcattaataa | 10 | 98 | 2 |
| 180304 | Coding | 18 | 2043 | agcatgtgtttacattggtc | 57 | 100 | 2 |
| 180305 | Coding | 18 | 2100 | atacagagatactcttcata | 31 | 101 | 2 |
| 180306 | Coding | 18 | 2110 | taaggttttcatacagagat | 47 | 102 | 2 |
| 180307 | Coding | 18 | 2121 | gagagaagcagtaaggtttt | 16 | 103 | 2 |
| 180308 | Coding | 18 | 2136 | tccttaggaactgaggagag | 58 | 224 | 2 |
| 180309 | Coding | 18 | 2203 | ggcttttcctagctctttga | 54 | 104 | 2 |
| 180311 | Coding | 18 | 2376 | gtgatgatttcagctaacat | 41 | 106 | 2 |
| 180314 | 3'UTR | 18 | 2500 | aagtttgtacagtaaaagct | 39 | 227 | 2 |
| 180320 | 5'UTR | 18 | 50 | ttggcaaatattaatgtgag | 3 | 231 | 2 |
| 180343 | 3'UTR | 18 | 4773 | ataaatttcacatccagctg | 48 | 254 | 2 |
| 180344 | 3'UTR | 18 | 4859 | taaatgtacaataatctatt | 0 | 255 | 2 |
| 223308 | Coding | 256 | 278 | taagtctggctgctgctgct | 41 | 257 | 2 |
| 223309 | Coding | 256 | 285 | ctttggataagtctggctgc | 48 | 258 | 2 |
| 223310 | Coding | 18 | 150 | aggcttttataaaagtccat | 48 | 259 | 2 |
| 223311 | Coding | 18 | 244 | atcaaggagaatcctctgct | 49 | 260 | 2 |
| 223312 | Coding | 18 | 1248 | gcccccaaggaagtcaggct | 48 | 261 | 2 |
| 223313 | Coding | 18 | 1407 | ccgtaatgacatcctgaagc | 41 | 262 | 2 |
| 223314 | Coding | 18 | 2156 | actcttggctcttcagacct | 44 | 263 | 2 |
| 223315 | Stop Codon | 18 | 2445 | taaggcagtcatttttgatg | 53 | 264 | 2 |
| 223316 | 3'UTR | 18 | 2472 | aactttctttaaggcaacct | 44 | 265 | 2 |
| 223317 | 3'UTR | 18 | 2586 | cctctataaaccacatgtac | 52 | 266 | 2 |
| 223318 | 3'UTR | 18 | 2637 | tgtcatcacttcagagtgtt | 30 | 267 | 2 |
| 223319 | 3'UTR | 18 | 2685 | aactgttagtttctgtgata | 52 | 268 | 2 |
| 223320 | 3'UTR | 18 | 2799 | tagaaagttttacccagcca | 58 | 269 | 2 |
| 223321 | 3'UTR | 18 | 3202 | ctatgtaattctccatggaa | 50 | 270 | 2 |
| 223322 | 3'UTR | 18 | 3266 | ctggactaggtgctctacac | 44 | 271 | 2 |
| 223323 | 3'UTR | 18 | 3366 | attgaagagatggtgcatta | 55 | 272 | 2 |
| 223324 | 3'UTR | 18 | 3473 | ggctttatcagagctggcta | 62 | 273 | 2 |
| 223325 | 3'UTR | 18 | 3542 | ctgtattagcgatttagttg | 53 | 274 | 2 |
| 223326 | 3'UTR | 18 | 3604 | accatgagagctagaccaat | 35 | 275 | 2 |
| 223327 | 3'UTR | 18 | 3686 | gcacatgtagggatgtgtag | 46 | 276 | 2 |
| 223328 | 3'UTR | 18 | 3880 | agtttttctattacacaaat | 21 | 277 | 2 |
| 223329 | 3'UTR | 18 | 3992 | cagtagccctttccctttcc | 11 | 278 | 2 |
| 223330 | 3'UTR | 18 | 4117 | catcaatatttctttgaccc | 23 | 279 | 2 |
| 223331 | 3'UTR | 18 | 4576 | aatggactattgaagggtgg | 44 | 280 | 2 |
| 223332 | 3'UTR | 18 | 4609 | agaaaacataagcatgtcct | 33 | 281 | 2 |
| 223333 | 3'UTR | 18 | 4702 | gaacaatcccttttagagag | 49 | 282 | 2 |
| 223334 | 3'UTR | 18 | 4848 | taatctatttttgagaagct | 52 | 283 | 2 |
| 223335 | 3'UTR | 18 | 5039 | tacgcttcaaggaaagcttc | 59 | 284 | 2 |
| 223336 | 3'UTR | 18 | 5183 | ccgagtctcactgaagttat | 55 | 285 | 2 |
| 223337 | 3'UTR | 18 | 5220 | tctttcaagatcggtcatga | 36 | 286 | 2 |
| 223338 | 3'UTR | 18 | 5274 | ccaaggcctaaaataaccag | 44 | 287 | 2 |
| 223339 | 3'UTR | 18 | 5390 | ctttgggtactctcacttat | 15 | 288 | 2 |
| 223340 | 3'UTR | 18 | 5430 | cctgactcatccttagaccc | 23 | 289 | 2 |
| 223341 | 3'UTR | 18 | 5606 | tctcaagctccatgatcctt | 4 | 290 | 2 |
| 223342 | 3'UTR | 18 | 5680 | cgccttctaacactgaaacc | 27 | 291 | 2 |
| 223343 | 3'UTR | 18 | 5686 | ctgtttcgccttctaacact | 45 | 292 | 2 |
| 223344 | 3'UTR | 18 | 5740 | gtttgggaatgagaagactt | 44 | 293 | 2 |
| 223345 | 3'UTR | 18 | 5785 | tagcagctggtcaccagtcc | 27 | 294 | 2 |
| 223346 | 3'UTR | 18 | 5858 | attttcatacagccatttat | 38 | 295 | 2 |
| 223347 | 3'UTR | 18 | 5908 | tattgacacactgaaatctc | 16 | 296 | 2 |
| 223348 | 3'UTR | 18 | 6119 | tagaaagacggatttttaaa | 0 | 297 | 2 |
| 223349 | 3'UTR | 18 | 6214 | tgtggtttggtaataccaag | 56 | 298 | 2 |
| 223350 | 3'UTR | 18 | 6244 | actaacatttactgccaatt | 28 | 299 | 2 |

As shown in Table 4, SEQ ID NOs 69, 70, 71, 74, 76, 77, 78, 79, 80, 81, 82, 83, 84, 85, 86, 87, 89, 90, 94, 95, 96, 97, 100, 101, 102, 104, 106, 222, 224, 227, 254, 257, 258, 259, 260, 261, 262, 263, 264, 265, 266, 267, 268, 269, 270, 271, 272, 273, 274, 275, 276, 280, 281, 282, 283, 284, 285, 286, 287, 291, 292, 293, 294, 295, 298 and 299 demonstrated at least 26% inhibition of rat glucocorticoid receptor expression in this experiment and are therefore preferred. As these "preferred target segments" have been found by experimentation to be open to, and accessible for, hybridization with the antisense compounds of the present invention, one of skill in the art will recognize or be able to ascertain, using no more than routine experimentation, further embodiments of the invention that encompass other compounds that specifically hybridize to these preferred target segments and consequently inhibit the expression of glucocorticoid receptor.

According to the present invention, antisense compounds include antisense oligomeric compounds, antisense oligonucleotides, ribozymes, external guide sequence (EGS) oligonucleotides, alternate splicers, primers, probes, and other short oligomeric compounds which hybridize to at least a portion of the target nucleic acid.

### Example 18

### Western blot analysis of glucocorticoid receptor protein levels

Western blot analysis (immunoblot analysis) is carried out using standard methods. Cells are harvested 16-20 h after oligonucleotide treatment, washed once with PBS, suspended in Laemmli buffer (100 ul/well), boiled for 5 minutes and loaded on a 16% SDS-PAGE gel. Gels are run for 1.5 hours at 150 V, and transferred to membrane for western blotting. Appropriate primary antibody directed to glucocorticoid receptor is used, with a radiolabeled or fluorescently labeled secondary antibody directed against the primary antibody species. Bands are visualized using a PHOSPHORIMAGER™ (Molecular Dynamics, Sunnyvale CA).

### Example 19

### Antisense inhibition of mouse glucocorticoid receptor: dose response study in b.END cells

In a further embodiment of the invention, ISIS 180271, ISIS 180272, ISIS 180277, ISIS 180280, ISIS 180281 and ISIS 180314 were tested in a dose response experiment. ISIS 118920 (GTTCATTCTAAAGTGGTCAC, SEQ ID NO: 300) targets protein phosphatase catalytic subunit 2α and was used as a control. b.END cells were plated in 24-well plates at a density of 40,000 cells per well. Cells were then treated with 1, 5, 10, 25, 50, 100 or 200 nM of antisense oligonucleotide, mixed with 3 µl of LIPOFECTIN (Invitrogen Life Technologies, Carlsbad, CA) per 100 nM oligonucleotide per 1 ml of media, as described by other examples herein. Expression of mouse glucocorticoid receptor was measured by real-time PCR as described by other examples herein. Data are expressed as percent inhibition of mouse glucocorticoid receptor mRNA, normalized to untreated control cells. The results are the average of three experiments and are shown in Table 5. A "+" preceding the numbers in the control oligonucleotide treated results indicates that gene expression increased.

**Table 5**

| **Antisense inhibition of mouse glucocorticoid receptor: dose response in b.END cells** | | | | | | | | |
|---|---|---|---|---|---|---|---|---|
| | | **% Inhibition of mouse glucocorticoid receptor** | | | | | | |
| | | **Dose of oligonucleotide** | | | | | | |
| **ISIS #** | **SEQ ID NO** | **1 nM** | **5 nM** | **10 nM** | **25 nM** | **50 nM** | **100 nM** | **200 nM** |
| **180271** | **68** | 11 | 39 | 50 | 71 | 79 | 80 | 81 |
| **180272** | **69** | 7 | 33 | 50 | 71 | 77 | 80 | 78 |
| **180277** | **74** | 17 | 51 | 56 | 78 | 88 | 82 | 71 |
| **180280** | **77** | 12 | 60 | 64 | 84 | 86 | 85 | 86 |
| **180281** | **78** | 23 | 72 | 73 | 82 | 89 | 83 | 84 |
| **180314** | **227** | 12 | 24 | 36 | 67 | 74 | 77 | 79 |
| **118920** | **300** | 2 | 1 | +36 | +11 | +20 | +15 | 4 |

As demonstrated in Table 5, the antisense compounds tested in this experiment inhibited mouse glucocorticoid receptor mRNA expression in b.END cells in a dose-dependent manner.

### Example 20

### Antisense inhibition of mouse glucocorticoid receptor: dose response study in primary mouse hepatocytes

In accordance with the present invention, ISIS 180271, ISIS 180272 and ISIS 180280 were tested in a dose-response experiment in primary mouse hepatocytes, which were treated with 50, 100, 200 or 400 nM of antisense oligonucleotide. ISIS 129685 (AATATTCGCACCCCACTGGT, SEQ ID NO: 301), ISIS 129686 (CGTTATTAACCTCCGTTGAA, SEQ ID NO: 302) and ISIS 129695 (TTCTACCTCGCGCGATTTAC, SEQ ID NO: 303) are oligonucleotides that target protein phosphatase 2A and were used as control oligonucleotides. Cells were treated and mRNA expression levels were measured as described by other examples herein. The data are normalized to untreated control cells and are expressed as percent change in mouse glucocorticoid receptor, where a "-" indicates a decrease in expression and a "+" indicates an increase in expression. The results are the average of 3 experiments and are shown in Table 6.

**Table 6**

| **Antisense inhibition of mouse glucocorticoid receptor: dose response experiment in primary mouse hepatocytes** | | | | | |
|---|---|---|---|---|---|
| | | **% Change in mouse glucocorticoid receptor expression** | | | |
| | | **Dose of oligonucleotide** | | | |
| **ISIS #** | **SEQ ID NO** | **50 nM** | **100 nM** | **200 nM** | **400 nM** |
| **180271** | **68** | -42 | -68 | -77 | -86 |
| **180272** | **69** | -49 | -66 | -73 | -84 |
| **180280** | **77** | -55 | -64 | -74 | -84 |
| **129685** | **301** | +6 | +12 | +3 | -31 |
| **129686** | **302** | +14 | +12 | +5 | +1 |
| **129695** | **303** | -6 | -19 | -10 | -21 |

These data demonstrate that in primary mouse hepatocytes, ISIS 180271, ISIS 180272 and ISIS 180280, unlike the control oligonucleotides, inhibited mouse glucocorticoid receptor expression in a dose-dependent manner.

### Example 21

### Effect of antisense inhibitors of glucocorticoid receptor on Lean mice (db/db +/- mice)

db/db +/- mice are heterozygous littermates of db/db mice, often referred to as lean littermates because they do not display the db (obesity and hyperglycemia) phenotype. Six-week old db/db +/- male mice were dosed twice weekly with 50 mg/kg of antisense oligonucleotide, given subcutaneously. A total of five doses were given. Glucocorticoid antisense oligonucleotides used were ISIS 180272 (SEQ ID NO: 69) and ISIS 180280 (SEQ ID NO: 77). ISIS 116847 (CTGCTAGCCTCTGGATTTGA; SEQ ID NO: 304), targeted to mouse PTEN, was used as a positive control. Antisense compounds were prepared in buffered saline and sterilized by filtering through a 0.2 micron filter. Blood samples were obtained from mice and rats via tail snip. Plasma glucose levels were measured before the initial dose (day -6) and the day before the mice were sacrificed (day 15). After sacrifice, serum lipids were measured and target reduction in liver was also measured.

In lean mice treated with ISIS 180272 (SEQ ID NO: 69), an antisense inhibitor of glucocorticoid receptor, plasma glucose levels were approximately 220 mg/dL at day -6 and 170 mg/dL at day 15. In lean mice treated with ISIS 180280 (SEQ ID NO: 77), another antisense inhibitor of glucocorticoid receptor, plasma glucose levels were approximately 230 mg/dL at day -6 and 160 mg/dL at day 15. Lean mice treated with saline alone had fed plasma glucose levels of approximately 240 mg/dL at day -6 and 180 mg/dL at day 15. While plasma glucose levels decreased slightly, the mice did not become hypoglycemic.

Serum lipids were also measured at the end of the study. Cholesterol levels were approximately 90 mg/dL for saline treated lean mice, 115 mg/dL for ISIS 180272-treated lean mice and 100 mg/dL for ISIS 180280-treated lean mice. Triglycerides were approximately 155 mg/dL for saline treated lean mice and substantially reduced to 100 mg/dL for ISIS 180272-treated lean mice and 90 mg/dL for ISIS 180280-treated lean mice.

Glucocorticoid receptor mRNA levels in liver were measured at the end of study using RiboGreen™ RNA quantification reagent (Molecular Probes, Inc. Eugene, OR) as taught in previous examples above. Glucocorticoid receptor mRNA levels were reduced by approximately 45% in lean mice treated with ISIS 180272, and by approximately 25% in lean mice treated with ISIS 180280, when compared to saline treatment.

### Example 22

### Effect of antisense inhibitors of glucocorticoid receptor on ob/ob mice

Ob/ob mice have a mutation in the leptin gene which results in obesity and hyperglycemia. As such, these mice are a useful model for the investigation of obesity and diabetes and treatments designed to treat these conditions. In accordance with the present invention, compounds targeted to glucocorticoid receptor are tested in the ob/ob model of obesity and diabetes.

Seven-week old male C57B1/6J-Lep ob/ob mice (Jackson Laboratory, Bar Harbor, ME) are fed a diet with a fat content of 10-15% and are subcutaneously injected with oligonucleotides at a dose of 25 mg/kg two times per week for 5 weeks. Glucocorticoid antisense oligonucleotides used were ISIS 180272 (SEQ ID NO: 69) and ISIS 180280 (SEQ ID NO: 77). ISIS 116847 (CTGCTAGCCTCTGGATTTGA; SEQ ID NO: 304), targeted to mouse PTEN, was used as a positive control and ISIS 141923 (CCTTCCCTGAAGGTTCCTCC; SEQ ID NO: 305), an unrelated oligonucleotide, was used as the negative oligonucleotide control. Saline-injected animals also serve as controls.

To assess the physiological effects resulting from antisense inhibition of target mRNA, the ob/ob mice that receive antisense oligonucleotide treatment are further evaluated at the end of the treatment period for serum lipids, serum free fatty acids, serum cholesterol, liver triglycerides, fat tissue triglycerides and liver enzyme levels. Hepatic steatosis, or clearing of lipids from the liver, is assessed by measuring the liver triglyceride content. Hepatic steatosis is assessed by routine histological analysis of frozen liver tissue sections stained with oil red O stain, which is commonly used to visualize lipid deposits, and counterstained with hematoxylin and eosin, to visualize nuclei and cytoplasm, respectively.

The effects of target inhibition on glucose and insulin metabolism are evaluated in the ob/ob mice treated with antisense oligonucleotides. Plasma glucose is measured prior to antisense oligonucleotide treatment (day -1) and following two and four weeks of treatment (day 12 and 27, respectively). Both fed and fasted plasma glucose levels are measured. Plasma insulin is also measured at the beginning of the treatment, and following 2 weeks and 4 weeks of treatment. Glucose and insulin tolerance tests are also administered in fed and fasted mice. Mice receive intraperitoneal injections of either glucose or insulin, and the blood glucose and insulin levels are measured before the insulin or glucose challenge and at 15, 20 or 30 minute intervals for up to 3 hours.

In ob/ob mice treated with ISIS 180272 (SEQ ID NO: 69), an antisense inhibitor of glucocorticoid receptor, fed plasma glucose levels were approximately 345 mg/dL at day -1, 350 mg/dL at day 12 and 245 mg/dL at day 27. In mice treated with ISIS 180280 (SEQ ID NO: 77), another antisense inhibitor of glucocorticoid receptor, fed plasma glucose levels were approximately 350 mg/dL at day -1, 340 mg/dL at day 12 and 255 mg/dL at day 27. In contrast, mice treated with saline alone had fed plasma glucose levels of approximately 350 mg/dL at day -1, 420 mg/dL at day 12 and 400 mg/dL at day 27. Mice treated with a positive control oligonucleotide, ISIS 116847 (SEQ ID NO: 304), targeted to PTEN, had fed plasma glucose levels of approximately 340 mg/dL at day -1, 230 mg/dL at day 12 and 200 mg/dL at day 27. Mice treated with negative control oligonucleotide ISIS 141923 had fed plasma glucose levels of approximately 360 mg/dL at day -1, 480 mg/dL at day 12 and 430 mg/dL at day 27. Thus fed plasma glucose levels were reduced after treatment with antisense inhibitors of glucocorticoid receptor. Hypoglycemia was not seen.

In fasted ob/ob mice, plasma glucose levels were measured on day 19 (after a 16 hour fast) and day 29 (after a 12 hour fast). In ob/ob mice treated with ISIS 180272 (SEQ ID NO: 69), an antisense inhibitor of glucocorticoid receptor, fasted plasma glucose levels were approximately 190 mg/dL at day 19 and 220 mg/dL at day 29. In mice treated with ISIS 180280 (SEQ ID NO: 77), another antisense inhibitor of glucocorticoid receptor, fasted plasma glucose levels were approximately 195 mg/dL at day 19 and 270 mg/dL at day 29. In contrast, mice treated with saline alone had fasted plasma glucose levels of approximately 320 mg/dL at day 19 and 320 mg/dL at day 29. Mice treated with a positive control oligonucleotide, ISIS 116847 (SEQ ID NO: 304), targeted to PTEN, had fasted plasma glucose levels of approximately 170 mg/dL at day 19 and 190 mg/dL at day 29. Mice treated with negative control oligonucleotide ISIS 141923 had fasted plasma glucose levels of approximately 245 mg/dL at day 19 and 340 mg/dL at day 29. Thus fasted plasma glucose levels were also reduced after treatment with antisense inhibitors of glucocorticoid receptor. Hypoglycemia was not observed.

Serum lipids in ob/ob mice were also measured at the end of the study. Cholesterol levels were approximately 270 mg/dL for saline treated mice, 305 mg/dL for ISIS 180272-treated mice, 250 mg/dL for ISIS 180280-treated mice, 285 mg/dL for ISIS 116847-treated mice and 265 mg/dL for ISIS 141923-treated mice. Triglycerides were approximately 120 mg/dL for saline treated mice, 115 mg/dL for ISIS 180272-treated mice, 105 mg/dL for ISIS 180280-treated mice, 95 mg/dL for ISIS 116847-treated mice and 95 mg/dL for ISIS 141923-treated mice.

Glucocorticoid receptor mRNA levels in ob/ob mouse livers were measured at the end of study using RiboGreen™ RNA quantification reagent (Molecular Probes, Inc. Eugene, OR) as taught in previous examples above. Glucocorticoid receptor mRNA levels were reduced by approximately 80% in mice treated with ISIS 180272, and by approximately 70% in mice treated with ISIS 180280, when compared to saline treatment. Glucocorticoid receptor mRNA levels were not significantly decreased in mice treated with the positive control oligonucleotide, ISIS 116847, and were slightly increased (120% of control) in mice treated with the negative control oligonucleotide, ISIS 141923.

Glucocorticoid receptor mRNA levels in ob/ob mouse white adipose tissue were measured at the end of study using RiboGreen™ RNA quantification reagent (Molecular Probes, Inc. Eugene, OR) as taught in previous examples above. Glucocorticoid receptor mRNA levels in fat were reduced by approximately 40% in mice treated with ISIS 180272, and by approximately 52% in mice treated with ISIS 180280, when compared to saline treatment. Glucocorticoid receptor mRNA levels in fat were slightly decreased (by approx. 13%) in mice treated with the positive control oligonucleotide, ISIS 116847, and were slightly increased (120% of control) in mice treated with the negative control oligonucleotide, ISIS 141923.

### Example 23

### Effect of antisense inhibitors of glucocorticoid receptor in leptin receptor-deficient mice (db/db mice)

Leptin is a hormone produced by fat that regulates appetite. Deficiencies in this hormone in both humans and non-human animals lead to obesity. db/db mice have a mutation in the leptin receptor gene which results in obesity and hyperglycemia. As such, these mice are a useful model for the investigation of obesity and diabetes and treatments designed to treat these conditions. db/db mice, which have lower circulating levels of insulin and are more hyperglycemic than ob/ob mice which harbor a mutation in the leptin gene, are often used as a rodent model of type 2 diabetes. In accordance with the present invention, oligomeric compounds of the present invention are tested in the db/db model of obesity and diabetes.

Seven-week old male C57B1/6J-Lepr db/db mice (Jackson Laboratory, Bar Harbor, ME) were fed a diet with a fat content of 15-20% and are subcutaneously injected with one or more of the oligomeric compounds of the invention or a control compound at a dose of 25 mg/kg two times per week for 5 weeks. Glucocorticoid antisense oligonucleotides used were ISIS 180272 (SEQ ID NO: 69) and ISIS 180280 (SEQ ID NO: 77). ISIS 116847 (SEQ ID NO: 304), targeted to mouse PTEN, was used as a positive control and ISIS 141923 (SEQ ID NO: 305), an unrelated oligonucleotide, was used as the negative oligonucleotide control. Oligonucleotides were prepared in buffered saline and sterilized by filtration through a 0.2 micron filter. Saline-injected animals, leptin receptor wildtype littermates (i.e. lean littermates) and db/db mice fed a standard rodent diet serve as controls. After the treatment period, mice are sacrificed and target levels are evaluated in liver, brown adipose tissue (BAT) and white adipose tissue (WAT). RNA isolation and target mRNA expression level quantitation are performed as described by other examples herein.

To assess the physiological effects resulting from inhibition of target mRNA, the db/db mice are further evaluated at the end of the treatment period for serum triglycerides, serum lipoproteins, serum free fatty acids, serum cholesterol, serum apolipoproteins, liver tissue triglycerides, fat tissue triglycerides and serum transaminase levels. Triglycerides, lipoproteins, cholesterol and transaminases are measured by routine clinical analyzer instruments (e.g. Olympus Clinical Analyzer, Melville, NY). Serum free fatty acids are measured using a Wako Chemicals kit for non-esterified free fatty acids (Richmond, VA). Tissue triglyceride levels are measured using a Triglyceride GPO Assay from Roche Diagnostics (Indianapolis, IN). Liver triglyceride levels are used to assess hepatic steatosis, or clearing of lipids from the liver. Hepatic steatosis is also assessed by routine histological analysis of frozen liver tissue sections stained with oil red O stain, which is commonly used to visualize lipid deposits, and counterstained with hematoxylin and eosin, to visualize nuclei and cytoplasm, respectively.

The effects of target inhibition on glucose and insulin metabolism are evaluated in the db/db mice treated with the oligomeric compounds of the invention. Plasma glucose (fed and fasted) is measured at the start of the treatment and weekly during treatment. Plasma insulin is similarly measured. Glucose and insulin tolerance tests are also administered in fed and fasted mice. Mice receive intraperitoneal injections of either glucose or insulin, and the blood glucose and insulin levels are measured before the insulin or glucose challenge and at 15, 20 or 30 minute intervals for up to 3 hours. Glucose levels are measured using a YSI glucose analyzer (YSI Scientific, Yellow Springs, OH) and insulin levels are measure using an Alpco insulin-specific ELISA kit from (Windham, NH).

In db/db mice treated with ISIS 180272 (SEQ ID NO: 69), an antisense inhibitor of glucocorticoid receptor, fed plasma glucose levels were approximately 300 mg/dL at day -1, 445 mg/dL at day 5, 450 mg/dL at day 12 and 450 mg/dL at day 26. In mice treated with ISIS 180280 (SEQ ID NO: 77), another antisense inhibitor of glucocorticoid receptor, fed plasma glucose levels were approximately 300 mg/dL at day -1, 480 mg/dL at day 5, 440 mg/dL at day 12 and 480 mg/dL at day 26. Mice treated with saline alone had fed plasma glucose levels of approximately 300 mg/dL at day -1, 470 mg/dL at day 5, 510 mg/dL at day 12 and 500 mg/dL at day 26. db/db mice treated with a positive control oligonucleotide, ISIS 116847 (SEQ ID NO: 304), targeted to PTEN, had fed plasma glucose levels of approximately 300 mg/dL at day -1, 405 mg/dL at day 5, 300 mg/dL at day 12 and 350 mg/dL at day 26. Mice treated with negative control oligonucleotide ISIS 141923 had fed plasma glucose levels of approximately 300 mg/dL at day -1, 405 mg/dL at day 5, 425 mg/dL at day 12 and 500 mg/dL at day 26.

In fasted db/db mice, plasma glucose levels were measured on day 19 (after a 16 hour fast) and day 29 (after a 12 hour fast). In db/db mice treated with ISIS 180272 (SEQ ID NO: 69), an antisense inhibitor of glucocorticoid receptor, fasted plasma glucose levels were approximately 200 mg/dL at day 19 and 210 mg/dL at day 29. In mice treated with ISIS 180280 (SEQ ID NO: 77), another antisense inhibitor of glucocorticoid receptor, fasted plasma glucose levels were approximately 260 mg/dL at day 19 and 235 mg/dL at day 29. In contrast, mice treated with saline alone had fasted plasma glucose levels of approximately 320 mg/dL at day 19 and 300 mg/dL at day 29. Mice treated with a positive control oligonucleotide, ISIS 116847 (SEQ ID NO: 304), targeted to PTEN, had fasted plasma glucose levels of approximately 320 mg/dL at day 19 and 195 mg/dL at day 29. Mice treated with negative control oligonucleotide ISIS 141923 had fasted plasma glucose levels of approximately 300 mg/dL at day 19 and 260 mg/dL at day 29. Thus fasted plasma glucose levels were reduced after treatment with antisense inhibitors of glucocorticoid receptor and hypoglycemia was not seen.

Serum lipids in db/db mice were also measured at the end of the study. Cholesterol levels were approximately 170 mg/dL for saline treated mice, 125 mg/dL for ISIS 180272-treated mice, 120 mg/dL for ISIS 180280-treated mice, 150 mg/dL for ISIS 116847-treated mice and 195 mg/dL for ISIS 141923-treated mice. Triglycerides were approximately 220 mg/dL for saline treated mice, 95 mg/dL for ISIS 180272-treated mice, 105 mg/dL for ISIS 180280-treated mice, 105 mg/dL for ISIS 116847-treated mice and 245 mg/dL for ISIS 141923-treated mice. Serum lipids, especially triglycerides, were thus decreased in db/db mice treated with antisense inhibitors of glucocorticoid receptors.

A second experiment was conducted similarly to that described above in this example, with fed plasma glucose measurements conducted on days -1, 6, 13 and 26. In these db/db mice treated with ISIS 180272 (25 mg/kg x 2 weekly s.c.; SEQ ID NO: 69), an antisense inhibitor of glucocorticoid receptor, fed plasma glucose levels were approximately 260 mg/dL at day -1, 290 mg/dL at day 6, 405 mg/dL at day 13 and 305 mg/dL at day 26. In mice treated with ISIS 180280 (25 mg/kg x 2 weekly s.c.; SEQ ID NO: 77), another antisense inhibitor of glucocorticoid receptor, fed plasma glucose levels were approximately 260 mg/dL at day -1, 375 mg/dL at day 6, 345 mg/dL at day 13 and 355 mg/dL at day 26. Mice treated with saline alone had fed plasma glucose levels of approximately 260 mg/dL at day -1, 465 mg/dL at day 6, 480 mg/dL at day 13 and 510 mg/dL at day 26. db/db mice treated with a positive control oligonucleotide, ISIS 116847 (SEQ ID NO: 304), targeted to PTEN, had fed plasma glucose levels of approximately 250 mg/dL at day -1, 355 mg/dL at day 6, 325 mg/dL at day 13 and 225 mg/dL at day 26. Mice treated with negative control oligonucleotide ISIS 141923 had fed plasma glucose levels of approximately 260 mg/dL at day -1, 430 mg/dL at day 6, 402 mg/dL at day 13 and 480 mg/dL at day 26.

Serum lipids in db/db mice were also measured at the end of the study. Cholesterol levels were approximately 170 mg/dL for saline treated mice, 190 mg/dL for ISIS 180272-treated mice, 155 mg/dL for ISIS 180280-treated mice, 180 mg/dL for ISIS 116847-treated mice and 185 mg/dL for ISIS 141923-treated mice. Triglycerides were approximately 220 mg/dL for saline treated mice, 120 mg/dL for ISIS 180272-treated mice, 115 mg/dL for ISIS 180280-treated mice, 190 mg/dL for ISIS 116847-treated mice and 180 mg/dL for ISIS 141923-treated mice. Serum triglycerides were thus decreased in db/db mice treated with antisense inhibitors of glucocorticoid receptors.

Glucocorticoid receptor mRNA levels in db/db mouse livers were measured at the end of study using RiboGreen™ RNA quantification reagent (Molecular Probes, Inc. Eugene, OR) as above. Glucocorticoid receptor mRNA levels were reduced by approximately 53% in mice treated with ISIS 180272, and by approximately 65% in mice treated with ISIS 180280, when compared to saline treatment. Glucocorticoid receptor mRNA levels were not decreased in mice treated with the positive control oligonucleotide, ISIS 116847 or the negative control oligonucleotide, ISIS 141923.

Plasma corticosterone levels were measured in these mice as a marker for stimulation of the hypothalamic pituitary axis. Corticosterone levels (measured by ELISA kit, ALPCO, Windham NH, following manufacturer's instructions) were 180 ng/ml in mice treated with saline; 225 ng/ml in mice treated with the PTEN inhibitor ISIS 116847, 175 ng/ml in mice treated with the glucocorticoid receptor inhibitor ISIS 180272, 170 ng/ml in mice treated with the glucocorticoid receptor inhibitor ISIS 180280 and 220 ng/ml in mice treated with control oligonucleotide ISIS 141923.

### Example 24

### Effect of antisense inhibitors of glucocorticoid receptor on lean (ZDF +/-) rats

ZDF +/- rats are heterozygous littermates of Zucker Diabetic Fatty rats, often referred to as lean littermates because they do not display the impaired insulin sensitivity phenotype of the homozygous ZDF fa/fa rat. Homogygous ZDF rats harbor a mutation in the leptin receptor which makes them a useful animal model of impaired insulin sensitivity.

Six week old ZDF +/- (lean) male rats were dosed twice weekly with 37.5 mg/kg of antisense oligonucleotide, given subcutaneously. Oligonucleotides were prepared in buffered saline and filter-sterilized. A total of five doses were given. Glucocorticoid antisense oligonucleotides used were ISIS 180277 (SEQ ID NO: 74) and ISIS 180281 (SEQ ID NO: 78), targeted to rat glucocorticoid receptor. Plasma glucose levels were measured before the initial dose in week 1 and the day before the rats were sacrificed in week 3. After sacrifice, serum lipids were measured and target reduction in liver was also measured.

In lean ZDF +/- rats treated with ISIS 180277 (SEQ ID NO: 74), an antisense inhibitor of rat glucocorticoid receptor, plasma glucose levels were approximately 170 mg/dL at week 1 and 120 mg/dL at week 3. In lean ZDF +/- rats treated with ISIS 180281 (SEQ ID NO: 78), another antisense inhibitor of glucocorticoid receptor, plasma glucose levels were approximately 140 mg/dL at week 1 and 140 mg/dL at week 3. Lean rats treated with saline alone had fed plasma glucose levels of approximately 142 mg/dL at week 1 and 135 mg/dL at week 3. While plasma glucose levels decreased with 180277 treatment, the rats did not become hypoglycemic.

Serum lipids were also measured at the end of the study. Cholesterol levels were approximately 110 mg/dL for saline treated lean rats, 70 mg/dL for ISIS 180277-treated lean rats and 25 mg/dL for ISIS 180281-treated lean rats. Triglycerides were approximately 115 mg/dL for saline treated lean rats and substantially reduced to approximately 15 mg/dL for ISIS 180277-treated lean rats and 20 mg/dL for ISIS 180281-treated lean rats.

Glucocorticoid receptor mRNA levels in lean ZDF +/- rat livers were measured at the end of study using RiboGreen™ RNA quantification reagent (Molecular Probes, Inc. Eugene, OR) as taught in previous examples above. Glucocorticoid receptor mRNA levels were reduced by approximately 59% in rats treated with ISIS 180277, and by approximately 65% in rats treated with ISIS 180281, when compared to saline treatment.

### Example 25

### Effect of antisense inhibitors of glucocorticoid receptor on Zucker Diabetic Fatty (ZDF) rats

The leptin receptor deficient (fa/fa) Zucker diabetic fatty (ZDF) rat is another useful model for the investigation of type 2 diabetes. Diabetes develops spontaneously in these male rats at ages 8-10 weeks, and is associated with hyperphagia, polyuria, polydipsia, and impaired weight gain, symptoms which parallel the clinical symptoms of diabetes. Phillips MS, Liu Q, Hammond HA, Dugan V, Hey PJ, Caskey CJ, Hess JF, 1996, Nat Genet 13, 18-19.

Six week old ZDF male rats were subcutaneously injected with oligonucleotides at a dose of 37.5 mg/kg two times per week for 7 weeks. Glucocorticoid antisense oligonucleotides used were ISIS 180277 (SEQ ID NO: 74) and ISIS 180281 (SEQ ID NO: 78). ISIS 116847 (GCGACAGCTGCTCCACCTTC; SEQ ID NO: 304), targeted to rat, mouse and human PTEN, was used as a positive control and ISIS 141923 (CCTTCCCTGAAGGTTCCTCC; SEQ ID NO: 305), an unrelated oligonucleotide, was used as the negative oligonucleotide control. Saline-injected animals also serve as controls. Oligonucleotides were prepared in buffered saline and filter-sterilized.

In ZDF rats treated with ISIS 180277 (SEQ ID NO: 74), an antisense inhibitor of glucocorticoid receptor, fed plasma glucose levels were approximately 210 mg/dL at day -1, 280 mg/dL at day 6, 390 mg/dL at day 13, 395 mg/dL at day 26 and 420 mg/dL at day 40. In rats treated with ISIS 180281 (SEQ ID NO: 78), another antisense inhibitor of glucocorticoid receptor, fed plasma glucose levels were approximately 210 mg/dL at day -1, 290 mg/dL at day 6, 395 mg/dL at day 13, 410 mg/dL at day 26 and 435 mg/dL at day 40. In contrast, rats treated with saline alone had fed plasma glucose levels of approximately 210 mg/dL at day -1, 260 mg/dL at day 6, 405 mg/dL at day 13, 410 mg/dL at day 26 and 445 mg/dL at day 40. Rats treated with a positive control oligonucleotide, ISIS 116847 (SEQ ID NO: 304), targeted to PTEN, had fed plasma glucose levels of approximately 210 mg/dL at day -1, 190 mg/dL at day 6, 150 mg/dL at day 13, 110 mg/dL at day 26 and 130 mg/dL at day 40.

In fasted ZDF rats, plasma glucose levels were measured on day 21 (after a 16 hour fast) and day 33 (after a 12 hour fast). In rats treated with ISIS 180277 (SEQ ID NO: 74), an antisense inhibitor of glucocorticoid receptor, fasted plasma glucose levels were approximately 145 mg/dL at day 21 and 130 mg/dL at day 33. In mice treated with ISIS 180281 (SEQ ID NO: 78), another antisense inhibitor of glucocorticoid receptor, fasted plasma glucose levels were approximately 170 mg/dL at day 19 and 140 mg/dL at day 29. In contrast, rats treated with saline alone had fasted plasma glucose levels of approximately 270 mg/dL at day 19 and 255 mg/dL at day 29. Rats treated with a positive control oligonucleotide, ISIS 116847 (SEQ ID NO: 304), targeted to PTEN, had fasted plasma glucose levels of approximately 115 mg/dL at day 19 and 120 mg/dL at day 29. Thus fasted plasma glucose levels were reduced after treatment with antisense inhibitors of glucocorticoid receptor. Hypoglycemia was not observed.

Serum lipids in ZDF fa/fa rats were also measured at the end of the study. Cholesterol levels were approximately 190 mg/dL for saline treated rats, 130 mg/dL for ISIS 180277-treated rats, 70 mg/dL for ISIS 180281-treated rats and 140 mg/dL for ISIS 116847-treated rats. Triglycerides were approximately 520 mg/dL for saline treated rats, 360 mg/dL for ISIS 180277-treated rats, 125 mg/dL for ISIS 180281-treated rats and 910 mg/dL for ISIS 116847-treated rats. Thus both antisense inhibitors of glucocorticoid receptor had lipid-lowering effects.

Serum free fatty acids were also measured in ZDF rats after antisense treatment. Free fatty acids were approximately 0.68 mEq/l for saline-treated rats, 0.48 mEq/l for ISIS 180277-treated rats and 0.31 mEq/l for ISIS 180281-treated rats.

A reduction in epididymal fat pad weights by glucocorticoid receptor antisense oligonucleotide was also observed in ZDF rats (saline 3.8 ± 0.07 grams vs. antisense 2.6 ± 0.06 grams *p<0.05).* The effects of glucocorticoid receptor antisense inhibition were not accompanied by any changes in food intake or body weight in these animals. To understand the mechanism underlying the lipid lowering effects of the glucocorticoid receptor antisense oligonucleotide, the expression of several lipogenic genes was investigated in these models. Glucocorticoid receptor antisense treatment caused a reduction in the expression of 3-hydroxy-3-methylglutaryl-coenzyme A (HMG-CoA) reductase, a rate limiting enzyme in cholesterol biosynthesis, thus explaining in part the effects of the glucocorticoid receptor antisense compound on cholesterol levels. The expression of several other lipogenic genes, including squalene synthase, sterol regulatory element binding protein-1c (SREBP-1c), HMGCoA synthase, remained unchanged.

Liver enzymes (AST/ALT) were also measured in these rats as a marker for liver toxicity. Liver enzymes were not increased by antisense treatment and actually decreased compared to saline-treated animals.

Glucocorticoid receptor mRNA levels in ZDF fa/fa rat livers and white adipose tissue were measured at the end of study using RiboGreen™ RNA quantification reagent (Molecular Probes, Inc. Eugene, OR) as taught in previous examples above. Glucocorticoid receptor mRNA levels were reduced by approximately 60% in livers of rats treated with ISIS 180277, and by approximately 63% in rats treated with ISIS 180281, when compared to saline treatment. Glucocorticoid receptor mRNA levels were also reduced by approximately 60% in fat of rats treated with ISIS 180281.

### Example 26

### Effect of antisense inhibitors of glucocorticoid receptor in the High Fat Diet/Streptozotocin (HFD/STZ) rat model

The HFD/STZ rat model (based on Reed et al., 2000, Metabolism, 49, 1390-1394) closely mimics human type 2 diabetes with the dietary component induced by the high fat diet (also known as DIO or diet-induced obesity) and hyperglycemia induced by streptozotocin. Unlike the ob/ob and db/db models, the diabetes phenotype is not caused by mutations of either the leptin or leptin receptor gene.

Seven week old male Sprague Dawley rats (weighing 160-180 grams) were fed with high fat diet consisting of 40% fat, 41% carbohydrate and 18% protein (Harlan Teklad Adjusted Fat Diet 96132, Harlan Teklad, Madison WI) for 2 weeks. During this feeding schedule, animals were monitored for glucose, insulin, triglycerides, and free fatty acids from plasma samples obtained by tail bleeding. HFD treatment when compared to normal chow treatment, resulted in significant elevation of insulin, triglycerides and free fatty acids but no increase in plasma glucose indicating that the animals had acquired the insulin resistance phenotype, which was further confirmed by oral glucose tolerance test. In order to induce hyperglycemia, the rats were injected intraperitoneally with 50 mg/kg of freshly prepared STZ. At this dose, STZ causes a moderate destruction of insulin-producing β-cells in the pancreas resulting in hyperglycemia measured after 3 days. Animals with plasma glucose values ranging between 350-450 mg/dL were selected to test the therapeutic effects of antisense drugs. In the first study, antisense oligonucleotide inhibitors of PTEN and glucocorticoid receptor were tested in this model. These compounds were administered by subcutaneous route with doses 25 mg/kg twice weekly for 4 weeks. Oligonucleotides were prepared in buffered saline and filter-sterilized. Plasma glucose and triglycerides were measured weekly.

In HFD/STZ rats treated with ISIS 180281 (SEQ ID NO: 78), an antisense inhibitor of glucocorticoid receptor, fed plasma glucose levels were 450 mg/dL at week 1, 487 mg/dL at week 2, and 446 mg/dL at week 4. Rats treated with saline alone had fed plasma glucose levels of approximately 432 mg/dL at week 1, 470 mg/dL at week 2, and 487 mg/dL at week 4. Rats treated with a positive control oligonucleotide, ISIS 116847 (SEQ ID NO: 304), targeted to PTEN, had fed plasma glucose levels of 446 mg/dL at week 1, 552 mg/dL at week 2 and 398 mg/dL at week 4. Rats treated with negative control oligonucleotide ISIS 141923 had fed plasma glucose levels of approximately 443 mg/dL at week 1, 525 mg/dL at week 2 and 398 mg/dL at week 4.

In fasted HFD/STZ rats, plasma glucose levels were 100 mg/dL in rats treated with ISIS 180281 (SEQ ID NO: 78), an antisense inhibitor of glucocorticoid receptor, 320 mg/dL in rats treated with saline alone, 155 mg/dL in rats treated with a positive control oligonucleotide, ISIS 116847 (SEQ ID NO: 304), targeted to PTEN, and 225 mg/dL in rats treated with negative control oligonucleotide ISIS 141923. Thus fasted plasma glucose levels were reduced after treatment with an antisense inhibitor of glucocorticoid receptor.

Serum lipids in HFD/STZ rats were also measured at the end of the study. Cholesterol levels were approximately 100 mg/dL for saline treated rats, 50 mg/dL for ISIS 180281-treated rats, 75 mg/dL for ISIS 116847-treated rats and 95 mg/dL for ISIS 141923-treated rats. Triglycerides were approximately 230 mg/dL for saline treated rats, 30 mg/dL for ISIS 180281-treated rats, 125 mg/dL for ISIS 116847-treated rats and 175 mg/dL for ISIS 141923-treated rats. Thus antisense inhibition of glucocorticoid receptor had lipid-lowering effects. Glucocorticoid receptor antisense also significantly reduced plasma free fatty acids in HFD-STZ rats (saline 0.93 ± 0.12 mEQ/L vs. antisense 0.52 ± 0.04 mEQ/L, p<0.05).

Plasma corticosterone levels were measured in these STZ-HFD rats and were unchanged by treatment with antisense inhibitor of glucocorticoid receptor, compared to saline treatment.

A reduction in epididymal fat pad weights by glucocorticoid receptor antisense oligonucleotide was also observed in HFD-STZ rats (saline 2.41 ± 0.23 grams vs. antisense 1.8 ± 0.63 grams). The effects of glucocorticoid receptor antisense inhibition were not accompanied by any changes in food intake or body weight in these animals. To understand the mechanism underlying the lipid lowering effects of the glucocorticoid receptor antisense oligonucleotide, we investigated the expression of several lipogenic genes in these models. Glucocorticoid receptor antisense treatment caused a reduction in the expression of 3-hydroxy-3-methylglutaryl-coenzyme A (HMG-CoA) reductase, a rate limiting enzyme in cholesterol biosynthesis, thus explaining in part the effects of the glucocorticoid receptor antisense compound on cholesterol levels. The expression of several other lipogenic genes, including squalene synthase, sterol regulatory element binding protein-1c (SREBP-1c), HMGCoA synthase, remained unchanged.

Glucocorticoid receptor levels in liver were also measured following treatment. Compared to saline controls, glucocorticoid mRNA levels were reduced by 50% by ISIS 180281 (SEQ ID NO: 78) and were not reduced at all by ISIS 141923. The PTEN antisense oligonucleotide, ISIS 116847 caused no inhibition and in fact caused a slight increase in glucocorticoid receptor mRNA levels (121% of saline control).

Thus treatment with antisense to glucocorticoid receptor resulted in 50% reduction in glucocorticoid receptor expression in the liver, approximately 50% decrease in fasted glucose and 68% reduction in plasma glucose. These results are consistent with the data that were obtained in Zucker Diabetic Fatty (ZDF) rat model.

### Example 27

### Effects of antisense inhibition of glucocorticoid receptor on hepatic and systemic response to glucocorticoids

Glucocorticoid receptor antagonists that have systemic inhibitory effects can cause undesirable side effects, such as stimulation of the hypothalamic pituitary axis. A significant advantage of antisense inhibitors is that reduced expression of the glucocorticoid receptor by antisense oligonucleotides is observed to a large extent in liver and fat, which are desirable target tissues for inhibition of glucocorticoid receptor, but to a lesser extent systemically. Thus there is believed to be reduced likelihood of undesirable systemic side effects (primarily stimulation of the hypothalamic pituitary axis) with oligonucleotide inhibitors, in comparison to other classes of inhibitors of this target.

To confirm that glucocorticoid receptor expression could be modulated in the liver without being modulated in the pituitary, ZDF rats were treated with the antisense inhibitor of glucocorticoid receptor (ISIS 180281) at 37.5 mg/kg administered subcutaneously twice a week for 5 weeks. RNA was isolated from the pituitary gland and glucocorticoid receptor levels were measured by Ribogreen™ analysis as described in previous examples. In rats treated with antisense to glucocorticoid receptor, levels of glucocorticoid receptor in the pituitary were identical to those in saline-treated rats. Thus it can be shown that antisense inhibition of this target can be achieved in specific organs (e.g., liver) which would lead to desired effects, without inhibition in undesirable site of inhibition for this target (e.g., pituitary). Pituitary gland proopiomelanocortin (POMC-1) RNA expression was also measured by RT-PCR and was not significantly affected by antisense inhibitors of glucocorticoid receptor expression.

Corticosterone levels were also measured as a marker for stimulation of the hypothalamic pituitary axis. No significant changes in corticosterone levels were seen (35 ng/ml for ZDF rats treated with antisense to glucocorticoid receptor, vs 45 ng/ml for saline treated rats and 30 ng/ml for control oligonucleotide-treated rats). Corticosterone levels were similarly found to be unchanged in mice treated with antisense inhibitors of glucocorticoid receptor.

To further test this hypothesis, normal male Sprague Dawley rats were treated with antisense inhibitor of glucocorticoid receptor (ISIS 180281; SEQ ID NO: 78) or a control oligonucleotide (ISIS 141923; SEQ ID NO: 305) at a dose of 50 mg/kg twice a week for 4 weeks. Subsequently, the animals were fasted overnight and were injected with saline or dexamethasone (4 mg/kg i.p.). Four hours after the injection, the animals were sacrificed and liver tissue was harvested to examine changes in tyrosine aminotransferase/TAT mRNA expression (a steroid responsive gene that was used as a marker of hepatic steroid activity) by RT-PCR. In addition, blood was sampled for measurement of circulating lymphocytes, which is a marker of systemic glucococorticoid effects. As expected, dexamethasone caused a significant increase in TAT expression and a decrease in circulating lymphocytes (i.e., lymphopenia). Treatment with the glucocorticoid receptor antisense inhibitor led to about a 75% reduction in hepatic glucocorticoid receptor expression, which was accompanied by a prevention of dexamethasone induced increase in TAT expression. However, no effect was observed on the dexamethasone-induced decrease in circulating lymphocytes (i.e., no lymphopenia).

These results along with the lack of effect on corticosterone levels in the previous example indicate that antisense inhibition of glucocorticoid receptor expression leads to functional antagonism of glucocorticoid effects in the liver without altering systemic glucocorticoid effects.

Levels of plasma adrenocorticotropic hormone (ACTH) were also examined after dexamethasone challenge. ACTH levels were measured using an ELISA kit (ALPCO, Windham NH) according to manufacturer's instructions. Glucocorticoid antisense oligonucleotide (ISIS 180281) did not affect basal ACTH levels (saline 9.79 pg/ml vs ASO 9.96 pg/ml). ISIS 180281 also reduced dexamethasone-induced PEPCK expression in the liver. Liver glycogen after dexamethasone challenge was also changed by antisense treatment with ISIS 180281. In mice treated with ISIS 180281, liver glycogen levels were approximately 1.5 mg/g in animals given dexamethasone challenge, compared to approx 1 mg/g in animals given saline in place of dexamethasone. In mice treated with the control oligonucleotide ISIS 141923, liver glycogen levels were approximately 13.5 mg/g in animals given dexamethasone challenge, compared to approximately 4 mg/g in animals given saline in place of dexamethasone. In mice treated with saline (no oligonucleotide), liver glycogen levels were approximately 9 mg/g in animals given dexamethasone challenge, compared to approx 3 mg/g in animals given saline in place of dexamethasone.

### Example 28

### Effect of antisense inhibitors of glucocorticoid receptor on diet-induced obesity in rats

Antisense inhibitors of glucocorticoid receptor expression are expected to reduce obesity or weight gain. This is tested in the high fat diet (HFD) model, also known as the DIO (diet-induced obesity) model.

Seven week old male Sprague Dawley rats (weighing 160-180 grams) are fed with high fat diet consisting of 40% fat, 41% carbohydrate and 18% protein (Harlan Teklad Adjusted Fat Diet 96132) for 2 weeks. During this feeding schedule, animals are weighed at regular intervals.

Mice treated with an antisense inhibitor of glucocorticoid receptor gained less weight on the high fat diet than did saline-treated animals. Therefore the antisense-treated mice are less likely to be obese.

### Example 29

### Glucocorticoid receptor antisense inhibition decreases hepatic glucose production and gluconeogenesis

Ex vivo hepatic glucose production was measured in liver slices from Sprague Dawley rats treated with glucocorticoid receptor antisense oligonucleotide (ISIS 180281). Control and antisense-treated rats were fasted for 24 hours and administered either vehicle or dexamethasone (Bausch & Lomb, Tampa, FL) at a dose of 12.5 mg/kg. Six hours after treatment, precision-cut liver slices were prepared using a Krumdieck

Tissue Slicer (Alabama Research and Development, Munford, AL), and incubated in glucose-free DMEM (Invitrogen, Carlsbad, CA) supplemented with 0.1% BSA, 10 mM lactate, 1 mM sodium pyruvate, 10 mM alanine, and 10 mM glycerol (NGS medium). After a 1 hour pre-incubation, individual slices were transferred to separate wells of a 24-well plate containing 0.5 ml NGS medium, and glucose released into the medium after 1.5 hours was determined by a Hitachi 912 clinical chemistry analyzer. Liver slices were weighed, and glucose production per milligram of liver tissue was determined.

Rats treated with antisense to glucocorticoid receptor (ISIS 180281) showed a significant reduction in basal glucose production (control antisense compound 0.86 ± 0.16 vs. 0.35 ± 0.01 glucose (g)/hour/liver slice (mg), *p*<*0.05*). To directly evaluate the effects of the antisense compound on glucocorticoid-mediated glucose production, assays were performed in antisense-treated rats that were injected with dexamethasone 6 hours prior to necropsy. Glucocorticoid receptor antisense compound dramatically inhibited dexamethasone-induced glucose production (control antisense +dexamethasone 5.61 ± 0.68 vs. glucocorticoid receptor antisense +dexamethasone 0.61 ± 0.04 glucose (g)/hour/liver slice (mg), *p*<*0.05*).

### Example 30

### Additional cross-species glucocorticoid receptor antisense oligonucleotides

Additional candidate antisense inhibitors of mouse glucocorticoid receptor were evaluated in comparison to previously screened oligonucleotides for ability to reduce expression of glucocorticoid receptor mRNA in vivo in various species. This is shown in Table 7. Shown are oligonucleotide sequence and position (position of 5' most nucleobase) on SEQ ID NO: 4 (human glucocorticoid receptor mRNA, GenBank accession no. NM_000176.1). Also shown is complementarity to human, cynomolgus monkey, rat and mouse glucocorticoid receptor mRNA ("yes" means perfect complementarity, and "1 mm" means one mismatch from perfect complementarity).

All compounds shown are chimeric oligonucleotides ("gapmers") 20 nucleotides in length, composed of a central "gap" region consisting often 2'-deoxynucleotides, which is flanked on both sides (5' and 3' directions) by five-nucleotide "wings". The wings are composed of 2'-methoxyethyl (2'-MOE) nucleotides. The internucleoside (backbone) linkages are phosphorothioate (P=S) throughout the oligonucleotide. All cytosine residues are 5-methylcytosines.

**Table 7**

| **Glucocorticoid receptor cross-species oligonucleotides** | | | | | | | |
|---|---|---|---|---|---|---|---|
| ISIS # | SEQ ID NO | Sequence | Pos'n on SEQ ID NO: 4 | Perfect complement to: | | | |
| | | | | Human | Monkey | Rat | Mouse |
| 361137 | 306 | cgacctattgaggtttgcaa | 509 | yes | yes | yes | yes |
| 180276 | 73 | ctttggtctgtggtatacaa | 679 | yes | 1 mm | 1 mm | yes |
| 345198 | 307 | gtcaaaggtgctttggtctg | 689 | yes | yes | yes | yes |
| 180304 | 100 | agcatgtgtttacattggtc | 2053 | yes | yes | yes | yes |
| 180275 | 72 | ctgtggtatacaatttcaca | 672 | yes | 1 mm | 1 mm | yes |
| 361141 | 308 | gcagacattttattaccaat | 1066 | yes | yes | yes | 1 mm |
| 180281 | 78 | gcccagtttctcttgcttaa | 1007 | yes | yes | yes | yes |
| 361151 | 309 | gtacatctgtcctccagagg | 1109 | yes | yes | yes | yes |
| 180274 | 71 | ctggtcgacctattgaggtt | 514 | yes | yes | yes | yes |
| 361156 | 310 | gctgtattcatgtcatagtg | 1129 | yes | yes | yes | yes |

These compounds were tested at a range of doses in cynomolgus monkey and rat primary hepatocytes as well as human cells for inhibition of glucocorticoid receptor expression. Experiments were done twice. IC50s were calculated and are shown in Table 8.

**Table 8**

| **Glucocorticoid receptor cross species oligonucleotides-IC50s (nM)** | | | | | | | | | | |
|---|---|---|---|---|---|---|---|---|---|---|
| **ISIS #** | **SEQ ID NO** | | **Rat expt 1 IC50** | **Rat expt 2 IC50** | | **Monkey expt 1 IC50** | **Monkey expt 2 IC50** | | **Human expt 1 IC50** | **Human expt 2 IC50** |
| 361137 | 306 | | 19 | 26 | | 12 | 11 | | 5 | 9 |
| 180276 | 73 | | 29 | 38 | | 27 | 24 | | 5 | 6 |
| 345198 | 307 | | 22 | 25 | | 35 | 31 | | 6 | 11 |
| 180304 | 100 | | 32 | 39 | | 29 | 28 | | 7 | 6 |
| 180275 | 72 | | 32 | 41 | | 35 | 41 | | 9 | 12 |
| 361141 | 308 | | 21 | 26 | | 15 | 12 | | 11 | 15 |
| 180281 | 78 | | 21 | 28 | | 24 | 22 | | 11 | 14 |
| 361151 | 309 | | 37 | 51 | | 135 | 128 | | 12 | 11 |
| 180274 | 71 | | 24 | 34 | | 105 | 115 | | 22 | 24 |
| 361156 | 310 | | 22 | 25 | | 169 | 158 | | 36 | 31 |

Five of these compounds (ISIS 180281, ISIS 180304, ISIS 345198, ISIS 361137 and ISIS 361141) were further tested at various doses in lean (nondiabetic) rats for their ability to reduce glucocorticoid receptor RNA levels in liver. Results are shown in Tables 9a and 9b (separate experiments). Liver enzyme (AST/ALT) levels were also measured in these rats, as a measure of oligonucleotide hepatotoxicity. With the exception of the 50 mg/kg dose of ISIS 180281, none of these compounds caused a significant increase in AST or ALT levels at any dose tested. Rats given a 50 mg/kg dose of ISIS 180281 had both AST and ALT levels nearly twice those of control-treated rats.

**Table 9a**

| **Rat lean screen- glucocorticoid receptor antisense oligonucleotides** | | | |
|---|---|---|---|
| **Compound** | **% reduction in glucocorticoid receptor mRNA in rat liver after antisense treatment at doses shown (compared to saline)** | | |
| | 50 mg/kg | 25 mg/kg | 12.5 mg/kg |
| ISIS 180281 | 68 | 65 | 48 |
| ISIS 180304 | 52 | 34 | 0 |
| ISIS 345198 | 63 | 58 | 52 |

**Table 9b**

| **Rat lean screen- glucocorticoid receptor antisense oligonucleotides** | | | |
|---|---|---|---|
| **Compound** | **% reduction in glucocorticoid receptor mRNA in rat liver after antisense treatment at doses shown (compared to saline)** | | |
| | 50 mg/kg | 25 mg/kg | 12.5 mg/kg |
| ISIS 180281 | 62 | 62 | 59 |
| ISIS 361137 | 59 | 47 | 32 |
| ISIS 361141 | 61 | 49 | 22 |

ISIS 345198 (GTCAAAGGTGCTTTGGTCTG; SEQ ID NO: 307) was chosen for further evaluation in mouse models of diabetes. This compound is perfectly complementary to mouse, rat, human, monkey, rabbit and guinea pig glucocorticoid receptor RNA.

### Example 31

### Additional studies showing that glucocorticoid receptor antisense treatment reduces glucocorticoid receptor expression in vivo

The effects of the lead murine glucocorticoid receptor antisense oligonucleotides on glucocorticoid receptor mRNA levels in murine models of type 2 diabetes were examined. After four weeks of systemic administration of glucocorticoid receptor antisense oligonucleotide (ISIS 345198) to ob/ob mice, a dose-dependent reduction of hepatic glucocorticoid receptor mRNA expression was observed. Oligonucleotide was administered s.c. twice a week for four weeks at doses of 6.25 mg/kg, 12.5 mg/kg or 25 mg/kg. Glucocorticoid receptor mRNA levels were reduced by 54%, 67% and 72%, respectively, at these doses, indicating a dose-dependent inhibition of glucocorticoid receptor expression. Control oligonucleotide had no effect on glucocorticoid receptor expression.

### Example 32

### Glucocorticoid receptor antisense treatment with ISIS 345198 lowers plasma glucose levels in ob/ob mice

In addition to reducing the level of glucocorticoid receptor mRNA, glucocorticoid receptor antisense oligonucleotide treatment decreased plasma glucose and circulating lipid levels in diabetic models. In saline and control antisense compound-treated ob/ob mice, hyperglycemia continued to worsen throughout the study duration, whereas ISIS 345198-treated animals showed a significant dose-dependent reduction in plasma glucose levels. After 4 weeks of treatment, plasma glucose levels were approximately 225 mg/dL, 250 mg/dL, and 300 mg/dL for mice treated with ISIS 345198 at 25 mg/kg, 12.5 mg/kg and 6.25 mg/kg, respectively. After 4 weeks of saline treatment, mice had plasma glucose levels of approximately 600 mg/dL and mice treated with control oligonucleotide had plasma glucose levels of approximately 490 mg/dL.

Due to the role of glucocorticoid receptor in regulating gluconeogenesis, the effects of glucocorticoid receptor antisense compound on fasted glucose levels were examined. A significant reduction in fasted glucose levels was observed in ob/ob mice (saline 321 ± 16.2 mg/dL vs. ISIS 345198 -treated 220 ± 8.3 mg/dL, *p<0.05)* and db/db mice (saline 320 ± 26.9 mg/dL vs. ISIS 180272-treated 204 ± 24.6 mg/dL, *p<0.05).* In both models, control antisense compound did not show significant effect on fasted glucose levels. The effects of glucocorticoid receptor antisense inhibition were not accompanied by changes in food intake, body weight or liver glycogen level, measured as described in Desai et al., 2001, Diabetes, 50, 2287-2295 (briefly, liver samples were homogenized in 0.03 N HCl to a final concentration of 0.5 g/ml. The homogenate (100 µl) was mixed with 400 µl of 1.25 N HCl and heated for 1 h at 100°C. Samples were centrifuged at 14,000 rpm, and 10 µl of supernatant was mixed with 1 ml of glucose oxidase reagent (Sigma). After a ten-minute incubation at 37°C, the absorbance was read at 505 nm).

### Example 33

### Effect of glucocorticoid receptor antisense oligonucleotide on body composition, plasma resistin, adiponectin, TNF alpha, insulin, and IL-6 levels in ob/ob mice treated with ISIS 345198

Although no change in body weight was observed, densitometric analysis of body composition was performed to accurately measure changes in body fat mass. Body composition analysis was conducted using Lunar X-ray densitometer (GE Medical Systems, Madison, WI 53717) in ob/ob mice that were treated with glucocorticoid receptor antisense oligonucleotide (ISIS 345198), 25 mg/kg twice a week for four weeks. Glucocorticoid receptor antisense compound significantly reduced body fat mass after a 4-week treatment period (saline 50.7 ± 0.4 vs. glucocorticoid receptor antisense 45.7 ± 0.5, *p<0.05).* This reduction was also reflected as a decrease in epididymal fat pad weight (saline 5.09 ± 0.10 grams vs. glucocorticoid receptor antisense 4.3 ± 0.12 grams, *p*<*0.05*). Plasma adiponectin, resistin, TNF-alpha and insulin levels were measured by ELISA using kits from Linco Research (St. Charles, MO and ALPCO. Windham, NH); plasma interleukin-6 (IL-6) levels were measured by ELISA (R&D Systems, Minneapolis, MN).

The decrease in adiposity was accompanied by a 20% decrease in plasma resistin levels (saline 22 ± 1.4 ng/ml vs. glucocorticoid receptor antisense 18 ± 0.94 ng/ml), whereas adiponectin levels remained unchanged (saline 8.73 ± 0.14 ug/ml vs. 8.72 ± 0.35 ug/ml). A more robust effect on lowering of TNF alpha (saline 42 ± 2.29 pg/ml vs. glucocorticoid receptor antisense 27 ± 0.86 pg/ml, *p<0.05*) and insulin levels (saline 43 ± 5.95 ng/ml vs. glucocorticoid receptor antisense 16 ± 1.43 ng/ml, *p<0.05*) was observed in glucocorticoid receptor antisense-treated mice. Glucocorticoid receptor antisense compound treatment did not result in any significant change in the circulating IL-6 levels (saline 6.27 ± 0.74 pg/ml vs. glucocorticoid receptor antisense-treated 5.37 ± 1.22 pg/ml)

In a separate study, lean, normoglycemic mice received the glucocorticoid receptor antisense compound (ISIS 345198) at 50 mg/kg/week for six weeks. Glucocorticoid receptor inhibition caused a significant reduction in glucocorticoid receptor mRNA expression in the liver (saline 100 ± 5.98 vs. glucocorticoid receptor antisense treated 24.4 ± 2, *p<0.05)* and white adipose tissue (saline 100 ± 4 vs. glucocorticoid receptor antisense 31 ± 6, *p<0.05*) without causing hypoglycemia (24h fasted levels, saline 147 ± 8 mg/dL vs. glucocorticoid receptor antisense 112 ± 5 mg/dL).
1. An antisense compound 8 to 80 nucleobases in length targeted to a nucleic acid molecule encoding glucocorticoid receptor, wherein said compound is complementary to said nucleic acid molecule encoding glucocorticoid receptor, and wherein said compound inhibits the expression of glucocorticoid receptor mRNA.
2. The antisense compound of embodiment 1 which is 13 to 50 nucleobases in length.
3. The antisense compound of embodiment 1 which is 15 to 30 nucleobases in length.
4. The antisense compound of embodiment 1 comprising an oligonucleotide.
5. The antisense compound of embodiment 1 comprising a DNA oligonucleotide.
6. The antisense compound of embodiment 1 comprising an RNA oligonucleotide.
7. The antisense compound of embodiment 1 comprising a double-stranded RNA oligonucleotide.
8. The antisense compound of embodiment 1 comprising a chimeric oligonucleotide.
9. The antisense compound of embodiment 1 wherein at least a portion of said compound hybridizes with RNA to form an oligonucleotide-RNA duplex.
10. The antisense compound of embodiment 1 having at least 90% complementarity with said nucleic acid molecule encoding glucocorticoid receptor.
11. The antisense compound of embodiment 1 having at least 95% complementarity with said nucleic acid molecule encoding glucocorticoid receptor.
12. The antisense compound of embodiment 1 having at least 99% complementarity with said nucleic acid molecule encoding glucocorticoid receptor.
13. The antisense compound of embodiment 1 having at least one modified internucleoside linkage, sugar moiety, or nucleobase.
14. The antisense compound of embodiment 1 having at least one 2'-O-methoxyethyl sugar moiety.
15. The antisense compound of embodiment 1 having at least one phosphorothioate internucleoside linkage.
16. The antisense compound of embodiment 1 wherein at least one cytosine is a 5-methylcytosine.
17. The antisense compound of embodiment 1 having at least one Locked Nucleic Acid (LNA) sugar moiety, ENA or PNA.
18. The antisense compound of embodiment 1, wherein said antisense compound comprises at least an 8-nucleobase portion of SEQ ID NO 32, 33, 34, 36, 37, 39, 40, 43, 44, 46, 48, 50, 51, 52, 53, 54, 55, 57, 58, 59, 60, 61, 62, 63, 65, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 104, 105, 106, 107, 108, 109, 110, 111, 112, 113, 114, 115, 116, 117, 118, 119, 120, 121, 122, 123, 124, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 166, 167, 168, 169, 170, 171, 172, 173, 174, 175, 176, 177, 179, 182, 183, 185, 186, 188, 189, 190, 191, 192, 193, 194, 195, 196, 197, 198, 199, 200, 202, 203, 204, 206, 208, 209, 210, 211, 212, 213, 214, 216, 221, 222, 223, 224, 225, 226, 227, 228, 232, 233, 234, 236, 237, 239, 240, 241, 242, 243, 244, 245, 246, 247, 248, 249, 250, 251, 252, 253, 254, 257, 258, 259, 260, 261, 262, 263, 264, 265, 266, 267, 268, 269, 270, 271, 272, 273, 274, 275, 276, 280, 281, 282, 283, 284, 285, 286, 287, 291, 292, 293, 294, 295, 298, 299, 306, 307, 308, 309 or 310.
19. The antisense compound of embodiment 1, wherein said antisense compound has a sequence selected from the group consisting of 32, 33, 34, 36, 37, 39, 40, 43, 44, 46, 48, 50, 51, 52, 53, 54, 55, 57, 58, 59, 60, 61, 62, 63, 65, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 104, 105, 106, 107, 108, 109, 110, 111, 112, 113, 114, 115, 116, 117, 118, 119, 120, 121, 122, 123, 124, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 166, 167, 168, 169, 170, 171, 172, 173, 174, 175, 176, 177, 179, 182, 183, 185, 186, 188, 189, 190, 191, 192, 193, 194, 195, 196, 197, 198, 199, 200, 202, 203, 204, 206, 208, 209, 210, 211, 212, 213, 214, 216, 221, 222, 223, 224, 225, 226, 227, 228, 232, 233, 234, 236, 237, 239, 240, 241, 242, 243, 244, 245, 246, 247, 248, 249, 250, 251, 252, 253, 254, 257, 258, 259, 260, 261, 262, 263, 264, 265, 266, 267, 268, 269, 270, 271, 272, 273, 274, 275, 276, 280, 281, 282, 283, 284, 285, 286, 287, 291, 292, 293, 294, 295, 298, 299, 306, 307, 308, 309 and 310.
20. An antisense compound having a nucleobase sequence of SEQ ID NO: 36, 50, 60 71, 72, 73, 78, 100, 306, 307, 308, 309 or 310.
21. An antisense compound 8 to 80 nucleobases in length targeted to a nucleic acid molecule encoding human glucocorticoid receptor, wherein said compound is targeted to nucleotides 13-119 in the 5' UTR, nucleotides 114-151 in the start codon region, nucleotides 351-533, 667-845, 877-1243, 1356- 1488, 1552-1756, 1819-1999, 2008-2139, 2146-2194, 2201-2301, or 2386-2416 in the coding region or nucleotides 2488-2685, 2723-3435 ,3499-3789, 3826-3860, 3886-3905, 3918-3937, 4031-4072, 4082- 4193 or 4244-4758 in the 3' UTR, all of SEQ ID NO:4; or nucleotides 104562-104648 in the 3' UTR of SEQ ID NO:25.
22. An antisense compound 8 to 80 nucleobases in length targeted to a nucleic acid molecule encoding mouse glucocorticoid receptor, wherein said compound is targeted to nucleotides 2-20 in the start codon region, 301-1405, 1459-2043 or 2050-2309 in the coding region, nucleotides 2376-2433 or 2521-2546 in the 3' UTR, all of SEQ ID NO: 11; nucleotides 227-297 in the 5' UTR of SEQ ID NO:219; or nucleotides 14909-18389 in the 3' UTR of SEQ ID NO:220.
23. An antisense compound 8 to 80 nucleobases in length targeted to a nucleic acid molecule encoding rat glucocorticoid receptor, wherein said compound is targeted to nucleotides 150-2129 or 2136-2395 in the coding region, or nucleotides 2472-3705, 4576-4867, 5039-5293, 5680-5877 or 6214- 6263 in the 3' UTR, all of SEQ ID NO: 18; or nucleotides 278-304 in the coding region of SEQ ID NO:256.
24. A method of inhibiting the expression of glucocorticoid receptor in a cell or tissue comprising contacting said cell or tissue with the antisense compound of embodiment 1 so that expression of glucocorticoid receptor is inhibited.
25. A method of treating an animal having a disease or condition associated with glucocorticoid receptor comprising administering to said animal a therapeutically or prophylactically effective amount of the antisense compound of embodiment 1 so that expression of glucocorticoid receptor is inhibited.
26. The method of embodiment 25 wherein the disease or condition is diabetes, obesity, metabolic syndrome X, hyperglycemia or hyperlipidemia.
27. The method of embodiment 26 wherein said diabetes is Type 2 diabetes.
28. The method of embodiment 26 wherein said hyperlipidemia is high blood cholesterol levels or high blood triglyceride levels.
29. The method of embodiment 26 wherein said inhibition of expression of glucocorticoid receptor is in the liver or fat.
30. The method of embodiment 25 wherein glucocorticoid receptor expression in the pituitary is not inhibited.
31. The method of embodiment 25 wherein basal levels of plasma corticosterone and plasma ACTH in the animal are unaffected by antisense treatment.
32. A method of decreasing blood glucose levels in an animal comprising administering to said animal a therapeutically or prophylactically effective amount of an antisense compound 8 to 80 nucleobases in length targeted to a nucleic acid molecule encoding glucocorticoid receptor, wherein said antisense compound inhibits the expression of glucocorticoid receptor.
33. The method of embodiment 32 wherein the animal is a human or a rodent.
34. The method of embodiment 32 wherein the blood glucose levels are plasma glucose levels or serum glucose levels.
35. The method of embodiment 32 wherein the animal is a diabetic animal.
36. The method of embodiment 32 wherein blood glucose levels are decreased without occurrence of lymphopenia.
37. The method of embodiment 32 wherein basal levels of plasma corticosterone and plasma ACTH in the animal are unaffected by antisense treatment.
38. A method of preventing or delaying the onset of a disease or condition associated with glucocorticoid receptor in an animal comprising administering to said animal a therapeutically or prophylactically effective amount of an antisense compound 8 to 80 nucleobases in length targeted to a nucleic acid molecule encoding glucocorticoid receptor, wherein said antisense compound inhibits the expression of glucocorticoid receptor.
39. The method of embodiment 38 wherein the animal is a human or a rodent.
40. The method of embodiment 38 wherein the disease or condition is a metabolic disease or condition.
41. The method of embodiment 40 wherein the metabolic disease or condition is diabetes, obesity, hyperlipidemia, hyperglycemia or metabolic syndrome X.
42. The method of embodiment 41 wherein said diabetes is Type 2 diabetes.
43. The method of embodiment 41 wherein said hyperlipidemia is high blood cholesterol or high blood triglyceride levels.
44. The method of embodiment 38 wherein the onset of said disease or condition is prevented or delayed without occurrence of lymphopenia.
45. The method of embodiment 38 wherein basal levels of plasma corticosterone and plasma ACTH in the animal are unaffected by antisense treatment.
46. A method of preventing or delaying the onset of an increase in blood glucose levels in an animal comprising administering to said animal a therapeutically or prophylactically effective amount of an antisense compound 8 to 80 nucleobases in length targeted to a nucleic acid molecule encoding glucocorticoid receptor, wherein said antisense compound inhibits the expression of glucocorticoid receptor.
47. The method of embodiment 46 wherein the animal is a human or a rodent.
48. The method of embodiment 46 wherein the animal is obese.
49. The method of embodiment 46 wherein the animal is insulin-resistant as compared to a normal animal.
50. The method of embodiment 46 wherein the blood glucose levels are plasma glucose levels or serum glucose levels.
51. The method of embodiment 46 wherein the animal is a diabetic animal.
52. The method of embodiment 46 wherein the onset of said increase in blood glucose levels is prevented or delayed without occurrence of lymphopenia.
53. The method of embodiment 46 wherein basal levels of plasma corticosterone and plasma ACTH in the animal are unaffected by antisense treatment.
54. A method of decreasing blood lipid levels in an animal comprising administering to said animal a therapeutically or prophylactically effective amount of an antisense compound 8 to 80 nucleobases in length targeted to a nucleic acid molecule encoding glucocorticoid receptor, wherein said antisense compound inhibits the expression of glucocorticoid receptor.
55. The method of embodiment 54 wherein the animal is a human or a rodent.
56. The method of embodiment 54 wherein the blood lipid levels are blood cholesterol levels or blood triglyceride levels.
57. The method of embodiment 54 wherein the animal is a diabetic animal.
58. The method of embodiment 54 wherein said blood lipid levels are decreased without the occurrence of lymphopenia.
59. The method of embodiment 54 wherein basal levels of plasma corticosterone and plasma ACTH in the animal are unaffected by antisense treatment.
60. A method of preventing or delaying the onset of an increase in blood lipid levels in an animal comprising administering to said animal a therapeutically or prophylactically effective amount of an antisense compound 8 to 80 nucleobases in length targeted to a nucleic acid molecule encoding glucocorticoid receptor, wherein said antisense compound inhibits the expression of glucocorticoid receptor.
61. The method of embodiment 60 wherein the animal is a human or a rodent.
62. The method of embodiment 60 wherein the animal is obese.
63. The method of embodiment 60 wherein the animal is insulin-resistant as compared to a normal animal.
64. The method of embodiment 60 wherein the blood lipid levels are blood cholesterol levels or blood triglyceride levels.
65. The method of embodiment 60 wherein the animal is a diabetic animal.
66. The method of embodiment 60 wherein the onset of said increase in blood lipid levels is prevented or delayed without the occurrence of lymphopenia.
67. The method of embodiment 60 wherein basal levels of plasma corticosterone and plasma ACTH in the animal are unaffected by antisense treatment.
68. A method of decreasing glucocorticoid receptor expression in the liver or fat but not the pituitary gland of an animal comprising administering to said animal a therapeutically or prophylactically effective amount of a compound which inhibits the expression of glucocorticoid receptor in the liver or fat but not the pituitary gland.
69. The method of embodiment 68 wherein said compound is an antisense compound 8 to 80 nucleobases in length targeted to a nucleic acid molecule encoding glucocorticoid receptor.
70. The method of embodiment 68 wherein basal levels of plasma corticosterone and plasma ACTH in the animal are unaffected by antisense treatment.
71. A method of decreasing body fat mass in an animal comprising administering to said animal a therapeutically or prophylactically effective amount of an antisense compound 8 to 80 nucleobases in length targeted to a nucleic acid molecule encoding glucocorticoid receptor, wherein said antisense compound inhibits the expression of glucocorticoid receptor.
72. The method of embodiment 71 wherein the animal is a human or a rodent.
73. The method of embodiment 71 wherein said body fat mass is decreased without the occurrence of lymphopenia.
74. The method of embodiment 71 wherein basal levels of plasma corticosterone and plasma ACTH in the animal are unaffected by antisense treatment.
75. A method of preventing or delaying the onset of an increase in body fat mass in an animal comprising administering to said animal a therapeutically or prophylactically effective amount of an antisense compound 8 to 80 nucleobases in length targeted to a nucleic acid molecule encoding glucocorticoid receptor, wherein said antisense compound inhibits the expression of glucocorticoid receptor.
76. The method of embodiment 75 wherein the animal is a human or a rodent.
77. The method of embodiment 75 wherein the onset of said increase in body fat mass is prevented or delayed without the occurrence of lymphopenia.
78. The method of embodiment 75 wherein basal levels of plasma corticosterone and plasma ACTH are unaffected by antisense treatment.

## Claims

1. An antisense oligonucleotide 15 to 30 nucleobases in length targeted to a nucleic acid molecule encoding glucocorticoid receptor, wherein said oligonucleotide is complementary to said nucleic acid molecule encoding glucocorticoid receptor, and wherein said oligonucleotide inhibits the expression of glucocorticoid receptor mRNA.

2. The antisense oligonucleotide of claim 1, wherein said oligonucleotide is targeted to:
(i) an intron of a nucleic acid molecule encoding glucocorticoid receptor;
(ii) an intron-exon junction of a nucleic acid molecule encoding glucocorticoid receptor; or
(iii) an exon-intron junction of a nucleic acid molecule encoding glucocorticoid receptor.

3. The antisense oligonucleotide of claim 1, wherein said oligonucleotide is targeted to:
(i) the 5' untranslated region (5' UTR) of a nucleic acid molecule encoding glucocorticoid receptor;
(ii) the start codon region of a nucleic acid molecule encoding glucocorticoid receptor;
(iii) the coding region of a nucleic acid molecule encoding glucocorticoid receptor;
(iv) the stop codon region of a nucleic acid molecule encoding glucocorticoid receptor; or
(v) the 3' UTR of a nucleic acid molecule encoding glucocorticoid receptor.

4. The antisense oligonucleotide of any preceding claim, having at least 90% complementarity, at least 95% complementarity, at least 99% complementarity, or 100% complementarity, with said nucleic acid molecule encoding glucocorticoid receptor.

5. The antisense oligonucleotide of any of claims 1-4, wherein the oligonucleotide is a chimeric oligonucleotide.

6. The antisense oligonucleotide of any of claims 1-4, wherein the oligonucleotide has at least one modified internucleoside linkage, sugar moiety, or nucleobase.

7. The antisense oligonucleotide of any of claims 1-4, having at least one 2'-O-methoxyethyl sugar moiety.

8. The antisense oligonucleotide of any of claims 1-4, having at least one phosphorothioate internucleoside linkage.

9. The antisense oligonucleotide of any of claims 1-4, wherein at least one cytosine is a 5-methylcytosine.

10. The antisense oligonucleotide of any of claims 1-4, having at least one Locked Nucleic Acid (LNA) sugar moiety, ENA or PNA.

11. A method of inhibiting the expression of glucocorticoid receptor in a cell or tissue comprising contacting said cell or tissue *in vitro* with the antisense oligonucleotide of any of claims 1-10 so that expression of glucocorticoid receptor is inhibited.

12. An antisense oligonucleotide 15 to 30 nucleobases in length targeted to a nucleic acid molecule encoding glucocorticoid receptor, wherein said oligonucleotide is complementary to said nucleic acid molecule encoding glucocorticoid receptor, and wherein said oligonucleotide inhibits the expression of glucocorticoid receptor mRNA, for use in:
(i) decreasing blood glucose levels in an animal by administering to said animal a therapeutically or prophylactically effective amount of the antisense oligonucleotide, or preventing or delaying the onset of an increase in blood glucose levels in an animal by administering to said animal a therapeutically or prophylactically effective amount of the antisense oligonucleotide;
(ii) treating an animal having a disease or condition associated with glucocorticoid receptor by administering to said animal a therapeutically or prophylactically effective amount of the antisense oligonucleotide so that expression of glucocorticoid receptor is inhibited, or preventing or delaying the onset of a disease or condition associated with glucocorticoid receptor in an animal by administering to said animal a therapeutically or prophylactically effective amount of the antisense oligonucleotide so that expression of glucocorticoid receptor is inhibited;
(iii) decreasing blood lipid levels in an animal by administering to said animal a therapeutically or prophylactically effective amount of the antisense oligonucleotide, or preventing or delaying the onset of an increase in blood lipid levels in an animal by administering to said animal a therapeutically or prophylactically effective amount of the antisense oligonucleotide;
(iv) decreasing body fat mass in an animal by administering to said animal a therapeutically or prophylactically effective amount of the antisense oligonucleotide, or preventing or delaying the onset of an increase in body fat mass in an animal by administering to said animal a therapeutically or prophylactically effective amount of the antisense oligonucleotide.

13. The antisense oligonucleotide of claim 12, wherein:
(a) the disease or condition is diabetes, obesity, metabolic syndrome X, hyperglycemia or hyperlipidemia, and wherein said hyperlipidemia is optionally high blood cholesterol levels or high blood triglyceride levels;
(b) the blood glucose levels are plasma glucose levels or serum glucose levels;
(c) the blood lipid levels are blood cholesterol levels or blood triglyceride levels;
(d) said inhibition of expression of glucocorticoid receptor is in the liver or fat;
(e) glucocorticoid receptor expression in the pituitary is not inhibited;
(f) basal levels of plasma corticosterone and plasma ACTH in the animal are unaffected by antisense treatment;
(g) the animal is insulin-resistant as compared to a normal animal;
(h) the animal is a diabetic animal; and/or
(i) the animal is obese.

14. The antisense oligonucleotide of claim 12, wherein:
(a) said disease or condition is treated without occurrence of lymphopenia, or the onset of said disease or condition is prevented or delayed without occurrence of lymphopenia;
(b) blood glucose levels are decreased without occurrence of lymphopenia, or the onset of said increase in blood glucose levels is prevented or delayed without occurrence of lymphopenia;
(c) said blood lipid levels are decreased without the occurrence of lymphopenia, or the onset of said increase in blood lipid levels is prevented or delayed without the occurrence of lymphopenia; or
(d) said body fat mass is decreased without the occurrence of lymphopenia, or the onset of said increase in body fat mass is prevented or delayed without the occurrence of lymphopenia.

15. The antisense oligonucleotide of any of claims 12-14, wherein the animal is a human or a rodent.


SEQUENCE LISTING

<110> ISIS PHARMACEUTICALS, INC.

<120> MODULATION OF GLUCOCORTICOID RECEPTOR
  EXPRESSION

<130> P056259EP

<150> 60/538,173
<151> 2004-01-20

<150> 60/550,191
<151> 2004-03-03

<150> EP 05722546.8
<151> 2005-01-20

<160> 310

<170> FastSEQ for Windows Version 4.0

<210> 1
<211> 20
<212> DNA
<213> Artificial Sequence

<220>
<223> Antisense Compound

<400> 1
tccgtcatcg ctcctcaggg                                                20

<210> 2
<211> 20
<212> DNA
<213> Artificial Sequence

<220>
<223> Antisense Compound

<400> 2
gtgcgcgcga gcccgaaatc                                                20

<210> 3
<211> 20
<212> DNA
<213> Artificial Sequence

<220>
<223> Antisense Compound

<400> 3
atgcattctg cccccaagga                                                20

<210> 4
<211> 4788
<212> DNA
<213> Artificial Sequence

<220>
<223> H. sapiens

<400> 4
tttttagaaa aaaaaaatat atttccctcc tgctccttct gcgttcacaa gctaagttgt 60
ttatctcggc tgcggcggga actgcggacg gtggcgggcg agcggctcct ctgccagagt 120

tgatattcac tgatggactc caaagaatca ttaactcctg gtagagaaga aaaccccagc 180
agtgtgcttg ctcaggagag gggagatgtg atggacttct ataaaaccct aagaggagga 240
gctactgtga aggtttctgc gtcttcaccc tcactggctg tcgcttctca atcagactcc 300
aagcagcgaa gacttttggt tgattttcca aaaggctcag taagcaatgc gcagcagcca 360
gatctgtcca aagcagtttc actctcaatg ggactgtata tgggagagac agaaacaaaa 420
gtgatgggaa atgacctggg attcccacag cagggccaaa tcagcctttc ctcgggggaa 480
acagacttaa agcttttgga agaaagcatt gcaaacctca ataggtcgac cagtgttcca 540
gagaacccca agagttcagc atccactgct gtgtctgctg cccccacaga gaaggagttt 600
ccaaaaactc actctgatgt atcttcagaa cagcaacatt tgaagggcca gactggcacc 660
aacggtggca atgtgaaatt gtataccaca gaccaaagca cctttgacat tttgcaggat 720
ttggagtttt cttctgggtc cccaggtaaa gagacgaatg agagtccttg gagatcagac 780
ctgttgatag atgaaaactg tttgctttct cctctggcgg gagaagacga ttcattcctt 840
ttggaaggaa actcgaatga ggactgcaag cctctcattt taccggacac taaacccaaa 900
attaaggata atggagatct ggttttgtca agccccagta atgtaacact gccccaagtg 960
aaaacagaaa aagaagattt catcgaactc tgcacccctg gggtaattaa gcaagagaaa 1020
ctgggcacag tttactgtca ggcaagcttt cctggagcaa atataattgg taataaaatg 1080
tctgccattt ctgttcatgg tgtgagtacc tctggaggac agatgtacca ctatgacatg 1140
aatacagcat ccctttctca acagcaggat cagaagccta tttttaatgt cattccacca 1200
attcccgttg gttccgaaaa ttggaatagg tgccaaggat ctggagatga caacttgact 1260
tctctgggga ctctgaactt ccctggtcga acagtttttt ctaatggcta ttcaagcccc 1320
agcatgagac cagatgtaag ctctcctcca tccagctcct caacagcaac aacaggacca 1380
cctcccaaac tctgcctggt gtgctctgat gaagcttcag gatgtcatta tggagtctta 1440
acttgtggaa gctgtaaagt tttcttcaaa agagcagtgg aaggacagca caattaccta 1500
tgtgctggaa ggaatgattg catcatcgat aaaattcgaa gaaaaaactg cccagcatgc 1560
cgctatcgaa aatgtcttca ggctggaatg aacctggaag ctcgaaaaac aaagaaaaaa 1620
ataaaaggaa ttcagcaggc cactacagga gtctcacaag aaacctctga aaatcctggt 1680
aacaaaacaa tagttcctgc aacgttacca caactcaccc ctaccctggt gtcactgttg 1740
gaggttattg aacctgaagt gttatatgca ggatatgata gctctgttcc agactcaact 1800
tggaggatca tgactacgct caacatgtta ggagggcggc aagtgattgc agcagtgaaa 1860
tgggcaaagg caataccagg tttcaggaac ttacacctgg atgaccaaat gaccctactg 1920
cagtactcct ggatgtttct tatggcattt gctctggggt ggagatcata tagacaatca 1980
agtgcaaacc tgctgtgttt tgctcctgat ctgattatta atgagcagag aatgactcta 2040
ccctgcatgt acgaccaatg taaacacatg ctgtatgttt cctctgagtt acacaggctt 2100
caggtatctt atgaagagta tctctgtatg aaaaccttac tgcttctctc ttcagttcct 2160
aaggacggtc tgaagagcca agagctattt gatgaaatta gaatgaccta catcaaagag 2220
ctaggaaaag ccattgtcaa gagggaagga aactccagcc agaactggca gcggttttat 2280
caactgacaa aactcttgga ttctatgcat gaagtggttg aaaatctcct taactattgc 2340
ttccaaacat ttttggataa gaccatgagt attgaattcc ccgagatgtt agctgaaatc 2400
atcaccaatc agataccaaa atattcaaat ggaaatatca aaaaacttct gtttcatcaa 2460
aagtgactgc cttaataaga atggttgcct taaagaaagt cgaattaata gcttttattg 2520
tataaactat cagtttgtcc tgtagaggtt ttgttgtttt attttttatt gttttcatct 2580
gttgttttgt tttaaatacg cactacatgt ggtttataga gggccaagac ttggcaacag 2640
aagcagttga gtcgtcatca cttttcagtg atgggagagt agatggtgaa atttattagt 2700
taatatatcc cagaaattag aaaccttaat atgtggacgt aatctccaca gtcaaagaag 2760
gatggcacct aaaccaccag tgcccaaagt ctgtgtgatg aactttctct tcatactttt 2820
tttcacagtt ggctggatga aattttctag actttctgtt ggtgtatccc ccccctgtat 2880
agttaggata gcatttttga tttatgcatg gaaacctgaa aaaaagttta caagtgtata 2940
tcagaaaagg gaagttgtgc cttttatagc tattactgtc tggttttaac aatttccttt 3000
atatttagtg aactacgctt gctcattttt tcttacataa ttttttattc aagttattgt 3060
acagctgttt aagatgggca gctagttcgt agctttccca aataaactct aaacattaat 3120
caatcatctg tgtgaaaatg ggttggtgct tctaacctga tggcacttag ctatcagaag 3180
accacaaaaa ttgactcaaa tctccagtat tcttgtcaaa aaaaaaaaaa aaaaagctca 3240
tattttgtat atatctgctt cagtggagaa ttatataggt tgtgcaaatt aacagtccta 3300
actggtatag agcacctagt ccagtgacct gctgggtaaa ctgtggatga tggttgcaaa 3360
agactaattt aaaaaataac taccaagagg ccctgtctgt acctaacgcc ctatttttgc 3420
aatggctata tggcaagaaa gctggtaaac tatttgtctt tcaggacctt ttgaagtagt 3480
ttgtataact tcttaaaagt tgtgattcca gataaccagc tgtaacacag ctgagagact 3540
tttaatcaga caaagtaatt cctctcacta aactttaccc aaaaactaaa tctctaatat 3600
ggcaaaaatg gctagacacc cattttcaca ttcccatctg tcaccaattg gttaatcttt 3660
cctgatggta caggaaagct cagctactga tttttgtgat ttagaactgt atgtcagaca 3720
tccatgtttg taaaactaca catccctaat gtgtgccata gagtttaaca caagtcctgt 3780
gaatttcttc actgttgaaa attattttaa acaaaataga agctgtagta gccctttctg 3840
tgtgcacctt accaactttc tgtaaactca aaacttaaca tatttactaa gccacaagaa 3900
atttgatttc tattcaaggt ggccaaatta tttgtgtaat agaaaactga aaatctaata 3960
ttaaaaatat ggaacttcta atatattttt atatttagtt atagtttcag atatatatca 4020
tattggtatt cactaatctg ggaagggaag ggctactgca gctttacatg caatttatta 4080
aaatgattgt aaaatagctt gtatagtgta aaataagaat gatttttaga tgagattgtt 4140

ttatcatgac atgttatata ttttttgtag gggtcaaaga aatgctgatg gataacctat 4200
atgatttata gtttgtacat gcattcatac aggcagcgat ggtctcagaa accaaacagt 4260
ttgctctagg ggaagaggga gatggagact ggtcctgtgt gcagtgaagg ttgctgaggc 4320
tctgacccag tgagattaca gaggaagtta tcctctgcct cccattctga ccacccttct 4380
cattccaaca gtgagtctgt cagcgcaggt ttagtttact caatctcccc ttgcactaaa 4440
gtatgtaaag tatgtaaaca ggagacagga aggtggtgct tacatcctta aaggcaccat 4500
ctaatagcgg gttactttca catacagccc tcccccagca gttgaatgac aacagaagct 4560
tcagaagttt ggcaatagtt tgcatagagg taccagcaat atgtaaatag tgcagaatct 4620
cataggttgc caataataca ctaattcctt tctatcctac aacaagagtt tatttccaaa 4680
taaaatgagg acatgttttt gttttctttg aatgcttttt gaatgttatt tgttattttc 4740
agtattttgg agaaattatt taataaaaaa acaatcattt gctttttg 4788

<210> 5
<211> 27
<212> DNA
<213> Artificial Sequence

<220>
<223> PCR Primer

<400> 5
aggttgtgca aattaacagt cctaact 27

<210> 6
<211> 23
<212> DNA
<213> Artificial Sequence

<220>
<223> PCR Primer

<400> 6
tagtcttttg caaccatcat cca 23

<210> 7
<211> 30
<212> DNA
<213> Artificial Sequence

<220>
<223> PCR Probe

<400> 7
agcacctagt ccagtgacct gctgggtaaa 30

<210> 8
<211> 19
<212> DNA
<213> Artificial Sequence

<220>
<223> PCR Primer

<400> 8
gaaggtgaag gtcggagtc 19

<210> 9
<211> 20
<212> DNA
<213> Artificial Sequence

<220>
<223> PCR Primer

<400> 9
gaagatggtg atgggatttc 20

<210> 10
<211> 20
<212> DNA
<213> Artificial Sequence

<220>
<223> PCR Probe

<400> 10
caagcttccc gttctcagcc                                                    20

<210> 11
<211> 2575
<212> DNA
<213> Artificial Sequence

<220>
<223> M. musculus

<400> 11
ggaagttaat atttgccaat ggactccaaa gaatccttag ctccccctgg tagagacgaa 60
gtccccagca gtttgcttgg ccgggggagg ggaagcgtga tggacttgta taaaaccctg 120
aggggtggag ctacagtcaa ggtttctgcg tcttcaccct cagtggctgc tgcttctcag 180
gcagattcca agcagcagag gattctcctt gatttttcaa aaggctcagc aagcaatgca 240
cagcagcagc agcagcagca gcagccgcag ccagatttat ccaaagccgt ttcactgtcc 300
atgggactgt atatgggaga gaccgaaaca aaagtgatgg ggaatgactt gggctaccca 360
cagcagggcc agcttggcct ctcctctggg gaaacagact ttcggcttct ggaagaaagc 420
attgcaaacc tcaataggtc gaccagccgt ccagagaatc ccaagagttc aacacctgca 480
gctgggtgtg ctaccccgac agagaaggag tttccccaga ctcactctga tccatcttca 540
gaacagcaaa atagaaaaag ccagcctggc accaacggtg gcagtgtgaa attgtatacc 600
acagaccaaa gcacctttga catcttgcag gatttggagt tttctgccgg gtccccaggt 660
aaagagacaa acgagagtcc ttggaggtca gacctgttga tagatgaaaa cttgctttct 720
cctttggcgg gagaagatga tccattcctt ctggaagggg acgtgaatga ggattgcaag 780
cctcttattt taccggacac taaacctaaa attcaggata ctggagatac aatcttatca 840
agccccagca gtgtggcact gccccaagtg aaaacagaga aagatgattt cattgagctt 900
tgcacccctg gggtaattaa gcaagagaaa ctgggcccgg tttattgcca ggcaagcttt 960
tctgggacaa atataattgg gaataaaatg tctgccattt ctgttcatgg cgtgagtacc 1020
tctggaggac agatgtacca ctatgacatg aatacagcat ccctttctca gcagcaggat 1080
cagaagcctg tttttaatgt cattccacca attcctgttg gttctgaaaa ctggaatagg 1140
tgccaagggt ctggagagga caacctgact tccttggggg ctatgaactt cgcaggccgc 1200
tcagtgtttt ctaatggata ttcaagccct ggaatgagac cagatgtgag ttctcctccg 1260
tccagctcct ccacagcaac gggaccacct cccaaactct gcctggtgtg ctccgatgaa 1320
gcttcggtat gccattatgg ggtgctgacg tgtggaagct gtaaagtctt ctttaaaaga 1380
gcagtggaag gacagcacaa ttacctttgt gctggaagaa atgattgcat cattgataaa 1440
attcgaagaa aaaactgtcc agcatgccgc tatcgaaaat gtcttcaagc tggaatgaac 1500
ctggaagctc gaaaaacgaa gaaaaaaatt aaaggaattc agcaagccac tgcaggagtc 1560
tcacaagaca cttctgaaaa cgctaacaaa acaatagttc ctgccgcgct gccacagctt 1620
acccctaccc tggtgtcact gctggaggtg atcgagcctg aggtgttata tgcaggatat 1680
gacagctctg ttccagactc agcatggaga attatgacca cgctcaacat gttaggtggg 1740
cgccaagtga ttgccgcagt gaaatgggca aaggcgatac caggattcag aaacttacac 1800
ctggatgacc aaatgaccct tctacagtac tcatggatgt ttctcatggc atttgccctg 1860
ggttggagat catacagaca agcaagtgga aacctgctat gctttgctcc tgatctgatt 1920
attaatgagc agagaatgac tctaccctgc atgtatgacc aatgtaaaca catgctgttt 1980
atctccactg aattacaaag attgcaggta tcctatgaag agtatctctg tatgaaaacc 2040
ttactgcttc tctcctcagt tcctaaggaa ggtctgaaga gccaagagtt atttgatgag 2100
attcgaatga cttatatcaa agagctagga aaagccattg tcaaaaggga aggaaactcc 2160
agtcagaatt ggcagcggtt ttatcaactg acaaaacttt tggactccat gcatgatgtg 2220
gttgaaaatc tccttagcta ctgcttccaa acatttttgg ataagtccat gagtattgaa 2280
ttcccagaga tgttagctga aatcatcact aatcagatac caaaatactc aaatggaaat 2340
atcaaaaagc ttctgtttca tcagaaatga ctgccttact aagaaaggct gccttaaaga 2400
aagttgaatt tatagctttt actgtacaaa cttatcaact tgtcttgtag atgttttgtc 2460
gttctttttg tttgtcttgt ttgttttcta tacgcactac atgtggtctc tagagggcca 2520
agacttggca acagaagcag atgagccatc acttttcagt gacaggaaag cagac 2575

<210> 12
<211> 23
<212> DNA

<213> Artificial Sequence

<220>
<223> PCR Primer

<400> 12
gacatcttgc aggatttgga gtt                                              23

<210> 13
<211> 23
<212> DNA
<213> Artificial Sequence

<220>
<223> PCR Primer

<400> 13
aacaggtctg acctccaagg act                                              23

<210> 14
<211> 27
<212> DNA
<213> Artificial Sequence

<220>
<223> PCR Probe

<400> 14
cgggtcccca ggtaaagaga caaacga                                          27

<210> 15
<211> 20
<212> DNA
<213> Artificial Sequence

<220>
<223> PCR Primer

<400> 15
ggcaaattca acggcacagt                                                  20

<210> 16
<211> 20
<212> DNA
<213> Artificial Sequence

<220>
<223> PCR Primer

<400> 16
gggtctcgct cctggaagat                                                  20

<210> 17
<211> 27
<212> DNA
<213> Artificial Sequence

<220>
<223> PCR Probe

<400> 17
aaggccgaga atgggaagct tgtcatc                                          27

<210> 18
<211> 6322
<212> DNA
<213> Artificial Sequence

<220>
<223> R. norvegicus

<220>
<221> misc_feature
<222> 24, 3663, 3680, 3684, 3685, 3791, 3805, 3806, 3813, 3854,
3861, 4162, 4177, 4205, 4206, 4240, 4246, 4247, 4262, 4283,
4284, 4293, 4295, 4311, 4354, 4358, 4359, 4360, 4398, 6010,
6011, 6013, 6014, 6065, 6069, 6145, 6161
<223> n = A,T,C or G

<400> 18
gacgctgcgg gggtggggga cctncggcgg cacggagtcc ccccccgggc tcacattaat 60
atttgccaat ggactccaaa gaatccttag ctccccctgg tagagacgaa gtccctggca 120
gtttgcttgg ccaagggagg gggagcgtaa tggactttta taaaagcctg aggggaggag 180
ctacagtcaa ggtttctgca tcttcgccct cagtggctgc tgcttctcag gcagattcca 240
agcagcagag gattctcctt gatttctcga aaggctccac aagcaatgtg cagcagcgac 300
agcagcagca gcagcagcag cagcagcagc agcagcagca gcagcagcag cagcagccag 360
gcttatccaa agccgtttca ctgtccatgg ggctgtatat gggagagaca gaaacaaaag 420
tgatggggaa tgacttgggc tacccacagc agggccaact tggcctttcc tctggggaaa 480
cagactttcg gcttctggaa gaaagcattg caaacctcaa taggtcgacc agcgttccag 540
agaaccccaa gagttcaacg tctgcaactg ggtgtgctac cccgacagag aaggagtttc 600
ccaaaactca ctcggatgca tcttcagaac agcaaaatcg aaaaagccag accggcacca 660
acggaggcag tgtgaaattg tatcccacag accaaagcac ctttgacctc ttgaaggatt 720
tggagttttc cgctgggtcc ccaagtaaag acacaaacga gagtccctgg agatcagatc 780
tgttgataga tgaaaacttg ctttctcctt tggcgggaga agatgatcca ttccttctcg 840
aagggaacac gaatgaggat tgtaagcctc ttattttacc ggacactaaa cctaaaatta 900
aggatactgg agatacaatc ttatcaagtc ccagcagtgt ggcactaccc caagtgaaaa 960
cagaaaaaga tgatttcatt gaactttgca cccccggggt aattaagcaa gagaaactgg 1020
gcccagttta ttgtcaggca agcttttctg ggacaaatat aattggtaat aaaatgtctg 1080
ccatttctgt tcatggtgtg agtacctctg gaggacagat gtaccactat gacatgaata 1140
cagcatccct ttctcagcag caggatcaga agcctgtttt taatgtcatt ccaccaattc 1200
ctgttggttc tgaaaactgg aataggtgcc aaggctccgg agaggacagc ctgacttcct 1260
tgggggctct gaacttccca ggccggtcag tgttttctaa tgggtactca agccctggaa 1320
tgagaccaga tgtaagctct cctccatcca gctcgtcagc agccacggga ccacctccca 1380
agctctgcct ggtgtgctcc gatgaagctt caggatgtca ttacggggtg ctgacatgtg 1440
gaagctgcaa agtattcttt aaaagagcag tggaaggaca gcacaattac ctttgtgctg 1500
gaagaaacga ttgcatcatt gataaaattc gaaggaaaaa ctgcccagca tgccgctatc 1560
ggaaatgtct tcaggctgga atgaaccttg aagctcgaaa aacaaagaaa aaaatcaaag 1620
ggattcagca agccactgca ggagtctcac aagacacttc ggaaaatcct aacaaaacaa 1680
tagttcctgc agcattacca cagctcaccc ctaccttggt gtcactgctg gaggtgattg 1740
aacccgaggt gttgtatgca ggatatgata gctctgttcc agattcagca tggagaatta 1800
tgaccacact caacatgtta ggtgggcgtc aagtgattgc agcagtgaaa tgggcaaagg 1860
cgatactagg cttgagaaac ttacacctcg atgaccaaat gaccctgcta cagtactcat 1920
ggatgtttct catggcattt gccttgggtt ggagatcata cagacaatca agcggaaacc 1980
tgctctgctt tgctcctgat ctgattatta atgagcagag aatgtctcta ccctgcatgt 2040
atgaccaatg taaacacatg ctgtttgtct cctctgaatt acaaagattg caggtatcct 2100
atgaagagta tctctgtatg aaaaccttac tgcttctctc ctcagttcct aaggaaggtc 2160
tgaagagcca agagttattt gatgagattc gaatgactta tatcaaagag ctaggaaaag 2220
ccatcgtcaa aagggaaggg aactccagtc agaactggca acggttttac caactgacaa 2280
agcttctgga ctccatgcat gaggtggttg agaatctcct tacctactgc ttccagacat 2340
ttttggataa gaccatgagt attgaattcc cagagatgtt agctgaaatc atcactaatc 2400
agataccaaa atattcaaat ggaaatatca aaaagcttct gtttcatcaa aaatgactgc 2460
cttactaaga aaggttgcct taaagaaagt tgaatttata gcttttactg tacaaactta 2520
tcaatttgtc ttgtagatgt tttgttgttc tttttgtttc tgtcttgttt tgttttaaac 2580
acgcagtaca tgtggtttat agagggccaa gacttggcga cagaagcagt tgagtcaaca 2640
ctctgaagtg atgacacagc acacagtgaa gtgtattgtt ggtgtatcac agaaactaac 2700
agttacgtgg aggcatggcc actgtcagag agggaccgca cctaaaccac cgtgcccaag 2760
tccatgtggt tcaactttct gactcagaac tttacagttg gctgggtaaa actttctaga 2820
ctttctgttg gtgtattttt cccatgtata gttaggatgg tattttgatt tatgcatgca 2880
aacctgaaaa aagtttacaa gtgtatatca gaaaagggaa gttgtgcctt ttatagctat 2940
tactgtctgg ttttaacaat ttcctttata ttcagtgaac tatgcttgct cgtttctctt 3000
caataatttt tgtattccag ttattgtaca gctgtttaag atgggcagct gcttcacagc 3060
tttcctagac gctaacatta atttccgtgt gaaaatgggt cggtgcttct accctgttgg 3120
caccagctat cagaagacca cagaaattga ctcagatctc cagtattctt gttaaaaagc 3180
tcttactctg tatatatctg cttccatgga gaattacata ggctgagcag attacatagg 3240

ctgagcagat taaccgtcct aactggtgta gagcacctag tccagtgacc ttctgggtaa 3300
accgtggatg atggttacag aagactggtg ggaaaacagt aactaccaaa aggccccttt 3360
ccatctaatg caccatctct tcaatgggga gatagcaacc aagcccgtaa atcagctctt 3420
tcaggacctt ctggagtggt ttgcataaca ttttaaaatg tattattcca gatagccagc 3480
tctgataaag ccgagagatt gtttaatcag accaagtaac ttctctcatt aaacttaccc 3540
ccaactaaat cgctaataca gcaagaatgg ctagacaccc attttcacat ctcacccgca 3600
ccgattggtc tagctctcat ggtggtcagg agaatcagct actgattttt gttacttaga 3660
atnttcagga ctcgcatttn tccnnctaca catccctaca tgtgccatag aatttaacac 3720
aagtcctgtg aacttcttca cattgagaat tatcatttta aacaaaacag aagcagtagt 3780
agccctttct ntgtgcacct taccnncttt ctntgactca aagcttaata tgcttactaa 3840
gccacaagaa atcngatttc nacttaaagg cgccaaatta tttgtgtaat agaaaaactg 3900
aaaatctaat attaaaaata tgaaacttct aatatatttt tatatttagt tatagtttcg 3960
atatatatca tatcggtatt cactgatctt gggaaaggga aagggctact gcagctttac 4020
atgcaattta ttaactgact gtaaaatagc tgtatagtaa taagaatgac ttttagtgag 4080
attgctttat catgacatgt tatatatttt tcgtaggggt caaagaaata ttgatggata 4140
tgatagccta tatgatttaa tngtatataa aagcatncaa acaggcctta acgcgtcttg 4200
gaaannaaaa tacctttgtt ctaagctagg gaagggagcn ggagannggc cccgtgtgta 4260
tnggaggttc cgaggctcgg atnnaagaga tcnanagggg atctaattcc ntacctccat 4320
ctaattacct caccacccat gatcctgtca gtgnaggnnn ggttattaaa tcccccgtta 4380
tactaatata aatagganag aagggtggcg ctcacgtctg ttccaggcgc cgcagtagca 4440
gggttatttt ccatgcagcc tcccgacaag gttagcagag ggaggctttg gcaagtttgg 4500
cgtggcgtgc atagaggcac cagcaacatg taaacctaaa gagcccatag gaagccaaga 4560
atacactaat cctccccacc cttcaatagt ccatttccaa gtaagatgag gacatgctta 4620
tgttttcttt gaatgctttt agaatgttgt tattttcagt attttgcaga aattatttaa 4680
taaaaaagta taatttgaat tctctctaaa agggattgtt cagtttgtaa tggtttaaat 4740
tggtctcaaa gtactttaag ataattgtaa cccagctgga tgtgaaattt atggtgccta 4800
agaaatacca cttgaatatt atcaagacag tgttaagttt taaaatgagc ttctcaaaaa 4860
tagattattg tacatttatg gaatgttata tggttaaacc caaaaaagca catcacacat 4920
aaatctgctt tcagcttggc tttcaaaaat agagctccaa aaacgaaaaa ggagaagaaa 4980
aagtatatat atgcgttgtt attaacagaa ggcaacagac attcataaaa ctactaccga 5040
agctttcctt gaagcgtata aagagccatg ctcctttagt atgtggggaa gaagagagcc 5100
gtcatagttt cgagtacaga gagaagatgc ggtactgtct ccgtgtgtgg cttcataccg 5160
ttcctaacta tttaggttta taataacttc agtgagactc ggtgacatgc ctgtatgact 5220
catgaccgat cttgaaagat atctttaatt actggtagga caaaagggac actctggtta 5280
ttttaggcct tggcttggga tactgtatat ccagaagaaa ggagacagga aacttgggga 5340
agggaaggga acctaggaag cactgccttc tgtaggaaag aacacaccaa taagtgagag 5400
tacccaaagg gacaaggcca cacagtgtgg ggtctaagga tgagtcaggg tgagctctgg 5460
tgggcatgga gaagccagca actccagtgc tacagagcag ggcagggcag ggatgggaca 5520
agatggatgc ggatcccagt cccagtagtt tgctccctct tatttaccat gggatgaacc 5580
atggagtatt gatctgtcag cactcaagga tcatggagct tgagattccg gttggtcacc 5640
ccaacggtaa gctgagattg aatgtgtttc ttatgtgccg gtttcagtgt tagaaggcga 5700
aacagagtgt acagaagaca ctgcaaaccg gtcagatgaa agtcttctca ttcccaaact 5760
attttcagtc agcctgctct atcaggactg gtgaccagct gctaggacag ggtcggcgct 5820
tctgtctaga atatgcctga aaggatttta ttttctgata aatggctgta tgaaaatacc 5880
ctcctcaata acctgcttaa ctacatagag atttcagtgt gtcaatattc tattttgtat 5940
attaaacaaa ggctatataa tggggacaaa tctatattat actgtgtatg gcattattaa 6000
gaagcttttn nannattttt tatcacagta atttttaaat gtgtaaaaaa ttaaaaatta 6060
gtgantccng tttaaaaata aaagttgtag ttttttattc atgctgaata acctgtagtt 6120
taaaaatccg tctttctacc tacanagtga aatgtcagac ngtaaaattt tgtgtggaaa 6180
tgtttaactt ttatttttct ttaaatttgc tgtcttggta ttaccaaacc acacattgta 6240
ctgaattggc agtaaatgtt agtcagccat ttacagcaat gccaaatatg gataaacatc 6300
ataataaaat atctgctttt tc 6322

<210> 19
<211> 25
<212> DNA
<213> Artificial Sequence

<220>
<223> PCR Primer

<400> 19
aaacaatagt tcctgcagca ttacc 25

<210> 20

<211> 23
<212> DNA
<213> Artificial Sequence

<220>
<223> PCR Primer

<400> 20
catacaacac ctcgggttca atc                                              23

<210> 21
<211> 23
<212> DNA
<213> Artificial Sequence

<220>
<223> PCR Probe

<400> 21
acccctacct tggtgtcact gct                                              23

<210> 22
<211> 23
<212> DNA
<213> Artificial Sequence

<220>
<223> PCR Primer

<400> 22
tgttctagag acagccgcat ctt                                              23

<210> 23
<211> 21
<212> DNA
<213> Artificial Sequence

<220>
<223> PCR Primer

<400> 23
caccgacctt caccatcttg t                                                21

<210> 24
<211> 24
<212> DNA
<213> Artificial Sequence

<220>
<223> PCR Probe

<400> 24
ttgtgcagtg ccagcctcgt ctca                                             24

<210> 25
<211> 32767
<212> DNA
<213> Artificial Sequence

<220>
<223> H. sapeins

<400> 25
aagcttatgg tttatgggtg ttacattcaa gacatttgta ggacacattc taaaatgcca 60
tccaatttca ggctctttcc agcagaaact gtggaatatt tttccgttca ttcagcattt 120
acttagtgcc tgctctgcca ggaattgaag agaaagccca aagacaggca gaccttacct 180
gagaggtagt gaactgacca ggatgactgt gggcagtaga cttgtttccc aaactagcct 240

caccatttct gtatttgcat atacgaggaa aggattagat atagggattc atgtcagcat 300
acaccccagg gacatttgtt tttagtgaaa ggtgccagtc ttcatccctg tacccagtac 360
acaaaccacg aagaagtatg ctcccgtcat tgtcaaagaa tcatagaatt ccaaatggag 420
ctagttttga tatccagatc tcacttcata tgaggaaact aggtccagta ttgtgagtaa 480
gaattaggac tcttcagatt ccctgggtat gaatctgact aacaactgtg tgaacttgac 540
caaattcata accctgtaaa ctctgtttcc tcacttttaa aatgggcaca acaaagtgat 600
gcatgtaaac tgcatagcac agtgtctggc acttaaaaag cactcctgaa gttattttta 660
gtgatgtgtt ttaagattag acaactcctt aatgccaaag gtttttactt gagaactctg 720
tctgtgtgcc atactacacg ctgttcataa gataagcctt tttcattaat tgatctcaaa 780
ctggcttcat tatgatctta actttatttc agttttattt ttaaaattta tttttaattt 840
ttatgggtat atagtaggca tatatattta tggggtacag gtcatgtttt aatgcaagca 900
tgcaattgtg ggggtgatat ataattgact ggggtgagat atctcattgt agttttgatt 960
tgcatttctc tgatgattaa ggatgttgaa catttcttca tacacctgtt ggccatttgt 1020
atgtcttttg agaaatgtct attcagatct tttgtccatt ttttaagttg gattgtttga 1080
ttttttcctg ttgtctgaac tctttatata ttctagttat taatcccttc tcagatgggt 1140
agcttgcaaa tattttcttc cattttgtgg gttgcttctt tgttgtttcc gttgctgtgc 1200
agaagttttt tagcttgatg tgatcccatt tgtccatttt tgcattggtt gcctgtgcat 1260
ttgaggtatt actaaagaaa tctttgccca taccagtgtc ctggagagct tcccaaatgt 1320
tttcttttag tatcctagtt tcaggtctta gatttagggc tttagtccat ttttatttga 1380
tttttatatg tggtgagaga taggggtcta gtttcattct gcctatggat atccagtttt 1440
cccagcacca tttattgaag agactgtcct ttccctagtg tatgttcttg gcacctttgc 1500
tgaaaatgag ttcactgtag gtgtatgaat ttgtttctgg gttctctagg tctgtgtatc 1560
tgtttttatg ctagaactat gttgtttggg ttattatagt tttgtagcat aatttgaagt 1620
cagataatgt aattcctcca gttttatttt ttttgttcag gatggctttg gctattccgg 1680
ggcttttgtg gttccatata aatcctatga tttttttttt ctatttctgt gaagaatgtc 1740
attgatattt attaataaag attgcattga atctgtagat tgctttgggt agtatggaca 1800
ttttaacaat attgattctt ccaatccatg agcatggact atctttcttt ttttgtgtgt 1860
cctcttcaat atttttcctc agtgttttat tgttttcatt gtagagctct ttcacttctt 1920
tcgttgagtt tattcctagg tgttttattt tatctgtagc tattgtaaat gagattactt 1980
tctgatttct tttttagatt gtcctctgtt ggcatctaga aatgccacag atttttgtat 2040
gttgattttg tatcctgtaa ctgtactgaa tttatctgtt ctaatatttt tttggtggag 2100
tctttaggct tttccaataa gatcatacag tctgcaaaca agaataattt gacttcttcc 2160
attccatttt ggattccctt tatatctttc tcttgtctga ttactctagg taggtcttcc 2220
agtacttcca gttgaataac agtgggcact cttgtcttgt tgtagatctt agaagaaagg 2280
ctttcagttt ttccccattc agtatgatac tagctgtcag tttgttgcag atggcataac 2340
tttcaaacta attgattata gttaggaagt ggatacttta acttgtggta ccattatcag 2400
atttatattt cggccataag cttgaagagg agctgaaaaa tgcatatgtg atgcatatgc 2460
ttcctatttg gctctcttct cccacccccc tgccctataa tccacacaag ttcctctctc 2520
agtcactcat caactacttg aacctctgag gaacttgggg ttaaggtaaa ttagaataaa 2580
actgtctgaa gaagagcaag cctttcatgt cttgagaaat tcttggggtt ttagaaataa 2640
cttcattgct ttttttctcc agttactttg gcttcttctt aaagagaata ctaacacttt 2700
gaacgtcata atactaaggt tctgcctctt caaataaaga ctttaaaaaa aaatggtttt 2760
tgtatgattc agtgtgaatt aaatcccaca gtgtaaagga ctttactttc ttaatgtaga 2820
ttttcaaata cacaattact gatgtttata agtagattta ttacaccaaa gcacctagca 2880
aattcttgaa tggatcaggt cttatttttc agtcttactt tgcaaattta agccaaataa 2940
ttaaggattt gttaaatatt tgtcttaata tcaagctttt gcatatcggg gccctctttt 3000
ataagcttta taagcaatct tttgttttct ctgcttgctc aaagtagcta tgtttgttgt 3060
atctgttagt atttgctcta taacaaacat actgggtgcc ttcccactta gatttggcaa 3120
ttatcactcc tgtaaatgag atattacata agataggaaa aagaacagta tctttccaag 3180
aagaatagta tccttccata ttaacagttt agagctgact gcttttaaaa tttagtggct 3240
ttaaaataac aaccatttat tattcttcat gagtctacaa atgaggtggg cagttctgct 3300
gatctggcca agctgaactt atctcagctg ggcacattca gcgtatctgc tgtcagttgg 3360
ctggttggct gtagcaatga atggtgaaag taggctgccc ttaacttttt cacacagtag 3420
cattagagtt acaaaagaac cagcagaacc atgcaaaact ctttaagacc taggcttgga 3480
acaactatat ttctaccaca ttctattggt caaagcaaat cacggggcta gtctagattc 3540
aagtgggtgg aggagctgca attacactgc aaaggagtgt gactgtaggg agaggtgttt 3600
ttttattttt atttttttgc gatttgtcac agtagttgta ggaatcaggt gtatttaaaa 3660
ttctgatcct tctgtgatat ccgaattgtt catgaacctt gcctctggtg gaaaggcaga 3720
atcattgtga cagaaggata aaatcttgga atttagagac taacaaaggt tcagattcca 3780
gctccatcac ttatttctgc aatcctgcag aagttaatct tcctgatagg cattcagtaa 3840
tgattgattc acctgaacct cagattcttt atgtatttta aagaaagggc taggtaaatg 3900
caaagcactt atgtaactgc ttttattatt gcaaacctgg ctcccacact ccattcaagg 3960
tgtaagactc agtgtcttcc ttgaattaaa aaggaagaga aagtgtgtta gggaaaggaa 4020
gagaaatatt tgactaattg tggccccaat aaagtgacca ctcactgggg gtattttcct 4080
gtaagaaaag aatggttgag gctcagagtt aagagataca aatccaaaag tctccttggg 4140
gtaggattcc ctgtgattca tgggttgaga ggtgtaacat tagacacagt cccagtctag 4200
attttttttt taaagaattg tggtccatcc tatacacact gggtgcctta atactatatg 4260

tggcaattat cactcctata aatcaggttt tacataagat aggaaaaaga acagtatcat 4320
tccacattaa caattgaaag atgactgctt ttaaaaaatt aaaagggcca tatagaaata 4380
aaatcacata aatttcttgt gttaaacata gttgtcatat tggatgagga ctaaacacct 4440
aaattcatcc aactagtagt aatagaaaag atgaaacaca cacacagtaa aactagatta 4500
atttaattta tacaaagggc cagatatctc agaattcaga cagtcagaga tgttgactag 4560
agttaatgcc tcttttagga gaggtaccag gtaagtgttc tcaaagaact ggaaactgag 4620
accaccacct ctggcattat ctgtttgtga acacaagcaa gtctgaattt ttccgcacca 4680
tagctacctt tcatgtaagc ttcttttctt agaagaaaag aaggtaacat ttgggtgtaa 4740
ttttttatta agggtgaaat ttagtgtaga gagtaaaggc atttggcata gaagccctta 4800
gttttttttg tttttaagtt gaactgccag cctttatgga ttgcagtctt cgctgttttg 4860
attgacattt cccaattcat tttgtattat ttattttttt aagagacagg gtctcactct 4920
gttacccagg ctggagtgca atggggcaaa cttggatcac tgcagccttg aactcctggg 4980
ctcaagcaat cctcccacct cagcctccca agtagcttgg actataggtg tgcaccacca 5040
tccttggcta attttttaaa tcttttgtag agacagggta gtgctctgtt gcccaggctg 5100
gtctcacatt cctggcctca gttgatgctc tggtctcagc cttccaaaat gctgggatta 5160
caagtgtgag ccactgcacc tggcccccaa tttcatcctt tacaaagact actttcaacc 5220
ataaatcaac ggaaacttca gctccctcag acatatttgg gatccaagga tattttccca 5280
aatgattaat gctaatttca tatcaataca tttttgcaaa acctacaaaa atggactagt 5340
aaagaaagac tcttaatttg ggaaagacag ttacttggag agaagagaaa cttaagaggc 5400
aggtcgagtt cagtgttcag aaatgagagg atcataaaga gatagccata aaaatgtttc 5460
tccctatatt gcctgctgat agggtgtatc agtgaaggtc ttactaagga ccttgtacct 5520
tttcagcgct gcactgcgtg ctcataggga ggaaagataa atcatgtgtt ttttctgacc 5580
tcaaaggagc ctgtatctgg ctagagagac atgatgcaga cacatgaaat aattaagaaa 5640
caattaactg tagcaggtgc tgaagaatat accaggaggt cagagaatgg tagagctagt 5700
gtgggcgaag gtatagccca gagcatcatc agatgattct tccttatgca aattcacatc 5760
tcctctgggt caagtatcat cctggcatgc agcagctcca taggtaatgc cctaaggcta 5820
gcctgaggca agttgcaaaa gccatcatat tgagtcatgg cctttttttg tgtgggggga 5880
ggggaatggc atccccttcc tgtctgccaa atcaaggaat acagtgccct cctaaacctg 5940
ctttgtttta gtggattgtt aaaaagaagt gaatgaattt atgcttcatt agggaaaggt 6000
tacagtggaa tactgaggag taaggggtat ttctatttaa caaatgacat aacttgaagg 6060
aatgaaatca taaggatgga atttcaggca ttaataaaaa gctgatgaga gatactttga 6120
gacaaaagag ccttcccagt gtaaccgaga tcacagcacc tacttcacat acacaggaaa 6180
ccagtcctat ctgtctctcc catagagcag tagctgcctt gtttttcctc cctcctccat 6240
cattcattct aaatctccag tcctccaccg caccttatcc aaaccctgat acccttaagt 6300
cacagatggt gaatcagtca aaagtagtat taaaaactag tggtacacag ctacacctgg 6360
aatgcagtaa gaaaaatacg gatttctgta catcatcttc cctccctgct cttaccccca 6420
tttaagagtt acagggtcag aacccaagag tgtgagtttt tgaaagtccc taaaaatttt 6480
ggatgatcac ctacatttag aaccactgca ctaagaagga caacaaatat gccaataaat 6540
tctgttgcca aggaggtgat tatgcaagct ggaaccctga taacatgagg agaatcccac 6600
aatagccaaa tagtccatgt actagttaca ttataataaa gccaaaagca gcaggcctac 6660
ctgactttct cctgaggtct atcatgagct tagagagaag gaacgtggac atacagaggt 6720
agctctagat ggagaagggc actaggtgtc atggaaagaa tcatgtgcaa gaagtaaaga 6780
ggtgctctga atgtcctagc cctgcttagg tgtctgtgtc ctcacatgag aatttatcca 6840
cagttctttc ccgctgtaac aatctttggt tccaactgca tttgtgagac agcaaaaagc 6900
tatggtccag tctccttcca ttgtatcatc tcatcaatgt atttctccca ctacccttgt 6960
gtgaaataca aacttttttg gcttattgtg attatgcaag gtgtatgcca actttttttt 7020
ttctccacat ctttcagctt tctgatgggt aaaaattttc cttattttgc tttagaaaaa 7080
ttctcattgg catagatcta atttcaggga gcctcccttg aaagctaaat aacattgaga 7140
attcatgaaa atataatgta gagcattatg cctgttagca tattagttta aatagaagtg 7200
gttcatgaaa atttttgaaa tgccagaccc tgtcctgtgt tttgtattct cccaaatact 7260
catccagata ctgttcagaa tgtaacatga ttattttgaa ataaagattt tcccctagtt 7320
tttaaaaaag ttactttata cattaaccct tatgttcctc tttgatcaat ttttccagta 7380
gtgtaaacag tcttcaggga agtagatttc ttacagaaat tgtcaagtgg ctctctgctg 7440
ttagcatggt tactaatctt ttggttactt ttcatatttt ttatactttc tggaagtgga 7500
caacttactt gtaaataaaa gtgcataatt tgtattaaaa atttttagta acaatctaat 7560
ttgtaaaata gatgtgagca gcatgaatgt gtgtgatatg cgtacatacg aattatgtct 7620
cttaaaaatg tatcacagac atctttccgt gtccaaacaa atctacctca ttctttctaa 7680
tagccatatg ggtataccat aatatattta actaggcccc tattaaaaga attttgactc 7740
ttttgtagct actatagtgt tgcagtgtgt atctgtgtat gtatctttgt gtgtgtatct 7800
ttgtacgagt gtacatatat tttccccttg gctatttcag attttttttt aggtttaaat 7860
cttaggaaag gttttgaaat tgtcttaagt attttcagaa gcattaaatc atggtttttt 7920
tacatttttc ttttagaagt tttatgtcat ctctatgagt agctttcagt aatttgttct 7980
gcataaaatt cccgaaaact tccatttaaa aataggtggc atgactagac tttctcagcc 8040
gaaagagtga ggtcccagga aggattttgg agaagctgtg ttcaaatata gctgctgacc 8100
tgatgtctgc ctagagtctg gcaaggtgat gtgttgaatc tagtgtctgc ctgcatgcca 8160
gcatcccttt actgatgaga tttgtggttt tcatcacttc atggtaatca tcccaagtta 8220
taagatggag tctctagaaa atcagtagag tatgaaggcc caagtaaaat acatgtgagt 8280

gcatgtatgt gtgcatacaa attacttctc ttaaaaacgt atcctgggca tttaaagaat 8340
gaggacctcc gaaggatttt gtggaagctg tgttcaagta cagctgctga gcgtatgtca 8400
gcctggagcc tggcaaggtg aagtgttgaa tctagtgtct ttttgactca ctgttttttt 8460
tgactcactg tgctttgaag cccttgtcat ttgggctcat aaaatagatt tctgtatact 8520
gtctctcctc cctgccctcg cccccattta aaagtatagt ggcagaaccc aagaatcaga 8580
gttactaaaa actctctaga aaatttggat gatcacccac ctgatcatgt cttttttact 8640
cactatgttt tttttttttt tgagacagag tctcgctctg tcgcccaggc tggagtgcag 8700
tggcatgatc ttggctcact gcaagctccg cctccctggt tcacgccatt ctcctgcctc 8760
agcctcccat gtagctggga ctacagggcc tgccaccgcg cccggctaat tttttgtatt 8820
tttagtagag tcggggtttc actgtgttag ccaggatggt cccgatctcc tgacctcgtg 8880
atccacccgc ctcggcctcc caaagtgctg ggattacagg cgtgagccac cacacccggc 8940
cctttactca ctatgttttt aagcccttgt tttcatttgc tccactgtaa aacattcccc 9000
aagccaatct ggagctgagg caaattttta acaatttaaa atctggggaa tataaatatt 9060
ggataatgat catcctgaaa aaacaatgaa ggtagtagca taatacttta tatatcaata 9120
aaatggcaaa ataagacagt tgttgaagga cagaaagagt aactgaagtt aggagcttat 9180
cttaacacat tttttgtgtc ataccatagg catcatattt tttaaatttt ttttatttca 9240
tacacatagg aaaatatatg tgtgtaagaa ataataaaca cctctttgta cctaccaccc 9300
aacttaagga acagctcatt gctattccct ttggtgctcg ctggatgccc tttcccagtc 9360
acatccccct cccttcccat ctgcaggact atactagtaa attttgtatt ttttgcatta 9420
ttttgctttg ttttatgatt ttactaccta tctacatatc cctaaataat acattattta 9480
gtttcatatg ttttaacttt atgttgtgga atcacattaa atgtagtctt ttttttttat 9540
attatacttt aagttctagg gtacatgtgc acaacgtgca ggtttgttac gtaggtatac 9600
atgcgccatg ttggtttgct gcacccatca actcgtcatt tacactgggt atttctccta 9660
atgctatccc tcccctagcc ccccaccccc cgataaatgt agtctttata acttgttttt 9720
ttaactcaac attgtttgta agattcatcc atgtaagctg aagctttttt atagagatct 9780
ttgttaagcc ttttaatgaa tacagtacat acatttctct gttcccctgt tagtggacac 9840
ttggattgtt tccagagttt tgctgttttg aacaacgctg ctgtgaaaat gtctcctgaa 9900
acacatttat aagagttttt ttttccccaa gggaattata cctagaaatt gaataactag 9960
atcacaaggc atacacatct acaacttctg ctaggtaatg ccaaattgtt tccaaggagc 10020
gttagaagtg ttctcatcaa cttttactag tgctagtctt ttacatttgt ggcagtatgg 10080
tgggtgtgaa atatttatgt ttagtttttc ttggtgccat ttaataattt ttataaaaaa 10140
tatttagaag tcaaggcagt tttttgtttt tgtttttatt ttttgcttgt tttgttttaa 10200
tgcagacatt gagattacga cttggaataa acattggttg caaagttcct aaaaggaaaa 10260
ctttttttgg tattctggag cttttctggt actgaataaa ataagtatgt taaattatgc 10320
atgtgtagtt tagaagtcag agcaataatt gtgattgttg aacagaatgg cagtaaaaag 10380
tttctaaacg attgtactgt acaagggaca cttgttgtgg gtcagtttta gcctccccaa 10440
cttttatgtt aaaagttgca acaaggttta agggcttatg tttgataggc cagatggtga 10500
ccagctgtga taaaacacag ggaacccttg caaaggattt caaaatttat gcagtagtcc 10560
gccttatctg cagttttgct ttccaaggtt tcagttaccc gcagtcaact gtgttctgaa 10620
aatattaagt gaaaaattac agaaataaag aatcgaagag ttttaaattt tatgcttccc 10680
acccatccca cctgggatgt gaatcattcc tttgttcagc atctccatgc tgtaggtgct 10740
gcctgcccct tagtcacttg gtagccatcc aggttatcag attgactctt ctagtattac 10800
aacacttggc ttcaagtaat ccttatttta cttcatagtg gccccaaagt gcaggagtgg 10860
tgatcctggc aattcagata tgtcaaagag aagctgtaaa ttgcttccct taagtgaaag 10920
atgaaaattc tagacttata tataaagaaa agaaatcata tgctgagact gctaagatct 10980
atgataagaa tgaatctttt atacatgaaa ttgtgaagaa tgaaaaagaa atgcgtgctg 11040
gttttgctgt catatctcag actgcaaaag tttgcagcca atgtgtatga taagtgctta 11100
gttaaaagga aaaaggcatt taaggtaagt atatatagtg tttggtacta cctgtgattt 11160
caggcatcca ttgggggtct cctgagtata aggggagact actcttttag tgttaaatga 11220
acactaagga acagagatgg ggaagaggtt ggagaagatt agttcagcag tttgagtata 11280
ggtaaacagt tgtttgagaa agaagaaaaa tgtgattagt attttacctt agcaatagtg 11340
gcatagataa tgataaatta tagtcacaca gaactcttag tatttacaga acgttcacat 11400
ttgtgatccc atttaacaat aactctgaaa gaaaggtatc atctaccact gctttattga 11460
taaagatata aaaggtaaga gagatgaaac atattggcca atgataccca tctggtaaga 11520
gacagggatg gggtgggacc ccaaggctct tctcgccaag cccacggttt ttttgcttta 11580
tacttttttg cctcctgatc accatggctg cagtttctac tgtggacaat gtctgtcagc 11640
aagcattgat cccctgcctt cagcactctt acgtcttagc aaggactgga aagaaaaagc 11700
caggagttta cagtctgctg gagcaacaga aaagaatgat atgaaatatg aagagaccaa 11760
aatgatttat aataaggtgc tagactatgt agtaaaaatc tgctttagct gtaagtcaaa 11820
agcaagagca gtcttttcag aatggaatag aaatgttgga attaaaggaa ttttcaaagt 11880
tgtgaatttt tttcaagata aacatgtttt attttggtaa ttatggtatt actaatttga 11940
taaccttcag ggagccacct aatattatag aagatgtaca tataatgaca aaagcaaaca 12000
ttttattttt aaggaccaca atctaatcta aaacaaaatt tccccctttt ctggtctttg 12060
gttaattaag gacttattta aatatcaaag aaagacacat agaaaacatt tagtatattt 12120
ctatactttt attaatgtcc tccatacctt acacagatac ttgacttggc tatggtctag 12180
ataatccatg aaaatttaaa ggacagattt taacaacttt atgctaaatt gatagatctc 12240
taggatcaga ttgccatcac tctcagatgc gaagcttcca accacttata ggttcctgat 12300

atcttgcttt tatacagacc taatttctct tcctttaaac tttcttttcc tcagttgcta 12360
tttgattgaa atattgagtc attaaaaatt tccaagtggg aatttttgtg tttcttcatc 12420
tatcatgaag ctgctcaaat aagtaggtgt ttgaatagga gtagaaacag taataggctg 12480
aagccagacc aatacagctt cagctaaatg ccgaccttgc taaagtctgg gaggaccggt 12540
gtggtattct acaatgtaca agtctgtagc cggtgccctt aatatgttgg cttcatgtct 12600
catgactctc ttctgtaaat atgcagttta aaaaatacaa gttattctgc tgtagaagat 12660
acatttgcaa aattgatgta tcccctctaa gtaaagttgg ctaaacaata aggacatatt 12720
tataattaat gaatttgaga agaatgctga cgatatgcat tattctttga agttaacatt 12780
tttcaggtcc taaataaaca aaaagtaggt tacttctgtc tggagtgtat gcaaggggta 12840
ccatcttgtc cttggttcct ggctgctatt ccaaggtgct ataaagtcag ctaaagagag 12900
caatcataat acattgatag catccctcaa tgtgtttctg agctacttga gaatcttatt 12960
tttgaatagg tagcaggaaa ccatctttgc agggcagcat gggcaaaggg attggaggga 13020
ctattattat aaagatccac tgaactgctt cagtatcata atatcttaaa ctaaaggact 13080
ggaaagagcc agattccaat ttaatctgct cttctatgaa ttcttagctg ggttcattta 13140
aaaagaaaaa acttgaagat tgcaagattt tgaagacatc ttaaaatagg tgaactccaa 13200
ggtgcacttt aaacttgaga ctgataactg aatactcctt caccttttga tctgatattg 13260
tcaaaatgaa tgaggactta gtgctctagt aagtttggaa cagaatgata ttaatttatt 13320
ttctcatgat tgattctttt ttgcttttta atagattaaa cttcaccgta gaacagtttc 13380
tcaacctctg gactattgac atttttgatt ggataattct ttgctgtcag ggctgttctg 13440
tgtgttgcag gatagttagc aacatccctg acaatcacaa atgttacttt ctgtctctat 13500
ggatttgcct attctggaca tttcgtataa atagaatcat atatatgtgg cttcttgtac 13560
ctggcttatt tcacttaaca tgttttcaag gttcatccat attgtagcat gtaacagcac 13620
ttcattttct ttttatggct gagtaatatt ctgttatgtg gatatactac catattttgt 13680
ctatccactc cttagctgat ggtcttttag gttgtgtcca ttctttggct attataaata 13740
atgctgttaa gaacattcat atacaagttt ctgtgtagac atatatcttt atttctcttg 13800
tgtggatacc taggagtaga attactggat catatgataa ctctatgtgt taccttttga 13860
ggaactgcca aacatttttc tacagtggct gtatcatttt acactcccat cagcaatgta 13920
taagaattcc aatttctctg tccttgccta tatttattaa ctgtcttttc ttattagcca 13980
actgctgtgg ttcgaatgtt tgtcccctcc aaaactcatg ttggaacata atccccaatg 14040
tggcagtatt gagatgtgag gcctttaaga agtgcttggg tcatcagagg tctgccctca 14100
tgaataggct aatccattca tgagttaatg tactaatggg ttatcactgg attgggacta 14160
gtggctttat aagaagagga agagaactaa tctagtaagc tcagccttct cactatgtga 14220
ttgctgccct gtgtcacctt gggactctgc agagagtcct ccagcagcaa gaagttcttc 14280
atcagctgtg gccccttgac cttggacttc ccagcctcca gaaatgtaag aaatccattt 14340
ctttttttta ataaattaca cagtctcacg tattcagtta taccaacaga acacagacta 14400
agacaccatc ctattgggta tgggtatctc attgtgtttt ttatttgtgt ctcccaaatg 14460
actaacgatg ttgaacatct tttcatctgc tttttggaca tttgtgtatt ttctttgaag 14520
aaatgtcttt aacattcttt gcccatttta aaattaggtt gtctttttat tgttgagttg 14580
tcggtgtgtg tgtgtgtgtg tgtgtgtgtg tgtgtatcta gaatatatgt gtatgtatat 14640
atgcagatat attctaaaca ctagaccctt atgaaatata taatttgagg acaatttctc 14700
ccatttaaaa ggccatcttt tcacttcttg atagtgtcat ttgactcaca agtttttaat 14760
ttttatgaag tccaatttat tttttaattc tttgtttttg gcactgtatc tttaaaaagt 14820
tgcctgatct aaggtcaaac tgattttcac ctatgttttc atctaagaat tatagtttta 14880
gctcttacat ttaggccttt gatccatttt gaattaattt gtgtatatgg tgtgaagtag 14940
ggctctaact tattcttttg tgtaatgata cctagttgtc ccagcaccat ttgttgaaaa 15000
gattattctt tccccattga atggtcttga taccttgttg aaatcaactg accataaata 15060
tataggctta ttcctggact cacaattcta tgagtctgta tgtctaatct tatgccagta 15120
ccacactgtt ttgattatta catctttgta caaagttttg aaattgggaa atgtgagtct 15180
tccaactttg ttctttttta agattacttt gcctatattc cgtgttcgtt gcaaactcat 15240
atgaatttta aatcaactct ccatttctgg aagaaaaaaa gaggcaattg aagttcagat 15300
agggattgca ttgaacctgt agatcagttt ggggaatatt gccatcataa caattagtag 15360
gtcttccaac ccatgaatac aagacttctt tccatttctg tagatattta gtttctttca 15420
ttaatatttt gtagttttca atataaaagt cttgtacttc gattaaattt attcttgaat 15480
attttgggtt ttgatgcttt tatgaatttg ttttcttaat ttcactttaa gattgttcat 15540
tgctactgat tagtaatgca actgattttt gtgtgttgat ttttgtatcc tgcaacctag 15600
ctgaaatcat tgattagcat aatagagtat ttaatagatt taggatttct atatataaga 15660
tcatgtcatc tgcaattaga gataatttta cttcttccct ttcaatctgg acatttttta 15720
cttctttttc ttgcctagtt gccctagcta gaacctccag tgcagtgttg aatagcagtg 15780
gtgagaatga gcatctttgt gttggtcttc atcttgtggg gaaacctttc agtttaagtg 15840
tgttgttgtg gggttttcat agttgtcctt tatcagattg agaatgttcc tttctgttcc 15900
tagtttgttg agtgttttct ttttgattgt tttaatcagg aaagggcatt agattttgtc 15960
aaatgctttt tctgcagcta ttgagatttt tgtgtgtttt tctggtcttt tatggtttat 16020
cacattaatt gattttcata tgtcaaacaa accctgtgtt cttgggtttc atctcacttg 16080
gttatggttt ataatccttt ttatatactt gtagattcag tttgccagta ttttgttgag 16140
gatgcttgca tttatattta taagggatat tggtctgttg tagctgacca gtaagtatag 16200
taagctgtat agtttactaa gtgttccctc tgttttgggg gagactttga gaagaaggat 16260
tgttggtaat tgttctttaa acatttggta aaattcacta gtgaagccat ctggggtctt 16320

ctttggaagt tttttgatta ctaacttaat gtctttactt gtttgttata agtccattca 16380
gatttttttc tccttgagtc atttttgaca gttggttgag gaatttgttc atttcatgta 16440
gttatctaat tggttagtgt ataattattc atagtattcc tttataatct tatttttttg 16500
ctgtaaggtc agtcataatg ttcactcttt catttcggat tctggtaatt taagagtctt 16560
ctctcctttt ttttcttggt cagtctagct aaagtaaagt tttgtccgtt ttcaggggaa 16620
cagctttttt tttttttttg aggcagaatt tccatcttgt cacccagtct agagtgcagt 16680
ggtgcaatct cggctcattg cagcctccgc ttcccgggtt caagagattc tcctgcctca 16740
gcttgccaag tagctgggat tacaagcgcc caccaccacg cctggctaat tttttatatt 16800
tttagtagag acggggtttc accatgttgg gcaggctggt ctcgaactcc tgacctcagg 16860
tgatctgcct gccttggcct cccaaagtgc tgggattaca ggtgtgagct accgtgccca 16920
acccagcttt ggttattttt gttgacctac tctattgttt ttctcttctc tatttcactt 16980
atttctacac tggtctttat tattttcttc cttatgcttg ctttggactt agttcttctt 17040
tttctagtct cttaaggtgg ataattaagt tcctgatttg aattcttact tctttgtaag 17100
gtggtcatgt actgctatga atttccttct cagaaatgta tatgctttca ctgcatccct 17160
taagatttgg tatgttgtat ttttgttttc atttgtctca aggtatagtc ttctgatttc 17220
cattgtgatt tcttccccct ctaacccgtt tattatttag gaacttgttg atttccacat 17280
acctgtgaac tttccagatt tccttctttg ttaattctca gtgtcattcc attctggtcc 17340
gagaacatac tttgtatgat ttctatcttt taaaatttat ttggcttgtc ttatgaccta 17400
atacattgtc tatcctggag gatgtttcat gtacacttga gaagaatgtg tattctgctt 17460
ttgttgggta gagtgtttga caggtgtgtt ggtacatagt tctgttcaaa tctgtttcct 17520
tgcagatttc tatctagttg ttctgtctat tggaagtagg atattgaaat ctccaactaa 17580
tattgctgaa ttgtttattg ttttcttcag ttctgtcact ttttgcttta tatattttga 17640
aattctattg ttaggtacaa gtaagtttat gattattata tcttcttgat agattgattc 17700
ttttatcatt atacagtgcc ctataagaac aatttttatc ttaagtctat tggtctatat 17760
tagtatagcc acttcagctt tcttttgttt actgtttgca tggaatattt tcttctttta 17820
ctttctattt gtgttcttga gtctaaggtg aatctctgta gatagcaatt ggatctgcca 17880
atctttgctt tttatttggg gagtttaaac cattgacatt taatgtaatt attgatgagg 17940
aagattactt ctgatatttt gccatttgtt tcctttattt tgtgtctctt gttcttaaat 18000
tcttccatta ctaccttctt tcttttgtat tacatatttt ctagtgtaac gattttaatt 18060
tctttgtcat ttcttttgtt gtatgttttt agttattttc ttagtggttg ccacggagat 18120
tttattgtca ttttaacagc ctaggttggg cacagtggct catgcctgta atcccagcac 18180
tttgggagac tgaggcagga ggatagcttg agtccaggag ttcaagacca gcctgggcaa 18240
cttactgaga tactgtctct acaaaaaaat acaaaaatta gccaggcatg gtggtgtgtg 18300
cctgtagtcc cagatgcttg agaggctgag ttgggaggat agcttgagcc caggaggttg 18360
aggctgcagt gaactttgat cacaccactg cactccagcc tgggtaccag ggcaaaacta 18420
gcccaaagaa atgaaggaaa aaaaaaatct aatttagatt aatatcaact caacttcaac 18480
agtgtataaa aactttgcct ctgtatacct cttctgcttc cactctgtgc tgttattgtc 18540
atagattttc atctttctac actgtgtgtt tatcaatgta gatttaaaaa tattgcttag 18600
tagttgtctt tagaatccga tacggagaaa aggagatata aacaaaagat gcatttttac 18660
tgtcttgtat gtttacttat gtaattccct ttcctgatgt tgtatttcta aaggcaaagt 18720
agggttattg tgagtgtcct tttgtttcaa cctgaaagac tccttttagc atgtgttgga 18780
gatatgctaa tgatggactc tcacagtttt tgttatctgg gaatgtgtta atttatcctt 18840
catttttgaa ggatagtgtt ggcaggatac agaattcttg gttgacatgt aattctttca 18900
gcattatgaa tatgtcatcg tactgtcttc tgacctccat ggtttctgat aaggaatcaa 18960
ctgttaatct tattgaggat cacttgtttg taatgacttg cttgtcgtgc tgctttcaag 19020
attcattctt tgcctttagc ttttggtagt ttgattgtga tgcatttagg tgtgtacttt 19080
attagtctgt tctacttgga gtttgttgag ctttgtagat gtatttcatc agatgtgtca 19140
agttcttttg ccactatttt ttttttaaat aatctttttg cccctttccg ctccttctgt 19200
cactctgatt atttgtgtgt tgctttgttt ggtggtgtcc cagaagtctc tgagactctg 19260
tccagttttt tcctccccat tcttttttct ttcacttcct cagactggat gatctcaatt 19320
tgacctatct tcgagttcat ggattttctc ttctccaagt gacatctgtg agatgaattt 19380
ttttctagag aatttttcat ttcagttatt ctacttcaaa atttctcttt ggttcagttt 19440
tatcattgct atctttatat tattctcagt ttaatgagat actgttttat actttccttt 19500
agttctttag acatagttta tgtcactgaa tatatttaaa atagctgatt ttaagtcttt 19560
tttttttatt tttttggaga tggagtctcg ctctgtcacc caggctggag tgcagtggca 19620
cgatctcagc tcactgcaag ctccacctcc tgggttcacg caatgatttt aagtctttgt 19680
ctatgaagtc tagtatctgg gcttcctcag gcatagtttc tgttttcttt ctttcttttc 19740
ctgtgtactt cgtttctttg tataccttgt aattgttgtt gttaactgga cattttgaat 19800
attatagtgt aacaactctg gcagtcagac tgtctcccct ccccagtatt tgttgttggt 19860
gagtattgta gatgtttgtt tagtgacttt tcatggctaa ttctgtaaat tttatattct 19920
ttgaagattg tgggcaccct gaagtctctg tttgttagtt tagtggtcac ctaataatta 19980
acagagattt cattaaatgc ctagaagcaa aatatcttcc agtctttgcc catggcctct 20040
gtgtatgcat tagggcaggc cttgaactct tacccaggga gtttacaacc ctgccttagc 20100
ctttactacc agcttctgca gagcattaag gtcaacaggt ggtgagagtt tggagcctac 20160
tccatctttc ctgagcgtat acacagccct actcatgcat gtggccctct agatttccag 20220
gaatatgttg gaccctttca aagcccttac agactcccca gcttttcctc tcaatcttta 20280
gactagtgtg ttgttttctt caacagttat ctgtcaggca gcagcaaatt aagagattag 20340

cataaatgtt ttcaactcct ccacccgtca tgtgccccag ggaagcacta agccagttct 20400
aagttaggca aaataaagac aatccttttg aggtggtctt ccatggagtc accagacagg 20460
taaaccaaat aattaattac aagtctttgg ctggatacag tggctcacac ctgtaatccc 20520
ggcactttgg gaggctgagg caggtggatc acaaggtcag gagattgaga ccatcctggc 20580
taacacggtg aaaccctgtc tctactaaaa aatacgaaaa aataggtggc tgtggtggcg 20640
ggcgcctgta gtcccagcta ctcgggaggc tgaggcagga gaatggaatg aacccaggag 20700
gtggagcttg ccgtgagccg agatcacact actgcactcc agcctgggtg acagagcaag 20760
actccgtctc aacaaaaaaa aaaaaaaaca agtcttcatg aaagaggtcc attctgctgt 20820
ctttcatacc aggaatgtgg aatgtggact gttattttca tggctactgc taagctagga 20880
atcaagggat agatggggac tgggtaaaac accacagagt ttgctgttct taccaagaat 20940
aagctgggga agagggttgt ttttgttttt cagtaaaaat tccctgggct gcttcaagcc 21000
gttgattaat tttcaggttc cgaaaaagtt cagtttgaca gtttttgccc tttttatttg 21060
cttttatgga tatgtagaac ttgagttctt ttttccacca gttttgctga cattgtttta 21120
aaagcacttt ttgtaaaacc caaatgttgt ctctctcaag gctagccaat aattaaaaat 21180
actgttactc ccctttgatt ttggaaatga attcgtattg accaaaattc aatactagag 21240
gtctttcaag ctgttttacc atttatctaa actttagaat ctaatgattc ctgtacattg 21300
tctagcatac tggtggtcct caattgtcat aagttcaact ttggaacaaa tgaacttttt 21360
gtgtgcaagt ttccaattgt ttggaaatta cattgatgcc ccctccatca aactgttatt 21420
cgtgggacat ctaggaattt cttacagcag ctgacaaata tttcaagtca gtgcctggta 21480
gtactgtcca ccaggcaaca gcttcagtag tagagcgatc tttatctata aggcagtgtt 21540
tgagcaattg tttattagtg ttttcctaac tactcagaag aactatcagg ggttatagag 21600
gtagctcaga gagttgggtg caagtagaga aatccacccg gcttgcatta cacatcttat 21660
ttctagagaa gctttccttt gaagaaagag ttctaaggtt taaaaaatta ccttgaatgc 21720
cacttatatt gcattttaat tttattttag agaaatcaat ggaaagtaga aaaattaagg 21780
cactgatact agtgttaaga atgttggtta aagcttctgg caattaattt tttatttcct 21840
tttttaattt tattaaaatt taacaatttt cagtttatgc tgtaatccag accaaggttt 21900
caatctaatg aagttaatgc cagtgttgct gctacctatt ttgtctttag tcattcagcc 21960
atgcttccta cttatactga ataagctagc ttaatctaac aatcaaaaaa gaaagctgtt 22020
gcctaagtta agaaaaacag tttgaactgt tttcaaacta aatacccagt agactctcta 22080
gttgttgaca ggagaatgct taattcagaa ttgtcctgca gtagatcatt ttatctcatt 22140
cctgttcttc tataggatag cttatttgtt tgaaattgta tttaatatgt tgtgattttt 22200
gtgtgcttgt ttctattttt cactggatag actcaagata aaacctggta ccctgcagtg 22260
tagctatcag tttatagcag aggaaattta cattagaact tggctgtgta tttacatgta 22320
tctaacttgg aggtcactct gcttactgtt gatatatcag tcatattaga tgagtcccta 22380
atgagatacc agaaaccccg gaaacatcat taggtggaac agtgtcctta atgctttatt 22440
aagtgttata ggtaagacaa agcctagtac tatttgtggc atcaaggtta ggtgtttaaa 22500
gacctgtatt cttctattgt catgttgaaa ttgttccctt gatgtagcaa tagaaaattt 22560
tagattaggc ttaagttaat cagcaaacaa agataaaagt ctgatactat cctaaatatt 22620
ttgtgtttct aaataattta acagtgatcc aattagctac tcctgtagaa atgtaattga 22680
taaacttttc actctctttt aaattgccat cttgaatttt acctgttttt taaagctgtc 22740
tcaagtcctc tctaaaaaaa ggcagtcatt tataaattta gaaaagcttg atagcacaga 22800
aagtcacaga aaaatgtaaa catagtttaa aactgaattg tatacaagcc actagaagta 22860
cttttattaa gtttacaaat attagtagag tggaactcat gcatttaata tgtttgaaac 22920
ttttgatcaa atactgtgct atgaaaaaca ttttagataa ttattcttta atcatgtgtg 22980
tgtaaaatgt ggcttttttt gacaaccaag tagcttttct gtgtgccaaa ctgtgacttt 23040
aaaattttaa agtactcaac agagtaaaca aaccacaaat accacttaaa ctgtacacat 23100
ttgcacatgc atttcctata aatagtacat gggtttcaag tcttcacttt tgaaattcag 23160
aaatgggttt tttctccttc cagtagaaat aaaaacttga tttattttat ttatttattt 23220
attttatttt tgagacggag tctcgttctg tggcccaggc tatggtgcag gagggtgatc 23280
tcagctcact gcaacctctg cctcctgggt tcaagtgatt ctcctgcctc agcctgccga 23340
gtagctggga ttacaggtgc ctgccaccat gcccagctaa tttttgtatt tttagtagag 23400
atggggtttc tccatgttgg gcaggctggt ctcgaactcc tggcctcagg tgatctgtct 23460
gtctcagcct tccaaagtgc tggggattac aggtgtgagc caccgcatcc agctaaaaac 23520
ttgattttta aaaatccaaa tcgaagacag aattgtgtat tttagtacat ttattagcag 23580
ccttgacgct ataccatatg gctgtttatc atttaaacag cttgtaaaag caaacacttc 23640
aggattcatg agtggcagaa ggactgagta ctttgggaaa taagagagaa cttttgttga 23700
ggatggttga ggaagagtcc aagacaataa taggcagaat aagcaaaaat ctagagactc 23760
attgtaggca ctcaagtatg tatttgttag aatgaatggc tgaacttggt atattgagga 23820
acactgagaa agccatactg actggaagat agttcctaca agaaactggt gagacatatg 23880
ttacagtcta gattttggtg agccttgtta aagtttgggc tttattttta tacggggaga 23940
aagtttcaca ggggtttgga aatgaggctt ggagctgtta atggggacac agtgaggttt 24000
tagggtagtg gctttcaaac tgtttaaatc caaactttga tgataaccct gacataacta 24060
ttgtttataa cttccatttc agttgtattg gttttatcaa aacatcttca ttgatcttac 24120
tgattgcttc ctatgcagat taatattata aatttgaatg tacaaaggaa gctttagcag 24180
taaaatagca acttttatct gtcttacgta ttggaggttc tgcataagat ttaatttttt 24240
ttttttttga aatggagttt tgctcttgtt cacggggctg gagtgcaatg gtgtgatctc 24300
ggctcaccac aacctctgcc tcccgggttt aagtgattct cctggctcag cctcccaagt 24360

agctgggatt acaggcatgt gccaccatgc ccggctaatt ttgaatttta gtagagacgg 24420
ggtttctcca tgttggtcag gctggtctcg aactcctgac ctcaggtgat ccgcctgcct 24480
cagcctccca aagtgctggg attacaggcg tgagccaccg cgcccggcca agatttaatt 24540
ttttaaaaga aaatattttg ctaagggttt ggaaactctt gttttagcaa gaatggatta 24600
agactgatta aaactaaagg caaagaggag gctcttatgt ttggaattct ttgctaatat 24660
ttacacaata taattctctc cacaaatatt taatggtacc agatattaga tggttataat 24720
ggcaaaagtg ttcaaaggat gctatcatat tcatgattca tgatcaaaat gaacattata 24780
aggctatccc tcttcagaat taaatacgtt actcctgtgg aaaacttgct tttaatgtag 24840
aagttgtccc agagcctttc ttcctttctc atgtcctctt atgtccactg ctgagctaac 24900
atgggtctca ctgaatgatt aagaaaaaac atcttaggtg gggagttctg tatatagtaa 24960
atgtttaatt tattggggtg gtgaacggga agtgctgctg gcaagagagg atgggaagag 25020
aaatctaccc aaatccttac ccgctttaca gaacataaac ttcctattca gtagtacaca 25080
ataacttaac gatcaaggca tcttaacttt tctgttttca gatgaaagaa ctatcgtttg 25140
gcttgatcaa gtatttagta tttattcgtt cactcaagtg cttacgtttt tttgttatct 25200
cagggtttta cgttagttat taaccaaaag aactagtttt agttctggaa gtctaaaata 25260
tataagagaa ggtgaggagt aataagagaa gatgaaggga gactttcgga atggcctatg 25320
aacttctagt aactatacca ccttaaaata gacaaattac aatgcagtta tgaagatatg 25380
tatttttcag tgaagacaac taaaatgttt gcacagaatt ttctttttta ttgagtgtta 25440
gaaattctat tttggagata ctaccttgca caacataaaa agaaaaagtg agtgtggaat 25500
ctaggaatct acgtggctct aggaaatttt ttaagtgtgg aaactgaagg agagcaagag 25560
aaagggagca tggcattccc ctgtttgtag ttcatgaggt gggtttaaat tgccttttgc 25620
caatgcagct gcacactgag gattacagaa ttctttttaa atgtttgtag aattattttt 25680
cacttattag gtaaaacgtg tattttttga ttttctccaa tttcagcttt ctcatgttgc 25740
tatgctcaat tttgtatacc atatatagtt ttgttaaatt gacaaagtgg tgttttttgt 25800
tcttcttttt cccattggtt aaaatttaaa gagaaagtgg aagctagaaa tttatctaaa 25860
aaatgtaact ttccctgtaa ttattaaagt atcaaatcta aatttgaatt ttctttgtgc 25920
ataatctttt ttcaagctat ttaccatgtt gacaaacttg ctttcctgtg gcaaatacac 25980
tagcaatacg ttataaatat gtaactttca acctatttac agttgatgct tttttagccc 26040
tttggattta aaatacaagc actgaagagg tgaggaagta ccactgctgc ctcagcatta 26100
tttcgaaatt ctgtttataa actatacaat ttccaaggtc atgaatccag cacctttcca 26160
ggtactaact attgggacaa agatagaatt tgattttatt tatttaccta ttgactgaag 26220
tctaacttaa atcttgcacc tagtaagatc ttagaaataa cgtgtgtact ctgacctgta 26280
aactaatcct agtattctgt gtgtatattc tttctcattt gggctcttaa aaggaaaagt 26340
aacgtacatc tgatgatcat tagcactgag ctttttcagc aaaaagtata tgtttataaa 26400
gaagtatagg ataatttagt aatttaataa tgtgacaaca tttgcgtgtg tttttttttt 26460
tgagaaatac aaattgtgag aaacagaaaa gtaaaagaag cagcagcaga aatatcacta 26520
taggatcaaa agattgcagg aaccaaaact ccaaaattat tgggcataat gtactaaaaa 26580
cagggcagtg gaggaaaggg acagtccaga ctagctctga gggtccaaag aaagtattaa 26640
atattgttac tggagtgatt tgctctgcta tttgggcttg ggaattaagt gaaattgttg 26700
atatactaga cagatacttc ccacccattt ttctcttgat aatcagggtt cattttttct 26760
attttctatt tctctggatg ctccatttct taatattaat attaatatta agctctcagt 26820
ctttatgcta aaaattggtt atttaaaaca atttaaatca acttcagtct aattggctta 26880
agttcaaatc cattttaaga tcgatattgt gtcctttaaa aattttattt aaaagatatt 26940
taaactgatg agaggatact acccattcca ctgataaact attactgtaa gtttgtctat 27000
tgagggctag ttatttggtt taaaaatgct gagattatgg aaagtggatt ggaatatttt 27060
ggagcaatat taaaaacagt atctgtaaca atttaataaa cttataaatt cctctttctc 27120
tgttgatcta tcttgaaaag acactctatg tctctaggca ttccttctct gtggtgtgat 27180
tggtagacag ggagtaaaca acttactgta aatgggcacc atgccagttg gcttcaggca 27240
gcatcaagct tgtgactcac agtcagggtt aggaaaatgc cttttaactt gtttgtctct 27300
gcctctttta aacattaaag gcacaactgt actaattatt aagtatttca taaggtcttt 27360
tagggcttat aagatctttt aggaatggcc tggaagttat tagtactgtt tcattgaatc 27420
tgaatacctt taacatgata atgagaagtt tttaaagggt ggttttatag ttaaacggaa 27480
tttctcaaat tggcttgctc cttatgttga tttatttagg atcacatttg ggagtttctc 27540
tgccctactt tcaatgtatt taatttactg accatcacta tttgggggga aaatgttata 27600
tgatatttag aaaccaagag ttttggagtt tttcccccat tagatgtatt tatttattta 27660
tttattattt tttaaagaca gggtcttgct ctgtcaccca ggctggagca cagtggcatg 27720
atcctagctc actgtattct tgaactcctg ggctcagact gtcctcccac ctcagcccaa 27780
gtggctaagt atcaagtaag aatcacctgg caaattccaa ggctgtatac cagatttcct 27840
aaattagaat tttggggttg ggtatctgaa ttttagtaaa gccctccaaa tgtttctggt 27900
attgcttcta agaacaattg ataacataat agctgtggcc attatagggg tattctgtca 27960
tatttagata taggcatacc ttgttttatt gtacttccca aatattgcgt gtttattttg 28020
ttttgtttca cttacaaatt gaaggtttgt ggcaacccta tattaagcga gtctgtcagt 28080
gccatttttc caacagcttg tgctcatttt gtgtctctgt gtcacatttt ggtaattctc 28140
tcaatatatc aaactttttc atcatttttg tatctgttac gaccagtgat cagtgatctt 28200
tgattttttc tttttttttt tttttttgag acggactttt gctctgtcac ccaggctgga 28260
gtgcagtggt tcaatcttgg ctcacagcaa cctctgcctc ccaggttcaa gcaatcctcc 28320
tgcctcagcc tccccagtag ccgggcctac aggcgtgtgc caccacgcct ggctaatttt 28380

tgtattttta gtagagatgg ggattcccca tgttggccag gctggtctcg aactcctgac 28440
ctcaggtgat ccgctcacct tggcctccca aagtgctggg attaccgtgc cagcctgatg 28500
ttactatttt aattgttttc aggcaccata aacctcacct gtataaggca ccgtacttaa 28560
ttggtaaata ttgcgcatga tctgactgct cttccaactg gccattccct gtctgtctcc 28620
ctcttcctgg gactctcaaa tccctgagag acaataatat taaaattaag ctaattaata 28680
accctacagt ggcctctaag tgttgaagtg aaagagttgc atgtctctca ctttaaataa 28740
aaagctagaa gtggctaaac ttagtgagga aggcacatca aaagccaaga caggccaaaa 28800
gcaaggactc ttgtactaaa cagctaaatt gtgaatgcaa aggaaaagct cttgaaggaa 28860
ataactagtg ctactccagc aaacatgtga atgatcagaa agtgaaacag ccttcttgct 28920
gatacgaaga aagttttagt ggtctggaca gaagatcaaa ccattcacaa cattccttta 28980
agccaaagct taactctctt caattctatg aaggctgtga gaggtgagaa agctgcagaa 29040
gaaaaattgg aagctagcag aggtcggttg atgaggttta gggaaagaag ccagcgctgt 29100
aacataaaag tgtaaggtga agcagcaagt gctgatacag aaactgcagc aagttatgta 29160
gaagatctag ctaagattac taaataatag attttccatg tagatgaaaa agccttttgt 29220
tggaagaaga tgccatctag gactttcata gctagaaagg agtcaatgtc tggcttcaga 29280
ggacaggctg acattcttgt taggggctaa tgtagttggt gactttaagt tgaagccagg 29340
tctcatttac cactccaaaa atccgaagac ccttaagact tatgcttaat ctactctgct 29400
tgtactctag aaatgaaaca acaaagcctg gatgacagca catctgttta tagtatgctt 29460
cactgaatat tttaaggcca ctgtaaagac ctgttcaact gctcagaaaa aaatgattac 29520
tttcaaaata ttgctgttca ttgacagtgc acctgggctc acccaagagc tctaatggaa 29580
ttgtacaaca agatggatgt tgttctcatg cctgccaaca catcatccat ttgtagccca 29640
tgaatcaggg agtgatttca agtttcaaat cagtacattt tgtaaggcta tagctgctat 29700
agacagtgat tgctctggtg gacctgggca aagtaaatca aaaaccttct gaaaaggatt 29760
ggccattcta gatgctatta agaatttgtg attcgcagga ggaggtcaaa ggatcaacat 29820
tagtagcagt ttgaaagaag ttgattccaa cagttataga tgaatttgag gggttcaaca 29880
cttcagttta ggaagtcact gcagatgtgg tagaaacagc aagagaacta gaattagaag 29940
tggagcccga aaatgtgacg gaattgctgc aatctcatga gaaaacgtga atggatgagg 30000
agttgcttct tatggacaaa tgagcaaata aatttttttc ttgagatgga atctactcct 30060
ggtgaagatt ctgtgaacct tgttgaaata acaacaaagg atttagaata ttacataaac 30120
ttaattggta aagcagcagc atggtttgag tggattcatt ccagttttga aagagtttct 30180
actgtgggta aaatgctatc aaacagcatc tcgtgctaca aagaaatctt ttatgaaaag 30240
aaaagtgaaa cttcattgtt gtctacttta agaaattgcc acagccaccc caccttcagc 30300
aaccacctct ctgatcagtc agcaggcatc aacactgaag caagaccctc cacaaggaaa 30360
aagattacaa ctcactgaaa gttcaaatga ttgttagcat ttttaagcaa tattttaaga 30420
ttaaggtaaa tacattttta aagacacaat gctattgcac acttaataga ctacagtata 30480
gtataaatat aacttttata tgtagtggga aaccaaaaaa ttcgtctgac ttgctttgtt 30540
gcaatattca ctttattgtg gtctagaacc gaacctgaaa tatctcagag gtatgcctgt 30600
attaatatta ttttgcaagt aaaaaaccca gcatataaaa aaaacgtaga atatgttgag 30660
agttcagtaa tatggatgaa aatgtttttc tctaactgaa gaacatgata aattataatt 30720
agggaaggat ataaaccaag aaaatatgtc tgagatagcc aattcttgca gttcataata 30780
tgaaaactca ttataccaat ctcagtaaga atacttttaa tagctgttat ttctttggga 30840
tatagaattt ataaagtaca cagtaatctt cttatgatca atcctaggat cactttacaa 30900
ccacttaccc catattacaa tgtagtacca agacaagcag accaaattat agaaggacaa 30960
agtttttgct aagcatattt tgtcatcagc ataccgcatt gtgtgtgcat gcatgtgtgt 31020
gtttgtgcat gtgtgtgatt gtataaaata ttagaaagcc accccagaaa agttaaatga 31080
ctaggaatgt tgtgaaggga ttaagctacc cctaaaatta tataacaaaa ctctcttcat 31140
ctattattag gtcatcttta gaacatcttc tcttaaattt gttataggtc tctctcatct 31200
gtttggatta aaattggtct gaaagcctaa aatggctttt tacctatata attatttccc 31260
aactagcttg tagtataggt gcaaagctat cacacttgct aggttagtga agtatgtaaa 31320
aactaccatc tttcaattag gaaccatcgg atagcttcta caggattgct ggggagaacc 31380
tttataaaga aagttatatc tttataaatt ttttgtcatt ttacttagct gagaatataa 31440
aataagttag ctaataatag agtagaaatg ttttctgtaa cagattaata ttgatcaaat 31500
gtgttattaa atgctaaaac accatttttt ttctctgtaa gccatgtgtt tcatgccaca 31560
acacaaaagg gacaattgtc tgtgttttat gacagttctg ttctgtcaga tgctgtttgt 31620
tcattttggt gaataaatga agagagccct ggacacatct ttttttcctc aacaaaagag 31680
gaaaattatt cttgtctgta tgtctataat cctgactctt tgaatggctt taattttttt 31740
aaagtcagca tttttttata aagataggtg tttggaatgt gggcgatatg gctggacagt 31800
tagattggga ccaaataatg gaaggctttg aacatcatgc taagaggttt gggttttact 31860
ctgaaggcag tagagaacca ttatgttttt aagccaggat tgacttgttc taagctgtac 31920
cttagaaata ttactctggc agttgtacat aggatgagct gtatgttgct ttgttttgtt 31980
tggggagaca gttctcgaag agagactaca tacgaaggca gttatatgag tcattactaa 32040
aggtctggca agaagtagta aaagcattaa ctggagtggt agcagtaggg aaggaaataa 32100
aaggatagat gtgggagtca tttggaaagt atgaggcaat tcattgacct tacagaatca 32160
ctggttttct gcttccactc cattcacatt gacctttcca aggttatcag tgacctgctt 32220
gtccttaaat tcagtgggca ctttccagta acctactgtt ggcaccagcc ctgtgctaga 32280
caccaggatc ctgtttgtaa aggcatctgc cagtggtttc tgtgacacaa ttctgtttct 32340
agttttcctc cttctacttc tctagcctct tggcaagttc ttctttcaga gtttctcaga 32400

gctttgtgct aggccctctt ctcattttct ccttctctaa gtgatcccat ccttttctgt 32460
tgcttcagtt accatttgtc cttatgcaaa ggacagccat atctactgta tctccagctc 32520
agatgtatct ctttgcctcc tgacccatat ttccaactat ctaactgggt atcttttctt 32580
ggatgagtta taggtctctc aaacacaaca tgtccagaat aattcattga cttattctaa 32640
ggcctgcttc ctctttctcc tgtagtccct atctcaggaa atatatggtg ctatcaaccc 32700
caaagcagaa atctggacat aatccctaac tacccttttc ccctctctgt gcacataatt 32760
tcagtca 32767

<210> 26
<211> 3791
<212> DNA
<213> Artificial Sequence

<220>
<223> H. sapiens

<400> 26
tttttagaaa aaaaaaatat atttccctcc tgctccttct gcgttcacaa gctaagttgt 60
ttatctcggc tgcggcggga actgcggacg gtggcgggcg agcggctcct ctgccagagt 120
tgatattcac tgatggactc caaagaatca ttaactcctg gtagagaaga aaaccccagc 180
agtgtgcttg ctcaggagag gggagatgtg atggacttct ataaaaccct aagaggagga 240
gctactgtga aggtttctgc gtcttcaccc tcactggctg tcgcttctca atcagactcc 300
aagcagcgaa gacttttggt tgattttcca aaaggctcag taagcaatgc gcagcagcca 360
gatctgtcca aagcagtttc actctcaatg ggactgtata tgggagagac agaaacaaaa 420
gtgatgggaa atgacctggg attcccacag cagggccaaa tcagcctttc ctcgggggaa 480
acagacttaa agcttttgga agaaagcatt gcaaacctca ataggtcgac cagtgttcca 540
gagaacccca agagttcagc atccactgct gtgtctgctg cccccacaga gaaggagttt 600
ccaaaaactc actctgatgt atcttcagaa cagcaacatt tgaagggcca gactggcacc 660
aacggtggca atgtgaaatt gtataccaca gaccaaagca cctttgacat tttgcaggat 720
ttggagtttt cttctgggtc cccaggtaaa gagacgaatg agagtccttg gagatcagac 780
ctgttgatag atgaaaactg tttgctttct cctctggcgg gagaagacga ttcattcctt 840
ttggaaggaa actcgaatga ggactgcaag cctctcattt taccggacac taaacccaaa 900
attaaggata atggagatct ggttttgtca agccccagta atgtaacact gccccaagtg 960
aaaacagaaa aagaagattt catcgaactc tgcacccctg gggtaattaa gcaagagaaa 1020
ctgggcacag tttactgtca ggcaagcttt cctggagcaa atataattgg taataaaatg 1080
tctgccattt ctgttcatgg tgtgagtacc tctggaggac agatgtacca ctatgacatg 1140
aatacagcat ccctttctca acagcaggat cagaagccta tttttaatgt cattccacca 1200
attcccgttg gttccgaaaa ttggaatagg tgccaaggat ctggagatga caacttgact 1260
tctctgggga ctctgaactt ccctggtcga acagtttttt ctaatggcta ttcaagcccc 1320
agcatgagac cagatgtaag ctctcctcca tccagctcct caacagcaac aacaggacca 1380
cctcccaaac tctgcctggt gtgctctgat gaagcttcag gatgtcatta tggagtctta 1440
acttgtggaa gctgtaaagt tttcttcaaa agagcagtgg aaggacagca caattaccta 1500
tgtgctggaa ggaatgattg catcatcgat aaaattcgaa gaaaaaactg cccagcatgc 1560
cgctatcgaa aatgtcttca ggctggaatg aacctggaag ctcgaaaaac aaagaaaaaa 1620
ataaaaggaa ttcagcaggc cactacagga gtctcacaag aaacctctga aaatcctggt 1680
aacaaaacaa tagttcctgc aacgttacca caactcaccc ctaccctggt gtcactgttg 1740
gaggttattg aacctgaagt gttatatgca ggatatgata gctctgttcc agactcaact 1800
tggaggatca tgactacgct caacatgtta ggagggcggc aagtgattgc agcagtgaaa 1860
tgggcaaagg caataccagg tttcaggaac ttacacctgg atgaccaaat gaccctactg 1920
cagtactcct ggatgtttct tatggcattt gctctggggt ggagatcata tagacaatca 1980
agtgcaaacc tgctgtgttt tgctcctgat ctgattatta atgagcagag aatgactcta 2040
ccctgcatgt acgaccaatg taaacacatg ctgtatgttt cctctgagtt acacaggctt 2100
caggtatctt atgaagagta tctctgtatg aaaaccttac tgcttctctc ttcagttcct 2160
aaggacggtc tgaagagcca agagctattt gatgaaatta gaatgaccta catcaaagag 2220
ctaggaaaag ccattgtcaa gagggaagga aactccagcc agaactggca gcggttttat 2280
caactgacaa aactcttgga ttctatgcat gaaaatgtta tgtggttaaa accagaaagc 2340
acatctcaca cattaatctg attttcatcc caacaatctt ggcgctcaaa aaatagaact 2400
caatgagaaa aagaagatta tgtgcacttc gttgtcaata ataagtcaac tgatgctcat 2460
cgacaactat aggaggcttt tcattaaatg ggaaaagaag ctgtgccctt ttaggatacg 2520
tgggggaaaa gaaagtcatc ttaattatgt ttaattgtgg atttaagtgc tatatggtgg 2580
tgctgtttga aagcagattt atttcctatg tatgtgttat ctggccatcc caacccaaac 2640
tgttgaagtt tgtagtaact tcagtgagag ttggttactc acaacaaatc ctgaaaagta 2700
tttttagtgt ttgtaggtat tctgtgggat actatacaag cagaactgag gcacttagga 2760
cataacactt ttggggtata tatatccaaa tgcctaaaac tatgggagga aaccttggcc 2820
accccaaaag gaaaactaac atgatttgtg tctatgaagt gctggataat tagcatggga 2880

tgagctctgg gcatgccatg aaggaaagcc acgctccctt cagaattcag aggcagggag 2940
caattccagt ttcacctaag tctcataatt ttagttccct tttaaaaacc ctgaaaacta 3000
catcaccatg gaatgaaaaa tattgttata caatacattg atctgtcaaa cttccagaac 3060
catggtagcc ttcagtgaga tttccatctt ggctggtcac tccctgactg tagctgtagg 3120
tgaatgtgtt tttgtgtgtg tgtgtctggt tttagtgtca gaagggaaat aaaagtgtaa 3180
ggaggacact ttaaaccctt tgggtggagt ttcgtaattt cccagactat tttcaagcaa 3240
cctggtccac ccaggattag tgaccaggtt ttcaggaaag gatttgcttc tctctagaaa 3300
atgtctgaaa ggattttatt ttctgatgaa aggctgtatg aaaataccct cctcaaataa 3360
cttgcttaac tacatataga ttcaagtgtg tcaatattct attttgtata ttaaatgcta 3420
tataatgggg acaaatctat attatactgt gtatggcatt attaagaagc tttttcatta 3480
ttttttatca cagtaatttt aaaatgtgta aaaattaaaa ccagtgactc ctgtttaaaa 3540
ataaaagttg tagtttttta ttcatgctga ataataatct gtagttaaaa aaaaagtgtc 3600
tttttaccta cgcagtgaaa tgtcagactg taaaaccttg tgtggaaatg tttaactttt 3660
attttttcat ttaaatttgc tgttctggta ttaccaaacc acacatttgt accgaattgg 3720
cagtaaatgt tagccattta cagcaatgcc aaatatggag aaacatcata ataaaaaaat 3780
ctgctttttt c 3791

<210> 27
<211> 3557
<212> DNA
<213> Artificial Sequence

<220>
<223> H. sapiens

<400> 27
ggcgccgcct ccacccgctc cccgctcggt cccgctcgct cgcccaggcc gggctgccct 60
ttcgcgtgtc cgcgctctct tccctccgcc gccgcctcct ccattttgcg agctcgtgtc 120
tgtgacggga gcccgagtca ccgcctgccc gtcggggacg gattctgtgg gtggaaggag 180
acgccgcagc cggagcggcc gaagcagctg ggaccgggac ggggcacgcg cgcccggaag 240
ccccgacccg cggagcccgg cgcggggcgg agggctggct tgtcagctgg gcaatgggag 300
actttcttaa ataggggctc tccccccacc catggagaaa ggggcggctg tttacttcct 360
ttttttagaa aaaaaaaata tatttccctc ctgctccttc tgcgttcaca agctaagttg 420
tttatctcgg ctgcggcggg aactgcggac ggtggcgggc gagcggctcc tctgccagag 480
ttgatattca ctgatggact ccaaagaatc attaactcct ggtagagaag aaaaccccag 540
cagtgtgctt gctcaggaga ggggagatgt gatggacttc tataaaaccc taagaggagg 600
agctactgtg aaggtttctg cgtcttcacc ctcactggct gtcgcttctc aatcagactc 660
caagcagcga agacttttgg ttgattttcc aaaaggctca gtaagcaatg cgcagcagcc 720
agatctgtcc aaagcagttt cactctcaat gggactgtat atgggagaga cagaaacaaa 780
agtgatggga aatgacctgg gattcccaca gcagggccaa atcagccttt cctcggggga 840
aacagactta aagcttttgg aagaaagcat tgcaaacctc aataggtcga ccagtgttcc 900
agagaacccc aagagttcag catccactgc tgtgtctgct gcccccacag agaaggagtt 960
tccaaaaact cactctgatg tatcttcaga acagcaacat ttgaagggcc agactggcac 1020
caacggtggc aatgtgaaat tgtataccac agaccaaagc acctttgaca ttttgcagga 1080
tttggagttt tcttctgggt ccccaggtaa agagacgaat gagagtcctt ggagatcaga 1140
cctgttgata gatgaaaact gtttgctttc tcctctggcg ggagaagacg attcattcct 1200
tttggaagga aactcgaatg aggactgcaa gcctctcatt ttaccggaca ctaaacccaa 1260
aattaaggat aatggagatc tggttttgtc aagccccagt aatgtaacac tgccccaagt 1320
gaaaacagaa aaagaagatt tcatcgaact ctgcacccct ggggtaatta agcaagagaa 1380
actgggcaca gtttactgtc aggcaagctt tcctggagca aatataattg gtaataaaat 1440
gtctgccatt tctgttcatg gtgtgagtac ctctggagga cagatgtacc actatgacat 1500
gaatacagca tccctttctc aacagcagga tcagaagcct atttttaatg tcattccacc 1560
aattcccgtt ggttccgaaa attggaatag gtgccaagga tctggagatg acaacttgac 1620
ttctctgggg actctgaact tccctggtcg aacagttttt tctaatggct attcaagccc 1680
cagcatgaga ccagatgtaa gctctcctcc atccagctcc tcaacagcaa caacaggacc 1740
acctcccaaa ctctgcctgg tgtgctctga tgaagcttca ggatgtcatt atggagtctt 1800
aacttgtgga agctgtaaag ttttcttcaa aagagcagtg gaaggtagac agcacaatta 1860
cctatgtgct ggaaggaatg attgcatcat cgataaaatt cgaagaaaaa actgcccagc 1920
atgccgctat cgaaaatgtc ttcaggctgg aatgaacctg gaagctcgaa aaacaaagaa 1980
aaaaataaaa ggaattcagc aggccactac aggagtctca caagaaacct ctgaaaatcc 2040
tggtaacaaa acaatagttc ctgcaacgtt accacaactc acccctaccc tggtgtcact 2100
gttggaggtt attgaacctg aagtgttata tgcaggatat gatagctctg ttccagactc 2160
aacttggagg atcatgacta cgctcaacat gttaggaggg cggcaagtga ttgcagcagt 2220
gaaatgggca aaggcaatac caggtttcag gaacttacac ctggatgacc aaatgaccct 2280
actgcagtac tcctggatgt ttcttatggc atttgctctg gggtggagat catatagaca 2340
atcaagtgca aacctgctgt gttttgctcc tgatctgatt attaatgagc agagaatgac 2400
tctaccctgc atgtacgacc aatgtaaaca catgctgtat gtttcctctg agttacacag 2460

gcttcaggta tcttatgaag agtatctctg tatgaaaacc ttactgcttc tctcttcagt 2520
tcctaaggac ggtctgaaga gccaagagct atttgatgaa attagaatga cctacatcaa 2580
agagctagga aaagccattg tcaagaggga aggaaactcc agccagaact ggcagcggtt 2640
ttatcaactg acaaaactct tggattctat gcatgaagtg gttgaaaatc tccttaacta 2700
ttgcttccaa acatttttgg ataagaccat gagtattgaa ttccccgaga tgttagctga 2760
aatcatcacc aatcagatac caaaatattc aaatggaaat atcaaaaaac ttctgtttca 2820
tcaaaagtga ctgccttaat aagaatggtt gccttaaaga aagtcgaatt aatagctttt 2880
attgtataaa ctatcagttt gtcctgtaga ggttttgttg ttttattttt tattgttttc 2940
atctgttgtt ttgttttaaa tacgcactac atgtggttta tagagggcca agacttggca 3000
acagaagcag ttgagtcgtc atcacttttc agtgatggga gagtagatgg tgaaatttat 3060
tagttaatat atcccagaaa ttagaaacct taatatgtgg acgtaatctc cacagtcaaa 3120
gaaggatggc acctaaacca ccagtgccca aagtctgtgt gatgaacttt ctcttcatac 3180
tttttttcac agttggctgg atgaaatttt ctagactttc tgttggtgta tcccccccct 3240
gtatagttag gatagcattt ttgatttatg catggaaacc tgaaaaaaag tttacaagtg 3300
tatatcagaa aagggaagtt gtgcctttta tagctattac tgtctggttt taacaatttc 3360
ctttatattt agtgaactac gcttgctcat tttttcttac ataatttttt attcaagtta 3420
ttgtacagct gtttaagatg ggcagctagt tcgtagcttt cccaaataaa ctctaaacat 3480
taatcaatca tctgtgtgaa aatgggttgg tgcttctaac ctgatggcac ttagctatca 3540
gaagaccaca aaaattg 3557

<210> 28

<220>

<400> 28
000

<210> 29

<220>

<400> 29
000

<210> 30
<211> 20
<212> DNA
<213> Artificial Sequence

<220>
<223> Antisense Compound

<400> 30
ttgatgtagg tcattctaat 20

<210> 31
<211> 20
<212> DNA
<213> Artificial Sequence

<220>
<223> Antisense Compound

<400> 31
tggcttagta aatatgttaa 20

<210> 32
<211> 20
<212> DNA
<213> Artificial Sequence

<220>
<223> Antisense Compound

<400> 32
cttcccttcc cagattagtg 20

<210> 33
<211> 20
<212> DNA
<213> Artificial Sequence

<220>
<223> Antisense Compound

<400> 33
aaccatcatc cacagtttac                                                    20

<210> 34
<211> 20
<212> DNA
<213> Artificial Sequence

<220>
<223> Antisense Compound

<400> 34
agttggtaag gtgcacacag                                                    20

<210> 35
<211> 20
<212> DNA
<213> Artificial Sequence

<220>
<223> Antisense Compound

<400> 35
gaaacctggt attgcctttg                                                    20

<210> 36
<211> 20
<212> DNA
<213> Artificial Sequence

<220>
<223> Antisense Compound

<400> 36
accagacagt aatagctata                                                    20

<210> 37
<211> 20
<212> DNA
<213> Artificial Sequence

<220>
<223> Antisense Compound

<400> 37
tagcttgtga acgcagaagg                                                    20

<210> 38
<211> 20
<212> DNA
<213> Artificial Sequence

<220>
<223> Antisense Compound

<400> 38
cttgcagtcc tcattcgagt                                                    20

<210> 39

<211> 20
<212> DNA
<213> Artificial Sequence

<220>
<223> Antisense Compound

<400> 39
ttcactgcac acaggaccag                                                    20

<210> 40
<211> 20
<212> DNA
<213> Artificial Sequence

<220>
<223> Antisense Compound

<400> 40
acttagcttg tgaacgcaga                                                    20

<210> 41
<211> 20
<212> DNA
<213> Artificial Sequence

<220>
<223> Antisense Compound

<400> 41
tagaatccaa gagttttgtc                                                    20

<210> 42
<211> 20
<212> DNA
<213> Artificial Sequence

<220>
<223> Antisense Compound

<400> 42
agattagtga ataccaatat                                                    20

<210> 43
<211> 20
<212> DNA
<213> Artificial Sequence

<220>
<223> Antisense Compound

<400> 43
tgccgccctc ctaacatgtt                                                    20

<210> 44
<211> 20
<212> DNA
<213> Artificial Sequence

<220>
<223> Antisense Compound

<400> 44
tgattgagaa gcgacagcca                                                    20

<210> 45
<211> 20

<212> DNA
<213> Artificial Sequence

<220>
<223> Antisense Compound

<400> 45
gaaaatttca tccagccaac                                                   20

<210> 46
<211> 20
<212> DNA
<213> Artificial Sequence

<220>
<223> Antisense Compound

<400> 46
gtgagaggaa ttactttgtc                                                   20

<210> 47
<211> 20
<212> DNA
<213> Artificial Sequence

<220>
<223> Antisense Compound

<400> 47
cgactcaact gcttctgttg                                                   20

<210> 48
<211> 20
<212> DNA
<213> Artificial Sequence

<220>
<223> Antisense Compound

<400> 48
ctataccagt taggactgtt                                                   20

<210> 49
<211> 20
<212> DNA
<213> Artificial Sequence

<220>
<223> Antisense Compound

<400> 49
aataattttc aacagtgaag                                                   20

<210> 50
<211> 20
<212> DNA
<213> Artificial Sequence

<220>
<223> Antisense Compound

<400> 50
taccaggatt ttcagaggtt                                                   20

<210> 51
<211> 20
<212> DNA

<213> Artificial Sequence

<220>
<223> Antisense Compound

<400> 51
gcacacagaa agggctacta                                                    20

<210> 52
<211> 20
<212> DNA
<213> Artificial Sequence

<220>
<223> Antisense Compound

<400> 52
tctccacccc agagcaaatg                                                    20

<210> 53
<211> 20
<212> DNA
<213> Artificial Sequence

<220>
<223> Antisense Compound

<400> 53
actattgttt tgttaccagg                                                    20

<210> 54
<211> 20
<212> DNA
<213> Artificial Sequence

<220>
<223> Antisense Compound

<400> 54
agtcattctc tgctcattaa                                                    20

<210> 55
<211> 20
<212> DNA
<213> Artificial Sequence

<220>
<223> Antisense Compound

<400> 55
tccaaaaatg tttggaagca                                                    20

<210> 56
<211> 20
<212> DNA
<213> Artificial Sequence

<220>
<223> Antisense Compound

<400> 56
tggcccttca aatgttgctg                                                    20

<210> 57
<211> 20
<212> DNA
<213> Artificial Sequence

<220>
<223> Antisense Compound

<400> 57
agaaaatttc atccagccaa                                                    20

<210> 58
<211> 20
<212> DNA
<213> Artificial Sequence

<220>
<223> Antisense Compound

<400> 58
tcagctgtgt tacagctggt                                                    20

<210> 59
<211> 20
<212> DNA
<213> Artificial Sequence

<220>
<223> Antisense Compound

<400> 59
tggacagatc tggctgctgc                                                    20

<210> 60
<211> 20
<212> DNA
<213> Artificial Sequence

<220>
<223> Antisense Compound

<400> 60
attctccact gaagcagata                                                    20

<210> 61
<211> 20
<212> DNA
<213> Artificial Sequence

<220>
<223> Antisense Compound

<400> 61
cccctagagc aaactgtttg                                                    20

<210> 62
<211> 20
<212> DNA
<213> Artificial Sequence

<220>
<223> Antisense Compound

<400> 62
attgctggta cctctatgca                                                    20

<210> 63
<211> 20
<212> DNA
<213> Artificial Sequence

<220>
<223> Antisense Compound

<400> 63
tcagtgaata tcaactctgg                                                   20

<210> 64
<211> 20
<212> DNA
<213> Artificial Sequence

<220>
<223> Antisense Compound

<400> 64
cacatattaa ggtttctaat                                                   20

<210> 65
<211> 20
<212> DNA
<213> Artificial Sequence

<220>
<223> Antisense Compound

<400> 65
atatataaca tgtcatgata                                                   20

<210> 66
<211> 20
<212> DNA
<213> Artificial Sequence

<220>
<223> Antisense Compound

<400> 66
aacacttcag gttcaataac                                                   20

<210> 67
<211> 20
<212> DNA
<213> Artificial Sequence

<220>
<223> Antisense Compound

<400> 67
gggtgaagac gcagaaacct                                                   20

<210> 68
<211> 20
<212> DNA
<213> Artificial Sequence

<220>
<223> Antisense Compound

<400> 68
tctcccatat acagtcccat                                                   20

<210> 69
<211> 20
<212> DNA
<213> Artificial Sequence

<220>

<223> Antisense Compound

<400> 69
gtttgcaatg ctttcttcca                                                    20

<210> 70
<211> 20
<212> DNA
<213> Artificial Sequence

<220>
<223> Antisense Compound

<400> 70
acctattgag gtttgcaatg                                                    20

<210> 71
<211> 20
<212> DNA
<213> Artificial Sequence

<220>
<223> Antisense Compound

<400> 71
ctggtcgacc tattgaggtt                                                    20

<210> 72
<211> 20
<212> DNA
<213> Artificial Sequence

<220>
<223> Antisense Compound

<400> 72
ctgtggtata caatttcaca                                                    20

<210> 73
<211> 20
<212> DNA
<213> Artificial Sequence

<220>
<223> Antisense Compound

<400> 73
ctttggtctg tggtatacaa                                                    20

<210> 74
<211> 20
<212> DNA
<213> Artificial Sequence

<220>
<223> Antisense Compound

<400> 74
caaaggtgct ttggtctgtg                                                    20

<210> 75
<211> 20
<212> DNA
<213> Artificial Sequence

<220>
<223> Antisense Compound

<400> 75
gaaaactcca aatcctgcaa                                                    20

<210> 76
<211> 20
<212> DNA
<213> Artificial Sequence

<220>
<223> Antisense Compound

<400> 76
ggtttagtgt ccggtaaaat                                                    20

<210> 77
<211> 20
<212> DNA
<213> Artificial Sequence

<220>
<223> Antisense Compound

<400> 77
ttctcttgct taattacccc                                                    20

<210> 78
<211> 20
<212> DNA
<213> Artificial Sequence

<220>
<223> Antisense Compound

<400> 78
gcccagtttc tcttgcttaa                                                    20

<210> 79
<211> 20
<212> DNA
<213> Artificial Sequence

<220>
<223> Antisense Compound

<400> 79
gaaatggcag acattttatt                                                    20

<210> 80
<211> 20
<212> DNA
<213> Artificial Sequence

<220>
<223> Antisense Compound

<400> 80
ccatgaacag aaatggcaga                                                    20

<210> 81
<211> 20
<212> DNA
<213> Artificial Sequence

<220>
<223> Antisense Compound

<400> 81
tgtcctccag aggtactcac                                                   20

<210> 82
<211> 20
<212> DNA
<213> Artificial Sequence

<220>
<223> Antisense Compound

<400> 82
gtggtacatc tgtcctccag                                                   20

<210> 83
<211> 20
<212> DNA
<213> Artificial Sequence

<220>
<223> Antisense Compound

<400> 83
tcatgtcata gtggtacatc                                                   20

<210> 84
<211> 20
<212> DNA
<213> Artificial Sequence

<220>
<223> Antisense Compound

<400> 84
gatgctgtat tcatgtcata                                                   20

<210> 85
<211> 20
<212> DNA
<213> Artificial Sequence

<220>
<223> Antisense Compound

<400> 85
tgagaaaggg atgctgtatt                                                   20

<210> 86
<211> 20
<212> DNA
<213> Artificial Sequence

<220>
<223> Antisense Compound

<400> 86
tggtggaatg acattaaaaa                                                   20

<210> 87
<211> 20
<212> DNA
<213> Artificial Sequence

<220>
<223> Antisense Compound

<400> 87

ggaattggtg gaatgacatt                                                    20

<210> 88
<211> 20
<212> DNA
<213> Artificial Sequence

<220>
<223> Antisense Compound

<400> 88
gagcacacca ggcagagttt                                                    20

<210> 89
<211> 20
<212> DNA
<213> Artificial Sequence

<220>
<223> Antisense Compound

<400> 89
ctgtccttcc actgctcttt                                                    20

<210> 90
<211> 20
<212> DNA
<213> Artificial Sequence

<220>
<223> Antisense Compound

<400> 90
ggtaattgtg ctgtccttcc                                                    20

<210> 91
<211> 20
<212> DNA
<213> Artificial Sequence

<220>
<223> Antisense Compound

<400> 91
tttcgatagc ggcatgctgg                                                    20

<210> 92
<211> 20
<212> DNA
<213> Artificial Sequence

<220>
<223> Antisense Compound

<400> 92
tgaagacatt ttcgatagcg                                                    20

<210> 93
<211> 20
<212> DNA
<213> Artificial Sequence

<220>
<223> Antisense Compound

<400> 93
gtttttcgag cttccaggtt                                                    20

<210> 94
<211> 20
<212> DNA
<213> Artificial Sequence

<220>
<223> Antisense Compound

<400> 94
caggaactat tgttttgtta                                                    20

<210> 95
<211> 20
<212> DNA
<213> Artificial Sequence

<220>
<223> Antisense Compound

<400> 95
gcctttgccc atttcactgc                                                    20

<210> 96
<211> 20
<212> DNA
<213> Artificial Sequence

<220>
<223> Antisense Compound

<400> 96
taataatcag atcaggagca                                                    20

<210> 97
<211> 20
<212> DNA
<213> Artificial Sequence

<220>
<223> Antisense Compound

<400> 97
tgctcattaa taatcagatc                                                    20

<210> 98
<211> 20
<212> DNA
<213> Artificial Sequence

<220>
<223> Antisense Compound

<400> 98
cattctctgc tcattaataa                                                    20

<210> 99
<211> 20
<212> DNA
<213> Artificial Sequence

<220>
<223> Antisense Compound

<400> 99
cagggtagag tcattctctg                                                    20

<210> 100
<211> 20
<212> DNA
<213> Artificial Sequence

<220>
<223> Antisense Compound

<400> 100
agcatgtgtt tacattggtc                                                    20

<210> 101
<211> 20
<212> DNA
<213> Artificial Sequence

<220>
<223> Antisense Compound

<400> 101
atacagagat actcttcata                                                    20

<210> 102
<211> 20
<212> DNA
<213> Artificial Sequence

<220>
<223> Antisense Compound

<400> 102
taaggttttc atacagagat                                                    20

<210> 103
<211> 20
<212> DNA
<213> Artificial Sequence

<220>
<223> Antisense Compound

<400> 103
gagagaagca gtaaggtttt                                                    20

<210> 104
<211> 20
<212> DNA
<213> Artificial Sequence

<220>
<223> Antisense Compound

<400> 104
ggcttttcct agctctttga                                                    20

<210> 105
<211> 20
<212> DNA
<213> Artificial Sequence

<220>
<223> Antisense Compound

<400> 105
ttgacaatgg cttttcctag                                                    20

<210> 106

<211> 20
<212> DNA
<213> Artificial Sequence

<220>
<223> Antisense Compound

<400> 106
gtgatgattt cagctaacat                                                    20

<210> 107
<211> 20
<212> DNA
<213> Artificial Sequence

<220>
<223> Antisense Compound

<400> 107
tgccaagtct tggccctcta                                                    20

<210> 108
<211> 20
<212> DNA
<213> Artificial Sequence

<220>
<223> Antisense Compound

<400> 108
ctgcttctgt tgccaagtct                                                    20

<210> 109
<211> 20
<212> DNA
<213> Artificial Sequence

<220>
<223> Antisense Compound

<400> 109
caggagggaa atatattttt                                                    20

<210> 110
<211> 20
<212> DNA
<213> Artificial Sequence

<220>
<223> Antisense Compound

<400> 110
acaacttagc ttgtgaacgc                                                    20

<210> 111
<211> 20
<212> DNA
<213> Artificial Sequence

<220>
<223> Antisense Compound

<400> 111
ctctggcaga ggagccgctc                                                    20

<210> 112
<211> 20

<212> DNA
<213> Artificial Sequence

<220>
<223> Antisense Compound

<400> 112
tccatcagtg aatatcaact                                                    20

<210> 113
<211> 20
<212> DNA
<213> Artificial Sequence

<220>
<223> Antisense Compound

<400> 113
tttggagtcc atcagtgaat                                                    20

<210> 114
<211> 20
<212> DNA
<213> Artificial Sequence

<220>
<223> Antisense Compound

<400> 114
atgattcttt ggagtccatc                                                    20

<210> 115
<211> 20
<212> DNA
<213> Artificial Sequence

<220>
<223> Antisense Compound

<400> 115
ttatagaagt ccatcacatc                                                    20

<210> 116
<211> 20
<212> DNA
<213> Artificial Sequence

<220>
<223> Antisense Compound

<400> 116
acgcagaaac cttcacagta                                                    20

<210> 117
<211> 20
<212> DNA
<213> Artificial Sequence

<220>
<223> Antisense Compound

<400> 117
actgctttgg acagatctgg                                                    20

<210> 118
<211> 20
<212> DNA

<213> Artificial Sequence

<220>
<223> Antisense Compound

<400> 118
gtatacaatt tcacattgcc                                                    20

<210> 119
<211> 20
<212> DNA
<213> Artificial Sequence

<220>
<223> Antisense Compound

<400> 119
caaaatgtca aaggtgcttt                                                    20

<210> 120
<211> 20
<212> DNA
<213> Artificial Sequence

<220>
<223> Antisense Compound

<400> 120
aggtctgatc tccaaggact                                                    20

<210> 121
<211> 20
<212> DNA
<213> Artificial Sequence

<220>
<223> Antisense Compound

<400> 121
tccaaaagga atgaatcgtc                                                    20

<210> 122
<211> 20
<212> DNA
<213> Artificial Sequence

<220>
<223> Antisense Compound

<400> 122
ggcagacatt ttattaccaa                                                    20

<210> 123
<211> 20
<212> DNA
<213> Artificial Sequence

<220>
<223> Antisense Compound

<400> 123
tcctgctgtt gagaaaggga                                                    20

<210> 124
<211> 20
<212> DNA
<213> Artificial Sequence

<220>
<223> Antisense Compound

<400> 124
cagatccttg gcacctattc                                                   20

<210> 125
<211> 20
<212> DNA
<213> Artificial Sequence

<220>
<223> Antisense Compound

<400> 125
ccccagagaa gtcaagttgt                                                   20

<210> 126
<211> 20
<212> DNA
<213> Artificial Sequence

<220>
<223> Antisense Compound

<400> 126
ctgttgttgc tgttgaggag                                                   20

<210> 127
<211> 20
<212> DNA
<213> Artificial Sequence

<220>
<223> Antisense Compound

<400> 127
caggttcaat aacctccaac                                                   20

<210> 128
<211> 20
<212> DNA
<213> Artificial Sequence

<220>
<223> Antisense Compound

<400> 128
cgccctccta acatgttgag                                                   20

<210> 129
<211> 20
<212> DNA
<213> Artificial Sequence

<220>
<223> Antisense Compound

<400> 129
ttcctgaaac ctggtattgc                                                   20

<210> 130
<211> 20
<212> DNA
<213> Artificial Sequence

<220>
<223> Antisense Compound

<400> 130
aacacagcag gtttgcactt                                                   20

<210> 131
<211> 20
<212> DNA
<213> Artificial Sequence

<220>
<223> Antisense Compound

<400> 131
tccttaggaa ctgaagagag                                                   20

<210> 132
<211> 20
<212> DNA
<213> Artificial Sequence

<220>
<223> Antisense Compound

<400> 132
catcaaatag ctcttggctc                                                   20

<210> 133
<211> 20
<212> DNA
<213> Artificial Sequence

<220>
<223> Antisense Compound

<400> 133
ctctttgatg taggtcattc                                                   20

<210> 134
<211> 20
<212> DNA
<213> Artificial Sequence

<220>
<223> Antisense Compound

<400> 134
atccaagagt tttgtcagtt                                                   20

<210> 135
<211> 20
<212> DNA
<213> Artificial Sequence

<220>
<223> Antisense Compound

<400> 135
tttcaaccac ttcatgcata                                                   20

<210> 136
<211> 20
<212> DNA
<213> Artificial Sequence

<220>

<223> Antisense Compound

<400> 136
gtatctgatt ggtgatgatt                                                    20

<210> 137
<211> 20
<212> DNA
<213> Artificial Sequence

<220>
<223> Antisense Compound

<400> 137
taaggcagtc acttttgatg                                                    20

<210> 138
<211> 20
<212> DNA
<213> Artificial Sequence

<220>
<223> Antisense Compound

<400> 138
taattcgact ttctttaagg                                                    20

<210> 139
<211> 20
<212> DNA
<213> Artificial Sequence

<220>
<223> Antisense Compound

<400> 139
acaaactgat agtttataca                                                    20

<210> 140
<211> 20
<212> DNA
<213> Artificial Sequence

<220>
<223> Antisense Compound

<400> 140
gtgcgtattt aaaacaaaac                                                    20

<210> 141
<211> 20
<212> DNA
<213> Artificial Sequence

<220>
<223> Antisense Compound

<400> 141
taatttctcc aaaatactga                                                    20

<210> 142
<211> 20
<212> DNA
<213> Artificial Sequence

<220>
<223> Antisense Compound

<400> 142
aaaagtgatg acgactcaac                                                    20

<210> 143
<211> 20
<212> DNA
<213> Artificial Sequence

<220>
<223> Antisense Compound

<400> 143
ttacgtccac atattaaggt                                                    20

<210> 144
<211> 20
<212> DNA
<213> Artificial Sequence

<220>
<223> Antisense Compound

<400> 144
ttaggtgcca tccttctttg                                                    20

<210> 145
<211> 20
<212> DNA
<213> Artificial Sequence

<220>
<223> Antisense Compound

<400> 145
gcactggtgg tttaggtgcc                                                    20

<210> 146
<211> 20
<212> DNA
<213> Artificial Sequence

<220>
<223> Antisense Compound

<400> 146
tttgggcact ggtggtttag                                                    20

<210> 147
<211> 20
<212> DNA
<213> Artificial Sequence

<220>
<223> Antisense Compound

<400> 147
atttcatcca gccaactgtg                                                    20

<210> 148
<211> 20
<212> DNA
<213> Artificial Sequence

<220>
<223> Antisense Compound

<400> 148
ggatacacca acagaaagtc                                                    20

<210> 149
<211> 20
<212> DNA
<213> Artificial Sequence

<220>
<223> Antisense Compound

<400> 149
acaacttccc ttttctgata                                                    20

<210> 150
<211> 20
<212> DNA
<213> Artificial Sequence

<220>
<223> Antisense Compound

<400> 150
cagtaatagc tataaaaggc                                                    20

<210> 151
<211> 20
<212> DNA
<213> Artificial Sequence

<220>
<223> Antisense Compound

<400> 151
agcaagcgta gttcactaaa                                                    20

<210> 152
<211> 20
<212> DNA
<213> Artificial Sequence

<220>
<223> Antisense Compound

<400> 152
gctgcccatc ttaaacagct                                                    20

<210> 153
<211> 20
<212> DNA
<213> Artificial Sequence

<220>
<223> Antisense Compound

<400> 153
aagcaccaac ccattttcac                                                    20

<210> 154
<211> 20
<212> DNA
<213> Artificial Sequence

<220>
<223> Antisense Compound

<400> 154

ccatcaggtt agaagcacca                                                    20

<210> 155
<211> 20
<212> DNA
<213> Artificial Sequence

<220>
<223> Antisense Compound

<400> 155
ttctgatagc taagtgccat                                                    20

<210> 156
<211> 20
<212> DNA
<213> Artificial Sequence

<220>
<223> Antisense Compound

<400> 156
aagaatactg gagatttgag                                                    20

<210> 157
<211> 20
<212> DNA
<213> Artificial Sequence

<220>
<223> Antisense Compound

<400> 157
gctctatacc agttaggact                                                    20

<210> 158
<211> 20
<212> DNA
<213> Artificial Sequence

<220>
<223> Antisense Compound

<400> 158
ttacccagca ggtcactgga                                                    20

<210> 159
<211> 20
<212> DNA
<213> Artificial Sequence

<220>
<223> Antisense Compound

<400> 159
catccacagt ttacccagca                                                    20

<210> 160
<211> 20
<212> DNA
<213> Artificial Sequence

<220>
<223> Antisense Compound

<400> 160
tagtcttttg caaccatcat                                                    20

<210> 161
<211> 20
<212> DNA
<213> Artificial Sequence

<220>
<223> Antisense Compound

<400> 161
agggcctctt ggtagttatt                                                    20

<210> 162
<211> 20
<212> DNA
<213> Artificial Sequence

<220>
<223> Antisense Compound

<400> 162
tagccattgc aaaaataggg                                                    20

<210> 163
<211> 20
<212> DNA
<213> Artificial Sequence

<220>
<223> Antisense Compound

<400> 163
tgccatatag ccattgcaaa                                                    20

<210> 164
<211> 20
<212> DNA
<213> Artificial Sequence

<220>
<223> Antisense Compound

<400> 164
ctgaaagaca aatagtttac                                                    20

<210> 165
<211> 20
<212> DNA
<213> Artificial Sequence

<220>
<223> Antisense Compound

<400> 165
acaactttta agaagttata                                                    20

<210> 166
<211> 20
<212> DNA
<213> Artificial Sequence

<220>
<223> Antisense Compound

<400> 166
tggttatctg gaatcacaac                                                    20

<210> 167
<211> 20
<212> DNA
<213> Artificial Sequence

<220>
<223> Antisense Compound

<400> 167
acagctggtt atctggaatc                                                    20

<210> 168
<211> 20
<212> DNA
<213> Artificial Sequence

<220>
<223> Antisense Compound

<400> 168
agtctctcag ctgtgttaca                                                    20

<210> 169
<211> 20
<212> DNA
<213> Artificial Sequence

<220>
<223> Antisense Compound

<400> 169
gtgaaaatgg gtgtctagcc                                                    20

<210> 170
<211> 20
<212> DNA
<213> Artificial Sequence

<220>
<223> Antisense Compound

<400> 170
tgacagatgg gaatgtgaaa                                                    20

<210> 171
<211> 20
<212> DNA
<213> Artificial Sequence

<220>
<223> Antisense Compound

<400> 171
aaagattaac caattggtga                                                    20

<210> 172
<211> 20
<212> DNA
<213> Artificial Sequence

<220>
<223> Antisense Compound

<400> 172
tttcctgtac catcaggaaa                                                    20

<210> 173

<211> 20
<212> DNA
<213> Artificial Sequence

<220>
<223> Antisense Compound

<400> 173
tctatggcac acattaggga                                                    20

<210> 174
<211> 20
<212> DNA
<213> Artificial Sequence

<220>
<223> Antisense Compound

<400> 174
ttgtgttaaa ctctatggca                                                    20

<210> 175
<211> 20
<212> DNA
<213> Artificial Sequence

<220>
<223> Antisense Compound

<400> 175
aagaaattca caggacttgt                                                    20

<210> 176
<211> 20
<212> DNA
<213> Artificial Sequence

<220>
<223> Antisense Compound

<400> 176
gaaagttggt aaggtgcaca                                                    20

<210> 177
<211> 20
<212> DNA
<213> Artificial Sequence

<220>
<223> Antisense Compound

<400> 177
caaatttctt gtggcttagt                                                    20

<210> 178
<211> 20
<212> DNA
<213> Artificial Sequence

<220>
<223> Antisense Compound

<400> 178
ttgaatagaa atcaaatttc                                                    20

<210> 179
<211> 20

<212> DNA
<213> Artificial Sequence

<220>
<223> Antisense Compound

<400> 179
acacaaataa tttggccacc                                                    20

<210> 180
<211> 20
<212> DNA
<213> Artificial Sequence

<220>
<223> Antisense Compound

<400> 180
ctattacaca aataatttgg                                                    20

<210> 181
<211> 20
<212> DNA
<213> Artificial Sequence

<220>
<223> Antisense Compound

<400> 181
agtagccctt cccttcccag                                                    20

<210> 182
<211> 20
<212> DNA
<213> Artificial Sequence

<220>
<223> Antisense Compound

<400> 182
aaagctgcag tagcccttcc                                                    20

<210> 183
<211> 20
<212> DNA
<213> Artificial Sequence

<220>
<223> Antisense Compound

<400> 183
tgcatgtaaa gctgcagtag                                                    20

<210> 184
<211> 20
<212> DNA
<213> Artificial Sequence

<220>
<223> Antisense Compound

<400> 184
attttaataa attgcatgta                                                    20

<210> 185
<211> 20
<212> DNA

<213> Artificial Sequence

<220>
<223> Antisense Compound

<400> 185
caagctattt tacaatcatt                                                    20

<210> 186
<211> 20
<212> DNA
<213> Artificial Sequence

<220>
<223> Antisense Compound

<400> 186
atccatcagc atttctttga                                                    20

<210> 187
<211> 20
<212> DNA
<213> Artificial Sequence

<220>
<223> Antisense Compound

<400> 187
tataaatcat ataggttatc                                                    20

<210> 188
<211> 20
<212> DNA
<213> Artificial Sequence

<220>
<223> Antisense Compound

<400> 188
caaactgttt ggtttctgag                                                    20

<210> 189
<211> 20
<212> DNA
<213> Artificial Sequence

<220>
<223> Antisense Compound

<400> 189
ctgggtcaga gcctcagcaa                                                    20

<210> 190
<211> 20
<212> DNA
<213> Artificial Sequence

<220>
<223> Antisense Compound

<400> 190
taatctcact gggtcagagc                                                    20

<210> 191
<211> 20
<212> DNA
<213> Artificial Sequence

<220>
<223> Antisense Compound

<400> 191
aatgagaagg gtggtcagaa                                                    20

<210> 192
<211> 20
<212> DNA
<213> Artificial Sequence

<220>
<223> Antisense Compound

<400> 192
ctcactgttg gaatgagaag                                                    20

<210> 193
<211> 20
<212> DNA
<213> Artificial Sequence

<220>
<223> Antisense Compound

<400> 193
agtaaactaa acctgcgctg                                                    20

<210> 194
<211> 20
<212> DNA
<213> Artificial Sequence

<220>
<223> Antisense Compound

<400> 194
ctgtttacat actttacata                                                    20

<210> 195
<211> 20
<212> DNA
<213> Artificial Sequence

<220>
<223> Antisense Compound

<400> 195
agatggtgcc tttaaggatg                                                    20

<210> 196
<211> 20
<212> DNA
<213> Artificial Sequence

<220>
<223> Antisense Compound

<400> 196
atgtgaaagt aacccgctat                                                    20

<210> 197
<211> 20
<212> DNA
<213> Artificial Sequence

<220>
<223> Antisense Compound

<400> 197
ttctgaagct tctgttgtca                                                    20

<210> 198
<211> 20
<212> DNA
<213> Artificial Sequence

<220>
<223> Antisense Compound

<400> 198
ctggtacctc tatgcaaact                                                    20

<210> 199
<211> 20
<212> DNA
<213> Artificial Sequence

<220>
<223> Antisense Compound

<400> 199
gagattctgc actatttaca                                                    20

<210> 200
<211> 20
<212> DNA
<213> Artificial Sequence

<220>
<223> Antisense Compound

<400> 200
tagtgtatta ttggcaacct                                                    20

<210> 201
<211> 20
<212> DNA
<213> Artificial Sequence

<220>
<223> Antisense Compound

<400> 201
ttatttggaa ataaactctt                                                    20

<210> 202
<211> 20
<212> DNA
<213> Artificial Sequence

<220>
<223> Antisense Compound

<400> 202
aaaacatgtc ctcattttat                                                    20

<210> 203
<211> 20
<212> DNA
<213> Artificial Sequence

<220>

<223> Antisense Compound

<400> 203
gaagctcttt ttgaaactta                                                    20

<210> 204
<211> 20
<212> DNA
<213> Artificial Sequence

<220>
<223> Antisense Compound

<400> 204
tggttttaac cacataacat                                                    20

<210> 205
<211> 20
<212> DNA
<213> Artificial Sequence

<220>
<223> Antisense Compound

<400> 205
acataacatt ttcatgcata                                                    20

<210> 206
<211> 20
<212> DNA
<213> Artificial Sequence

<220>
<223> Antisense Compound

<400> 206
tttgttgtga gtaaccaact                                                    20

<210> 207
<211> 20
<212> DNA
<213> Artificial Sequence

<220>
<223> Antisense Compound

<400> 207
acactaaaaa tacttttcag                                                    20

<210> 208
<211> 20
<212> DNA
<213> Artificial Sequence

<220>
<223> Antisense Compound

<400> 208
aactccaccc aaagggttta                                                    20

<210> 209
<211> 20
<212> DNA
<213> Artificial Sequence

<220>
<223> Antisense Compound

<400> 209
ttcctgaaaa cctggtcact                                                    20

<210> 210
<211> 20
<212> DNA
<213> Artificial Sequence

<220>
<223> Antisense Compound

<400> 210
attaatctgc ataggaagca                                                    20

<210> 211
<211> 20
<212> DNA
<213> Artificial Sequence

<220>
<223> Antisense Compound

<400> 211
ttctaccaac ctgaagagag                                                    20

<210> 212
<211> 20
<212> DNA
<213> Artificial Sequence

<220>
<223> Antisense Compound

<400> 212
agaagaactc gtgatattat                                                    20

<210> 213
<211> 20
<212> DNA
<213> Artificial Sequence

<220>
<223> Antisense Compound

<400> 213
tccttaggaa ctaaaaggtt                                                    20

<210> 214
<211> 20
<212> DNA
<213> Artificial Sequence

<220>
<223> Antisense Compound

<400> 214
tttcaaccac ctgcaagaga                                                    20

<210> 215
<211> 20
<212> DNA
<213> Artificial Sequence

<220>
<223> Antisense Compound

<400> 215
ggtcccagct gcttcggccg                                                   20

<210> 216
<211> 20
<212> DNA
<213> Artificial Sequence

<220>
<223> Antisense Compound

<400> 216
ggagagcccc tatttaagaa                                                   20

<210> 217
<211> 4734
<212> DNA
<213> Artificial Sequence

<220>
<223> M. musculus


<400> 217
   gcactgggat gggttttcct tcttgaggtg tcaagcttcg gctcctttgc caagatggcg 60
   gccctggctc ccatggaggt agcgattgtg cagcacctct ggcccagggg ccgtcttaca 120
   gccacggcct acccgcgcaa agcggaagac acattgggca gcctttacat tttccatcca 180
   agaaagggcg cctcggtttt gaagctaaag agcacctctg ccaaaatggt gaccgtgtgg 240
   cgtcactgct ctttaccaag atggcggcga gagacttccg gcacgcgctt ccccaatcag 300
   agatctccaa gaggtcaggc agaggagacc gccctcggag tccaagtgct gcgcgatcgg 360
   ctgggggaga gaatggggtg gagccacggc agcctcagct ccgatcagaa gtgccaagcg 420
   ctggcacctg tgggggagca aaagttactt cctcgcaccc caaagcaaca ccataacacc 480
   ttactcccca acccccaggc ccctaaaccg tagcgtgcgg cgcgaggtag ggggcacggt 540
   cccagagtcg ccccagtctg ccaaaggggc gggcagctta ggggcaggtc cgcgcggcgg 600
   ctgcactgtc acccccacgc ccctctcctg tcctaggggg accggccacg tgtttctctt 660
   ggagacccgg ggctcgccct gggaacagct ggagggagct aaacgctgac gttgtaaaga 720
   tgcgtgtttt gttttatttg gaggggcaga ggggtccctg gaactcagaa agaaggcaga 780
   gcgaggcact gagcctggag cagcaaatgt caagatttgg gggaggggcc tccgcgggga 840
   gcttggatgc tggccccgaa gggggtggaa ggagaggtca ggagtttggg gtaagaggag 900
   ggcggacttc gccagcaact tactattccg tctgcaactt gcttctaggc ctgcacacac 960
   cccctcccgg ccccgcaagg cttccttaat cacaattttt ttcttttttc tttttttttc 1020
   tttttttttt ttaagtgcaa agaaacccag ctcgctaaga gggttttgca ttcgccgtgc 1080
   aacttcctcc gaatgtgagc gcgctggcag gcagggaggg agcggtgggg gggggggggg 1140
   gaggtttgaa cttggcaggc ggcgcctcct gctgccgccg ccgccgctgc tgccgcctcc 1200
   tctcagactc ggggaagagg gtgggggacg atcggggcgc gggggagggt gggttctgct 1260
   ttgcaacttc tcccggtggc gagcgagcgc gcgcgcagcg gcggcggcgg cagcggcggc 1320
   ggctgcagac ggggccgccc agacgatgcg ggggtggggg acctggcagc acgcgagtcc 1380
   ccccccgggc tcacagtatg tatgcgctga ccctctgctc tgccctcccg tcccctcccc 1440
   aagcctcccc ccaccccgag ggcgtgtctg ccgtcctgtc ccgagagcgg ccagggctct 1500
   gcggcaccgt ttccgtgcca tcccgtagcc cctctgctag agtgacacac ttcgcgcaac 1560
   tcggcagttg gcggacgcgg accacccctg cggctctgcc ggctggctgt caccccctcg 1620
   ggggctctgg cggcagaccc acggggcggg ctcccgagcg gttccaagcc gcggagctgg 1680
   gcgggggcgg gaaggagcca gggagaagag aaactaaaga aactcgcttt ccctcccagg 1740
   ccaggtcggc agccgctgcc gcactttttt tttttctcgt ccctcgggtg gggaaaaaag 1800
   gccaagaagc cgcgcggccc ccggcggtgg cgatgcccgg agccgcgcgg gcttgcaggc 1860
   gccgtcgggg ccgggctggc ggggcccgcg cgctgggcga gaagagggcg agggccgagg 1920
   gcgcccttgc agttgccgac agtcgccaac aggttgcacc gttccccgcg gccgccgcgc 1980
   ggcccctcgg gcggggagcg gccggggggt ggagtgggag cgcgtgtgtg cgagtgtgtg 2040
   cgcgccgtgg cgccgcctcc gcccgcccct cgctcggtcc cgctcgcctg ccgcggccgg 2100
   gcggcccttt cgcgtgtccg cgctcccccc ccttcccctc cgcctcctcc attttgcgag 2160
   ctcggagtca gtgcctggag cccgagtcgc cgcccgcccg tcggggacgg attctaagtg 2220
   ggtggaacaa gacgccgcag ccgggcggcg cggcgccggg acgggagaac gcgcgcgggg 2280
   agacgggagc ggcgcggggg cccggcttgt cagccgggaa ggggtgactt tccgcgctag 2340
   gggctctccc ctcccccatg gaaaagaggg ggcgactgtt gacttccttc tccgtgacac 2400
   gcgcgcctcc cgcgtccgca cgccgacttg tttatctggc tgcggtggga gcgcaggcgg 2460
   gcgggcgagc gcgcgggtgc tgaggtgagc gggggctggg cgagcgggcg cccgcgctga 2520
   ggtgagccgg actgggcgcg ctccccgagg ggctcggcag ccggggcggc gggacttggc 2580
   aaacttttgc cagcccgggc ttgggggcgg gggagggggg gtggaggctg gcgagggcag 2640
   ggtgacggtg acgaaagggc ctcggcggtg acagcgctgg cgcttcctct ccccgcaccg 2700
   ccatccctgg cccagcgcgc tgccccgccg cggagcctcg agcgcctggc gggagtctgg 2760
   cgtccttttc ggttttgctt tttttttttt ttttccttgt cgcaagccct ccggtctccg 2820
   ctgtcctcgg ggccgccagg acagccagct ttttccccct gggggaaggc agaggggcgg 2880
   ctgcgacccg cggtctcagg gcgcgcggct gcgggctgcg ggcttgtggg gtggattcgg 2940
   ggctcgctgc ctgcagccca gacttcgccc gcccggcctt atctgctaga agtgggcgtg 3000
   ccgcagagaa ctcaacaggt ctggacacat tctcccttca cctcgcacct ttctccctcc 3060
   ttctccccca accccacccc cggacaactt gggcactagc tttggggcat gatttcgaac 3120
   ctgacttttc tgagtggtcc ccttttagaa agagaccctc cctagccaca gttttttgat 3180
   gcagcgattt tttttttttt ttttttttta acaagtgcta ctttgacgtt tgaggttgca 3240
   ggctcaggaa ttgcggcctt accacttaag accctgggca tggtttcctg tgactaatgt 3300
   cacagggtta tttacagttt taaatggggg ataaatgtcg cttaagggag catcttgttt 3360
   tatgaagtgt tgttatggtt tcaggctgga aggggcagct gtcaaaaaag cgggtgtgaa 3420
   aattcattaa ggtcatttga taactaatgt ttagagaccg ttctcagagg cataggaccc 3480
   cggttctgag agacaagtcc tgtacaggga agaagcctac ggcaggtccc agccctctct 3540
   ccaccttcct caaattcccc ttccagagaa gaaggtcata cttagtgtct tggtacagga 3600
   aaaatttacc attttatggg aggggattcc aaatatattt gtcgtagtct ttctagcctc 3660
   tcaaaacatt ttgattatta ataatatcta gcagtctgga ggaataaata aaggtttaaa 3720
   actgcagaga aaattatctg agcatttctt gaatgggttt tatttgactt tatatgtgat 3780
   tctacagtcc agtttttctg tttttcctgg tatttacata tttggaaaga gaaatcttca 3840
   acttatagat aaaatatttt gtattgagca tattaaacaa atttttaaaa aaggaatata 3900
   actcagtaaa tcttcgtgtt aggaattttg ataagctctg ttctattaaa ctatttaaat 3960
   acgttaaaat tgtagggaag aagccagtac acactctact ctgcttttaa ggatgcacat 4020
   tttagtctgt atataatata tattatttct tatatcccaa gtttgaaggt aggtgatact 4080
   agacaggcat atttagttga aaatggagtt tcagagtaag agcttttccc tagccctgca 4140
   agacaagcag cccagcactg cttttttttt ttttccaata aaatgtgcat aattcttcaa 4200
   ggttgtacct tgaaaggttt agatgtgtgg ggattggcac aggtgaaatt gtgaatcatg 4260
   aggtgtgtaa acaattatag tatagcattt atcaaacggt ttatgtagtg gtttccagag 4320
   aggaatctct caattgaaag gggttggaaa tttaaggatc acgcctttaa aaaaaaaaaa 4380
   aaaaagttaa atactttgac gtaaatttga acctttgcaa taatcacaaa taacaaatta 4440
   caacctccat gcttaccaaa tgtgtacatt tagcgagtgg caggataaac agccaagttt 4500
   aaatcggtcg accgggcaga tgagctgctt tgtggatggg agcagggagg atatagcttt 4560
   attttagaag ataggaacta ttcttctgat aatgaagact ttggtttggg agttacctaa 4620
   agggtttatt taataggctg tctcttacta atcggatcag agataatgtg gttttatagc 4680
   ttattatgtc cttttttttt tttttgtagt taatatttgc caatggactc caaa 4734

<210> 218
<211> 836
<212> DNA
<213> Artificial Sequence

<220>
<223> M. musculus

<400> 218
ggacgcgtgg ggcggcggcg gcggcagcgg cggcggctgc agacggggcc gcccagacga 60
tgcgggggtg ggggacctgg cagcacgcga gtcccccccc gggctcacat taatatttgc 120
caatggactc caaagaatcc ttagctcccc ctggtagaga cgaagtcccc agcagtttgc 180
ttggccgggg gaggggaagc gtgatggact gtataaaacc ctgaggggtg gagctacagt 240
caaggtttct gcgtcttcac cctcagtggc tgctgcttct caggcagatt ccaagcagca 300
gaggattctc cttgattttt caaaaggctc agcaagcaat gcacagcagc agcagcagca 360
gcagctgccg cagccagatt tatccaaagc cgtttcactg tccatgggac tgtatatggg 420
agagaccgaa acaaaagtga tggggaatga cttgggctac ccacagcagg gccagcttgg 480
cctctcctct ggggaaacag actttcggct tctggaagaa agcattgaaa cctcaatagg 540
tcgaccagcc gtccagagaa tcccaagagt tcaacacctg cagctgggtg tgctaccccg 600
acagagaagg agtttcccca gactcactct gatccatctt cagaacagca aaatagaaaa 660
agcagctgga ccaaggtggc aggtgtgaaa attgtgtacc aagacaaagc acttttgaca 720
tctggaggga tgggggttct ggcgggtccc agggtaagag aaaagagagt cttggggggc 780
cacctgtggt tggatgcaaa ctggttcctt gggggggaaa aggtccccct gggggg 836

<210> 219
<211> 571
<212> DNA
<213> Artificial Sequence

<220>
<223> M. musculus


<400> 219
gcccgtcggg gacggattct aagtgggtgg aacaagacgc cgcagccggg cggcgcggcg 60
ccgggacggg agaacgcgcg cggggagacg ggagcggcgc gggggcccgg cttgtcagcc 120
gggaacgggt gactttccgc gctaggggct ctcccctccc ccatggaaaa gagggggcga 180
ctgttgactt ccttctccgt gacacgcgcg cctcccgcgt ccgcacgccg acttgtttat 240
ctggctgcgg tgggagccgc aggcgggcgg gcgagcgcgc cggtgctgag ttaatatttg 300
ccaatggact ccaaagaatc cttagctccc cctggtagag acgaagtccc cagcagtttg 360
cttggccggg ggaggggaag cgtgatggac ttgtataaaa ccctgagggg tggagctaca 420
gtcaaggttt ctgcgtcttc accctcagtg gctgctgctt ctcaggcaga ttccaagcag 480
cagaggattc tccttgattt ttcaaaaggc tcagcaagca atgcgcagca gcagcagcag 540
cagcagcagc agcagcagca gcagcagcag c 571

<210> 220
<211> 19000
<212> DNA
<213> Artificial Sequence

<220>
<223> M. musculus


<400> 220
aggattagag ggataagtaa caactcccag ctacagttac tattttttaa tttttgttcc 60
taaagaagca attgaaaaac attgttattt ctaactagag aaagaacaat ttttagttgt 120
gaatgtatta atcttttttt ttttttttgc atttgaggct tagcgcttcg ctaggctgaa 180
ataaaagcat gcataaagga tgaagagaca agcaacatcc agtctagctt attcctaagt 240
accatctctt agcaaagttc atagaaagag agtgctgatg tgtgtgaact ttcctatata 300
agtgataaac aacatgattt accatgagga gttccacacc cgacgagttg atactattca 360
tagtttttaa agtctgtttg agaggctaga acaagacgag gaattggcaa tataatcaga 420
agcaaaaggc tagattattt ttagcaacac atatgctttt gttccagagt cagcatgtat 480
ggcaaaagtt ccttaggaca attcaatatt gaaaatcatt atcacttaat ataagatgta 540
atagctaatt tatcaatttt tcataaacct tcgttgattc aggtaaagga taaatatcca 600
ttgttcttcc aaattctctg aaaaattaaa gactttgaat aagaacttta aactaataat 660
atatcttgct tattcccgtg ctggtaagaa gtttgcagaa tgaaagtgag ccctggtcag 720
agcagcagac tcggtttttg ttgttttctc aggatattct gattgagaga gagcaggacg 780
gttgtactca gaatagaaaa ctagagggtt tccaatatac atggtaatgt ttctaaacta 840
atctttccca agtcacaaga aactatcgtt tcagctagga tcaaactcta aggtgctatg 900
tggttttttt tccctatatt agagagcatg gatagaatga taatgagatt aatgtaagaa 960
cttaaaagga aaagaagatt gatgtttaag ggttagaagg aatttagggg tgtggtagag 1020
atatcatcct ttcttggttt ttatggtaat tatgggtcta atccattttt ttaatcttgc 1080
caatcacata cttagaaaat aatgcatttt taaaaaatag taattatatg ttaaatgaac 1140
cacaacatca ttctgtttag aagccagaga actaattggc tcttgcactg tgtattttat 1200
ttactgttgt gtgtatgttt cacatgcatg ggaatatgca aatatgtaga gaccagagca 1260
gcacataggg catcttctat agcgctccgc ctttctgcct tgagacaggc atgaatggta 1320
acctcactgt ctgtttgtga gctcttcata tccacctgac tctcttcctc aatgctggga 1380
ttataggcat gcacaattac ggccttcttt ttaagaaagt ggtaaagatt tgaacttata 1440
tcctcatgcc tgctaggcaa atgatcttaa ccactgtacc atcttgtcca tttaatgcat 1500
ttcatatttc atacagaaaa atagtatatt ccaatatatt gaaatcaggt attggtgctt 1560
gttagcactt aaaagacaga attcagtgtg aacatgctga catggaatga gaagggggaa 1620
gaaatatatt ctgacattcc tttcataatc atgccaagca taaccctgtt tatgcttcct 1680
atagccctgg aatgagacca gatgtgagtt ctcctccgtc cagctcctcc acagcaacgg 1740
gaccacctcc caaactctgc ctggtgtgct ccgatgaagc ttcgggatgc cattatgggg 1800
tgctgacgtg tggaagctgt aaagtcttct ttaaaagagc agtggaaggt agtgtgtgtt 1860
ttgaagagtt taatttttgt tcctatttct cacagtaaat gtttaactgt tcattgtata 1920
caaagtccac taatgataga cattagttca ggtgcaagca tggtggctct taaatcctcc 1980
attgggaagg gacagaagta aggagaagaa ctagaataag taattaagtt cagcctgaga 2040
aagaactaaa gcaagcattg taaggaacgc tgctgcacgg actggcagta cgctgagtct 2100
gggcagtccg agaatggtgg cttttcattc cctgtaagca agctggcttc aaagctacca 2160
gctataccag actgacaaaa tcagtgaccc tggggaggaa caataaggga gggacagcat 2220
tctttgcttt acttttttcc tgttaaataa ataaagaggt catgttaagt accacaatgg 2280
ctgaggccaa attaacaaag tacagagatt atcagatagg tgaagtctag aaatatggtg 2340
tttgaaagaa gatgactgca aggtgatcat gtgttgacgg gttcacattc cagagcagcc 2400
ggttaatatt agagatagtt tatataccct acccaaaagt atcttaatgg aattaaacca 2460

gaaaataaag ctacattgat ctttcaatat actacaattc acagcccatt gttcttattt 2520
aaaatgaact cattccctta tttttatgta aaaataagta ttagctatgc tatttataaa 2580
atgtttacca tttagttatt tcagcaaagc gtggtggcta gattacatta gtaaggaagc 2640
ctttagctgt gtacggtgtt aatgagtaag cctcttggct cagcttcctc cctaacttga 2700
gtttcagtgt tctcaaaggc actttccagc tctaacattc tgtaaaccct tgatactcta 2760
attcaaggaa aaaaatttat ctagacttgt aaaatccata acatagtttg tagcatttca 2820
tttttaaaat tattaaattt tatttgatat tacatacatt acatatataa cataacattt 2880
taacacatac tagaaattat acttaaaccc tgtattggca ttataaatac ccaaacataa 2940
atcagagtta agttttacac atttgttatt tatataagac aaatctattt tttaatccaa 3000
gatcctatga gttctccatg tccttatgcc cagctggtcc tttttacttg tattcatttt 3060
acctacaagt atctataact gtattggtag accataagtg gatagttacc aatgactctt 3120
cacttgccgt gtgtgaagta catgcatcca aaatcaatta gtggccctgt gaacacttag 3180
atcctgttag gatctgtgtc tgccagacta gcaaacaggt tcagaaagtt gaagtagcca 3240
agctgggatt gcaacaaagt ggtctccaag taaggtctta ttctcaacaa agatcctcaa 3300
agatgcatct gacaagccct tgatacctgc tagattataa ttcaggtaga tgctgaaata 3360
aaagtaatca caagagattt aactattagc tactatatac tgagtagtta tggtgtgttg 3420
agcacagtat cgtctgcata attacttcca agcttggcaa attttctaac agttcataag 3480
agaacaaact ccaaaaagtt aatgtttcca agatcagttt cttggcatgc cactatacca 3540
ctgctactga gaactgctat agaagcgtaa gctgatagta ttagtttata cttaatctcc 3600
aggtactcac agtacagtct gtagcctgca tcttttacat gtgttgtttc ccatttccta 3660
taccatgttg ttctatgcaa cattttattg gtcataggat ccagctttgg tatgattcta 3720
gaaaggatta cactacacct tattaggaga cagatatcac tggctatcct gtaagctggc 3780
tgctacacca atcataaata gcaaggcact gtcaagtgct aaaagtagat ttagccaaaa 3840
gcaccaacaa ccaacagcat taaagaggct ttggatgagc tgaaggctct tcccttgaca 3900
ttaagaagtt agaaaggaaa tttatcaggt ggcagaaaac aaaggaattc cagacgactg 3960
tcttaaaaag acatgtaatt ggtagtacat ggccattctc attctttgtg aaataatgaa 4020
cactagctca aatcccctgt tttgttacaa ggaatgaata gactagtgga tcattctatt 4080
atactaacct gagccctata gctaagattc tcagttatac tacacactgg ataaagaaat 4140
aaatatcagc ataatacaca gtcaatcttt tagttagaca taaataaaca ctgaattttt 4200
tctactctag aagtaatttt tccccagttt ttccagcaac actgatataa ggattctctg 4260
aaagacaatg gatgtagggg tgtttttatg ggtaaaattt ttagtgcttt aactgtggat 4320
acaatctgtt gtttctcctt ttgctgcatg atggaagttt ttaaaaagaa tttatttatc 4380
cttatttata tgtataaatg tttacctgta taaacactgt gtgcctggta cctgcagagc 4440
taaagggcat ctggttccct agaattggag ttatagacag ttgtgacctg ctgccatgtg 4500
ggtgctagga actgaaccaa agtcctttgc aagagcagca agtgttctta actactgagc 4560
catctctcca atcccatata ataggatttt attgagttag ttcttctatt ttgtgtttaa 4620
gaaaacagga ggcttataaa atacagaaag tatttgaaag agctagcata gatattaggc 4680
atgggaaagt aaccctgaaa taaagccatt atataactta actatatagc taagtgtata 4740
tatatatata tatatatata tatatatata tatatatata tatatatata tacacacaca 4800
tacatacata tgactattag tcaacttcgt aactgtatct tcacaaaaga ttagactaat 4860
acagttaact atgtactcca aactcatatt taaacaacat catgatttta catatcagga 4920
aaaactaaat gttttttttt taatccaagt cttagattgc cctaataaag aaaaaagtct 4980
cttcttagaa atcaggtcta tatatgttcc aactggtgaa ctgactgtta caattctttc 5040
tcatttcttt gaatatgatt tattctaata taagtaacac agattgttaa ttactggtgt 5100
atctatatag acagttaaat cttgtgtagg aagcttctat accccacccc agtcaaatag 5160
atccttcagt tgttttcatc aacatttaat actcagatta aagaagacag tgcatgtggg 5220
aaacagaaac acttttagcc ttttcctttt taacagtctt agaatttttt gccaggagag 5280
aactgaagtt cataatatgt tcttcagtag tggataccag aaaatggagc cactagctag 5340
cattaagtca gtatttgagg ccaaaggtga ctccatgcat ctttgtattc taaaagtata 5400
ttaaataatg atctttgtta gtttgtttag tttgccacat ttgtgattta cctaaccagt 5460
ggctagaagt tggtaacatt ttacttcatt taaaattggg aagaacttct tttacctaca 5520
catgataatg ttgagtgatg aattaaagtc atctttttcc ttagggattc caaaagaaaa 5580
tttaagcctg cttcctttta gtcacttcaa caatagatca gaattcataa taaaagtaat 5640
aattgcaaac cccatttata actgttaaat aactatagaa agcaggtttt aaagtttgat 5700
cttagagtat tcttatgatt tgaatacata tatatatgaa atactgtagt tgggttgttt 5760
tcattgattt taaattcgtt aagcagaaca agtcctctca acattgtaat tcatgcagat 5820
aaacagactt aacatcctta gtcagtgact ttgtgtcttg atgatagtct gctcaaaaca 5880
aaaacagaaa ggggataggc tttttctttt aaaatatcct tacagatctg tggaatttta 5940
acaatgccct tttaatctct tataggacag cacaattacc tttgtgctgg aagaaatgat 6000
tgcatcattg ataaaattcg aagaaaaaac tgtccagcat gccgctatcg aaaatgtctt 6060
caagctggaa tgaacctgga aggtaatgga agcttaagga actgtctgcc tgtcacatga 6120
gaactgatcc tttactttat aaaagctagc aagtgaacca tgtaatgaac tctatatatg 6180
cttaattagc aaaatgttat attaaaatgg ttttttccac gatcatatca ttttgtgtgt 6240
taattcaact acttttgtta tatacattga atttgggaca gccatggtgt atattaagta 6300
aagagattac ataatattga aaattacata acttggagca attttgtttt catccttctt 6360
actccttttt taaaactcaa aaccatttcc taagattgga aaagtattgt acatgagatc 6420
tagttcatat tcctctctta ctttgcactt caaaaaccac cacacctttt acttgaaaac 6480

atatgtttta ttacaactct gccaacaaca ttggatgggt tgttttatgg tttaagtata 6540
aaggaaaagt aaaaactact tgtaagttat aatttgatat tcctgcttat ataaaaagtt 6600
atataattaa cacttattta aaaacttccc tctggaaacg tcagggtttt gctgtagtgg 6660
aaatagcaag ctctgtgaaa gtagcattct tcctaactca tgttgttagt attgactgtt 6720
actgtggtac atcatagcaa acgctcagca aatataaaga gaatacaaat ttagtacctg 6780
agtctgagca gcttactttc tgtttatgct acagtgtcaa tcacaaaata gtatggcact 6840
ctacaaaggc aatactatta caggaccaag acggaaggcc atctgagcct gtcctgaaag 6900
tcagactcat cctggaagaa gtagcactga gctgcaaata cagaccttga ggaggaatta 6960
gatagccatg acagagacgt gctcatagtt gtactttata aagagcagtc tttgggatag 7020
acaaatggat cggtggcagg ggatccaact tcacttctca gcacccttat ttgctgagaa 7080
ctcacagctg cctctaactg ggaattgaac tcagcatctc tagaagagcg gtcagtgctc 7140
ttaactgctg agccatttct ccagcctgag actcaaaagt ttaaggtgaa cagtaaactc 7200
ttggttccct gttagaggaa acaagtcctg ggagggaatt cctatagaga atcctggaat 7260
tgggagttcc atgggtaaaa tcacttgcta ggcacgcatg aaagacccaa gttaggatcc 7320
cactacccac ttaaaaagcc aggtctagct atgctcttgc ctattaacca cagaattttg 7380
aaggataagg tggagacagg agtgtttcta gggcttactg gcctacctcc aagtccaacg 7440
agagagcctg cctcaaaaga gtgatagact tggataccca acttcttccc ctggccttga 7500
gtcatgcaca gatgtgtgca tatgcacgtg tacatacacc acactcacac atgtacacac 7560
agattcacat agacatgcca aaagttttaa atagtttact tctttcaata atagtgtcag 7620
aaattaaaga cctcttataa cttgaaagta taggctacaa agttgatttc gtatattttt 7680
ttcttttttc aatattcaca gctcgaaaaa cgaagaaaaa aattaaagga attcagcaag 7740
ccactgcagg agtctcacaa gacacttctg aaaacgctaa caaaacaata gttcctgccg 7800
cgctgccaca gcttacccct accctggtgt cactgctgga ggtgatcgag cctgaggtgt 7860
tatatgcagg atatgacagc tctgttccag actcagcatg gagaattatg accacgctca 7920
acatgttagg tgggcgccaa gtgattgccg cagtgaaatg ggcaaaggcg ataccaggta 7980
aggcatgaag gatgagccac ggcataaatc tttgtgtttt attgaataaa tgcactctga 8040
gttgggcttg gtgaataact tcgtgacatg ccatctgctt atcatacaca atccttgggg 8100
tcagtctgca gcaccctcgg aatagagctg ctctgagact ctaggcggtc tgtcctcctg 8160
acggagctca cggcagagct ggtttctttt actcacaagg acaaaagaga gatccaaagt 8220
atactggata ctgttccctg actttcaggt tacacatagt gtctcatttt acataagatc 8280
ttatattatc cgaactaaaa ttccattcac agcgacacct ggaagacagc atttagaaat 8340
tccatttatt ttcattcaga ttgtttttga agttgataaa cagcaggttt tgttgacttt 8400
gtcaaaattt actaacacaa agttatttta atttttcatt aaatgaaatg gcatctactg 8460
actataaact ttattctagc tttaaaggta tttttaaaca agtttcatgt tggaacttcg 8520
ggtgcagcag aggccaaggt taagcatccg cccttacttt acagatagca aagtggttgg 8580
cacgtttatg gctgcataca cctaagctgc catcacccca cctttttagc aaaagaagat 8640
ggatgtcagg aaattttaaa aatctaaatc tgttaagaaa taggaatcct aagcaaagac 8700
tctcggcttt ctcttttact atatattcta ctttgtgtgc acttaaatgg acaaaattgg 8760
ccaaaaaata gctattcata agtgttttta aatcattaaa gagggttgag tgcccaggcg 8820
ttgatctggt caatggctgt ctactaaaga tcacatccaa acaaggccaa cttgtcaact 8880
cgagtgttcc ttataccttt atttcccatc taggattcag aaacttacac ctggatgacc 8940
aaatgaccct tctacagtac tcatggatgt ttctcatggc atttgccctg ggttggagat 9000
catacagaca agcaagtgga aacctgctat gctttgctcc tgatctgatt attaatgagt 9060
aagttacatg gccttaaccc tccacaaaga actatagcaa ctaagcagtg ctgacaacag 9120
gagaaatggt tttccccaga gactaataca tcagttggtt atccagtacc attggtcatc 9180
cctagaagca tgcatacaag taacatcatg catactgagc aggtttcact tatgtattta 9240
ggaatatata tatgaatata catgcatgaa tgtaacagca attcatgcac aaagtggcca 9300
tcaatttgaa ggagagcaaa aagaggaatg tgaaaaggtt tagagagagg aaagggaagg 9360
gaaggggcaa atgactttgt aatctgagaa aaatgaaaga attttaaaat aaataagcag 9420
actaaaagac ctgtaaaaaa taactaatcc ctaatgtttc cataaaagta tgctgtaaag 9480
attccagaat tagatagctt attgagttct gagtatataa aggattcaca attttgttgt 9540
ttcaaaacag caaaaaccta gtgtcacatg taaagggtta ttattgaata gtctctccat 9600
gttctccaac attctagttc tataataggt tattgggaat agcagtaggt atgtttgcag 9660
ccatgacttc ataaatttaa aaaccaaccc aaaattcagc taatatttta gactagcact 9720
atttctttct aactcacatc tgtatttgtg tctcccaggc agagaatgac tctaccctgc 9780
atgtatgacc aatgtaaaca catgctgttt atctccactg aattacaaag attgcaggta 9840
tcctatgaag agtatctctg tatgaaaacc ttactgcttc tctcctcagg ttggtggacc 9900
acctactcac ttttaacttt cattctaaat atggcaaatg catgtgaaag ttgtataact 9960
attgtgcata tatgtgtata tatatgtatg tgtgtgtgta tatatatata tatatatata 10020
tatatatata tatatatatg ccaatttcat tgtgttttcc tagtaagatg atttttagaa 10080
aggcaaactg taaaaagggt atatatatat attaagtgtt atcaatatta gtaaaataaa 10140
taggtattgt agatctaggt gtgggatctg cccagggatc tctggtgatg aggctccata 10200
tagagtcttc cttcctgcta cccattcatc tatatctata taagatgtgg agaaagaatg 10260
ccaacagtaa tgagtctgtc tctctactgt acgagtgttt ctagataaac agtgacagct 10320
accatgaatg gggaatagct gagaaaataa tcacagtttg tttttcagaa caaccaaatg 10380
tttaaacttt agactttagc aagactactt tatattaaaa gtaaaaaagt tcaaaaaatt 10440
aaagaatctt gtctatatta gttaagtaat aaaaggaaag tcatacctga tattctctta 10500

aaatatacaa agatctcttc aagtcagcaa ttgaaacaac ccagttttta aggactggga 10560
atatgcaaag atgacttatt aaataagata gaataaagac agtaagtctg tcacattcca 10620
taaggacatg tctagaatga gccaatccag agtagtgagt acaccaaata ctggttgagg 10680
acacagagta caggaattct ttttcactgc tgaaagaata gcaaagtgga atgaccattt 10740
cagaagacag tttggtagtt tcctataaag gcgaacatag tctcaccatc tggtgtagca 10800
attgcttgat cagtatacaa aggaaatgaa aacttatgca gcatggatat ttatagccaa 10860
gctgtttgta tctggctcaa cttaggcgca atgagatata tcatccagta gacaaatcaa 10920
taaactgaca tatgcaaatg atggtattac tcaacactga aaagaaatgg gctgttgtgc 10980
caataaaaaa atattattga atcttaaata tatactatta aaaaaagtaa tttcttaaaa 11040
agaatctcat caactctaag aaacagatgg aggcagatga gatgacttgt gtaaaatgga 11100
agctcaacat gcccagcaag ccaactttcc tcagaaattt tacaggtgct tgtggtgaag 11160
ataagaggaa aactctcccg tttttatgtc agttagaaag ggggataaac tgttggaggt 11220
acatgtagta tttccaaagc aaaagcaagg cctgccctat aagagaacct catcaagacc 11280
ttatctgaca tggagcaaag gcaaataaag gattctagac ccttataata gccctcctga 11340
cttcacaggg aagaaaaggg gaaaatgggt ttccaggaag catttgggtg cagtaaaagt 11400
gaaatgtaat cttaagagaa aacatttcat tggagccttg cttacagttc ccagagctta 11460
gcttagttca ttatcgtgat ggcaggcaag catgattctg gaaaagtagc tgagtgctac 11520
actctgatcg gcaagcagag agagaaagag agtagcactg ggcctgttgt ggacttttga 11580
gacctcaaag cttatcctac agtgtcacac agtaacaggg ccacacctac tcaaataaga 11640
gcacacctcc caatccttct aatcctttca tagctcccct ccctggtaac taagcctatg 11700
ggaccattct tattcaaacc accacagtgc agataaccaa cattaaacac agtccagagc 11760
ctagccagat taccatacta tcttccacca aatgtcttaa ttccatcagt tctgatactc 11820
cttgtctagc tttcaataaa aaacaaagcc agcattaaat ctgccccaaa ccactggcat 11880
tatcacattg gaatttaaaa taactgcaat atttttgcta aggacactac aggaaaaata 11940
aagaccacat gagaattatg aatgaagagt catagaagct aaggaggttc ctctaaggga 12000
gcactagaga taacagatgc tgaaatgata atgcacagtg ctcctagcag gcagaccatc 12060
agttgtcata taaccaagaa agagtgagca tagggcaaca aaaacctcct agactcaaat 12120
aaaaagaggg aaaagaataa aaaaaaggtg tagagagatc ataaaaaata ttttaagtaa 12180
taaaagttca aaaatttcta aaattagtta tatgtaccaa aatgtggatg caggatgctc 12240
aaggataacc aagcaaagaa aaacaataac aacaaaaaaa tctatatttt ggcatatcct 12300
cttcaaatta taaaaaaaaa aaagattttt taccctgagt tatggtttta aaacagcaat 12360
gacaacaacc agacttctta tcagaaatca tgcaagtaag aatgtgaagt aaatagttag 12420
aaatgaaagc aaacaaccca ccagtcctga gacaatagat ccaacagaat tacctctcag 12480
aagtgaggaa gatggtgcca tgcacagtag tagacaccta gaattctagc actccagaga 12540
ctaaggactg aaattcaagc cagccatggc tacctatcaa gactgtctca agaaataaag 12600
taatgggtaa aataaaaaca ttctcaaaca aatgtgtctt tttaaaggga ttttgtggtc 12660
atctttacat ctgtcctaca tgagctattt taaaaattgt tcagaagaga ggaaaatatg 12720
tcagaattat gtgctgcata agaaagagat tatagctggg cagtggtggc gcacgccttt 12780
aatcccggca cttgggaggc agaggcaggt ggatttctga gttcgagacc agcctggtct 12840
acagagttcc aggacagcca gggctacaca gagaaaccct gtctcggaaa aagaaagaaa 12900
gaaaaggaag gaaagaagga agggagggag ggagggaggg attttaatag aaaagggatg 12960
cctgagagta aaatgtcaat tttcttctta atgttacata actgttatag tgataataat 13020
caggtatcag gggatctgga gaggtggctc agcagttccc agcacccaca taaccactca 13080
gaactgtcta actcaggtcc cagtgatcca ttgcctactt ctaacttctg gggtacatac 13140
ttgtggtgta caaacataca tcaggcaaaa cactcatgca cacagataca cacacacaca 13200
cacacacaca cacacacttt tatttatctt acaaattaac aatcgcagac tatgtgattg 13260
aaatacaggc atatgtacaa tgaatgacag aggtattgta gttaagttag gagggaaatt 13320
tcagatacac tctggcattg actttgcctg ttagagtagt tctagtgtat ttcggaaggg 13380
agaacaggtt agttgtaaat gtgtactgca gatgacagag cagtcactaa gagttcaaac 13440
tgtcaaaggg aacacgcaat catgagcatg tctgggacac tgaaggctga tgggagtctc 13500
cagagtctag tggttgaaag tgtgcaccac aggcagggtc cccagcttcc ccaaacctga 13560
tgtgcttggt cagaacagaa gcacctctcg gggccaagct aagggggaag ctccttcccc 13620
tcctcggggc aaagttggaa aaactgctaa gtgtgagcaa aggcaatccc cagaaacaaa 13680
aaagcaaaat ggattcttgg tgcaaaacta gtagcaagag cacaggtctg cttcagtgag 13740
ctccttgggg ctgtttctaa aatgtgttca tgggaggcac atctgctaag gaatgaaaaa 13800
ctgaagacaa caagaaagag gaatggaaac cagcatttcc tcctagggag attaagttca 13860
gtataatgga gaaagaagga aggagaagaa agcctggaag gagaatgaca gacttaattc 13920
cctggtgaca ctgcagacgc tggggagatg tgcggcagca atgatacaat ctagggaaag 13980
cctgctgaac ggaagaagaa ggacagcgag agttgtgccc acagcgataa gggagacagg 14040
ttcagtaaaa aggacagctg tgaagctttc aaatcagagg aagatgatta ataatgaagt 14100
ggatctcctt cagtgtgttc ttgtcaatga agtatttaaa caactccctt caaagaaaaa 14160
gactgaaatc aaaggctcta tagaagtgct tctaaactct ctagccgttt gctttgtaat 14220
agttaacaca cagtacagac accctttcct ggaggtactg acctcacctg gtacagtatg 14280
aaggttgtaa aatggaagat tgtttaaagc gggttctcac tggtacacat cacaaattag 14340
ctatacacag gatgacacgg tttgcatcct cgggtgtcgt caccgctccc gttataagat 14400
gggagacatt tggatctccc atggttatga accccaattc tcagttagcc tatactttca 14460
cttagctata gtcccaccat cctttgagaa tgcatgaatt ttagagtgta ttgaatgtac 14520

attgtaacga tgaaatacgc atgaaatgtg ctacttagta caagacaagt taaaaaaagg 14580
aaaatttaac tgacttcatc ttacctttta gttcctaagg aaggtctgaa gagccaagag 14640
ttatttgatg agattcgaat gacttatatc aaagagctag gaaaagccat tgtcaaaagg 14700
gaaggaaact ccagtcagaa ttggcagcgg ttttatcaac tgacaaaact tttggactcc 14760
atgcatgatg taagtaccaa acataaatcc aaaacaacag caaaaaagaa aaagtaggtt 14820
ggggtggtgg cagatgcctt taatcacagc agacacagag gtagagcaag tagatgtcta 14880
tgagtctgag gctgctctac atagggaatg ccaggacagt cagagatacg taagagatcc 14940
tatctcaaaa gcccaaagac agagacagag agagacagag acagaagaac attgtaggtc 15000
ctaggttatg gtttaattag tgtatgtgta tagttatata ccaatcagtc attatagtga 15060
ttattcaaga taaataattt tttacacatt tctcttaagt cttactagtc tggtcatttg 15120
cgtaaagatg actctgttag tacagagttc agtagctcct gattttaagt atccctgaag 15180
tacatgaatg agctatcaga ttacactagt ttcacctccc tgtgtatcag tactggtgca 15240
gtctcctttg agcctgctgc ctcgtctcct gactgctggg atcacaggca tgccaggctt 15300
cattctgcac acagtttaag atgccttttg ttcttatgtg cttattttat ttaaattatt 15360
ttcttatctg aggaattcca acaaagattg gtatatttgt gatcaaattg ttaaaatggc 15420
cagtagggca ggaatgcagg atctatatac caggtcttct gtgtgtatat tacgactttc 15480
agtttagtgt gtctgggatt ctggagtgtg ggaaccagta ggtccctgac tcttgttcct 15540
gttcttgggt tctccttttt ctctttgtct tttccaacaa tatgatggtt tttattttat 15600
tttgctatgt tttattttat tatcacctct tagaagcaca atctttttaa taagaggaga 15660
agttgagagg aactgggagg gtaagccaca atcaagatac attacatgag aaaagaattt 15720
attttcagta aacaaaaaaa tatgtcaaga cttggtgtgg ggagccgaga gatatgagcc 15780
attcgcagtt atgctttgaa gcctcaccat taccgtatct tctcttgcag gtggttgaaa 15840
atctccttag ctactgcttc caaacatttt tggataagtc catgagtatt gaattcccag 15900
agatgttagc tgaaatcatc actaatcaga taccaaaata ctcaaatgga aatatcaaaa 15960
agcttctgtt tcatcagaaa tgactgcctt actaagaaag gctgccttaa agaaagttga 16020
atttatagct tttactgtac aaacttatca acttgtcttg tagatgtttt gtcgttcttt 16080
ttgtttgtct tgtttgtttt ctatacgcac tacatgtggt ctctagaggg ccaagacttg 16140
gcaacagaag cagatgagcc atcacttttc agtgacagga aagcagacag tgatgtgcat 16200
tggctggtgt atcacagaaa ctagaacagt tagtggagac atgtccacta tcagagaagg 16260
accgcacctg aaccaccagt gcccaaagtc catgtgatca actttctgct caactttcag 16320
ttggctggat aacactttct agacttttct gttggtgtat ttttcccatg tatagttagg 16380
atagcatttt gatttatgca tggaaacctg aaaaaagttt acacgtgtat atcagaaaag 16440
ggaagttgtg ccttttatag ctattactgt ctggttttaa caatttcctt tatattcagt 16500
gaactatgct tgctcgtttt tcttaaataa tttttgtatt ctagttattg tatagctgtt 16560
taagatgggc agctgcctca cagctctcct agacgctaac attaatttcc gtgtgaaaat 16620
gggtcggtgc tcctaccctg atggcactca gctatcagaa gaccacagaa attgactcag 16680
atctccagta ttcttgtcaa aagctcttac tctgtatata tctgcttcca tggggaatta 16740
tataggttgt gcagattaac cgtcctaact ggtatagagc acctagtcca gtgacctgct 16800
gggtaaactg tggatgatgg ttacaaaaga ctaattgtaa aacagtgccc accaacaggc 16860
cccgtttgca cccaatgcac catctcttca gtggtgcgat agcaacaaag tttgtaactc 16920
agctctttca ggaccttcgg gagtagtttg tgtaacattt taaaatgtat tattccagat 16980
aaccagctgt gataaagccg agagattgtt ttaatcagac caagtaactt ctctcattaa 17040
acgttaccct caactaagtc tctaatatgg caagaatggc tagacaccca ttttcacatc 17100
ccacctgtca ccaattggtc tagctttcct ggtggtacag gaaaatcagc tactgatttt 17160
ttgttattta gaactgaatg tcaggcatcc atgtttgtcc aactatacat ccctacatgt 17220
gccatagaat ctaacacaag tcttgtgaac ttcttcacac tgagagttat cattttaaac 17280
aaaacagaag ctgtagtagc cctttctgtg tgcaccttac caactttctg tgactcaaag 17340
cttaacacac ttactaagcc acaagaaatc tgatttctac ttaaggtggc caaattattt 17400
gtgtaataga aaactgaaaa tctaatatta aaaatatgaa acttctaata tatttttata 17460
tttagttcta gtttcagata tatatcatat tggtattcac taatctggga agggaagggc 17520
tactgcagct gtacatgcaa tttattaaca tgattgtaaa atagctgtat agtgtaaaat 17580
aagaatgatt tttagatgag attgttttat catgacatgt tatatatttt ttgtaggggt 17640
caaagaaatg ttgatggata tcctataaga tttatagtat ataagagcat ccatacaggc 17700
ctcagtggtc ttggaaatta aaacaggttt gctctaagct agggagaggg agctgggact 17760
ggccctgtgt gcagtgcagg tcctgagggt ttgacccgat cagatcacag gggaactaat 17820
tccctcccat ctaaccatcc tcatccgacc atggccctgt cagtgcaggc tggctttatt 17880
aaatccagga cagaaaggtg gcgcttatgt acttagaggc accgtccagt aacagggttg 17940
ttcccacatg cagcctccgc acgggttaac agaaacagag gctttagaag tttggcaata 18000
atgtgcatag aggttccagc aatatgtaaa tactaaagaa tcgcatagga agccaataat 18060
acactaatcc tctccatcct acaagagtcc atttccaagt aagatgagga catgtttatg 18120
ttttctttga atgctttttg aatgttgtta ttttcagtat tttgcagaaa ttatttaata 18180
aaaaaaagta taatcatttg ctttttgaat tctctctaaa agggaatgtt cagtttgtaa 18240
tggtttaaat tggtctcaaa gtactttaaa ataattgtaa cccagctgga tgtgaaattt 18300
atggtgccta agaaatacca cttgaagatt atcaatgaca gtgttaagtt tcaaaatgag 18360
cttctcaaaa atagattatt gtacatttat ggaatgttat atggttaaac ccaaaaagca 18420
catcacacat aaatctgctt tcagttccaa ccagcttggc tttcaaaaat agagctccaa 18480
aaaaaaaaaa ggaaaaaaaa gatatatatg ctttgttatt aacagaaggc agcagacatt 18540

cataaaacta ctatcggaag ttttccatta gatgtataaa gagctatcct ttggtatgtg 18600
ggaaagaaga aagctgtcat aattctgatt gagtataagt gagagagata cggtactgtt 18660
tgagagcagc tccttttctg cgtgtggctt cataccgttc caaactatgt agattttata 18720
atagcttcag tgagaattgg taacatgcct gtatgactca caacagatct tgaaaactat 18780
ctttaattac tggtaggaca aaaagggaca ttctggttat tttaggcact ggcttggaac 18840
actgtatatg cagaagaaag aagacaggca atctggggaa aggaagggga cctgggaagc 18900
actgccttct ttaaggaaag acacaccaat agatgagatc atcccaaagg cacagggacc 18960
acagagtgtg agtccttagt gacgagtcag gtgagctctg 19000

<210> 221
<211> 20
<212> DNA
<213> Artificial Sequence

<220>
<223> Antisense Compound

<400> 221
cattggcaaa tattaacttc 20

<210> 222
<211> 20
<212> DNA
<213> Artificial Sequence

<220>
<223> Antisense Compound

<400> 222
tttggagtcc attggcaaat 20

<210> 223
<211> 20
<212> DNA
<213> Artificial Sequence

<220>
<223> Antisense Compound

<400> 223
tttctgaatc ctggtatcgc 20

<210> 224
<211> 20
<212> DNA
<213> Artificial Sequence

<220>
<223> Antisense Compound

<400> 224
tccttaggaa ctgaggagag 20

<210> 225
<211> 20
<212> DNA
<213> Artificial Sequence

<220>
<223> Antisense Compound

<400> 225
taaggcagtc atttctgatg 20

<210> 226

<211> 20
<212> DNA
<213> Artificial Sequence

<220>
<223> Antisense Compound

<400> 226
aaggcagcct ttcttagtaa                                                    20

<210> 227
<211> 20
<212> DNA
<213> Artificial Sequence

<220>
<223> Antisense Compound

<400> 227
aagtttgtac agtaaaagct                                                    20

<210> 228
<211> 20
<212> DNA
<213> Artificial Sequence

<220>
<223> Antisense Compound

<400> 228
gctcatctgc ttctgttgcc                                                    20

<210> 229
<211> 20
<212> DNA
<213> Artificial Sequence

<220>
<223> Antisense Compound

<400> 229
gcatacatac tgtgagcccg                                                    20

<210> 230
<211> 20
<212> DNA
<213> Artificial Sequence

<220>
<223> Antisense Compound

<400> 230
ctgggcggcc ccgtctgcag                                                    20

<210> 231
<211> 20
<212> DNA
<213> Artificial Sequence

<220>
<223> Antisense Compound

<400> 231
ttggcaaata ttaatgtgag                                                    20

<210> 232
<211> 20

<212> DNA
<213> Artificial Sequence

<220>
<223> Antisense Compound

<400> 232
agccagataa acaagtcggc                                                    20

<210> 233
<211> 20
<212> DNA
<213> Artificial Sequence

<220>
<223> Antisense Compound

<400> 233
atattaactc agcaccggcg                                                    20

<210> 234
<211> 20
<212> DNA
<213> Artificial Sequence

<220>
<223> Antisense Compound

<400> 234
agaatcttag ctatagggct                                                    20

<210> 235
<211> 20
<212> DNA
<213> Artificial Sequence

<220>
<223> Antisense Compound

<400> 235
catgccttac ctggtatcgc                                                    20

<210> 236
<211> 20
<212> DNA
<213> Artificial Sequence

<220>
<223> Antisense Compound

<400> 236
tgtaacttac tcattaataa                                                    20

<210> 237
<211> 20
<212> DNA
<213> Artificial Sequence

<220>
<223> Antisense Compound

<400> 237
tcacatagtc tgcgattgtt                                                    20

<210> 238
<211> 20
<212> DNA

<213> Artificial Sequence

<220>
<223> Antisense Compound

<400> 238
tccttaggaa ctaaaaggta                                                    20

<210> 239
<211> 20
<212> DNA
<213> Artificial Sequence

<220>
<223> Antisense Compound

<400> 239
tatctctgac tgtcctggca                                                    20

<210> 240
<211> 20
<212> DNA
<213> Artificial Sequence

<220>
<223> Antisense Compound

<400> 240
agcctttctt agtaaggcag                                                    20

<210> 241
<211> 20
<212> DNA
<213> Artificial Sequence

<220>
<223> Antisense Compound

<400> 241
acatcactgt ctgctttcct                                                    20

<210> 242
<211> 20
<212> DNA
<213> Artificial Sequence

<220>
<223> Antisense Compound

<400> 242
ggacatgtct ccactaactg                                                    20

<210> 243
<211> 20
<212> DNA
<213> Artificial Sequence

<220>
<223> Antisense Compound

<400> 243
tttgggcact ggtggttcag                                                    20

<210> 244
<211> 20
<212> DNA
<213> Artificial Sequence

<220>
<223> Antisense Compound

<400> 244
catcttaaac agctatacaa                                                    20

<210> 245
<211> 20
<212> DNA
<213> Artificial Sequence

<220>
<223> Antisense Compound

<400> 245
ctgatagctg agtgccatca                                                    20

<210> 246
<211> 20
<212> DNA
<213> Artificial Sequence

<220>
<223> Antisense Compound

<400> 246
agagatggtg cattgggtgc                                                    20

<210> 247
<211> 20
<212> DNA
<213> Artificial Sequence

<220>
<223> Antisense Compound

<400> 247
cctgacattc agttctaaat                                                    20

<210> 248
<211> 20
<212> DNA
<213> Artificial Sequence

<220>
<223> Antisense Compound

<400> 248
caaacatgga tgcctgacat                                                    20

<210> 249
<211> 20
<212> DNA
<213> Artificial Sequence

<220>
<223> Antisense Compound

<400> 249
ttagattcta tggcacatgt                                                    20

<210> 250
<211> 20
<212> DNA
<213> Artificial Sequence

<220>
<223> Antisense Compound

<400> 250
gttaagcttt gagtcacaga                                                    20

<210> 251
<211> 20
<212> DNA
<213> Artificial Sequence

<220>
<223> Antisense Compound

<400> 251
ctctccctag cttagagcaa                                                    20

<210> 252
<211> 20
<212> DNA
<213> Artificial Sequence

<220>
<223> Antisense Compound

<400> 252
tggacggtgc ctctaagtac                                                    20

<210> 253
<211> 20
<212> DNA
<213> Artificial Sequence

<220>
<223> Antisense Compound

<400> 253
gaaatggact cttgtaggat                                                    20

<210> 254
<211> 20
<212> DNA
<213> Artificial Sequence

<220>
<223> Antisense Compound

<400> 254
ataaatttca catccagctg                                                    20

<210> 255
<211> 20
<212> DNA
<213> Artificial Sequence

<220>
<223> Antisense Compound

<400> 255
taaatgtaca ataatctatt                                                    20

<210> 256
<211> 1552
<212> DNA
<213> Artificial Sequence

<220>

<223> R. norvegicus

<400> 256
atggactcca aagaatcctt agctccccct ggtagagacg aagtccctgg cagtttgctt 60
ggccagggga gggggagcgt aatggacttt tataaaagcc tgaggggagg agctacagtc 120
aaggtttctg catcttcgcc ctcagtggct gctgcttctc aggcagattc caagcagcag 180
aggattctcc ttgatttctc gaaaggctcc acaagcaatg tgcagcagcg acagcagcag 240
cagcagcagc agcagcagca gcagcagcag cagcagcagc agcagcagcc agacttatcc 300
aaagccgttt cactgtccat ggggctgtat atgggagaga cagaaacaaa agtgatgggg 360
aatgacttgg gctacccaca gcagggccaa cttggccttt cctctgggga aacagacttt 420
cggcttctgg aagaaagcat tgcaaacctc aataggtcga ccagcgttcc agagaacccc 480
aagagttcaa cgtctgcaac tgggtgtgct accccgacag agaaggagtt tcccaaaact 540
cactcggatg catcttcaga acagcaaaat cgaaaaagcc agaccggcac caacggaggc 600
agtgtgaaat tgtatcccac agaccaaagc acctttgacc tcttgaagga tttggagttt 660
tccgctgggt ccccaggtaa agacacaaac gagagtccct ggagatcaga cctgttgata 720
gatgaaaact tgctttctcc tttggcggga gaagatgatc cattccttct cgaaggggac 780
acgaatgagg attgtaagcc tcttatttta ccggacacta aacctaaaat taaggatact 840
ggagatacaa tcttatcaag tcccagcagt gtggcactgc cccaagtgaa aacagaaaaa 900
gatgatttca ttgaactttg cacccccggg gtaattaagc aagagaaact gggcccagtt 960
tattgtcagg caagcttttc tgggacaaat ataattggta ataaaatgtc tgccatttct 1020
gttcatggtg tgactacctc tggaggacag atgtaccact atgacatgaa tacagcatcc 1080
ctttctcagc agcaggatca gaagcctgtt tttaatgtca ttccaccaat tcctgttggt 1140
tctgaaaact ggaataggtg ccaaggctcc ggagaggaca gcctgacttc cttgggggct 1200
ctgaacttcc caggccggtc agtgttttct aatgggtact caagccctgg aatgagacca 1260
gatgtaagct ctcctccatc cagctcgtca gcagccacgg gaccacctcc caagctctgc 1320
ctggtgtgct ccgatgaagc ttcaggatgt cattacgggg tgctgacatg tggaagctgc 1380
aaagtattct ttaaaagagc agtggaagga cagcacaatt acctttgtgc tggaagaaac 1440
gattgcatca ttgataaaat tcgaaggaaa aactgcccag catgccgcta tcggaaatgt 1500
cttcaggctg gaatgaacct tgaagctcga aaaacaaaga aaaaaaaaaa aa 1552

<210> 257
<211> 20
<212> DNA
<213> Artificial Sequence

<220>
<223> Antisense Compound

<400> 257
taagtctggc tgctgctgct 20

<210> 258
<211> 20
<212> DNA
<213> Artificial Sequence

<220>
<223> Antisense Compound

<400> 258
ctttggataa gtctggctgc 20

<210> 259
<211> 20
<212> DNA
<213> Artificial Sequence

<220>
<223> Antisense Compound

<400> 259
aggcttttat aaaagtccat 20

<210> 260
<211> 20
<212> DNA

<213> Artificial Sequence

<220>
<223> Antisense Compound

<400> 260
atcaaggaga atcctctgct                                                    20

<210> 261
<211> 20
<212> DNA
<213> Artificial Sequence

<220>
<223> Antisense Compound

<400> 261
gcccccaagg aagtcaggct                                                    20

<210> 262
<211> 20
<212> DNA
<213> Artificial Sequence

<220>
<223> Antisense Compound

<400> 262
ccgtaatgac atcctgaagc                                                    20

<210> 263
<211> 20
<212> DNA
<213> Artificial Sequence

<220>
<223> Antisense Compound

<400> 263
actcttggct cttcagacct                                                    20

<210> 264
<211> 20
<212> DNA
<213> Artificial Sequence

<220>
<223> Antisense Compound

<400> 264
taaggcagtc atttttgatg                                                    20

<210> 265
<211> 20
<212> DNA
<213> Artificial Sequence

<220>
<223> Antisense Compound

<400> 265
aactttcttt aaggcaacct                                                    20

<210> 266
<211> 20
<212> DNA
<213> Artificial Sequence

<220>
<223> Antisense Compound

<400> 266
cctctataaa ccacatgtac                                                    20

<210> 267
<211> 20
<212> DNA
<213> Artificial Sequence

<220>
<223> Antisense Compound

<400> 267
tgtcatcact tcagagtgtt                                                    20

<210> 268
<211> 20
<212> DNA
<213> Artificial Sequence

<220>
<223> Antisense Compound

<400> 268
aactgttagt ttctgtgata                                                    20

<210> 269
<211> 20
<212> DNA
<213> Artificial Sequence

<220>
<223> Antisense Compound

<400> 269
tagaaagttt tacccagcca                                                    20

<210> 270
<211> 20
<212> DNA
<213> Artificial Sequence

<220>
<223> Antisense Compound

<400> 270
ctatgtaatt ctccatggaa                                                    20

<210> 271
<211> 20
<212> DNA
<213> Artificial Sequence

<220>
<223> Antisense Compound

<400> 271
ctggactagg tgctctacac                                                    20

<210> 272
<211> 20
<212> DNA
<213> Artificial Sequence

<220>
<223> Antisense Compound

<400> 272
attgaagaga tggtgcatta                                                    20

<210> 273
<211> 20
<212> DNA
<213> Artificial Sequence

<220>
<223> Antisense Compound

<400> 273
ggctttatca gagctggcta                                                    20

<210> 274
<211> 20
<212> DNA
<213> Artificial Sequence

<220>
<223> Antisense Compound

<400> 274
ctgtattagc gatttagttg                                                    20

<210> 275
<211> 20
<212> DNA
<213> Artificial Sequence

<220>
<223> Antisense Compound

<400> 275
accatgagag ctagaccaat                                                    20

<210> 276
<211> 20
<212> DNA
<213> Artificial Sequence

<220>
<223> Antisense Compound

<400> 276
gcacatgtag ggatgtgtag                                                    20

<210> 277
<211> 20
<212> DNA
<213> Artificial Sequence

<220>
<223> Antisense Compound

<400> 277
agtttttcta ttacacaaat                                                    20

<210> 278
<211> 20
<212> DNA
<213> Artificial Sequence

<220>

<223> Antisense Compound

<400> 278
cagtagccct ttccctttcc                                                    20

<210> 279
<211> 20
<212> DNA
<213> Artificial Sequence

<220>
<223> Antisense Compound

<400> 279
catcaatatt tctttgaccc                                                    20

<210> 280
<211> 20
<212> DNA
<213> Artificial Sequence

<220>
<223> Antisense Compound

<400> 280
aatggactat tgaagggtgg                                                    20

<210> 281
<211> 20
<212> DNA
<213> Artificial Sequence

<220>
<223> Antisense Compound

<400> 281
agaaaacata agcatgtcct                                                    20

<210> 282
<211> 20
<212> DNA
<213> Artificial Sequence

<220>
<223> Antisense Compound

<400> 282
gaacaatccc ttttagagag                                                    20

<210> 283
<211> 20
<212> DNA
<213> Artificial Sequence

<220>
<223> Antisense Compound

<400> 283
taatctattt ttgagaagct                                                    20

<210> 284
<211> 20
<212> DNA
<213> Artificial Sequence

<220>
<223> Antisense Compound

<400> 284
tacgcttcaa ggaaagcttc                                                    20

<210> 285
<211> 20
<212> DNA
<213> Artificial Sequence

<220>
<223> Antisense Compound

<400> 285
ccgagtctca ctgaagttat                                                    20

<210> 286
<211> 20
<212> DNA
<213> Artificial Sequence

<220>
<223> Antisense Compound

<400> 286
tctttcaaga tcggtcatga                                                    20

<210> 287
<211> 20
<212> DNA
<213> Artificial Sequence

<220>
<223> Antisense Compound

<400> 287
ccaaggccta aaataaccag                                                    20

<210> 288
<211> 20
<212> DNA
<213> Artificial Sequence

<220>
<223> Antisense Compound

<400> 288
ctttgggtac tctcacttat                                                    20

<210> 289
<211> 20
<212> DNA
<213> Artificial Sequence

<220>
<223> Antisense Compound

<400> 289
cctgactcat ccttagaccc                                                    20

<210> 290
<211> 20
<212> DNA
<213> Artificial Sequence

<220>
<223> Antisense Compound

<400> 290
tctcaagctc catgatcctt                                                    20

<210> 291
<211> 20
<212> DNA
<213> Artificial Sequence

<220>
<223> Antisense Compound

<400> 291
cgccttctaa cactgaaacc                                                    20

<210> 292
<211> 20
<212> DNA
<213> Artificial Sequence

<220>
<223> Antisense Compound

<400> 292
ctgtttcgcc ttctaacact                                                    20

<210> 293
<211> 20
<212> DNA
<213> Artificial Sequence

<220>
<223> Antisense Compound

<400> 293
gtttgggaat gagaagactt                                                    20

<210> 294
<211> 20
<212> DNA
<213> Artificial Sequence

<220>
<223> Antisense Compound

<400> 294
tagcagctgg tcaccagtcc                                                    20

<210> 295
<211> 20
<212> DNA
<213> Artificial Sequence

<220>
<223> Antisense Compound

<400> 295
attttcatac agccatttat                                                    20

<210> 296
<211> 20
<212> DNA
<213> Artificial Sequence

<220>
<223> Antisense Compound

<400> 296

tattgacaca ctgaaatctc                                                    20

<210> 297
<211> 20
<212> DNA
<213> Artificial Sequence

<220>
<223> Antisense Compound

<400> 297
tagaaagacg gatttttaaa                                                    20

<210> 298
<211> 20
<212> DNA
<213> Artificial Sequence

<220>
<223> Antisense Compound

<400> 298
tgtggtttgg taataccaag                                                    20

<210> 299
<211> 20
<212> DNA
<213> Artificial Sequence

<220>
<223> Antisense Compound

<400> 299
actaacattt actgccaatt                                                    20

<210> 300
<211> 20
<212> DNA
<213> Artificial Sequence

<220>
<223> Antisense Compound

<400> 300
gttcattcta aagtggtcac                                                    20

<210> 301
<211> 20
<212> DNA
<213> Artificial Sequence

<220>
<223> Antisense Compound

<400> 301
aatattcgca ccccactggt                                                    20

<210> 302
<211> 20
<212> DNA
<213> Artificial Sequence

<220>
<223> Antisense Compound

<400> 302
cgttattaac ctccgttgaa                                                    20

<210> 303
<211> 20
<212> DNA
<213> Artificial Sequence

<220>
<223> Antisense Compound

<400> 303
ttctacctcg cgcgatttac                                                    20

<210> 304
<211> 20
<212> DNA
<213> Artificial Sequence

<220>
<223> Antisense Compound

<400> 304
ctgctagcct ctggatttga                                                    20

<210> 305
<211> 20
<212> DNA
<213> Artificial Sequence

<220>
<223> Antisense Compound

<400> 305
ccttccctga aggttcctcc                                                    20

<210> 306
<211> 20
<212> DNA
<213> Artificial Sequence

<220>
<223> Antisense Compound

<400> 306
cgacctattg aggtttgcaa                                                    20

<210> 307
<211> 20
<212> DNA
<213> Artificial Sequence

<220>
<223> Antisense Compound

<400> 307
gtcaaaggtg ctttggtctg                                                    20

<210> 308
<211> 20
<212> DNA
<213> Artificial Sequence

<220>
<223> Antisense Compound

<400> 308
gcagacattt tattaccaat                                                    20

<210> 309
<211> 20
<212> DNA
<213> Artificial Sequence

<220>
<223> Antisense Compound

<400> 309
gtacatctgt cctccagagg                                                    20

<210> 310
<211> 20
<212> DNA
<213> Artificial Sequence

<220>
<223> Antisense Compound

<400> 310
gctgtattca tgtcatagtg                                                    20